(19) Europäisches Patentamt
European Patent Office
Office européen des brevets

(11) EP 3 888 651 A1

(12) **EUROPEAN PATENT APPLICATION**
published in accordance with Art. 153(4) EPC

(43) Date of publication:
**06.10.2021 Bulletin 2021/40**

(21) Application number: **19888637.6**

(22) Date of filing: **29.11.2019**

(51) Int Cl.:
*A61K 31/40* (2006.01) *A61K 31/401* (2006.01)
*A61K 31/4174* (2006.01) *A61K 31/422* (2006.01)
*A61K 31/437* (2006.01) *A61K 31/4439* (2006.01)
*A61K 31/444* (2006.01) *A61P 35/00* (2006.01)
*A61P 35/04* (2006.01) *A61P 43/00* (2006.01)
*C07D 207/36* (2006.01) *C07D 233/78* (2006.01)
*C07D 401/10* (2006.01) *C07D 401/12* (2006.01)
*C07D 405/06* (2006.01) *C07D 413/04* (2006.01)
*C07D 471/04* (2006.01) *C07D 213/71* (2006.01)
*C07D 213/73* (2006.01)

(86) International application number:
**PCT/JP2019/046862**

(87) International publication number:
**WO 2020/111252 (04.06.2020 Gazette 2020/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.11.2018 JP 2018225753**

(71) Applicant: **Kyushu University, National University Corporation**
**Nishi-ku**
**Fukuoka-shi**
**Fukuoka 819-0395 (JP)**

(72) Inventors:
- **FUKUI, Yoshinori**
  **Fukuoka-shi, Fukuoka 819-0395 (JP)**
- **URUNO, Takehito**
  **Fukuoka-shi, Fukuoka 819-0395 (JP)**
- **KANAI, Motomu**
  **Tokyo 113-8654 (JP)**
- **OISAKI, Kounosuke**
  **Tokyo 113-8654 (JP)**
- **TSUTSUMI, Ryosuke**
  **Tokyo 113-8654 (JP)**

(74) Representative: **Vossius & Partner Patentanwälte Rechtsanwälte mbB**
**Siebertstraße 3**
**81675 München (DE)**

(54) **DOCK1-INHIBITING COMPOUND AND USE THEREOF**

(57) Provided is a compound that is usable as an active ingredient of an anticancer agent. Preferably provided is a compound that has DOCK1-inhibiting activity and exerts an anticancer effect based on the activity. A compound represented by the following formula (A) or a salt thereof:

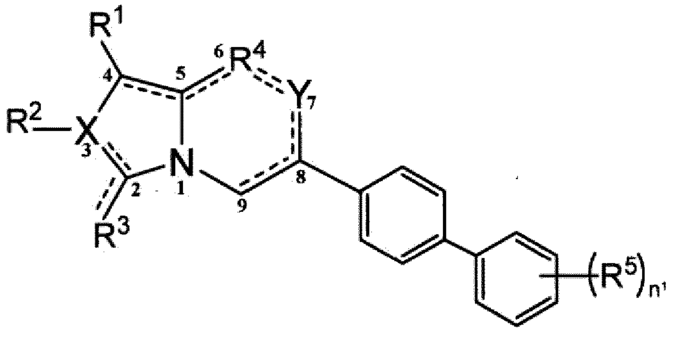

(A)

EP 3 888 651 A1

wherein
X represents a carbon atom or a nitrogen atom;
Y represents an oxygen atom, a hydroxy group, or a hydrocarbon group;
$R^1$ and $R^2$ are different, and each represents a hydrogen atom or a group represented by the following formula (A-1):

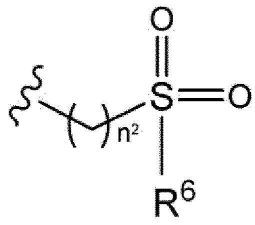

(A－1)

(wherein $R^6$ represents a pyrrolidino group or a phenyl group, and $n^2$ is 0 or 1) ;
$R^3$ represents -CO-$R^7$ (wherein $R^7$ is an alkoxy group, an alkyl group, or an alkylamino group), a 1,3-oxazole group, an alkylhydroxy group, a hydrogen atom, or an oxygen atom;
$R^4$ represents a hydrogen atom, an oxygen atom, or a hydrocarbon group in which one or more hydrogen atoms may be replaced by one or more substituents;
$R^5$ represents a halogen atom, a halogenated alkyl group, or a halogenated alkylthio group; and
$n^1$ is an integer of 0 to 5; and

## Description

Technical Field

The present invention relates to a DOCK 1-inhibiting compound, and a use thereof.

Background Art

Cancer is the leading cause of death in developed countries, including Japan. The number of cancer patients is increasing year by year. Therefore, cancer is an urgent issue for modern medicine to overcome. Accordingly, in order to develop new anticancer agents, Japanese, European, and American pharmaceutical companies are actively engaged in drug discovery research, including the search for new molecular targets.

DOCK1 is an activator of Rac of a low-molecular-weight G protein. DOCK1 is known to promote cytoskeletal reorganization and regulate various cellular responses, such as cell motility, morphological changes, proliferation, and differentiation, through Rac activation (Non-patent Literature (NPL) 1). In cancer cells, DOCK1 regulates invasive edge formation and macropinocytosis through Rac activation and contributes to invasive metastasis, and the survival and growth of cancer cells (Non-patent Literature (NPL) 1 to Non-patent Literature (NPL) 3). Accordingly, a compound that effectively inhibits the function of DOCK1 is expected to be effective as an active ingredient for a new type of anticancer agent that inhibits invasive metastasis and the survival and growth of cancer cells. On the other hand, DOCK2, which is one of the DOCK family molecules and which has a structure very similar to that of DOCK1, is expressed in immune cells; this DOCK2 plays an essential role in immune cell migration and antigen-stimulated response (Non-patent Literature (NPL) 4 and Non-patent Literature (NPL) 5). Accordingly, in order to use as an anticancer agent a compound that inhibits the function of DOCK1, there is a need to develop a compound whose influence on the immune system via DOCK2 is as small as possible; i.e., a compound that has an enhanced selectivity for DOCK1.

DOCK1 is known to act downstream of tyrosine kinase-type receptor signals in breast cancer and glioma to enhance invasive metastasis of cancer cells, and contribute to malignant transformation of cancer (Non-patent Literature (NPL) 1 and Non-patent Literature (NPL) 2). Furthermore, recent studies by the present inventors have revealed that even in cancers having mutant Rac, DOCK1 is involved in invasive metastasis and the survival and growth of cancer cells (Non-patent Literature (NPL) 6) .

Ras is an oncogene that was first found in humans (1982). Ras exists as three isoforms: H-Ras, K-Ras, and N-Ras (Non-patent Literature (NPL) 7 and Non-patent Literature (NPL) 8). Cancers having a mutation in Ras gene account for one-third of all human cancers. In particular, in pancreatic cancer, colorectal cancer, multiple myeloma, and lung cancer, mutations in Ras gene are observed at a high rate of 32 to 98% (NPL 7 and NPL 8). Mutant Ras has been shown to be an important driving factor from cancer initiation (onset) to progression (development), and the need for drug discovery that targets mutant Ras is extremely high. However, no therapeutic agents effective for cancers having mutant Ras have been developed, although 30 years or more have already passed since the discovery of mutant Ras. Therefore, there is an urgent need to address this issue. One of the reasons why drug discovery that targets mutant Ras is difficult is that the surface structure to which a low-molecular-weight compound is bound is extremely small, and is thus unsuitable as a target for drug discovery. In place of this approach, drug discovery approaches that target molecules involved in the intracellular localization regulation of mutant Ras and signal transduction pathways are considered promising.

Using 3LL mouse lung carcinoma cells, DLD-1 human colon carcinoma cells, and HT-1080 human fibrosarcoma cells, which are carcinoma cell lines having mutant Ras, the present inventors have clarified that DOCK1 is important for cell invasion response, macropinocytosis, and survival in low-nutrient conditions (Non-patent Literature (NPL) 3). Macropinocytosis is a phenomenon in which a portion of the cell membrane significantly bends, allowing extracellular fluid, and proteins and other components in the extracellular fluid, to enter the cell. Cytoskeletal reorganization via Rac activation is important for this macropinocytosis. Macropinocytosis is a process necessary to supply glutamine, which is a nutrient essential for survival, in cancer cells under low-nutrient conditions (Non-patent Literature (NPL) 9 and Non-patent Literature (NPL) 10).

The present inventors further showed that TBOPP (1-(2-oxo-2-(3'-(trifluoromethyl)-[1,1'-biphenyl]-4-yl)ethyl)-5-(pyrrolidin-1-ylsulfonyl)pyridin-2(1H)-one), which was previously developed as a DOCK1 selective inhibitor, inhibits Rac activation by DOCK1, and blocks cancer cell invasion response, macropinocytosis, and survival under low glutamine conditions, while not affecting immune cell migration. The inventors further demonstrated in an animal experiment using mice that the TBOPP blocks cancer invasive metastasis and proliferation (Non-Patent Literature (NPL) 3 and Patent Literature (PTL) 1). Similarly, in human-derived melanoma and breast cancer cell lines having active mutant Rac (P29S mutation), invasive response and macropinocytosis of these cancer cell lines can be suppressed by treatment for genetic deletion of DOCK1, or treatment with TBOPP (Non-patent Literature (NPL) 6).

Citation List

Patent Literature

PTL 1: WO1016/136985

Non-patent Literature

NPL 1: Genes Dev. 28: 533-547 (2014)
NPL 2: Proc. Natl. Acad. Sci. USA 110: 7434-7439 (2013)
NPL 3: Cell Reports 19: 969-980 (2017)
NPL 4: Nature 412: 826-831 (2001)
NPL 5: Exp. Cell Res. 319: 2343-2349 (2013)
NPL 6: Biochem. Biophys. Res. Commun. 497: 298-304 (2018)
NPL 7: Cancer Research 72: 2457-2467 (2012)
NPL 8: Nature Reviews Drug Discovery 13: 828-851 (2014)
NPL 9: Nature 497: 633-638 (2013)
NPL 10: Cell Res. 23: 982-983 (2013)

Summary of Invention

Technical Problem

An object of the present invention is to provide a compound that can be used as an active ingredient of an anticancer agent. Preferably, an object of the present invention is to provide a compound that has DOCK1-inhibiting activity, and that exerts anticancer activity based on this activity. More preferably, an object of the present invention is to provide a compound that has DOCK1-inhibiting activity, exerts anticancer activity based on this activity, and has little influence on the immune system via DOCK2, i.e., a compound with high selectivity for DOCK1. Even more preferably, an object of the present invention is to provide a compound that has a higher DOCK1-inhibiting effect (potency), DOCK1/DOCK2 selectivity (selectivity), and/or a higher effect of inhibiting cell invasion response (cell activity) than TBOPP, which is known as a DOCK1-selective inhibitor. Another object of the present invention is to provide use of these compounds as DOCK1-selective inhibitors, and use of these compounds as pharmaceutical agents based on their anticancer activity.

Solution to Problem

To achieve the above objects, the present inventors repeatedly conducted extensive research. As a result, the inventors found multiple compounds that have DOCK1-selective inhibition and/or a cell invasion response inhibitory effect, in addition to DOCK1-inhibiting activity; and confirmed that some of these compounds have a higher DOCK1-inhibiting effect, DOCK1/DOCK2 selectivity, and cell invasion response inhibitory effect than those achieved by TBOPP in at least one, preferably two or more, and more preferably all, of these effects. The present invention has been accomplished through further continued search based on these findings. The present disclosure provides inventions according to the following embodiments.

<u>(I) Pyrrole-type compounds having DOCK1-inhibiting activity</u>

(I-1) A compound represented by the following formula (A):

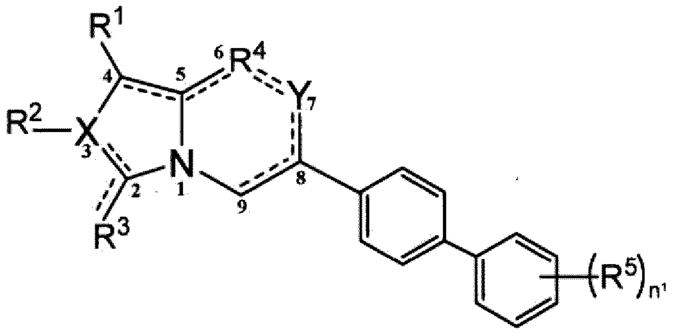

(A)

wherein

X represents a carbon atom or a nitrogen atom;
Y represents an oxygen atom, a hydroxy group, or a hydrocarbon group;
$R^1$ and $R^2$ are different, and each represents a hydrogen atom or a group represented by the following formula (A-1):

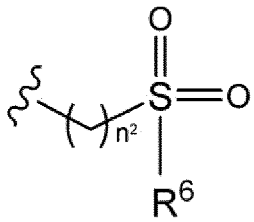

(A − 1)

(wherein $R^6$ is a pyrrolidino group or a phenyl group, and $n^2$ is 0 or 1) ;
$R^3$ represents -CO-$R^7$ (wherein $R^7$ is an alkoxy group, an alkyl group, or an alkylamino group), a 1,3-oxazole group, an alkylhydroxy group, a hydrogen atom, or an oxygen atom;
$R^4$ represents a hydrogen atom, an oxygen atom, or a hydrocarbon group in which one or more hydrogen atoms may be replaced by one or more substituents;
$R^5$ represents a halogen atom, a halogenated alkyl group, or a halogenated alkylthio group; and
$n^1$ is an integer of 0 to 5); and
in the skeleton of the compound of formula (A),
each single solid line represents a single bond;
each double line consists of a solid line and a dotted line represents a single bond or a double bond; and
two dotted lines represent no bond or a double bond; or
a salt thereof.

(1-2) The compound according to (I-1), wherein in formula (A),

X is a carbon atom;
Y is an oxygen atom, a hydroxy group, or hydrocarbon group;
$R^1$ and $R^2$ are different, and each represents a group represented by formula (A-1) (wherein $R^6$ is a pyrrolidino group and $n^2$ is 0) or a hydrogen atom;
$R^3$ is -CO-$R^7$ (wherein $R^7$ is an alkoxy group, an alkyl group, or an alkylamino group) or a hydrogen atom;
$R^4$ is a hydrogen atom or a hydrocarbon group in which one or more hydrogen atoms may be replaced by one or more substituents;
$R^5$ is a halogen atom, a halogenated alkyl group, or a halogenated alkylthio group; and
$n^1$ is an integer of 1 to 3).

The compound according to (1-2) can also be represented by the following formula (Ax):

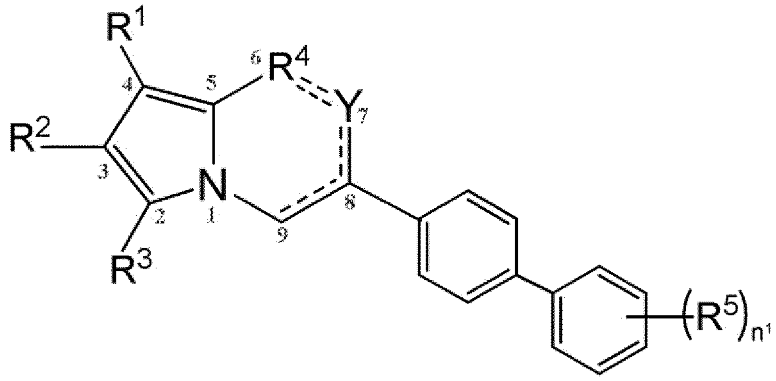

(A x)

wherein

Y represents an oxygen atom, a hydroxy group, or a hydrocarbon group;
$R^1$ and $R^2$ are different, and each represents a group represented by the following formula (A-2):

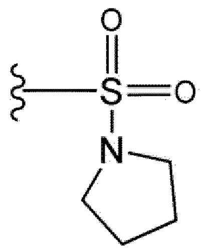

(A－2)

,

or a hydrogen atom;
$R^3$ represents -CO-$R^7$ (wherein $R^7$ represents an alkoxy group, an alkyl group, or an alkylamino group) or a hydrogen atom;
$R^4$ represents a hydrogen atom or a hydrocarbon group in which one or more hydrogen atoms may be replaced by one or more substituents;
$R^5$ represents a halogen atom, a halogenated alkyl group, or a halogenated alkylthio group;
$n^1$ is an integer of 1 to 3;
the double line between 6-position and 7-position in the skeleton of the compound, which consists of two dotted lines, represents no bond when Y is an oxygen atom or a hydroxy group, and the double line represents a double bond when Y is a hydrocarbon group;
the double line between 7-position and 8-position in the skeleton of the compound, which consists of a solid line and a dotted line, represents a double bond when Y is an oxygen atom, and the double line represents a single bond when Y is a hydroxy group or a hydrocarbon group;
the double line between 8-position and 9-position in the skeleton of the compound, which consists of a solid line and a dotted line, represents a single bond when Y is an oxygen atom or a hydroxy group, and the double line represents a double bond when Y is a hydrocarbon group.

In the present specification, a group of compounds represented by the above formula (Ax) may be collectively referred to as "Compound Ax."

(1-3) The compound according to (I-1), wherein in formula (A),

X is a carbon atom;
Y is an oxygen atom or a hydroxy group;
$R^1$ is a group represented by formula (A-1) (wherein $R^6$ is a pyrrolidino group and $n^2$ is 0) ;
$R^2$ is a hydrogen atom;
$R^3$ is -CO-$R^7$ (wherein $R^7$ is an alkoxy group, an alkyl group, or an alkylamino group), a 1,3-oxazole group, or an alkylhydroxy group;
$R^4$ is a hydrogen atom;

$R^5$ is a halogen atom, a halogenated alkyl group, or a halogenated thio group; and
$n^1$ is an integer of 1 to 3; and

in the skeleton of the compound,

the double line between 2-position and 3-position and the double line between 4-position and 5-position, which each consist of a solid line and a dotted line, represent a double bond;
the double line between 7-position and 8-position, which consists of a solid line and a dotted line, represents a single bond when Y represents is a hydroxy group, and the double line represents a double bond when Y is an oxygen atom;
the double line between 5-position and 6-position and the double line between 8-position and 9-position, which each consist of a solid line and a dotted line, represent a single bond;
the two dotted lines between 6-position and 7-position represent no bond; and
$R^3$ is bound to 2-position by a single bond.

The compound according to (1-3) can also be represented by the following formula (Aa):

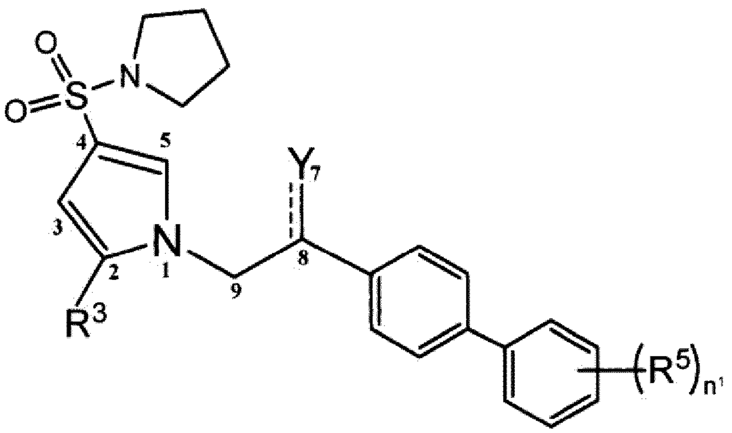

(A a)

wherein

Y is an oxygen atom or a hydroxy group;
$R^3$ is -CO-$R^7$ (wherein $R^7$ is an alkoxy group, an alkyl group, or an alkylamino group), a 1,3-oxazole group, an alkylhydroxy group, or a hydrogen atom;
$R^5$ is a halogen atom, a halogenated alkyl group, or a halogenated alkylthio group;
$n^1$ is an integer of 1 to 3; and
the double line between 7-position and 8-position in the skeleton of the compound, which consists of a solid line and a dotted line, represents a double bond when Y is an oxygen atom, and the double line represents a single bond when Y is a hydroxy group.

In the present specification, a group of compounds represented by the above formula (Aa) may be collectively referred to as "Compound Aa."
(1-4) The compound according to (I-1), wherein
in formula (A),

X is a carbon atom;
Y is an oxygen atom;
$R^1$ is a group represented by formula (A-1) (wherein $R^6$ is a pyrrolidino group and $n^2$ is 0) ;
$R^2$ is a hydrogen atom;
$R^3$ is -CO-$R^7$ (wherein $R^7$ is an alkoxy group, an alkyl group, or an alkylamino group), or a 1,3-oxazole group;
$R^4$ is a hydrogen atom;
$R^5$ is a halogenated alkyl group; and
$n^1$ is 1; and

in the skeleton of the compound,

the double line between 2-position and 3-position, the double line between 4-position and 5-position, and the double line between 7-position and 8-position, which each consist of a solid line and a dotted line, represent a double bond; the double line between 5-position and 6-position and the double line between 8-position and 9-position, which each consists of a solid line and a dotted line, represent a single bond;
the two dotted lines between 6-position and 7-position represent no bond; and
$R^3$ is bound to 2-position by a single bond.

The compound according to (1-4) can also be represented by the following formula ($Aa_1$):

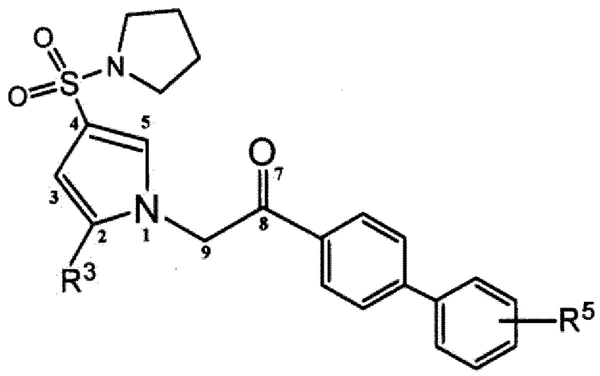

($Aa_1$)

wherein

$R^3$ is -CO-$R^7$ (wherein $R^7$ is an alkoxy group, an alkyl group, or an alkylamino group) or a 1,3-oxazole group; and
$R^5$ is a halogenated alkyl group.

In the present specification, a group of compounds represented by the above formula ($Aa_1$) may be collectively referred to as "Compound $Aa_1$."
(1-5) The compound according to (I-1), wherein
in formula (A),

X is a carbon atom;
Y is a hydroxy group;
$R^1$ is a group represented by formula (A-1) (wherein $R^6$ is a pyrrolidino group and $n^2$ is 0) ;
$R^2$ is a hydrogen atom;
$R^3$ is -CO-$R^7$ (wherein $R^7$ is an alkoxy group, an alkyl group, or an alkylamino group) or a 1,3-oxazole group;
$R^4$ is a hydrogen atom;
$R^5$ is a halogenated alkyl group; and
$n^1$ is an integer of 1; and

in the skeleton of the compound,

the double line between 2-position and 3-position and the double line between 4-position and 5-position, which each consist of a solid line and a dotted line, represent a double bond;
the double line between 5-position and 6-position, the double line between 7-position and 8-position, and the double line between 8-position and 9-position, which each consist of a solid line and a dotted line, represent a single bond;
the two dotted lines between 6-position and 7-position represent no bond; and
$R^3$ is bound to 2-position by a single bond.

The compound according to (1-5) can also be represented by the following formula ($Aa_2$):

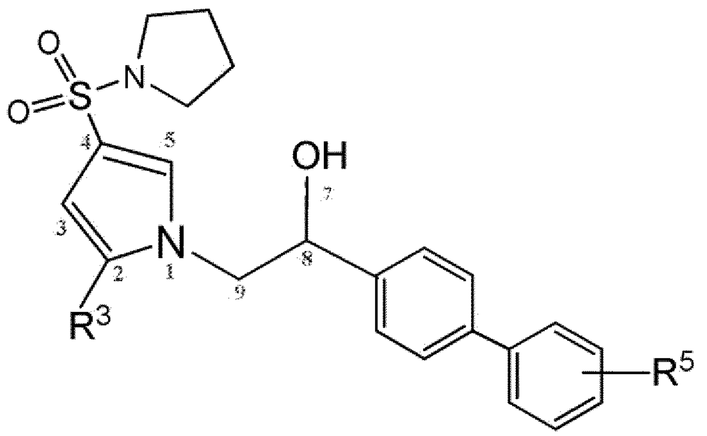

(Aa$_2$)

wherein

$R^3$ is -CO-$R^7$ (wherein $R^7$ is an alkoxy group, an alkyl group, or an alkylamino group) or a 1,3-oxazole group; and
$R^5$ is a halogenated alkyl group.

In the present specification, a group of compounds represented by the above formula (Aa$_2$) may be collectively referred to as "Compound Aa$_2$."

(1-6) The compound according to (I-1), wherein in formula (A),

X is a carbon atom;
Y is a hydrocarbon group;
$R^1$ is a hydroxy group;
$R^2$ is a group represented by formula (A-1) (wherein $R^6$ is a pyrrolidino group and $n^2$ is 0) ;
$R^3$ is hydrogen;
$R^4$ is a hydrocarbon group in which one or more hydrogen atoms may be replaced by one or more substituents;
$R^5$ is a halogen atom, a halogenated alkyl group, or a halogenated alkylthio group; and
$n^1$ is an integer of 1 to 3; and

in the skeleton of the compound,

the double line between 2-position and 3-position and the double line between 4-position and 5-position, which each consist of a solid line and a dotted line, represent a double bond;
the double line between 5-position and 6-position, which consists of a solid line and a dotted line, represents a single bond;
the two dotted lines between 6-position and 7-position represent a double bond;
the double line between 7-position and 8-position, which consists of a solid line and a dotted line, represents a single bond; and
the double line between 8-position and 9-position, which consists of a solid line and a dotted line, represents a double bond.

The compound according to (1-6) can also be represented by the following formula (Ab):

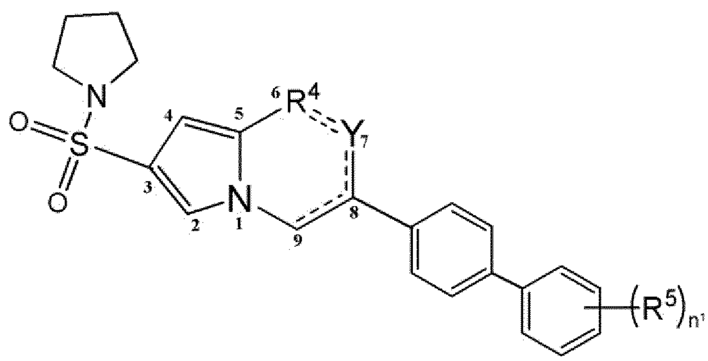

(A b)

wherein

Y is a hydrocarbon group;
$R^4$ is a hydrocarbon group wherein one or more hydrogen atoms may be replaced by one or more substituents;
$R^5$ is a halogen atom, a halogenated alkyl group, or a halogenated alkylthio group;
$n^1$ is an integer of 1 to 3;
the double line between 6-position and 7-position, which consists of a solid line and a dotted line, represents a double bond;
the double line between 7-position and 8-position, which consists of a solid line and a dotted line, represents a single bond; and
the double line between 8-position and 9-position, which consists of a solid line and a dotted line, represents a double bond.

In the present specification, a group of compounds represented by the above formula (Ab) may be collectively referred to as "Compound Ab."
(1-7) The pyrrole-type compound represented by formula (A) and having DOCK1-inhibiting activity (Compound A) includes any compounds selected from the group consisting of the following compounds, or salts thereof:

(A1) methyl 1-(2-oxo-2-(3'-(trifluoromethyl)-[1,1'-biphenyl]-4-yl)ethyl)-4-(pyrrolidin-1-yl-sulfonyl)-1H-pyrrole-2-carboxylate (RT-22),
(A2) 2-(2-acetyl-4-(pyrrolidin-1-yl-sulfonyl)-1H-pyrrol-1-yl)-1-(3'-(trifluoromethyl)-[1,1'-biphenyl]-4-yl)ethane (HS-16),
(A3) 1-(1-(2-oxo-2-(3'-(trifluoromethyl)-[1,1'-biphenyl]-4-yl)ethyl)-4-(pyrrolidin-1-yl-sulfonyl)-1H-pyrrol-2-yl)propan-1-one (HS-17),
(A4) N,N-dimethyl-1-(2-oxo-2-(3'-(trifluoromethyl)-[1,1'-biphenyl]-4-yl)ethyl)-4-(pyrrolidin-1)-yl-sulfonyl)-1H-pyrrole-2-carboxamide (RT-23),
(A5) 2-(2-(oxazol-2-yl)-4-(pyrrolidin-1-yl-sulfonyl)-1H-pyrrol-1-yl)-1-(3'-(trifluoromethyl)-[1,1'-biphenyl]-4-yl)ethan-1-one (RT-58),
(A6) methyl 1-(2-hydroxy-2-(3'-(trifluoromethyl)-[1,1'-biphenyl]-4-yl)ethyl)-4-(pyrrolidin-1-yl-sulfonyl)-1H-pyrrole-2-carboxylate (HS-6),
(A7) 2-(2-(hydroxymethyl)-4-(pyrrolidin-1-yl-sulfonyl)-1H-pyrrol-1-yl)-1-(3'-(trifluoromethyl)-[1,1'-biphenyl]-4-yl)ethan-1-ol (HS-14),
(A8) 2-(pyrrolidin-1-yl-sulfonyl)-6-(3'-(trifluoromethyl)-[1,1'-biphenyl]-4-yl)indolizin-8-yl acetate (HS-20), and
(A9) 3-(2-oxo-2-(3'-(trifluoromethyl)-[1,1'-biphenyl]-4-yl)ethyl)-5-((phenylsulfonyl)methyl)imidazolidine-2,4-dione (RT-35) .

(II) Substituted aza-alicyclic compounds having DOCK1-inhibiting activity

(II-1) A compound represented by the following formula (B):

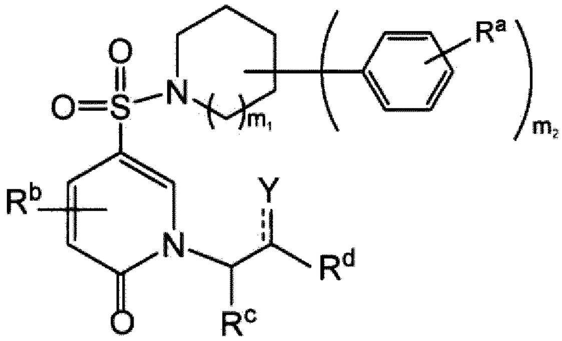

(B)

wherein

$R^a$ is one of the groups represented by the following formulas (B-1) to (B-4):

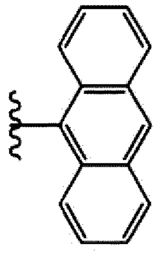

(B－1) ,

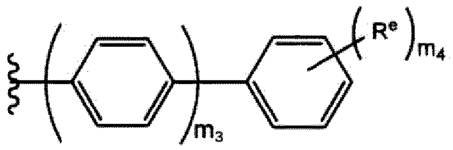

(B－2)

(wherein $R^e$ is the same or different, and each represents a halogen atom, a halogenated alkyl group, or a halogenated alkylthio group; $m_3$ is an integer of 0 to 2; and $m_4$ is an integer of 0 to 5),

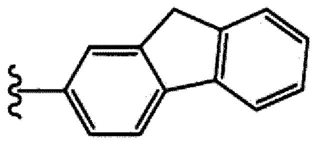

(B－3) ,

and

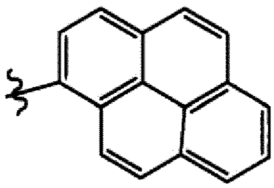

(B－4) ;

$R^b$ is a hydrogen atom or an amino group;
$R^c$ is a hydrogen atom or an alkyl group;
$R^d$ is a group represented by the following formula (B-5) :

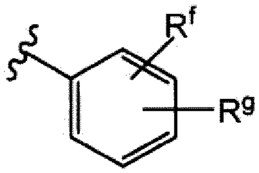

(B − 5)

(wherein $R^f$ is a hydrogen atom, a hydroxy group, an alkyl group, an acyloxy group, or an alkoxy group (wherein the alkoxy group may be linked to Y to form a ring), and $R^g$ is a group represented by the following formula (B-6) or a quinolyl group:

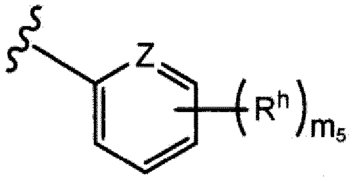

(B − 6)

(wherein Z is a carbon atom or a nitrogen atom, $R^h$ is the same or different, and each represents a halogen atom, a halogenated alkyl group, a halogenated alkylthio group, an alkyl group, an alkenyl group, or an alkylamino group; and $m_5$ is an integer of 1 to 3)); or
a group represented by the following formula (B-7):

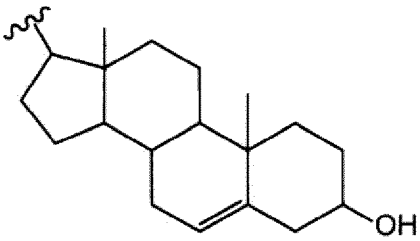

(B − 7) ;

Y is an oxygen atom, a hydroxy group, or an alkoxy group (wherein when $R^d$ is a group represented by formula (B-5), the alkoxy group may be linked to an alkoxy group represented by $R^f$ to form a ring);
$m_1$ means 0 or 1;
$m_2$ means 0 or 1; and
the double line consisting of a dotted line and a solid line in formula (B) represents a double bond when Y is an oxygen atom, and the double line represents a single bond when Y is a hydroxy group or an alkoxy group.

In the present specification, a group of compounds represented by the above formula (B) may be collectively referred to as "Compound B."

(II-2) The compound according to (II-1), wherein in formula (B),

$m_1$ and $m_2$ are both 1;
Y is an oxygen atom; and
$R^a$ is one of the groups represented by the following formulas (B-1) to (B-4):

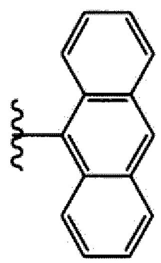

(B-1) ,

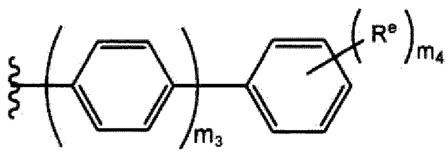

(B-2)

(wherein $R^e$ is the same or different, and each represents a halogen atom, a halogenated alkyl group, or a halogenated alkylthio group; $m_3$ is an integer of 0 to 2; and $m_4$ is an integer of 0 to 5),

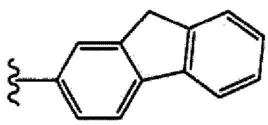

(B-3) ,

and

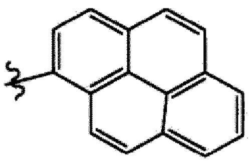

(B-4) ;

$R^b$ and $R^c$ are both a hydrogen atom;
$R^d$ is a group represented by the following formula (B-5):

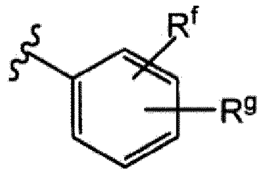

(B-5)

(wherein $R^f$ is a hydrogen atom, and $R^g$ is a group represented by the following formula (B-6):

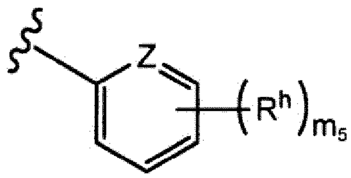

(B-6)

(wherein Z is a carbon atom; $R^h$ is the same or different, and each represents a halogen atom, a halogenated alkyl group, or a halogenated alkylthio group; and $m_5$ is an integer of 1 to 3)).

The compound according to (II-2) can also be represented by the following formula (Ba):

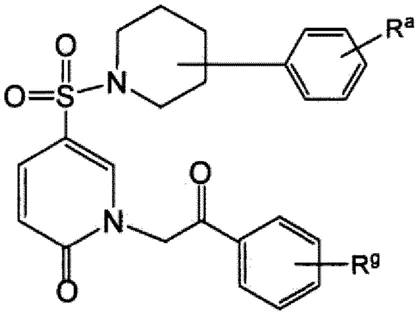

(Ba)

wherein

$R^a$ is one of the groups represented by the following formulas (B-1) to (B-4):

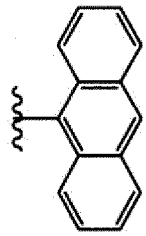

(B-1),

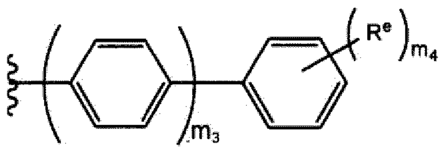

(B-2)

(wherein $R^e$ is the same or different, and each represents a halogen atom, a halogenated alkyl group, or a halogenated alkylthio group; $m_3$ is an integer of 0 to 2; and $m_4$ is an integer of 0 to 5),

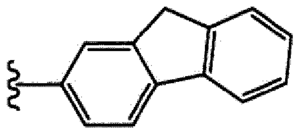

(B-3),

and

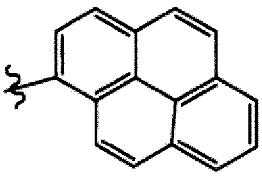

(B-4)

;

$R^g$ is a group represented by the following formula (B-6) :

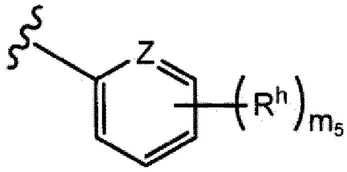

(B − 6)

(wherein Z is a carbon atom; $R^h$ is the same or different, and each represents a halogen atom, a halogenated alkyl group, or a halogenated alkylthio group; and $m_5$ is an integer of 1 to 3).

In the present specification, a group of compounds represented by the above formula (Ba) may be collectively referred to as "Compound B."

(II-3) The compound according to II-1, wherein in formula (B),

$m_1$ and $m_2$ are both 0;
$R^b$ is a hydrogen atom or an amino group;
$R^c$ is a hydrogen atom or an alkyl group;
$R^d$ is a group represented by the following formula (B-5) :

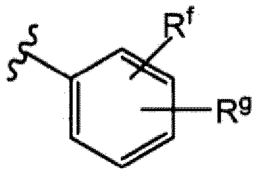

(B − 5)

(wherein $R^f$ is the same or different, and each represents a hydrogen atom, a hydroxy group, an alkyl group, an acyloxy group, or an alkoxy group (wherein the alkoxy group may be linked to Y to form a ring);
$R^g$ is a group represented by the following formula (B-6):

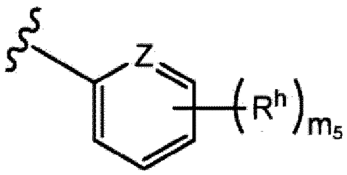

(B − 6)

(wherein Z is a carbon atom or a nitrogen atom; $R^h$ is the same or different, and each represents a halogen atom, a halogenated alkyl group, a halogenated alkylthio group, an alkyl group, an alkylene group, or an alkylamino group; $m_5$ is an integer of 1 to 3) or a quinolyl group; or
a group represented by the following formula (B-7):

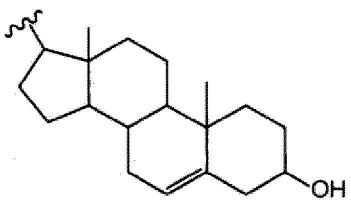

(B − 7) ;

Y is an oxygen atom, a hydroxy group, or an alkoxy group (wherein when $R^d$ is a group represented by formula (B-5), the alkoxy group may be linked to an alkoxy group represented by $R^f$ to form a ring) .

The compound according to (II-3) can also be represented by the following formula (Bb):

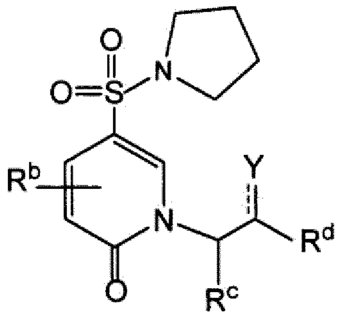

(B b)

wherein

$R^b$ is a hydrogen atom or an amino group;
$R^c$ is a hydrogen atom or an alkyl group;
$R^d$ is a group represented by the following formula (B-5) :

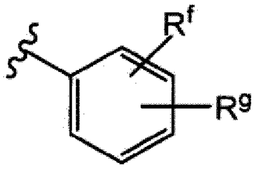

(B − 5)

(wherein $R^f$ is a hydrogen atom, a hydroxy group, an alkyl group, an acyloxy group, or an alkoxy group (wherein the alkoxy group may be linked to Y to form a ring) ; $R^g$ is a group represented by the following formula (B-6), or a quinolyl group:

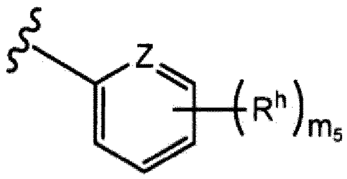

(B − 6)

(wherein Z is a carbon atom or a nitrogen atom; $R^h$ is the same or different, and each represents a halogen atom, a halogenated alkyl group, a halogenated alkylthio group, an alkyl group, an alkenyl group, or an alkyl amino group; and $m_5$ is an integer of 1 to 3)), or
a group represented by the following formula (B-7):

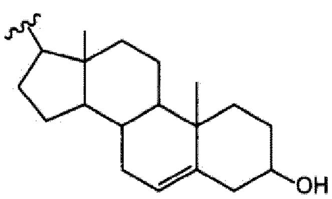

(B − 7) ;

Y is an oxygen atom, a hydroxy group, or an alkoxy group (wherein when $R^d$ is a group represented by formula (B-5), the alkoxy group may be linked to an alkoxy group represented by $R^f$ to form a ring) .

In the present specification, a group of compounds represented by formula (Bb) may be collectively referred to as "Compound Bb."

(II-4) The substituted aza-alicyclic compound represented by formula (Ba) and having DOCK1-inhibiting activity includes any compounds selected from the group consisting of the following compounds and salts thereof:

(B1) 5-((4-(4-(anthracen-9-yl)phenyl)piperidin-1-yl)sulfonyl)-1-(2-oxo-2-(3'-(trifluoromethyl)-[1,1'-biphenyl]-4-yl)ethyl)pyridin-2(1H)-one (KS-47)

(B2) 1-(2-oxo-2-(3'-(trifluoromethyl)-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one (KS-54),

(B3) 5-((4-([1,1':4',1":4",1"'-quaterphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one (KS-44),

(B4) 5-((4-([1,1'-biphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one (KS-43),

(B5) 1-(2-oxo-2-(3',4',5'-trifluoro-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one (KS-59),

(B6) 5-((4-(4-(9H-fluoren-2-yl)phenyl)piperidin-1-yl)sulfonyl)-1-(2-oxo-2-(3'-(trifluoromethyl)-[1,1'-biphenyl]-4-yl)ethyl)pyridin-2(1H)-one (KS-46), and

(B7) 1-(2-oxo-2-(3'-(trifluoromethyl)-[1,1'-biphenyl]-4-yl)ethyl)-5-((4-(4-(pyren-1-yl)phenyl)piperidin-1-yl)sulfonyl)pyridin-2(1H)-one (KS-45).

(II-5) The aza-alicyclic compound represented by formula (Bb) and having DOCK1-inhibiting activity includes any compound selected from the group consisting of the following compounds and salts thereof:

(B8) 1-(2-hydroxy-2-(3-hydroxy-3'-(trifluoromethyl)-[1,1'-biphenyl]-4-yl)ethyl)-5-(pyrrolidin-1-yl-sulfonyl)pyridin-2(1H)-one (AK-16),

(B9) 1-((2,2-dimethyl-7-(3-(trifluoromethyl)phenyl)-4H-benzo[d][1,3]dioxin-4-yl)methyl)-5-(pyrrolidin)-1-yl-sulfonyl)pyridin-2(1H)-one (AK-17),

(B10) 1-(2-oxo-2-(3'-((trifluoromethyl)thio)-[1,1'-biphenyl]-4-yl)ethyl)-5-(pyrrolidin-1-yl-sulfonyl)pyridin-2(1H)-one (KS-21),

(B11) 3-amino-1-(2-oxo-2-(3'-(trifluoromethyl)-[1,1'-biphenyl]-4-yl)ethyl)-5-(pyrrolidin-1-yl-sulfonyl)pyridin-2(1H)-one (RT-13),

(B12) 2-bromo-1-(3-methyl-3-(trifluoromethyl)-[1,1'-biphenyl]-4-yl)ethan-1-one (AK-5),

(B13) 1-(2-(3-hydroxy-3'-(trifluoromethyl)-[1,1'-biphenyl]-4-yl)-2-oxoethyl)-5-(pyrrolidin-1-yl-sulfonyl)pyridin-2(1H)-one (AK-15),

(B14) 1-(2-(3-methoxy-3'-(trifluoromethyl)-[1,1'-biphenyl]-4-yl)-2-oxoethyl)-5-(pyrrolidin-1-yl-sulfonyl)pyridin-2(1H)-one (AK-14),

(B15) 4-(2-(2-oxo-5-(pyrrolidin-1-yl-sulfonyl)pyridin-1(2H)-yl)acetyl)-3'-(trifluoromethyl)-[1,1'-biphenyl]-3-yl-acetate (AK-25),

(B16) 1-(2-(3-isobutoxy-3'-(trifluoromethyl)-[1,1'-biphenyl]-4-yl)-2-oxoethyl)-5-(pyrrolidin-1-yl-sulfonyl)pyridin-2(1H)-one (AK-22),

(B17) 1-(1-(3-(hexyloxy)-3'-(trifluoromethyl)-[1,1'-biphenyl]-4-yl)-1-oxooctan-2-yl)-5-(pyrrolidin-1-yl-sulfonyl)pyridin-2(1H)-one (AK-23),

(B18) 1-(2-oxo-2-(3-phenethoxy-3'-(trifluoromethyl)-[1,1'-biphenyl]-4-yl)ethyl)-5-(pyrrolidin-1-yl-sulfonyl)pyridin-2(1H)-one (AK-24),

(B19) 1-(2-oxo-2-(3'-vinyl-[1,1'-biphenyl]-4-yl)ethyl)-5-(pyrrolidin-1-yl-sulfonyl)pyridin-2(1H)-one (KS-26),

(B20) 1-(2-(4'-(dimethylamino)-3'-methyl-[1,1'-biphenyl]-4-yl)-2-oxoethyl)-5-(pyrrolidin-1-yl-sulfonyl)pyridin-2(1H)-one (KS-16),

(B21) 1-(2-oxo-2-(4-(6-(trifluoromethyl)pyridin-2-yl)phenyl)ethyl)-5-(pyrrolidin-1-yl-sulfonyl)pyridin-2(1H)-one (KS-22),

(B22) 1-(2-oxo-2-(4-(quinolin-7-yl)phenyl)ethyl)-5-(pyrrolidin-1-yl-sulfonyl)pyridin-2(1H)-one (KS-18),

(B23) 1-(2-oxo-2-(4-(quinolin-6-yl)phenyl)ethyl)-5-(pyrrolidin-1-yl-sulfonyl)pyridin-2(1H)-one (KS-23), and

(B24) 1-(2-((3S,10R,13S,17S)-3-hydroxy-10,13-dimethyl-2, 3, 4, 7, 8, 9,10,11,12,13,14,15,16,17-tetradecahydro-1H-cyclopenta[a]phenanthren-17-yl)-2-oxoethyl)-5-(pyrrolidin-1-yl-sulfonyl)pyridin-2(1H)-one (AK-20).

<u>(III) Applications of pyrrole-type compounds or substituted aza-alicyclic compounds having DOCK1-inhibiting activity</u>

(III-1) A DOCK1-selective inhibitor containing as an active ingredient at least one compound selected from the group consisting of the compounds according to (I-1) to (1-7) and (II-1) to (II-5), and salts thereof.

(III-2) A pharmaceutical composition comprising at least one compound selected from the compounds according to (I-1) to (1-7) and (II-1) to (II-5), and salts thereof; and a pharmaceutically acceptable carrier or an additive.

(III-3) The pharmaceutical composition according to (III-2), which is for use in the treatment and/or prevention of a

cancer.

(III-4) The pharmaceutical composition according to (III-3), wherein the cancer is an invasive or metastatic cancer.

Advantageous Effects of Invention

According to the present invention, a compound that can be used as an active ingredient of an anticancer agent can be provided. In particular, the compound of the present invention has DOCK1-inhibiting activity, preferably DOCK1-selective Rac activity-inhibiting activity, and can exert anticancer effects based on this activity. Preferable compounds of the present invention are characterized by having a high selectivity to DOCK1, and little influence on the immune system via DOCK2. More preferable compounds of the present invention have a higher DOCK1-inhibiting effect (potency), higher DOCK1/DOCK2 selectivity (selectivity) and/or higher cell invasion response inhibition (cell activity), as compared with TBOPP, which is known as a DOCK1-selective inhibitor; and can be used as a DOCK1-selective inhibitor, and as a drug having an anticancer effect with little side effects.

Brief Description of Drawings

Fig. 1 is a table showing the chemical formulas, the inhibitory activity against DOCK1 ($IC_{50}$ value in GEF assay), the DOCK1-selective inhibitory effect (DOCK1/DOCK2 selectivity), and the inhibitory effect on cancer cell invasion response (cell invasion inhibitory activity to 3LL cells) of nine representative compounds encompassed by Compounds A of the present invention.

Fig. 2 is a table showing the chemical formulas, the inhibitory activity against DOCK1 ($IC_{50}$ value in GEF assay), the DOCK1-selective inhibitory effect (DOCK1/DOCK2 selectivity), and the inhibitory effect on cancer cell invasion response (cell invasion inhibitory activity to 3LL cells) of seven representative compounds encompassed by Compounds Ba of the present invention.

Fig. 3-1 is a table showing the chemical formulas, the inhibitory activity against DOCK1 ($IC_{50}$ value in GEF assay), the DOCK1-selective inhibitory effect (DOCK1/DOCK2 selectivity), and the inhibitory effect on cancer cell invasion response (cell invasion inhibitory activity to 3LL cells) of representative compounds encompassed by Compounds Bb of the present invention.

Fig. 3-2 is a table showing the chemical formulas, the inhibitory activity against DOCK1 ($IC_{50}$ value in GEF assay), the DOCK1-selective inhibitory effect (DOCK1/DOCK2 selectivity), and the inhibitory effect on cancer cell invasion response (cell invasion inhibitory activity against 3LL cells) of representative compounds encompassed by Compounds Bb of the present invention.

Fig. 4 is graphs showing the results confirming the DOCK1-selective inhibitory effect of RT-22, which is a representative pyrrole-type compound of the present invention (Compound A), and KS-59, which is a representative substituted aza-alicyclic compound of the present invention (Compound B) (Pharmacological Test 1). (A) is a graph showing reaction inhibition curves plotting the measurement values of Rac activation by the mouse DOCK1 DHR-2 domain or DOCK2 DHR-2 domain in the presence of various concentrations of RT-22, taking the measurement values of that of DMSO (control: in the absence of RT-22) as 100%. The numerical values in the graph are $IC_{50}$ values ($\mu$M) for mouse DOCK1 and DOCK2 in order from the top, respectively. (B) is a graph showing reaction inhibition curves plotting the measurement values of Rac activation by the mouse DOCK1 DHR-2 domain or DOCK2 DHR-2 domain in the presence of various concentrations of KS-59, taking the measurement values of that of DMSO (control: in the absence of KS-59) as 100%. The numerical values in the graph are $IC_{50}$ values ($\mu$M) for mouse DOCK1 and DOCK2 in order from the top, respectively. (C) is a graph showing reaction inhibition curves plotting the measurement values of Rac activation by the human DOCK1 DHR-2 domain or DOCK2 DHR-2 domain in the presence of various concentrations of RT-22, taking the values of that of DMSO (control) as 100%. The numerical values in the graph are $IC_{50}$ values ($\mu$M) for human DOCK1 and DOCK2 in order from the top, respectively. (D) is a graph showing reaction inhibition curves plotting the measurement values of Rac activation by the human DOCK1 DHR-2 domain or DOCK2 DHR-2 domain in the presence of various concentrations of KS-59, taking the values of that of DMSO (control) as 100%. The numerical values in the graph are $IC_{50}$ values ($\mu$M) for human DOCK1 and DOCK2 in order from the top, respectively. (E) is a graph showing reaction inhibition curves plotting the measurement values of Rac activation by the mouse Trio DH-PH domain or Tiam1 DH-PH domain in the presence of various concentrations of RT-22, taking the values of that of DMSO (control) as 100%. The numerical values in the graph are $IC_{50}$ values ($\mu$M) for mouse Trio DH-PH domain and Tiam1 DH-PH domain in order from the top, respectively.

Fig. 5-1 is graphs showing the experimental results confirming the cancer cell invasion-inhibitory effect of RT-22, HS-6, and HS-20, which are representative pyrrole-type compounds of the present invention (Compounds A), and KS-59 and RT-13, which are representative substituted aza-alicyclic compounds of the present invention (Compounds B), in comparison with that of known TBOPP (TS-45) (Pharmacological Study 2). (A) is a graph showing quantified invasive potential of mouse lung carcinoma cell line (3LL) in the presence of RT-22, KS-59, or TBOPP. (B) is a graph showing quantified invasive potential of mouse lung carcinoma cell line (3LL) in the presence of HS-6, HS-20, RT-13, or TBOPP. The vertical axis represents percent cell invasion (%), taking the number of invading cells in the control test (DMSO) as 100%.

Fig. 5-2(C) is a graph showing quantified invasive potential of mouse pancreatic cancer cell line (PANC-02) in the presence of RT-22. Fig. 5-2(D) is a graph showing quantified invasive potential of human breast cancer cell line (MDA-MB-157) in the presence of RT-22. The vertical axis represents percent cell invasion (%), taking the number of invading cells in the control test (DMSO) as 100%.

Fig. 6 shows the experimental results of macropinocytosis inhibition of RT-22, which is a representative pyrrole-type compound of the present invention (Compound A) (Pharmacological Test 3). (A) is fluorescence microscope images based on analysis on macropinocytosis activity of mouse lung carcinoma cell line (3LL), using uptake of TMR-dextran (dextran labeled with TMR, red) as an index. The cell nuclei were stained with DAPI (blue). (B) is a graph showing quantified macropinocytosis activity in the presence of RT-22, based on the fluorescence microscope images in (A). (C) is fluorescence microscope images based on analysis on macropinocytosis activity of mouse pancreatic cancer cell line (PANC-02), using uptake of TMR-dextran (dextran labeled with TMR, red) as an index. The cell nuclei were stained with DAPI (blue). (D) is a graph showing quantified macropinocytosis activity in the presence of RT-22, based on the fluorescence microscope images in (C). The vertical axis represents the degree of dextran uptake under each condition, relative to the degree of dextran uptake by the cells in the control test (with the addition of DMSO) taken as 1.

Fig. 7 is a graph showing the experimental results confirming the effect of RT-22, which is a representative pyrrole-type compound of the present invention (Compound A), on migration of lymphocytes (Pharmacological Test 4). The vertical axis represents the number of cells that migrated to the lower chamber (cells that migrated towards CCL21) expressed as a percentage (%) in the total number of cells seeded in the Transwell under each condition.

Fig. 8 is a graph showing the experimental results confirming the effect on the viability of lymphocytes (T cells) (Pharmacological Test 5).

Fig. 9 is a graph showing the experimental results confirming the inhibitory effect of RT-22, which is a representative pyrrole-type compound of the present invention (Compound A), on tumor growth in a model to which a mouse lung carcinoma cell line (3LL) was subcutaneously administered (Pharmacological Test 6). In the graph, the vertical axis represents the volume of the tumor mass transplanted in the mice, and the horizontal axis represents the number of days elapsed. RT-22 or a solvent alone as a control was administered continuously using a microinfusion pump (intravenous administration, i.v.) (control group (solvent only): n = 6; RT-22 group: n = 5) .

## Description of Embodiments

### (1) Explanation of Substituents

The present specification describes compounds represented by various chemical formulas, wherein groups are represented by symbols. The meaning and specific examples of the groups represented by symbols are explained below.

Unless otherwise specified, examples of the "alkyl group" in the compound of the present invention usually include linear or branched alkyl groups having 1 to 6 carbon atoms. Examples of such alkyl groups include methyl, ethyl, propyl, isopropyl, butyl, isobutyl, s-butyl, t-butyl, pentyl, 1-methylbutyl, 2-methylbutyl, 3-methylbutyl, 1,1-dimethylpropyl, 1,2-dimethylpropyl, 2,2-dimethylpropyl, 1-ethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl group, and the like. Preferable examples include lower alkyl groups having 1 to 4 carbon atoms, such as methyl, ethyl, propyl, isopropyl, butyl, and isobutyl; and more preferably lower alkyl groups having 1 to 2 carbon atoms, such as methyl and ethyl.

Unless otherwise specified, examples of the "alkenyl group" in the compound of the present invention include groups having at least one double bond in the alkyl group mentioned above. More specifically, examples of the alkenyl group include linear or branched alkenyl group having 1 to 2 double bonds and 2 to 6 carbon atoms; and preferably linear or branched alkenyl groups having 2 to 4 carbon atoms and 1 double bond. Examples of such alkenyl groups include, but are not limited to, vinyl, allyl, propenyl, isopropenyl, 1-butenyl, 2-butenyl, and 2-methylallyl; more preferably vinyl, allyl, and propenyl; and even more preferably vinyl.

The "alkoxy group" in the compound of the present invention is, for example, a hydroxyl group whose hydrogen atom is replaced by an alkyl group having 1 to 6 carbon atoms (preferably 1 to 4 carbon atoms), or a hydroxyl group whose hydrogen atom is replaced by an aromatic hydrocarbon group. Examples of such alkoxy groups include methoxy, ethoxy, 1-propoxy, 2-propoxy, 1-butoxy, 2-butoxy, 2-methyl-1-propoxy, 2-methyl-2-propoxy, 1-pentyloxy, 2-pentyloxy,

3-pentyloxy, 2-methyl-2-butoxy, 3-methyl-2-butoxy, 1-hexyloxy, 2-hexyloxy, 3-hexyloxy, 2-methyl-1-pentyloxy, 3-methyl-1-pentyloxy, 2-ethyl-1-butoxy, 2,2-dimethyl-1-butoxy, and 2,3-dimethyl-1-butoxy, as well as benzoxy, phenethoxy, and the like. Preferable examples include methoxy, ethoxy, 1-propoxy, 2-propoxy, 1-butoxy, hexyloxy, and phenethoxy; and more preferably methoxy. In the compound of the present invention, the alkoxy group may be bound to another alkoxy group to form a ring.

Examples of the "alkoxy group" of the "alkoxycarbonyl group" in the compound of the present invention include the alkoxy groups mentioned above. Preferable examples include methoxy, ethoxy, 1-propoxy, and 2-propoxy; and more preferably methoxy.

The "alkylamino group" in the compound of the present invention is a monovalent functional group (-NHR, -NRR’) obtained by removing a hydrogen atom from a primary amine or a secondary amine whose hydrogen atom of ammonia is replaced by alkyl. Preferably, the alkylamino group is a dialkylamino group (-NRR’) obtained by removing a hydrogen atom from a secondary amine. Examples of the alkyl group represented by R or R’ include the alkyl groups mentioned above. Preferable examples include lower alkyl groups having 1 to 4 carbon atoms; and more preferably lower alkyl groups having 1 to 2 carbon atoms, such as methyl and ethyl. When the "alkylamino group" is a dialkylamino group represented by -NRR’, alkyl groups represented by R and R’ may be the same or different. The alkyl groups represented by R and R’ are preferably the same alkyl group selected from lower alkyl groups having 1 to 4 carbon atoms; more preferably a lower alkyl group having 1 to 2 carbon atoms; and particularly preferably methyl.

Examples of the "alkylhydroxy group" in the compound of the present invention include a monovalent functional group obtained by replacing at least one hydrogen atom of an alkyl group, such as those mentioned above, with at least one hydroxy group. Preferably, the alkylhydroxy group is a functional group (-R-OH) obtained by replacing one hydrogen atom of an alkyl group with a hydroxy group. The alkyl group is preferably a lower alkyl group having 1 to 4 carbon atoms; more preferably a lower alkyl group having 1 to 2 carbon atoms; and particularly preferably methyl.

Examples of the "halogen atom" in the compound of the present invention include a fluorine atom, a chlorine atom, a bromine atom, and an iodine atom. Preferable examples include a chlorine atom, a bromine atom, and a fluorine atom; and more preferably a fluorine atom.

Examples of the "halogenated alkyl group" in the compound of the present invention include a monovalent functional group in which at least one hydrogen atom of an alkyl group, such as those mentioned above, is replaced by at least one halogen atom. Examples of the halogen atom include, but are not limited to, the halogen atoms mentioned above. The halogen atom is preferably a chlorine atom, a bromine atom, or a fluorine atom, and more preferably a fluorine atom (a fluoroalkyl group). The alkyl group is preferably a lower alkyl group having 1 to 4 carbon atoms, more preferably a lower alkyl group having 1 to 2 carbon atoms, and particularly preferably methyl (a halogenated methyl group). The number of hydrogen atoms replaced by halogen atoms is not limited to one, and can be two or more. Examples of the halogenated alkyl group include a monohaloalkyl group ($-CH_3X$), a dihaloalkyl group ($-CHX_2$), and a trihaloalkyl group ($-CX_3$) in which 1 to 3 hydrogen atoms of a methyl group are replaced by one or more halogen atoms (X). Preferably, the halogenated alkyl group is a trihaloalkyl group. The halogen atoms by which hydrogen atoms are replaced do not have to be of the same kind, and may be a combination of two or more kinds. Preferably, the halogen atoms are of the same kind. In the compound of the present invention, the halogenated alkyl group is more preferably a trifluoromethyl group.

Examples of the "halogenated alkylthio group" in the compound of the present invention include monovalent functional groups in which a hydrogen atom of an alkylthio group (also referred to as an alkylsulfanyl group) is replaced by a halogen atom. Examples of the halogen atom include, but are not limited to, the halogen atoms mentioned above. Preferable examples include a chlorine atom, a bromine atom, and a fluorine atom; and more preferably a fluorine atom (a fluoroalkylthio group). Examples of the "alkyl group" of the alkylthio group include the alkyl groups mentioned above. Preferable examples include lower alkyl groups having 1 to 4 carbon atoms; more preferably lower alkyl groups having 1 to 2 carbon atoms; and particularly preferably methyl (a halogenated methylthio group). The number of hydrogen atoms replaced by halogen atoms is not limited to one, and can be two or more. Examples include those obtained by replacing one to three of the three hydrogen atoms of a methylthio group with halogen atoms (X), such as monohaloalkylthio groups ($-SCH_2X$), dihaloalkylthio groups ($-SCHX_2$), and trihaloalkylthio groups ($-SCX_3$). The halogenated alkylthio group is preferably a trihaloalkylthio group. The halogen atoms with which hydrogen atoms are replaced do not necessarily have to be of the same kind, and can be a combination of two or more kinds. Preferably, the halogen atoms are of the same kind. The halogenated alkyl group in the compound of the present invention is preferably a trifluoromethylthio group.

Examples of the acyl group of the "acyloxy group" (alkanoyl group: R-CO-) in the compound of the present invention include, but are not limited to, formyl (methanoyl), acetyl (ethanoyl), propionyl (propanoyl), benzoyl, and acrylyl (propenoyl). Preferable examples include formyl, acetyl, and propionyl; more preferably formyl and acetyl; and particularly preferably an acetoxy group having acetyl as an acyl group.

(2) Pyrrole-type Compound Having DOCK1-inhibiting Activity (Compound A)

The compound having a DOCK1-inhibiting activity according to the present invention includes compounds represented by the following formula (A) and salts thereof. In the present specification, such compounds are collectively referred to as "Compound A" or "pyrrole-type compounds." The compounds referred to as "Compound A" or "pyrrole-type compounds" include compounds having a nitrogen-containing cyclic structure that is not exactly a pyrrole ring, but is similar to a pyrrole ring (a benzo-condensed pyrrole ring, an imidazolidine-2,4-dione group).

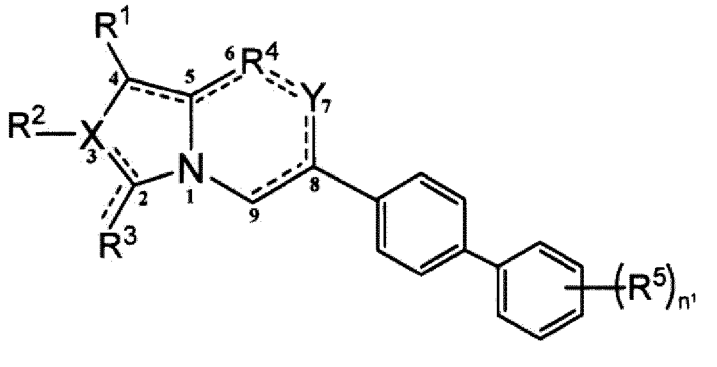

(A)

In formula (A), X represents a carbon atom or a nitrogen atom, and preferably a carbon atom. In the cyclic skeleton of the above formula, the double line between 2-position and 3-position and the double line between 4-position and 5-position, which each consist of a solid line and a dotted line, represent a double bond when X is a carbon atom, and the double lines represent a single bond when X is a nitrogen atom.

$R^1$ and $R^2$ are a hydrogen atom or a group represented by the following formula (A-1) . When one of $R^1$ and $R^2$ is a hydrogen atom, the other is a group represented by (A-1). When Y is a hydrocarbon group and the two dotted lines between 6-position and 7-position represent a double bond, $R^1$ is preferably a hydrogen atom and $R^2$ is preferably a group represented by (A-1). When Y is an oxygen atom or a hydroxy group and the two dotted lines between 6-position and 7-position represent no bond, $R^1$ is preferably a group represented by (A-1) and $R^2$ is preferably a hydrogen atom.

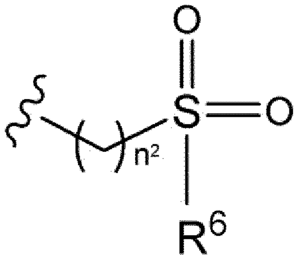

(A－1)

In the above formula (A-1), $R^6$ is a pyrrolidino group or a phenyl group. The pyrrolidino group and the phenyl group may be unsubstituted or substituted with one substituent, or two to four substituents. When the pyrrolidino group or the phenyl group has one or more substituents, examples of the substituents include halogen atoms, alkyl groups having 1 to 6 carbon atoms, alkoxy groups having 1 to 6 carbon atoms, a hydroxy group, a carboxy group, alkoxycarboxy groups, a cyano group, a $CF_3$ group, a $CF_3O$ group, a $CHF_2O$ group, a $CF_3CH_2O$ group, and the like. $R^6$ is preferably an unsubstituted pyrrolidino group or a phenyl group, and more preferably an unsubstituted pyrrolidino group. In formula (A-1), $n^2$ is 0 or 1, and preferably 0. Although there is no particular limitation, when $R^6$ is a pyrrolidino group, $n^2$ is preferably 0; and when $R^6$ is a phenyl group, $n^2$ is preferably 1.

$R^3$ is -CO-$R^7$, a 1,3-oxazole group, an alkylhydroxy group, a hydrogen atom, or an oxygen atom. In the above formula (A), the double line between 2-position in the cyclic skeleton and $R^3$, which consists of a solid line and a dotted line, represents a double bond when $R^3$ is an oxygen atom, and the double line represents a single bond when $R^3$ is a substituent or atom other than an oxygen atom.

$R^3$ is preferably -CO-$R^7$. $R^7$ is an alkoxy group, an alkyl group, or an alkylamino group. The alkoxy group represented by $R^7$ is as described above. Preferable examples include, but are not limited to, methoxy, ethoxy, 1-propoxy, and 2-propoxy; and more preferably methoxy. The alkyl group represented by $R^7$ is also as described above. Preferable examples include, but are not limited to, lower alkyl groups having 1 to 4 carbon atoms, such as methyl, ethyl, propyl, isopropyl, butyl, and isobutyl; and more preferably methyl and ethyl. The alkylamino group represented by $R^7$ is as

described above, and is preferably a dialkylamino group. Specific examples of preferable dialkylamino groups include, but are not limited to, lower dialkylamino groups having 1 to 4 carbon atoms, such as dimethylamino, diethylamino, methylethylamino, dipropylamino, and dibutylamino; and more preferably dimethylamino.

The 1,3-oxazole group represented by $R^3$ can be any of 1,3-oxazol-2-yl, 1,3-oxazol-4-yl, and 1,3-oxazol-5-yl; and is preferably 1,3-oxazol-2-yl. The alkylhydroxy group represented by $R^3$ is as described above. Preferable examples include lower alkylhydroxy groups having 1 to 4 carbon atoms, such as methylhydroxy, ethylhydroxy, propylhydroxy, and butylhydroxy; and more preferably methylhydroxy.

$R^4$ represents a hydrogen atom, an oxygen atom, or a hydrocarbon group in which at least one hydrogen atom may be replaced by at least one substituent. $R^4$ is preferably a hydrogen atom. The double line between 5-position and 6-position in the cyclic skeleton of the above formula (A), which consists of a solid line and a dotted line, represents a double bond when $R^4$ is an oxygen atom, and the double line represents a single bond when $R^4$ is an atom other than an oxygen atom. When $R^4$ is a hydrocarbon group in which at least one hydrogen atom may be replaced by at least one substituent, the substituent is preferably an acyloxy group. The acyloxy group is as described above. Preferable examples include formyloxy and acetoxy group; and more preferably acetoxy. Examples of other substituents include, but are not limited to, halogen atoms, a hydroxyl group, a cyano group, a $CF_3$ group, a $CF_3O$ group, a $CHF_2O$ group, a $CF_3CH_2O$ group, and the like. The hydrocarbon group is preferably methyl. When $R^4$ is a hydrocarbon group in which at least one hydrogen atom may be replaced by at least one substituent, $R^4$ may be bound to a hydrocarbon group represented by Y (described later) to form a ring. In this case, the double line between 6-position and 7-position in the cyclic skeleton of formula (A), which consists of two dotted lines, represents a double bond.

Y represents an oxygen atom, a hydroxy group, or a hydrocarbon group. Preferably, Y is an oxygen atom or a hydroxy group; and more preferably an oxygen atom. Examples of compounds wherein Y is an oxygen atom or a hydroxy group include Compound Aa represented by the above chemical formula (Aa). Examples of Compound Aa include Compound $Aa_1$ wherein Y is an oxygen atom, and Compound $Aa_2$ wherein Y is a hydroxy group. Examples of compounds wherein Y is a hydrocarbon group include Compound Ab represented by the above chemical formula (Ab).

In the above formula A, the double line between 7-position and 8-position, which consists of a solid line and a dotted line, represents a double bond when Y is an oxygen atom; and the double bond represents a single bond when Y is an atom other than an oxygen atom. In the above formula, the double line between 8-position and 9-position, which consists of a solid line and a dotted line, represents a single bond when Y is an oxygen atom or a hydroxy group. When Y is a hydrocarbon group, Y may be bound to $R^4$ that is a hydrocarbon group in which one or more hydrogen atoms may be replaced as described above, to form a ring. In this case, in the cyclic skeleton of formula (A), the double line between 8-position and 9-position, which consists of a solid line and a dotted line, and the double line between 6-position and 7-position, which consists of two dotted lines, represent a double bond.

$R^5$ is a halogen atom, a halogenated alkyl group, or a halogenated alkylthio group. The halogen atom, the halogenated alkyl group, and the halogenated alkylthio group are as described above. The halogen atom is preferably a fluorine atom. The halogenated alkyl group is preferably a trihaloalkyl group. The halogenated alkylthio group is preferably trihaloalkylthio. The halogen atom of the halogenated alkyl group and the halogen atom of the halogenated alkylthio group are both preferably a fluorine atom. Examples of the alkyl group include lower alkyl groups having 1 to 4 carbon atoms; preferably methyl and ethyl; and more preferably methyl. $R^5$ is preferably a halogenated alkyl group; more preferably a trihaloalkyl group; even more preferably a trifluoroalkyl group; and particularly preferably trifluoromethyl.

$n^1$ is an integer of 0 to 5. When $R^5$ is a halogen atom, $n^1$ is preferably an integer of 1 to 3. When $R^5$ is a halogenated alkyl group or a halogenated alkylthio group, $n^1$ is preferably 1. When $n^1$ is 1, $R^5$ can be located at a meta-, ortho-, or para-position of the benzene ring; preferably at a meta-position; and more preferably at 3'-position.

Examples of Compound A according to the present invention include Compound Ax represented by chemical formula Ax as a preferable group of compounds. As shown below, Compound A of the present invention can be classified into two groups of compounds depending on whether $R^4$ is a hydrogen atom. Compounds in which $R^4$ is a hydrogen atom can be further classified into compounds in which Y is an oxygen atom, and compounds in which Y is a hydroxy group.

(1) A group of compounds in which $R^4$ is a hydrogen atom (a group of these compounds is collectively referred to as "Compound Aa"), wherein

(Aa1) a group of compounds in which Y is an oxygen atom (a group of these compounds is collectively referred to as "Compound Aa1"); and
(Aa2) a group of compounds in which Y is a hydroxy group (a group of these compounds is collectively referred to as "Compound Aa2").

(2) A group of compounds in which $R^4$ is an atom or atomic group other than a hydrogen atom (a group of these compounds is collectively referred to as "Compound Ab"), wherein

(Ab1) a group of compounds in which $R^4$ is a hydrocarbon group in which one or more hydrogen atoms may be replaced by one or more substituents (a group of these compounds is collectively referred to as "Compound Abl"); and

(Ab2) a group of compounds in which $R^4$ is an oxygen atom (a group of these compounds is collectively referred to as "Compound Ab2").

The compounds in these groups are described in more detail below.

(2-1) Compound Aa

Compound Aa is represented by formula (A), wherein $R^4$ is a hydrogen atom, Y is an oxygen atom or a hydroxy group, and preferably X is a carbon atom; $R^1$ is a group represented by formula (A-1) (wherein $R^6$ is a pyrrolidino group, and $n^2$ is 0); $R^3$ is -CO-$R^7$ ($R^7$ is an alkoxy group, an alkyl group, or an alkylamino group), a 1,3-oxazole group, an alkylhydroxy group, or a hydrogen atom; $R^5$ is a halogen atom, a halogenated alkyl group, or a halogenated alkylthio group; and $n^1$ is an integer of 1 to 3), the double line between 2-position and 3-position and the double line between 4-position and 5-position in the skeleton of formula (A), which each consists of a solid line and a dotted line, represent a double bond; the double line between 7-position and 8-position, which consists of a solid line and a dotted line, represents a double bond when Y is an oxygen atom, and the double line represents a single bond when Y is a hydroxy group; the double line between 5-position and 6-position and the double line between 8-position and 9-position, which each consist of a solid line and a dotted line, represent a single bond; the two dotted lines between 6-position and 7-position represent no bond; and $R^3$ is bound to 2-position by a single bond.

Compound Aa can be represented by the following formula (Aa) :

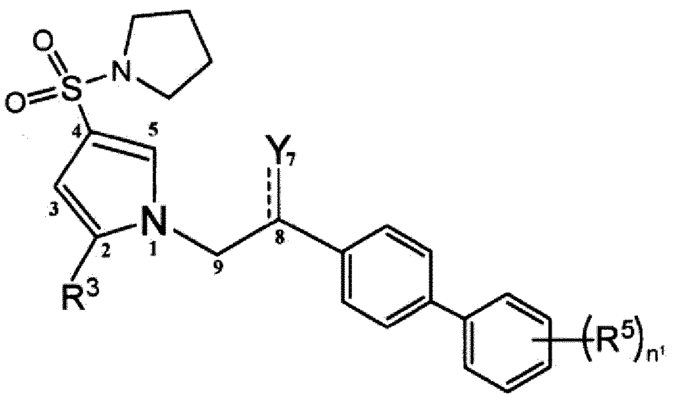

(A a)

wherein Y is an oxygen atom or a hydroxy group;

$R^3$ is -CO-$R^7$ (wherein $R^7$ is an alkoxy group, an alkyl group, or an alkylamino group), a 1,3-oxazole group, an alkylhydroxy group, or a hydrogen atom;

$R^5$ is a halogen atom, a halogenated alkyl group, or a halogenated alkoxy group;

$n^1$ is an integer of 1 to 3; and

the double line between 7-position and 8-position in the skeleton of the compound, which consists of a solid line and a dotted line, represents a double bond when Y is an oxygen atom, and the double line represents a single bond when Y is a hydroxy group.

As described above, Compound Aa can be classified according to whether Y is an oxygen atom or a hydroxy group. Examples of Compound Aa1 in which Y is an oxygen atom preferably include the following compounds.

Compound Aa1

Compound Aa1 refers to a group of compounds represented by formula (A) wherein $R^4$ is a hydrogen atom, and Y is an oxygen atom; and preferably X is a carbon atom; $R^1$ is a group represented by formula (A-1) (wherein $R^6$ is a pyrrolidino group, and $n^2$ is 0); $R^3$ is -CO-$R^7$ (wherein $R^7$ is an alkoxy group, an alkyl group, or an alkylamino group), or a 1,3-oxazole group; $R^5$ is a halogenated alkyl group; $n^1$ is 1; the double line between 2-position and 3-position, the double line between 4-position and 5-position, and the double line between 7-position and 8-position in the skeleton of the compound, which each consists of a solid line and a dotted line, represent a double bond; the double line between 5-position and 6-position and the double line between 8-position and 9-position, which each consist of a solid line and

a dotted line, represent a single bond; the two dotted lines between 6-position and 7-position represent no bond; and $R^3$ is bound to 2-position by a single bond.

Compound Aa1 can be represented by the following formula ($Aa_1$):

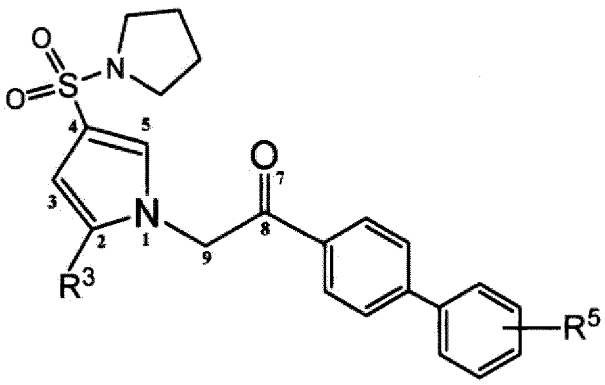

($Aa_1$)

wherein

$R^3$ is -CO-$R^7$ (wherein $R^7$ is an alkoxy group, an alkyl group, or an alkylamino group), or a 1,3-oxazole group; and $R^5$ is a halogenated alkyl group.

The alkyl group of the alkoxy group, alkyl group, or alkylamino group represented by $R^7$ is as described above. Preferable examples include lower alkyl groups having 1 to 3 carbon atoms; and more preferably methyl and ethyl; and even more preferably methyl. The number of alkyl groups in the alkylamino group can be, for example, one or two. Preferably, the alkylamino group is a dialkylamino group having two alkyl groups, and more preferably dimethylamino. The 1,3-oxazole group represented by $R^3$ is as mentioned above, and is preferably 1,3-oxazol-2-yl. $R^3$ in Compound Aa1 is preferably -CO-$R^7$ (wherein $R^7$ is an alkoxy group, and preferably methoxy) or a 1,3-oxazole group (preferably 1,3-oxazol-2-yl); and more preferably -CO-$R^7$ (wherein $R^7$ is an alkoxy group, and preferably methoxy).

The details of the halogenated alkyl group represented by $R^5$ are as described above. Preferably, the halogenated alkyl group is preferably an alkyl group having 2 to 3 halogen atoms, and more preferably a trihaloalkyl group. The halogen atom is preferably a fluorine atom. The alkyl group is preferably a lower alkyl group having 1 to 3 carbon atoms, preferably methyl or ethyl, and more preferably methyl. $R^5$ is preferably trifluoromethyl.

The pyrrole-type compound (Compound Aa1) having DOCK1-inhibiting activity, which is represented by the above formula ($Aa_1$), includes the following compounds and salts thereof:

(A1) methyl 1-(2-oxo-2-(3'-(trifluoromethyl)-[1,1'-biphenyl]-4-yl)ethyl)-4-(pyrrolidin-1-yl-sulfonyl)-1H-pyrrole-2-carboxylate (RT-22: Production Example 1A),
(A2) 2-(2-acetyl-4-(pyrrolidin-1-yl-sulfonyl)-1H-pyrrol-1-yl)-1-(3'-(trifluoromethyl)-[1,1'-biphenyl]-4-yl)ethane (HS-16: Production Example 4A),
(A3) 1-(1-(2-oxo-2-(3'-(trifluoromethyl)-[1,1'-biphenyl]-4-yl)ethyl)-4-(pyrrolidin-1-yl-sulfonyl)-1H-pyrrol-2-yl)propan-1-one (HS-17: Production Example 5A),
(A4) N,N-dimethyl-1-(2-oxo-2-(3'-(trifluoromethyl)-[1,1'-biphenyl]-4-yl)ethyl)-4-(pyrrolidin-1)-yl-sulfonyl)-1H-pyrrole-2-carboxamide (RT-23: Production Example 2A), and
(A5) 2-(2-(oxazol-2-yl)-4-(pyrrolidin-1-yl-sulfonyl)-1H-pyrrol-1-yl)-1-(3'-(trifluoromethyl)-[1,1'-biphenyl]-4-yl)ethan-1-one (RT-58: Production Example 3A).

Preferable examples of Compound Aa2 in which Y is a hydroxy group include the following compounds.

Compound Aa2

Compound Aa2 refers to a group of compounds represented by formula (A) wherein $R^4$ is a hydrogen atom and Y is a hydroxy group, and preferably X is a carbon atom; $R^1$ is a group represented by formula (A-1) (wherein $R^6$ is a pyrrolidino group and $n^2$ is 0); $R^3$ is -CO-$R^7$ (wherein $R^7$ is an alkoxy group) or an alkylhydroxy group; $R^5$ is a halogenated alkyl group; and $n^1$ is 1; the double line between 2-position and 3-position and the double line between 4-position and 5-position in the skeleton of the compound, which each consist of a solid line and a dotted line, represent a double bond; the double line between 5-position and 6-position, the double line between 8-position and 9-position,

and the double line between 7-position and 8-position, which each consist of a solid line and a dotted line, represent a single bond; the two dotted lines between 6-position and 7-position represent no bond; and $R^3$ is bound to 2-position by a single bond.

Compound Aa2 can be represented by the following formula ($Aa_2$) :

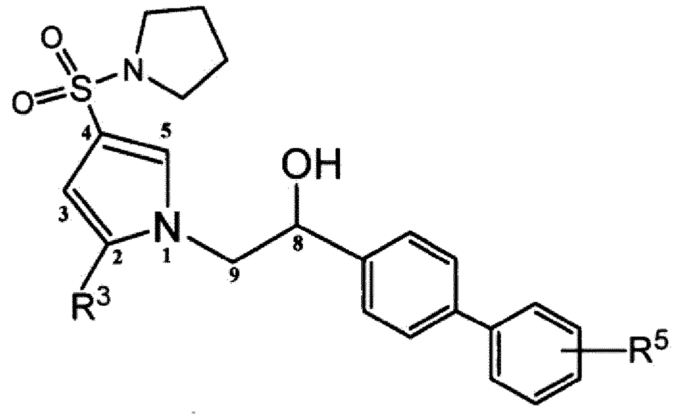

($Aa_2$)

wherein $R^3$ is -CO-$R^7$ (wherein $R^7$ is an alkoxy group), or an alkylhydroxy group; and $R^5$ is a halogenated alkyl group.

The alkyl group of the alkoxy group represented by $R^7$ is as described above. Preferable examples include lower alkyl groups having 1 to 3 carbon atoms; preferably methyl and ethyl; and more preferably methyl. The same applies to the alkyl group of the alkyhydroxy group represented by $R^3$. $R^3$ in Compound Aa2 is preferably -CO-$R^7$ (wherein $R^7$ is an alkoxy group, and is preferably methoxy).

The details of the halogenated alkyl group represented by $R^5$ are as described above. The halogenated alkyl group represented by $R^5$ is preferably an alkyl group having 2 to 3 halogen atoms, and is more preferably a trihaloalkyl group. The halogen atom is preferably a fluorine atom. The alkyl group is preferably a lower alkyl group having 1 to 3 carbon atoms, preferably methyl or ethyl, and is more preferably methyl. $R^5$ is preferably trifluoromethyl.

The pyrrole-type compound represented by the above formula ($Aa_2$) and having DOCK1-inhibiting activity represented (Compound Aa2) includes the following compounds and salts thereof:

(A6) methyl 1-(2-hydroxy-2-(3'-(trifluoromethyl)-[1,1'-biphenyl]-4-yl)ethyl)-4-(pyrrolidin-1-yl-sulfonyl)-1H-pyrrole-2-carboxylate (HS-6: Production Example 6A), and

(A7) 2-(2-(hydroxymethyl)-4-(pyrrolidin-1-yl-sulfonyl)-1H-pyrrol-1-yl)-1-(3'-(trifluoromethyl)-[1,1'-biphenyl]-4-yl)ethan-1-ol (HS-14: Production Example 7A).

(2-2) Compound Ab

As described above, Compound Ab includes "Compound Ab1" wherein $R^4$ is a hydrocarbon group in which one or more hydrogen atoms may be replaced by one or more substituents, and "Compound Ab2" wherein $R^4$ is an oxygen atom.

Compound Ab1

Preferable examples of Compound Ab1 wherein $R^4$ is a hydrocarbon group in which one or more hydrogen atoms may be replaced by one or more substituents include the following compounds.

The "hydrocarbon group in which one or more hydrogen atoms may be replaced by one or more substituents" includes -CH groups and atomic groups containing -CH groups whose one or more hydrogen atoms are replaced by any one or more functional groups. $R^4$ is preferably a hydrocarbon group in which one or more hydrogen atoms are replaced by any one or more substituents. Examples of substituents include acyloxy groups, halogen atoms, hydroxyl, cyano, $CF_3$, $CF_3O$, $CHF_2O$, $CF_3CH_2O$, and the like; preferably acyloxy groups; and more preferably acetoxy.

Examples of Compound Ab1 include compounds of formula (A) wherein $R^4$ is a hydrocarbon group; and Y is a hydrocarbon group, preferably -CH, and Y and $R^4$ that is a hydrocarbon group are bound to form a ring. Preferable examples include compounds wherein X is a carbon atom; $R^1$ is a hydrogen atom; $R^2$ is a group represented by formula (A-1) (wherein $R^6$ is a pyrrolidino group and $n^2$ is 0); $R^3$ is a hydrogen atom; $R^5$ is a halogenated alkyl group; $n^1$ is 1; the double line between 2-position and 3-position, the double line between 4-position and 5-position, the double line between 6-position and 7-position, and the double line between 8-position and 9-position in the skeleton of formula (A),

which each consist of a solid line and a dotted line, represent a double bond; the double line between 5-position and 6-position and the double line between 7-position and 8-position, which each consist of a solid line and a dotted line, represent a single bond; and $R^3$ is bound to 2-position by a single bond.

The details of the halogenated alkyl group represented by $R^5$ are as described above. Preferable examples of the alkyl group represented by $R^5$ are alkyl groups having 2 to 3 halogen atoms, and more preferably trihaloalkyl groups. The halogen atom is preferably a fluorine atom. The alkyl group is preferably a lower alkyl group having 1 to 3 carbon atoms, and is more preferably methyl. $R^5$ is preferably trifluoromethyl.

Examples of Compound Ab1 having DOCK1-inhibiting activity include the following compounds and salts thereof: (A8) 2-(pyrrolidin-1-yl-sulfonyl)-6-(3'-(trifluoromethyl)-[1,1'-biphenyl]-4-yl)indolizin-8-yl-acetate (HS-20: Production Example 8A) .

Compound Ab2

Examples of compound Ab2 wherein $R^4$ is an oxygen atom preferably include the following compounds.

Compound Ab2 includes compounds represented by formula (A) wherein $R^4$ is an oxygen atom, and preferably X is a nitrogen and; $R^1$ is a group represented by formula (A-1) (wherein $R^6$ is phenyl and $n^2$ is 1); $R^2$ is a hydrogen atom: $R^3$ is an oxygen atom; $R^5$ is a halogenated alkyl group; and $n^1$ is 1; and in the skeleton of the compound, the double line between position 5 and position 6 and the doule line between position 7 and position 8, which each consist of a solid line and a dotted line, represent a double bond; and the double line between 2-position and 3-position, the double line between 4-position and 5-position, and the double line between 8-position and 9-position, which each consist of a solid line and a dotted line, represent a single bond; the double line between 6-position and 7-position, which consists of a solid line and a dotted line, represents no bond; and $R^3$ is bound to 2-position by a double bond.

The details of the halogenated alkyl group represented by $R^5$ are as described above. Preferably, the halogenated alkyl group is an alkyl group having 2 to 3 halogen atoms, and is more preferably a trihaloalkyl group. The halogen atom is preferably a fluorine atom. The alkyl group is preferably a lower alkyl group having 1 to 3 carbon atoms, and more preferably methyl. $R^5$ is preferably trifluoromethyl.

Compound Ab2 having DOCK1-inhibiting activity includes the following compounds and salts thereof:
(A9) 3-(2-oxo-2-(3'-(trifluoromethyl)-[1,1'-biphenyl]-4-yl)ethyl)-5-((phenylsulfonyl)methyl)imidazolidine-2,4-dione (RT-35: Production Example 9A).

(3) Substituted Aza-alicyclic Compound Having DOCK1-inhibiting activity (Compound B)

The compound having DOCK1-inhibiting activity of the present invention includes compounds represented by the following formula (B) and salts thereof. These compounds are hereinafter collectively referred to as "Compound B" or "substituted aza-alicyclic compound."

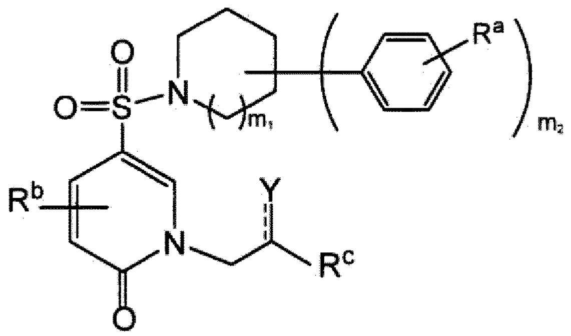

(B)

wherein $m_1$ is 0 or 1 and $m_2$ is 0 or 1.

More specifically, Compound B (substituted aza-alicyclic compound) includes pyrrolidine compounds that may have one or more substituents ($m_1$ = 0), and piperidine compounds that may have one or more substituents ($m_1$ = 1). Preferably, when $m_1$ is 1, $m_2$ is 1; and when $m_1$ is 0, $m_2$ is 0. More specifically, preferable examples of Compound B include piperidine compounds having a substituent ($m_1$ = 1 and $m_2$ = 1), and pyrrolidine compounds having no substituents ($m_1$ = 0 and $m_2$ = 0); and more preferably piperidine compounds having a substituent (substituted piperidine compounds).

In the above formula (B), $R^a$ is any of the groups represented by the following formulas (B-1) to (B-4):

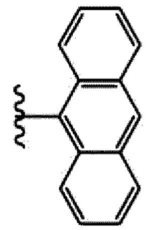

(B-1)

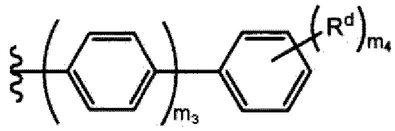

(B-2)

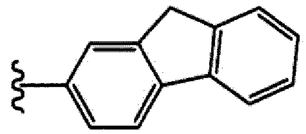

(B-3)

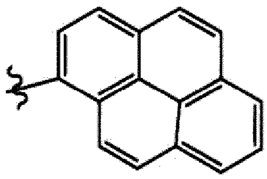

(B-4)

Preferable examples of $R^a$ include, but are not limited to, groups represented by formula (B-1) or (B-2).

In the above formula (B-2), $R^d$ is the same or different, and each represents a halogen atom, a halogenated alkyl group, or a halogenated alkylthio group. The halogen atom, halogenated alkyl group, and halogenated alkylthio group are as described above. The halogen atom is preferably a fluorine atom. The halogenated alkyl group is preferably a trihaloalkyl group. The halogenated alkylthio group is preferably a trihaloalkylthio group. The halogen atom of the halogenated alkyl group and the halogen atom of the halogenated alkylthio group are both preferably a fluorine atom (a fluoroalkyl group and a fluoroalkylthio group). Examples of alkyl groups include lower alkyl groups having 1 to 4 carbon atoms, preferably methyl and ethyl, and more preferably methyl (halogenated methyl). $R^d$ is preferably a halogen atom, and more preferably a fluorine atom.

In the above formula (B-2), $m_4$ is an integer of 0 to 5, and preferably 0 to 3. When the $R^d$ is a halogen atom, $m_4$ is preferably 1 to 3, and more preferably 3. In the above formula (B-2), $m_3$ is an integer of 0 to 2, and more preferably 0 or 2.

In the above formula (B), $R^c$ is a hydrogen atom or an alkyl group. The details of the alkyl group are as described above. Specific examples include lower alkyl groups having 1 to 6 carbon atoms, and preferably linear lower alkyl groups having 3 to 6 carbon atoms.

In the above formula (B), $R^d$ is a group represented by either the following formula (B-5) or (B-7):

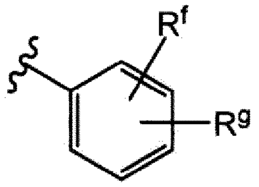

(B-5)

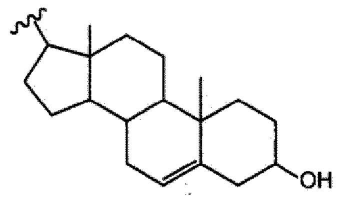

(B-7)

In the above formula (B-5), $R^f$ is a hydrogen atom, a hydroxy group, an alkyl group, an acyloxy group, or an alkoxy group, and $R^g$ is a quinolyl group or a group represented by the following formula (B-6):

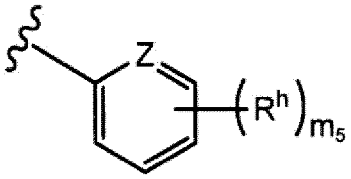

(B-6)

(wherein Z is a carbon atom or a nitrogen atom; $R^h$ is the same or different, and each represents a halogen atom, a halogenated alkyl group, a halogenated alkylthio group, an alkyl group, an alkenyl group, or an alkyl amino group; and $m_5$ is an integer of 1 to 3).

$R^d$ is preferably a group represented by formula (B-5). $R^f$ in formula (B-5) can be classified into a hydrogen atom and groups other than a hydrogen atom; and is preferably a hydrogen atom. In formula (B-5), $R^g$ is preferably a group represented by formula (B-6) wherein Z is a carbon atom. Although the positions at which $R^f$ and $R^g$ are located on the benzene ring are not limited, $R^f$ is preferably located at an ortho position; and $R^g$ is preferably located at a meta position or a para position, and is preferably at a para position.

The alkyl group represented by $R^f$ or $R^h$ in formulas (B-5) and (B-6) is as described above. Preferable examples include lower alkyls group having 1 to 3 carbon atoms; more preferably methyl or ethyl; and even more preferably methyl.

The acyloxy group represented by $R^f$ in formula (B-5) is as described above. Preferable examples include acyloxy groups in which the acyl group is formyl, acetyl, or propionyl; and more preferably an acetoxy group in which the acyl group is acetyl.

The alkoxy group represented by $R^f$ in formula (B-5) is as described above. Preferable examples include a hydroxyl group whose hydrogen atom is replaced by an alkyl group having 1 to 6 carbon atoms, or a hydroxyl group whose hydrogen atom is replaced by an aromatic hydrocarbon group. Preferable examples of the hydroxyl group whose hydrogen atom is replaced by an alkyl group having 1 to 6 carbon atoms include methoxy, ethoxy, 1-propoxy, 2-propoxy, 1-butoxy, and 2-butoxy. Examples of the hydroxyl group whose hydrogen atom is replaced by an aromatic hydrocarbon group include benzoxy and phenethoxy; and preferably phenethoxy. When the group represented by $R^f$ is an alkoxy group, the alkoxy group represented by $R^f$ may be bound to an alkoxy group represented by Y to form a ring.

Preferable examples of the quinolyl group represented by $R^g$ in formula (B-5) include, but are not limited to, quinolyl-6-yl and quinolyl-7-yl; and more preferably quinolyl-7-yl.

Examples of the halogen atom represented by $R^h$ in formula (B-6) include those mentioned above. Preferable examples include a fluorine atom, a chlorine atom, and a bromine atom; and more preferably a fluorine atom. When $R^h$ is a halogen atom, $m_5$ is 1 to 3, and preferably 3. When two or more halogen atoms are present, the halogen atoms may be the same or different. Examples of positions on the benzene ring at which $R^h$ is located include meta-positions and/or a para-position. The details of the halogenated alkyl group represented by $R^h$ are as described above. Preferable examples of the halogenated alkyl group include alkyl groups having 2 to 3 halogen atoms; and more preferably trihaloalkyl groups. The details of the halogenated alkylthio group represented by $R^h$ are as described above. Preferable examples of the halogenated alkylthio group include alkylthio groups having 2 to 3 halogen atoms; and more preferably trihaloalkylthio groups. The halogen atom is preferably a fluorine atom. Preferable examples of the alkyl group include lower alkyl groups having 1 to 3 carbon atoms; preferably methyl and ethyl; and more preferably methyl. When $R^g$ is a trihaloalkyl group or a trihaloalkylthio group, $m_5$ is 1 to 3, and is preferably 1. The position at which $R^h$ is located on the benzene ring can be, for example, a meta-position and/or a para-position; preferably a meta-position; and particularly 3'-position.

Examples of the alkenyl group represented by $R^h$ include those mentioned above. Preferable examples include linear alkenyl groups having two or three carbon atoms and one double bond. Examples of such alkenyl groups include vinyl, allyl, and propenyl; and preferably vinyl. Examples of the alkylamino group represented by $R^h$ include those mentioned above. Preferably, the alkylamino group is a dialkylamino group obtained by removing hydrogen from a secondary amine. Examples of alkyl groups include those mentioned above. Preferably, the alkyl groups are the same

or different kinds of lower alkyl groups having 1 to 4 carbon atoms; and more preferably may be the same or different and methyl or ethyl. The dialkylamino group is particularly preferably a dimethylamino group. When $R^h$ is an alkyl group, an alkenyl group, and/or an alkylamino group, $m_5$ is 1 or 2, and preferably 1. The position at which $R^h$ is located on the benzene ring can be, for example, a meta-position and/or a para-position; preferably a meta-position, and particularly 3'-position.

The groups represented by $R^h$ in formula (B-6) are the same or different and preferably represent a halogen atom, a halogenated alkyl group, or a halogenated alkylthio group; and more preferably a halogen atom or a halogenated alkyl group (preferably a trihaloalkyl group). When $R^h$ is a halogen atom, $m_5$ is 1 to 3, and is preferably 3. The position at which $R^h$ is located on the benzene ring can be, for example, meta-positions and/or a para-position; preferably meta-positions and a para-position, and particularly 3'-position to 5'-position. When $R^h$ is a halogenated alkyl group or a halogenated alkylthio group, $m_5$ is preferably 1. The position at which $R^h$ is located on the benzene ring can be, for example, a meta-position and/or a para-position; preferably a meta-position, and particularly 3'-position.

In formula (B-6), Z is preferably a carbon atom.

Compound B of the present invention can be classified into two groups according to whether $m_1$ is 1 or 0 in formula (B), as shown below. As stated above, in a group of compounds wherein $m_1$ is 1, $m_2$ is also preferably 1. This group of compounds can be referred to as a substituted piperidine compound (Compound Ba). In a group of compounds wherein $m_1$ is 0, $m_2$ is also preferably 0. This group of compounds can be referred to as a pyrrolidine compound (Compound Bb). Compound Bb can be further classified into the following four groups:

(1) a group of compounds in which $m_1$ is 1 and $m_2$ is 1 (Compound Ba) .
(2) a group of compound in which $m_1$ is 0 and $m_2$ is 0 (Compound Bb)

(Bb1) A group of compounds in which Y is other than an oxygen atom (Compound Bb1)
(Bb2) A group of compounds in which Y is an oxygen atom, $R^d$ is a substituent (B-5), and $R^f$ is other than a hydrogen atom (Compound Bb2).
(Bb3) A group of compounds in which Y is an oxygen atom, $R^d$ is a substituent (B-5), and $R^f$ is a hydrogen atom (Compound Bb3).
(Bb4) A group of compounds in which Y is an oxygen atom and $R^d$ is a substituent (B-7) (Compound Bb4).

These groups of compounds are described in more detail below.

(3-1) Compound Ba

Compound Ba is a preferable compound among the substituted aza-alicyclic compounds of the present invention (Compound B). The compound is preferably represented by the following formula (Ba).

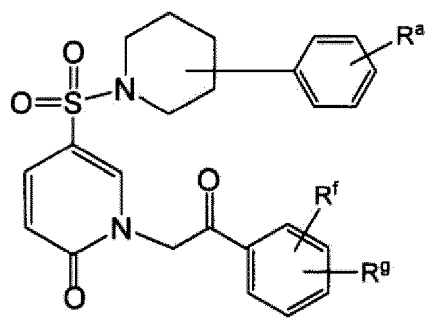

(B a)

In formula (Ba), $R^a$ is any one of the groups represented by the following formulas (B-1) to (B-4):

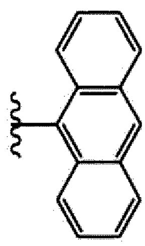

(B − 1) ,

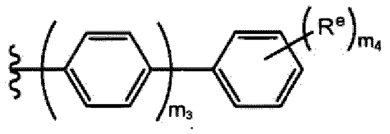

(B－2) ,

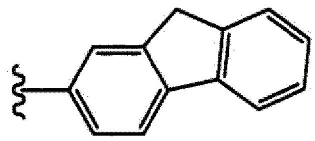

(B－3) ,

and

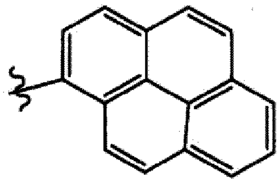

(B－4) .

Preferable examples of $R^a$ include, but are not limited to, groups represented by formula (B-1) or (B-2). $R^e$, $m_3$, and $m_4$ in formula (B-2) are as explained above.

In the above formula (Ba), $R^e$ is a hydrogen atom and $R^g$ is a group represented by the following formula (B-6):

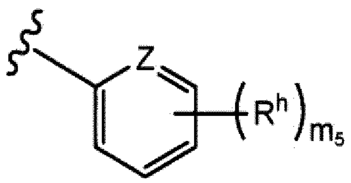

(B－6)

wherein Z is a carbon atom; $R^h$ is the same or different, and each represents a halogen atom, a halogenated alkyl group, or a halogenated alkylthio group; $m_5$ is an integer of 1 to 3. The halogen atom, halogenated alkyl group, and halogenated alkylthio group are as explained above. When $R^h$ is a halogen atom, $m^5$ is 1 to 3, and preferably 3. The position at which $R^h$ is located on the benzene ring can be, for example, meta-positions and/or a para-position; preferably a meta-position; and particularly 3'-position to 5'-position. When $R^h$ is a halogenated alkyl group or a halogenated alkylthio group, $m_5$ is preferably 1. The position at which $R^h$ is located on the benzene ring can be, for example, a meta-position and/or a para-position; preferably a meta-position; and particularly 3'-position. $R^h$ is preferably a fluorine atom or a trihalomethyl group (preferably trifluoromethyl).

The substituted aza-alicyclic compound represented by the above formula (B), particularly by formula (Ba), and having DOCK1-inhibiting activity (Compound Ba) includes the following compounds and salts thereof:

(B1) 5-((4-(4-(anthracen-9-yl)phenyl)piperidin-1-yl)sulfonyl)-1-(2-oxo-2-(3'-(trifluoromethyl)-[1,1'-biphenyl]-4-yl)ethyl)pyridin-2(1H)-one (KS-47: Production Example 2B);

(B2) 1-(2-oxo-2-(3'-(trifluoromethyl)-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one (KS-54: Production Example 3B);

(B3) 5-((4-([1,1': 4',1": 4",1'"-quaterphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one (KS-44: Production Example 5B);

(B4) 5-((4-([1,1'-biphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one (KS-43: Production Example 7B);

(B5) 1-(2-oxo-2-(3',4',5'-trifluoro-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one (KS-59: Production Example 4B);

(B6) 5-((4-(4-(9H-fluorene-2-yl)phenyl)piperidin-1-yl)sulfonyl)-1-(2-oxo-2-(3'-(trifluoromethyl)-[1,1'-biphenyl]-4-yl)ethyl)pyridin-2(1H)-one (KS-46: Production Example 1B); and

(B7) 1-(2-oxo-2-(3'-(trifluoromethyl)-[1,1'-biphenyl]-4-yl)ethyl)-5-((4-(4-(pyren-1-yl)phenyl)piperidin-1-yl)sulfonyl)pyridin-2(1H)-one (KS-45: Production Example 6B).

(3-1) Compound Bb

Preferable examples of Compound Bb include Compound Bb represented by the following formula (Bb):

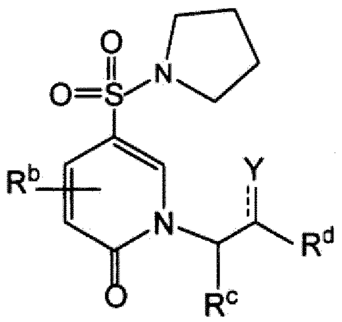

(B b)

wherein $R^b$ is a hydrogen atom or an amino group;
$R^c$ is a hydrogen atom or an alkyl group;
$R^d$ is a group represented by the following formula (B-5):

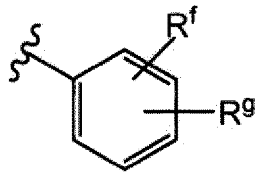

(B − 5)

(wherein $R^f$ is a hydrogen atom, a hydroxy group, an alkyl group, an acyloxy group, or an alkoxy group (wherein the alkoxy group may be linked to Y to form a ring) ; $R^g$ is a group represented by the following (B-6), or a quinolyl group:

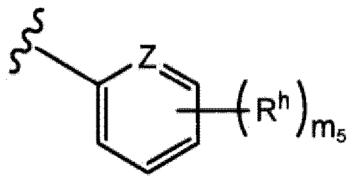

(B − 6)

(wherein Z is a carbon atom or a nitrogen atom; $R^h$ is the same or different, and each represents a halogen atom, a halogenated alkyl group, a halogenated alkylthio group, an alkyl group, an alkenyl group, or an alkylamino group; and $m_5$ is an integer of 1 to 3)), or
a group represented by the following formula (B-7):

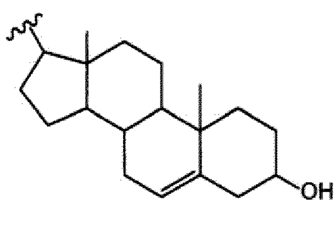

(B − 7)

;

Y is an oxygen atom, a hydroxy group, or an alkoxy group (wherein when $R^d$ is a group represented by formula (B-5), the alkoxy group may be linked to an alkoxy group represented by $R^f$ to form a ring).

As described above, Compound Bb can be classified according to whether Y is an oxygen atom. Preferable examples of Compound Bb1 in which Y is other than an oxygen atom include the following compounds.

Compound Bb1

In formula (Bb), Y is a hydroxy group or an alkoxy group; $R^b$ is a hydrogen atom; $R^c$ is a hydrogen atom; $R^d$ is a group represented by formula (B-5) (wherein $R^f$ is a hydroxy group or an alkoxy group); $R^g$ is a group represented by formula (B-6) (wherein Z is a carbon atom; $R^h$ is the same or different, and each represents a halogen atom, a halogenated alkyl group, or a halogenated alkylthio group; $m_5$ is an integer of 1 to 3). The double line consisting of a solid line and a dotted line in formula (Bb) represents a single bond. When Y is an alkoxy group, $R^f$ in formula (B-5) is also an alkoxy group, and these two alkoxy groups can be bound to each other to form a ring.
The compound can be represented by the following formula ($Bb_1$) :

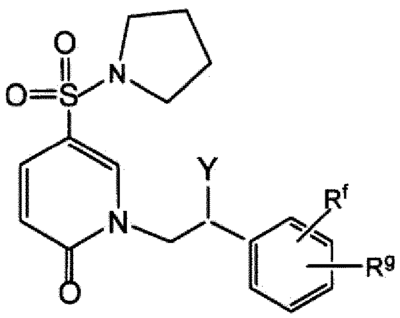

($Bb_1$)

wherein Y is a hydroxy group or an alkoxy group; $R^f$ is a hydroxy group or an alkoxy group (wherein the alkoxy group may be linked to an alkoxy group represented by Y to form a ring); and $R^g$ is a group represented by the following formula (B-6):

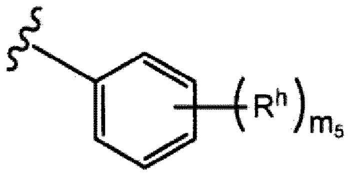

(B-6)

(wherein $R^h$ is the same or different, and each represents a halogen atom, a halogenated alkyl group, or a halogenated alkylthio group; and $m_5$ is an integer of 1 to 3).
In the formula, the alkoxy group, halogen atom, halogenated alkyl group, and halogenated alkylthio group are as described above.
The substituted aza-alicyclic compound represented by the above formula ($Bb_1$) and having DOCK1-inhibiting activity (Compound Bb1) includes the following compounds and salts thereof:

(B8) 1-(2-hydroxy-2-(3-hydroxy-3'-(trifluoromethyl)-[1,1'-biphenyl]-4-yl)ethyl)-5-(pyrrolidin-l-yl-sulfonyl)pyridin-2(1H)-one (AK-16: Production Example 18B); and
(B9) 1-((2,2-dimethyl-7-(3-(trifluoromethyl)phenyl)-4H-benzo[d][1,3]dioxin-4-yl)methyl)-5-(pyrrolidin)-1-yl-sulfonyl)pyridin-2(1H)-one (AK-17: Production Example 19B).

Among Compound Bb, preferable examples of Compound Bb2 in which Y is an oxygen atom, $R^d$ is a substituent (B-5), and $R^f$ is other than a hydrogen atom include the following compounds.

Compound Bb2

In formula (Bb), Y is an oxygen atom; $R^d$ is a group represented by formula (B-5) (wherein $R^f$ is a hydroxy group, an alkyl group, an acyloxy group, or an alkoxy group); and preferably $R^b$ is a hydrogen atom; $R^c$ is a hydrogen atom or an alkyl group; $R^g$ is a group represented by formula (B-6) (wherein Z is a carbon atom; $R^h$ is the same or different, and each represents a halogen atom, a halogenated alkyl group, or a halogenated alkylthio group; and $m_5$ is an integer of 1 to 3). The double line consisting of a solid line and a dotted line in formula (Bb) represents a double bond.
Compound Bb2 can be represented by the following formula ($Bb_2$) :

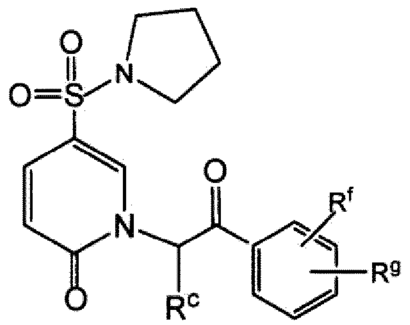

(Bb$_2$)

wherein

$R^c$ is a hydrogen atom or an alkyl group;
$R^f$ is a hydroxy group, an alkyl group, an acyloxy group, or an alkoxy group;
$R^g$ is a group represented by the following formula (B-6):

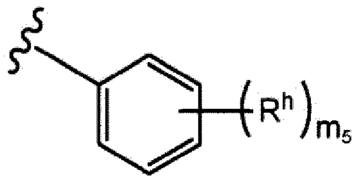

(B-6)

(wherein $R^h$ is the same or different, and each represents a halogen atom, a halogenated alkyl group, a halogenated alkylthio group, an alkyl group, an alkenyl group, or an alkylamino group; and $m^5$ is an integer of 1 to 3).

In the above formula, the alkyl group, the acyloxy group, the alkoxy group, the halogen atom, the halogenated alkyl group, and the halogenated alkylthio group are as described above.

The substituted aza-alicyclic compound represented by the above formula (Bb$_2$) and having DOCK1-inhibiting activity (Compound Bb2) includes the following compounds and salts thereof:

(B10) 2-Bromo-1-(3-methyl-3'-(trifluoromethyl)-[1,1'-biphenyl]-4-yl)ethan-1-one (AK-5: Production Example 15B);
(B11) 1-(2-(3-Hydroxy-3'-(trifluoromethyl)-[1,1'-biphenyl]-4-yl)-2-oxoethyl)-5-(pyrrolidin-1-yl-sulfonyl)pyridin-2(1H)-one (AK-15: Production Example 17B);
(B12) 1-(2-(3-Methoxy-3'-(trifluoromethyl)-[1,1'-biphenyl]-4-yl)-2-oxoethyl)-5-(pyrrolidin-1-yl-sulfonyl)pyridin-2(1H)-one (AK-14: Production Example 16B);
(B13) 4-(2-(2-oxo-5-(pyrrolidin-1-yl-sulfonyl)pyridin-1(2H)-yl)acetyl)-3'-(trifluoromethyl)-[1,1'-biphenyl]-3-yl)acetate (AK-25: Production Example 23B);
(B14) 1-(2-(3-isobutoxy-3'-(trifluoromethyl)-[1,1'-biphenyl]-4-yl)-2-oxoethyl)-5-(pyrrolidin-1-yl-sulfonyl)pyridin-2(1H)-one (AK-22: Production Example 20B);
(B15) 1-(1-(3-(hexyloxy)-3'-(trifluoromethyl)-[1,1'-biphenyl]-4-yl)-1-oxooctan-2-yl)-5-(pyrrolidin-1-yl-sulfonyl)pyridin-2(1H)-one (AK-23: Production Example 21B); and
(B16) 1-(2-oxo-2-(3-phenethoxy-3'-(trifluoromethyl)-[1,1'-biphenyl]-4-yl)ethyl)-5-(pyrrolidin-1-yl-sulfonyl)pyridin-2(1H)-one (AK-24: Production Example 22B).

Among Compound Bb, preferable examples of Compound Bb3 in which Y is an oxygen atom, $R^d$ is a substituent (B-5), and $R^f$ is a hydrogen atom include the following compounds.

Compound Bb3

In the above formula (Bb), Y is an oxygen atom; $R^d$ is a group represented by formula (B-5) (wherein $R^f$ is a hydrogen atom), preferably $R^b$ is a hydrogen atom or an amino group; $R^c$ is a hydrogen atom; and $R^g$ is a group represented by formula (B-6) (wherein Z is a carbon atom or a nitrogen atom; $R^h$ is the same or different, and each represents a halogen atom, a halogenated alkyl group, or a halogenated alkylthio group; $m^5$ is an integer of 1 to 3) or a quinolyl group. The double line consisting of a solid line and a dotted line in formula (Bb) represents a double bond.

Compound Bb3 can be represented by the following formula ($Bb_3$) :

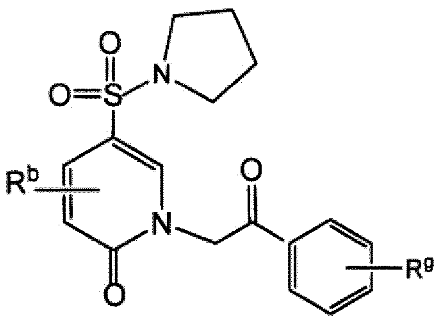

(B b 3)

wherein $R^b$ is a hydrogen atom or an amino group;
$R^g$ is a group represented by the following formula (B-6):

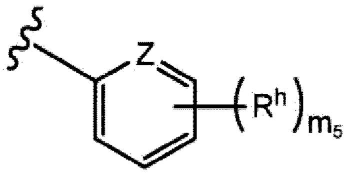

(B − 6)

(wherein Z is a carbon atom or a nitrogen atom; $R^h$ is the same or different, and each represents a halogen atom, a halogenated alkyl group, a halogenated alkylthio group; and $m_5$ is an integer of 1 to 3) or a quinolyl group.

The halogen atom, halogenated alkyl group, and halogenated alkylthio group are as described above. A preferable example of the quinolyl group in formula ($Bb_3$) is such that a quinolyl group whose 7-position or 6-position is bound to a benzene nucleus (quinolin-7-yl and quinolin-6-yl).

The substituted aza-alicyclic compound represented by the above formula ($Bb_3$) and having DOCK1-inhibiting activity (Compound Bb3) includes the following compounds and salts thereof:

(B17) 1-(2-oxo-2-(3'-((trifluoromethyl)thio)-[1,1'-biphenyl]-4-yl)ethyl)-5-(pyrrolidin-1-yl-sulfonyl)pyridin-2(1H)-one (KS-21: Production Example 8B);
(B18) 3-Amino-1-(2-oxo-2-(3'-(trifluoromethyl)-[1,1'-biphenyl]-4-yl)ethyl)-5-(pyrrolidin-1-yl-sulfonyl)pyridin-2(1H)-one (RT-13: Production Example 14B);
(B19) 1-(2-oxo-2-(3'-vinyl-[1,1'-biphenyl]-4-yl)ethyl)-5-(pyrrolidin-1-yl-sulfonyl)pyridin-2(1H)-one (KS-26: Production Example 13B);
(B20) 1-(2-(4'-(dimethylamino)-3'-methyl-[1,1'-biphenyl]-4-yl)-2-oxoethyl)-5-(pyrrolidin-1-yl-sulfonyl)pyridin-2(1H)-one (KS-16: Production Example 9B);
(B21) 1-(2-oxo-2-(4-(6-(trifluoromethyl)pyridin-2-yl)phenyl)ethyl)-5-(pyrrolidin-1-yl-sulfonyl)pyridin-2(1H)-one (KS-22: Production Example 11B);
(B22) 1-(2-oxo-2-(4-(quinolin-7-yl)phenyl)ethyl)-5-(pyrrolidin-1-yl-sulfonyl)pyridin-2(1H)-one (KS-18): Production Example 10B); and
(B23) 1-(2-oxo-2-(4-(quinolin-6-yl)phenyl)ethyl)-5-(pyrrolidin-1-yl-sulfonyl)pyridin-2(1H)-one (KS-23): Production Example 12B).

Among Compound Bb, preferable examples of Compound Bb4 wherein Y is an oxygen atom and $R^d$ is a substituent (B-7) include the following compounds.

Compound Bb4

In formula (Bb), Y is an oxygen atom; $R^d$ is a group represented by formula (B-7), and preferably $R^b$ is a hydrogen atom or an amino group; and $R^c$ is a hydrogen atom. The double line consisting of a solid line and a dotted line in formula (Bb) represents a double bond.

Compound Bb4 can be represented by the following formula ($Bb_4$).

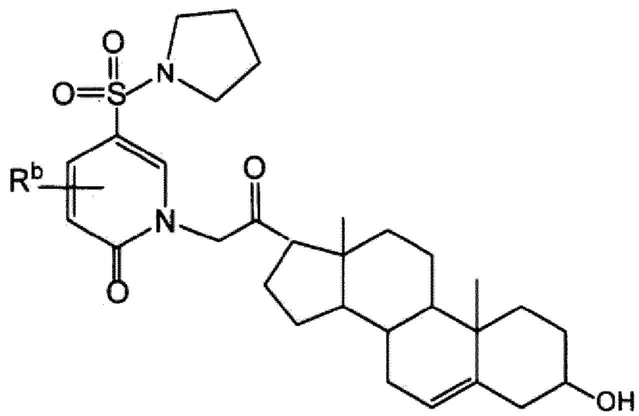

(Bb4)

wherein $R^b$ is a hydrogen atom or an amino group. Preferably, $R^b$ is a hydrogen atom.

The substituted aza-alicyclic compound (Compound $Bb_4$) having DOCK1-inhibiting activity and represented by the above formula ($Bb_4$) includes the following compound and salts thereof: (B24) 1-(2-((3S,10R,13S,17S)-3-hydroxy-10,13-dimethyl-2, 3, 4,7, 8, 9,10,11,12,13,14,15,16,17-tetradecahydro-1H-cyclopenta[a]phenanthren-17-yl)-2-oxoethyl)-5-(pyrrolidin-1-yl-sulfonyl)pyridin-2(1H)-one (AK-20: Production Example 24B).

In the present invention, the salt of Compound A or the salt of Compound B described above can be any pharmacologically acceptable salt, and is not particularly limited as long as it is a pharmaceutically acceptable salt. For example, such salts include the following forms of salts. Among the above compounds, for example, compounds having an acidic group can easily form salts with basic compounds. Examples of such basic compounds include metal hydroxides such as sodium hydroxide, potassium hydroxide, calcium hydroxide, and lithium hydroxide; and alkali metal carbonates such as sodium hydrogen carbonate, and sodium carbonate; and alkali metal alcoholates such as sodium methylate, and potassium ethylate. Among the compounds described above, compounds having a basic group can easily form salts with acids. Examples of such acids include inorganic acids such as nitric acid, hydrochloric acid, hydrobromic acid, and sulfuric acid; and organic acids such as acetic acid, methanesulfonic acid, oxalic acid, maleic acid, fumaric acid, p-toluenesulfonic acid, succinic acid, benzoic acid, and citric acid. These salts can be used in the same manner as the compounds in free form.

The compound of the present invention includes isomers such as stereoisomers and optical isomers. These isomers are also included within the scope of Compound A or Compound B of the present invention.

## (4) Production Method

The methods for producing the pyrrole-type compound represented by the above chemical formula (A) (Compound A), the substituted aza-alicyclic compound represented by chemical formula (B) (Compound B), and salts of these compounds are not particularly limited. The method for producing the pyrrole-type compound can be performed with reference to Production Examples 1A to 7A in the Production Examples described later, and Reference Examples related thereto. The method for producing the substituted aza-alicyclic compound can also performed with reference to Production Examples 1B to 25B in the Production Examples described later, and Reference Examples related thereto. However, these production methods are only examples, and the pyrrole-type compound and the substituted aza-alicyclic compound can also be produced by referring to these production methods and modifying the methods as appropriate, based on common technical knowledge of persons skilled in the art.

The compound of the present invention produced by the above method can be isolated and purified from the reaction mixture by applying known isolation and/or purification means. Examples of such separation and purification means include distillation, recrystallization, solvent extraction, column chromatography, ion exchange chromatography, gel chromatography, affinity chromatography, preparative thin-layer chromatography, and like methods.

## (5) DOCK1 Selective Inhibitor

The DOCK1 selective inhibitor according to the present invention is characterized by containing a pyrrole-type compound (Compound A), a substituted aza-alicyclic compound (Compound B), or a salt of any of these compounds as an active ingredient.

Inhibiting DOCK1 means inhibiting the activity of DOCK1. This activity is not particularly limited, and examples of such activity include an activity of inhibiting the activity of converting Rac-GDP to Rac-GTP (GEF activity). The GEF activity of the compound or DOCK1 selective inhibitor can be confirmed by the method shown in Pharmacological Test

Example 1 below or using a known method similar thereto.

The selective inhibition referred to herein means that the activity of DOCK1 is inhibited more strongly than the activity of DOCK2, which constitutes a DOCK-A subfamily. The selective inhibition is not limited to inhibit only DOCK1. In this sense, the DOCK1 selective inhibitor may have the action of inhibiting the activity of DOCK5 and/or DOCK2, which constitute DOCK-A subfamilies.

The DOCK1 selective inhibitor according to the present invention may consist of the above pyrrole-type compound (Compound A), substituted aza-alicyclic compound (Compound B), or a salt of any one of these compounds, according to the present invention. However, the DOCK1 selective inhibitor may further contain components such as carriers, excipients (bulking agents), or additives commonly used in the art, as long as the DOCK1 selective inhibitory activity is not impaired. The proportion of the compound of the present invention contained in the DOCK1 selective inhibitor can be appropriately selected and adjusted within the range of 1 to 100% by mass so that the DOCK1 selective inhibitor can have DOCK1 selective inhibitory activity. For example, the proportion of the compound of the present invention can be appropriately selected with reference to the additives and content ratios described in the Pharmaceutical Composition described below.

(6) Pharmaceutical Composition

The pharmaceutical composition according to the present invention contains the pyrrole-type compound (Compound A), the substituted aza-alicyclic compound (Compound B), or a pharmaceutically acceptable salt of any one of these compounds. The substituted aza-alicyclic compound (compound B) is preferably Compound Ba represented by formula (Ba). The pharmaceutically acceptable salt can be appropriately selected with reference to those described in detail above.

The pharmaceutical composition according to the present invention may consist of only the compound of the present invention or a pharmaceutically acceptable salt thereof described above. It may also be a pharmaceutical composition prepared by combining the compound of the present invention or a pharmaceutically acceptable salt thereof with any carrier or additive; and formulating the mixture, by a known method, into a form suitable for the desired application, in terms of, for example, the route of administration or the method of administration.

Specific examples of dosage forms include tablets, pills, powders, liquids, suspensions, emulsions, granules, capsules, suppositories, injections (e.g., liquids, suspensions, etc.), and the like.

The amount of the compound of the present invention or a pharmaceutically acceptable salt thereof in the pharmaceutical composition of the present invention is not particularly limited. For example, the amount can be within the range of about 0.001 to 99 mass%, preferably about 0.01 to 50 mass%, and more preferably about 0.05 to 10 mass%, per 100 mass% of the pharmaceutical composition.

The disease to be treated by the pharmaceutical composition according to the present invention is not particularly limited. The target disease is, for example, a cancer. Examples of types of cancer to be treated include, but are not limited to, lung cancer, breast cancer, pancreatic cancer, colon cancer, gastric cancer, ovarian cancer, glioma, glioblastoma, melanoma, esophageal cancer, and the like. The Examples described below show that since the compound of the present invention provides the effect of inhibiting cancer cell invasion, the target disease is, among cancers, preferably a metastatic cancer.

The Examples described below further confirm the effect of the compound of the present invention to suppress macropinocytosis caused by cancer cells. Accordingly, the pharmaceutical composition according to the present invention is preferably used for a type of cancer, among cancers, in which a macropinocytosis phenomenon is observed.

The subject to whom the pharmaceutical composition according to the present invention is administered may be a patient suffering from the above-described diseases, or a human who may have a potential to be affected by the diseases.

The dosage of the pharmaceutical composition of the present invention is usually about 5 mg to 500 mg, preferably about 5 mg to 250 mg, more preferably about 5 mg to 100 mg, and even more preferably about 5 mg to 50 mg, per day in terms of the compound of the present invention or a pharmaceutically acceptable salt thereof.

Further, the pharmaceutical composition according to the present invention may contain the DOCK1-selective inhibitor described above. In such a pharmaceutical composition, the content of the DOCK1-selective inhibitor, the target disease, the dosage form, the subject of administration, the dosage, etc., can be as described above.

Examples

The present invention is further clarified below with reference to Production Examples and Pharmacological Test Examples regarding the compounds of the present invention. However, the present invention is not limited to these. The following Production Examples and Pharmacological Tests were performed under conditions of room temperature (25±5°C) and atmospheric pressure, unless otherwise noted.

Production Examples

(I) General Methods

1D NMR spectra were recorded on JEOL JNM-LA 500, JEOL ECX500 (500 MHz for $^1$H NMR), and JEOL ECS400 (400 MHz for $^1$H NMR) spectrometers. Chemical shifts were recorded in parts per million (ppm) relative to the residual solvent peaks at $\delta_H$ 7.26 in $CDCl_3$, $\delta_H$ 2.50 in DMSO-$d_6$, and $\delta_H$ 2.05 in acetone-$d_6$, and coupling constants (J) are shown in hertz (Hz). The following abbreviations are used for spin multiplicity: s = singlet, d = doublet, t = triplet, q = quartet, m = multiplet, and br = broad.

Electrospray ionization (ESI) mass spectra were measured on a Shimadzu LCMS-2020 spectrometer (for LRMS) and a JEOL JMS-T100LC AccuTOF spectrometer (for HRMS). Column chromatographies were performed with silica gel 60 (spherical) 40-50 μm (Kanto Chemicals) or by Biotage® Isolera (registered trademark) One 3.0 with pre-packed column of Biotage® SNAP Ultra.

Chemicals and solvents were obtained from commercial sources. All moisture-sensitive reactions were performed under an argon atmosphere. All new compounds were determined to be >95% purity by $^1$H NMR, unless otherwise noted.

(II) Production Methods for Substituted Aza-alicyclic Compounds (Compounds Ba: KS-43 to KS-47, KS-54, and KS-59; Compounds Ba: KS-21 to KS-23, KS-16, KS-18, and KS-26)

Reference Example 1

Production of tert-butyl 4-(4-bromophenyl)piperidine-1-carboxylate

The titled compound was synthesized by following the reported procedure of boronic acid-sulfonyl hydrazone coupling (Allwood, D.M.; Blakemore, D.C.; Brown, A.D.; Ley, S.V.; J. Org. Chem. 2014, 79, 328).

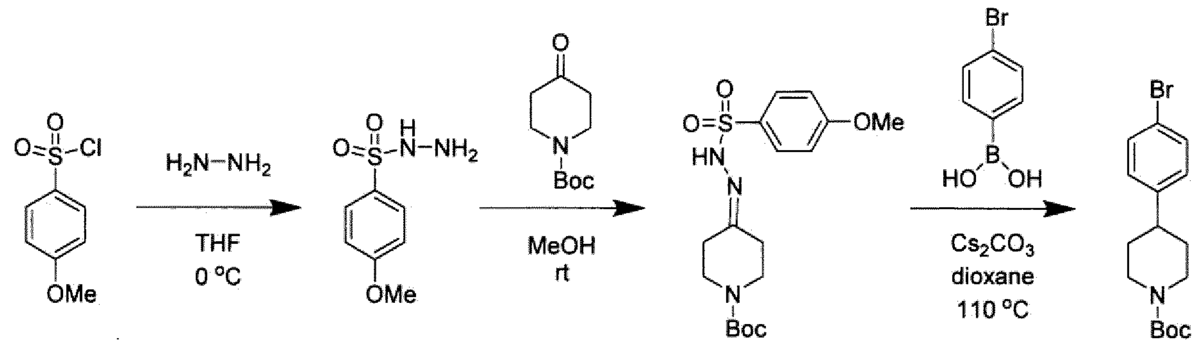


Step 1: To a solution of 4-methoxybenzenesulfonyl chloride (413.3 mg, 2.0 mmol, 1.0 equiv) in THF (10 mL, 0.2 M) at 0°C was added hydrazine hydrate (243 μL, 5.0 mmol, 2.5 equiv) dropwise. The reaction mixture was stirred at 0°C until complete conversion was observed by TLC. The mixture was diluted with EtOAc, washed with brine, and dried over $Na_2SO_4$. After filtration, all the volatiles were removed *in vacuo* to give 4-methoxybenzenesulfonohydrazide as an off-white amorphous solid (378 mg, 1.87 mmol, 93.4% yield). Spectral data were identical to those reported in the literature (Allwood, D.M.; Blakemore, D.C.; Brown, A.D.; Ley, S.V.; J. Org. Chem., 2014, 79, 328).
$^1$H NMR (400 MHz, $CDCl_3$) δ 7.84 (2H, d, J = 8.8 Hz), 7.02 (2H, d, J = 8.8 Hz), 5.69 (1H, br s), 3.88 (3H, s), 3.59 (2H, br s); HRMS (ESI+) calculated for $C_7H_{11}N_2O_3S$ $[M + H]^+$ 203.0490, found 203.0481.

Step 2: To a solution of 4-methoxybenzenesulfonohydrazide (378.0 mg, 1.87 mmol, 1.0 equiv) in MeOH (3.74 mL, 0.5 M) was added 1-Boc-4-piperidone (372.6 mg, 1.87 mmol, 1.0 equiv). The reaction mixture was stirred at room temperature until complete conversion was observed by TLC. Solvents were removed *in vacuo* to give *tert*-butyl 4-(2-((4-methoxy-phenyl)sulfonyl)hydrazono)piperidine-1-carboxylate (914.4 mg, 100% yield). This sample was used for the next step, without further purification.

Step 3: tert-Butyl 4-(2-((4-methoxyphenyl)sulfonyl)hydrazono)piperidine-1-carboxylate (709.4 mg, 1.85 mmol, 1.0 equiv), p-bromophenylboronic acid (557.3 mg, 2.78 mmol, 1.5 equiv), and cesium carbonate (904.2 mg, 2.78 mmol, 1.5 equiv) were placed in an oven-dried tube *in vacuo* for 30 min. The tube was backfilled with argon, and dry, degassed 1,4-dioxane (7.5 mL, 0.25 M) was added. The tube was sealed and heated at 110°C for 18 h. Thereafter, the resulting product was cooled to room temperature, quenched with saturated aqueous sodium bicarbonate solution (2 mL), and extracted with $CH_2Cl_2$ (5 mL × 3). The combined organic layer was dried over $Na_2SO_4$. After filtration, all of the volatiles

were removed *in vacuo.* The obtained crude mixture was purified by column chromatography (EtOAc/hexane = 10-33%) to give tert-butyl 4-(4-bromophenyl)piperidine-1-carboxylate as a colorless oil (209 mg, 0.615 mmol, 33.3% yield). Spectral data were identical to those reported in the literature (Allwood, D.M.; Blakemore, D.C.; Brown, A.D.; Ley, S.V.; J. Org. Chem., 2014, 79, 328).
$^1$H NMR (400 MHz, $CDCl_3$) δ 7.41 (2H, d, J = 8.5 Hz), 7.06 (2H, d, J = 8.5 Hz), 4.23 (2H, br), 2.78 (2H, br t, J = 12.3 Hz), 2.59 (1H, tt, J = 12.2, 3.5 Hz), 1.78 (2H, br d, J = 13.1 Hz), 1.58 (2H, td, J = 12.7, 4.3 Hz), 1.47 (9H, s); HRMS (ESI+) calculated for $C_{16}H_{22}NO_2Br$ $[M + Na]^+$ 362.0726, found 362.0717.

Reference Example 2

General procedure for Suzuki-Miyaura reaction 1 (exemplified by the synthesis of 4-(4-(9H-fluoren-2-yl)phenyl)piperidine-1-carboxylate)

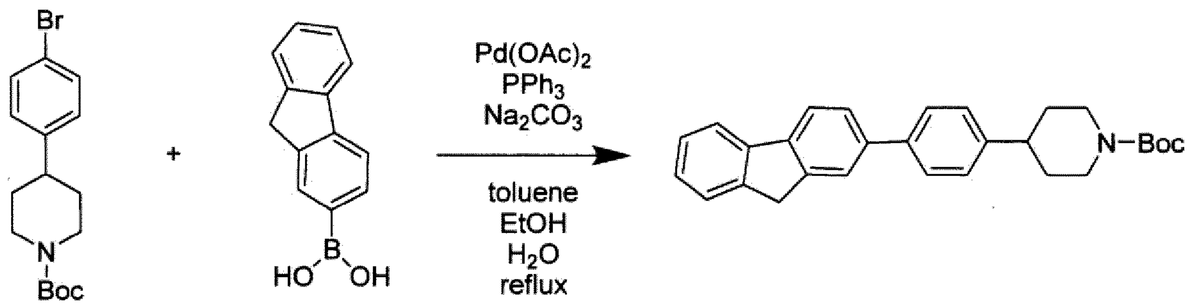


A mixture of the aryl bromide compound (tert-butyl 4-(4-bromophenyl)piperidine-1-carboxylate obtained in Reference Example 1) (119.5 mg, 0.35 mmol, 1.0 equiv), palladium(II) acetate (8.0 mg, 0.03 mmol, 10 mol%), triphenylphosphine (28.3 mg, 0.1 mmol, 30 mol%), sodium carbonate (152.6 mg, 1.44 mmol, 4.0 equiv), and a boronic acid compound (151.0 mg, 0.7 mmol, 2.0 equiv) in a liquid mixture of toluene (8.0 mL), EtOH (1.0 mL), and degassed water (2.2 mL) was heated at reflux overnight. The reaction mixture was diluted with brine, and extracted with toluene. The combined organic layer was dried over $Na_2SO_4$, filtered, and concentrated *in vacuo.* The obtained residue was purified by column chromatography ($EtOAc/CH_2Cl_2$ = 30%) to give the titled 4-(4-(9H-fluoren-2-yl)phenyl)piperidine-1-carboxylate as a brown solid (55.0 mg, 0.12 mmol, yield 36.8%).
$^1$H NMR (400 MHz, $CDCl_3$) δ 7.79-7.86 (2H, m), 7.76 (1H, s), 7.59-7.61 (2H, m), 7.55 (1H, m), 7.37-7.41 (1H, m), 7.28-7.33 (3H, m), 4.27 (2H, br), 3.96 (2H, s), 2.80-2.83 (2H, m), 2.67-2.73 (1H, m), 1.86 (2H, br d, J = 12.6 Hz), 1.63-1.71 (2H, m), 1.50 (9H, s) .

Reference Example 3

Production of tert-butyl 4-(4-(anthracen-9-yl)phenyl)piperidine-1-carboxylate

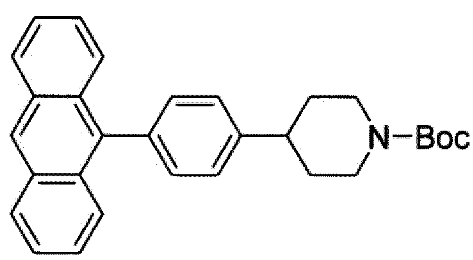

According to the general procedure for the Suzuki-Miyaura reaction 1 described in Reference Example 2 and using 9-anthracenboronic acid (178.0 mg, 0.8 mmol, 2.0 equiv) as the boronic acid compound instead, the titled tert-butyl 4-(4-(anthracen-9-yl)phenyl)piperidine-1-carboxylate was obtained as a brown solid (52.1 mg, 0.119 mmol, 29.8% yield).
$^1$H NMR (400 MHz, $CDCl_3$) δ 8.48 (1H, s), 8.02 (2H, d, J = 8.6 Hz), 7.66 (2H, d, J = 9.0 Hz), 7.45 (2H, m), 7.34-7.38

(6H, m), 4.31 (2H, m), 2.77-2.88 (3H, m), 1.97 (2H, br d, J = 12.9 Hz), 1.71-1.81 (2H, m), 1.51 (9H, s).

Reference Example 4

Production of *tert*-butyl 4-(3',4',5'-trifluoro-[1,1'-biphenyl]-4-yl)piperidine-1-carboxylate

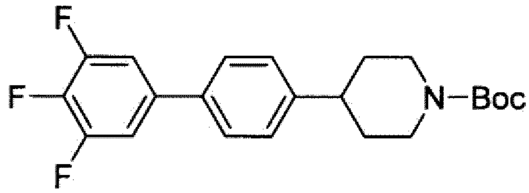

A mixture of *tert*-butyl 4-(4-bromophenyl)piperidine-1-carboxylate (660.7 mg, 1.94 mmol, 1.0 equiv), palladium(II) acetate (22.0 mg, 0.1 mmol, 5 mol%), Amphos (39.0 mg, 0.15 mmol, 7.5 mol%), potassium carbonate (1.00 g, 7.6 mmol, 4.0 equiv), and 3,4,5-trifluorophenylboronic acid (CAS: 143418-49-9: purchased from Wako Pure Chemical Industries, Ltd.) (682.0 mg, 3.8 mmol, 2.0 equiv) in a liquid mixture of toluene (18 mL) and degassed water (1.8 mL) was stirred at 100°C overnight. The reaction mixture was diluted with water, and extracted with $CH_2Cl_2$. The combined organic layer was washed with water, dried over $Na_2SO_4$, filtered, and concentrated *in vacuo.* The residue was purified by column chromatography (EtOAc/hexane = 2-18%) to give tert-butyl 4-(3',4',5'-trifluoro-[1,1'-biphenyl]-4-yl)piperidine-1-carboxylate (597.3 mg, 1.53 mmol, 78.9% yield).
$^1H$ NMR (400 MHz, $CDCl_3$) $\delta$ 7.43 (2H, d, J = 8.5 Hz), 7.27 (2H, d, J = 8.5 Hz), 7.17 (2H, dd, J = 8.8, 2.2 Hz), 4.26 (2H, m), 2.79 (2H, br t, J = 11.2 Hz), 2.66 (1H, tt, J = 12.1, 3.5 Hz), 1.82 (2H, br d, J = 12.6 Hz), 1.62-1.66 (2H, m), 1.48 (9H, s).

Reference Example 5

General procedure for Boc removal (exemplified by the synthesis of 4-(4-(9H-fluoren-2-yl)phenyl)piperidine)

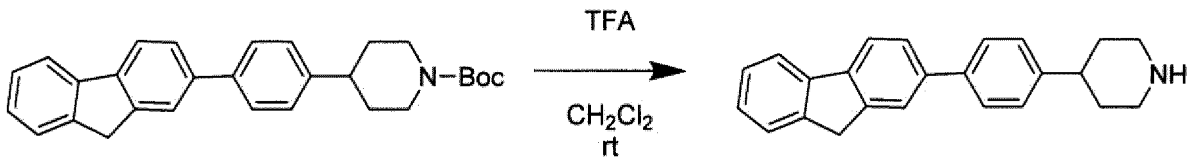


The Boc-protected amine compound (4-(4-(9H-fluoren-2-yl)phenyl)piperidine-1-carboxylate produced in Reference Example 2) (55.0 mg, 0.129 mmol) and trifluoroacetic acid (1.3 mL) were added to $CH_2Cl_2$ (1.3 mL), and the mixture was stirred at room temperature for 2 hours. A 3 N NaOH aqueous solution was added slowly, and the titled organic compound was extracted with $CH_2Cl_2$. The combined organic layer was dried over $Na_2SO_4$, filtered, and concentrated *in vacuo* to give the titled 4-(4-(9H-fluoren-2-yl)phenyl)piperidine as a brown oil (24.8 mg, 59% yield).
$^1H$ NMR (400 MHz, $CDCl_3$) $\delta$ 7.76-7.84 (3H, m), 7.55-7.63 (4H, m), 7.29-7.41 (5H, m), 3.96 (2H, s), 3.22 (2H, br d, J = 12.1 Hz), 2.75 (2H, td, J = 12.1, 2.2 Hz), 2.65 (1H, tt, J = 12.1, 3.1 Hz), 1.88 (2H, br d, J = 12.1 Hz), 1.68 (2H, td, J = 12.1, 4.0 Hz).

Reference Example 6

Production of 4-(4-(anthracen-9-yl)phenyl)piperidine

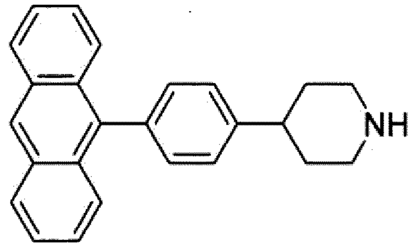

According to the general procedure for Boc-removal and using tert-butyl 4-(4-(anthracen-9-yl)phenyl)piperidine-1-carboxylate produced in Reference Example 3 (52.1 mg, 0.119 mmol) instead, the titled 4-(4-(anthracen-9-yl)phenyl)piperidine was obtained as a brown oil (30.5 mg, 0.09 mmol, 75.9% yield).
$^1$H NMR (400 MHz, $CDCl_3$) δ 8.48 (1H, s), 8.03 (2H, br d, J = 8.5 Hz), 7.68 (2H, br d, J = 9.1 Hz), 7.40-7.45 (4H, m), 7.35-7.36 (4H, m), 3.25 (2H, br d, J = 10.3 Hz), 2.61-2.84 (4H, m), 1.71-1.87 (3H, m).

Reference Example 7

Production of 4-(3',4',5'-trifluoro-[1,1'-biphenyl]-4-yl)piperidine

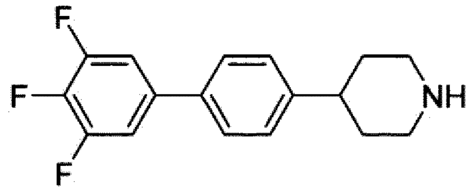

According to the general procedure for Boc-removal and using tert-butyl 4-(3',4',5'-trifluoro-[1,1'-biphenyl]-4-yl)piperidine-1-carboxylate produced in Reference Example 4 (597.3 mg, 1.53 mmol) instead, the titled 4-(3',4',5'-trifluoro-[1,1'-biphenyl]-4-yl)piperidine was obtained as a brown oil (435.8 mg, 1.49 mmol, 97% yield).
$^1$H NMR (400 MHz, $CDCl_3$) δ 7.43 (2H, d, J = 8.0 Hz), 7.29 (2H, d, J = 8.5 Hz), 7.14 (2H, dd, J = 8.9, 2.2 Hz), 3.19 (2H, br d, J = 12.1 Hz), 2.73 (2H, m), 2.63 (1H, tt, J = 12.1, 3.5 Hz), 1.83 (2H, br d, J = 13.9 Hz), 1.64 (2H, m) .

Reference Example 8

Production of 2-methoxypyridine-5-sulfonyl chloride

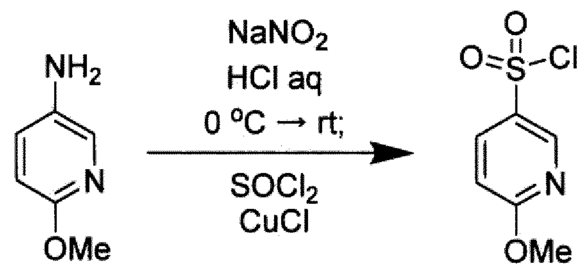


(a) Thionyl chloride (3.1 mL, 42.6 mmol, 4.26 equiv to amine) was added dropwise over 60 min to a degassed water (18.7 mL), while maintaining the temperature of the mixture at 0°C. The solution was allowed to warm to room temperature over 17 h. Copper(I) chloride (99 mg, 1.0 mmol, 0.1 equiv) was added to the mixture, and the resulting yellow-green suspension was cooled to -3°C using an ice bath.
(b) Hydrochloric acid (36% w/w, 10.1 mL) was added with stirring to 2-amino-5-methoxypyridine (1.24 g, 10 mmol, 1.0 equiv), while maintaining the temperature of the mixture equal to or below 30°C with an ice bath. The mixture was cooled to 0°C using an ice bath, and a solution of sodium nitrite (0.75 g, 0.1 mmol, 0.01 equiv) in degassed

water (3.0 mL) was added dropwise over 45 min, while maintaining the temperature of the reaction mixture at 0°C. The resulting slurry was cooled to 0°C and stirred for 10 min.

(c) The slurry from step (b) was cooled to 0°C and added to the solution obtained from step (a) over 95 min, while maintaining the temperature of the reaction mixture at 0°C. As the reaction proceeded, a solid began to precipitate. When the addition was complete, the reaction mixture was stirred at 0°C for 75 min. The reaction mixture was extracted with $CH_2Cl_2$, and washed with water and brine. The obtained organic layer was dried over $MgSO_4$, filtered, and concentrated *in vacuo* to give the titled 2-methoxypyridine-5-sulfonyl chloride. The spectral data were identical to those reported in the literature (Hogan, P.J.; Cox, B.G.; Org. Proc. Res. Dev. 2009, 13, 875).

$^1$H NMR (400 MHz, $CDCl_3$) δ 8.82 (d, J = 2.7 Hz, 1H), 8.10 (dd, J = 9.0, 2.7 Hz, 1H), 6.90 (d, J = 9.0 Hz, 1H), 4.04 (s, 3H).

Reference Example 9

General procedure for sulfonamide synthesis (exemplified by the synthesis of 5-((4-(4-(9H-fluoren-2-yl)phenyl)piperidin-1-yl)sulfonyl)-2-methoxypyridine)

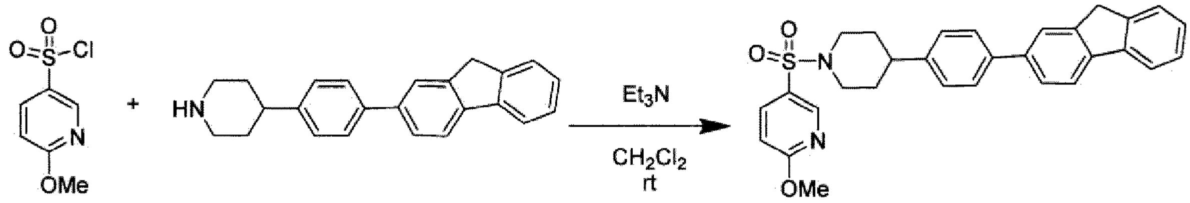


To a solution of a sulfonyl chloride compound (2-methoxypyridine-5-sulfonyl chloride produced in Reference Example 8) (15.8 mg, 0.0762 mmol, 1.0 equiv) in $CH_2Cl_2$ (18.0 mL) was added a secondary amine compound (4-(4-(9H-fluoren-2-yl)phenyl)piperidine obtained in Reference Example 5) (24.8 mg, 0.076 mmol, 1.0 equiv) and triethylamine (10 μL, 0.076 mmol, 1.0 equiv). The mixture was stirred at room temperature for 2 days. The reaction mixture was extracted with EtOAc, and the combined organic layer was washed with water and brine. The organic layer was dried over $Na_2SO_4$, filtered, and concentrated *in vacuo* to give the titled 5-((4-(4-(9H-fluoren-2-yl)phenyl)piperidin-1-yl)sulfonyl)-2-methoxypyridine as a brown oil (49.0 mg, 100% yield).

$^1$H NMR (400 MHz, $CDCl_3$) δ 8.61 (1H, d, J = 2.2 Hz), 7.91 (1H, dd, J = 8.5, 2.2 Hz), 7.74-7.84 (4H, m), 7.56-7.61 (4H, m), 7.36-7.40 (1H, m), 7.31 (2H, d, J = 7.1 Hz), 6.86 (1H, d, J = 8.0 Hz), 3.95 (3H, s), 2.40-2.52 (3H, m), 1.89-1.95 (6H, m).

Reference Example 10

Production of 5-((4-(4-(anthracen-9-yl)phenyl)piperidin-1-yl)sulfonyl)-2-methoxypyridine

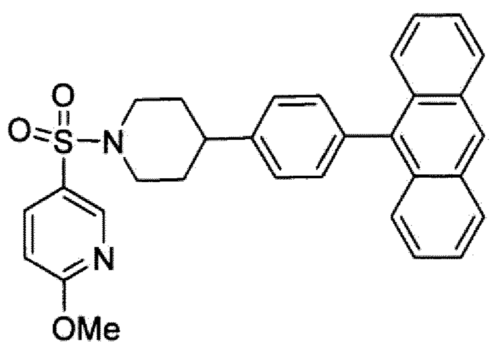

According to the general procedure for sulfonamide synthesis described in Reference Example 9 and using 4-(4-(anthracen-9-yl)phenyl)piperidine produced in Reference Example 6 (30.5 mg, 0.09 mmol, 1.0 equiv) as the secondary amine compound instead, the titled 5-((4-(4-(anthracen-9-yl)phenyl)piperidin-1-yl)sulfonyl)-2-methoxypyridine was obtained as a brown solid (18.3 mg, 0.036 mmol, 40% yield).

$^1$H NMR (400 MHz, $CDCl_3$) δ 8.64 (1H, d, J = 2.7 Hz), 8.49 (1H, s), 8.03 (2H, d, J = 8.5 Hz), 7.94 (1H, dd, J = 8.5, 2.7 Hz), 7.63 (2H,d, J = 8.5 Hz), 7.43 (2H, t, J = 8.0 Hz), 7.30 (2H, s), 6.88 (1H, d, J = 8.9 Hz), 4.04 (3H, s), 2.45-2.64 (3H, m), 1.94-2.02 (6H, m).

Reference Example 11

Production of 2-methoxy-5-((4-(3',4',5'-trifluoro-[1,1'-biphenyl]-4-yl)piperidin-1-yl) sulfonyl)pyridine

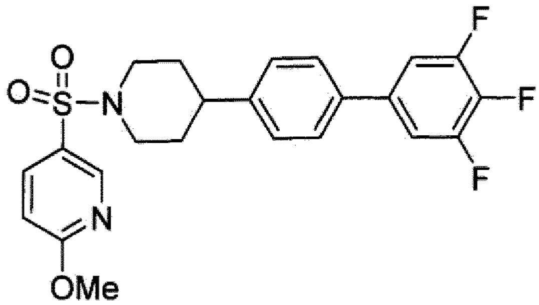

According to the general procedure for sulfonamide synthesis described in Reference Example 9 and using 4-(3',4',5'-trifluoro-[1,1'-biphenyl]-4-yl)piperidine produced in Reference Example 7 (210 mg, 0.72 mmol, 1.0 equiv) as the secondary amine compound instead, the titled 2-methoxy-5-((4-(3',4',5'-trifluoro-[1,1'-biphenyl]-4-yl)piperidin-1-yl)sulfonyl)pyridine was obtained as a brown oil (248.8 mg, 0.538 mmol, 74.7% yield).
$^1$H NMR (400 MHz, $CDCl_3$) δ 8.60 (1H, d, J = 2.2 Hz), 7.90 (1H, dd, J = 8.5, 2.7 Hz), 7.42 (2H, dd, J = 6.2, 1.7 Hz), 7.24 (2H, d, J = 8.0 Hz), 7.13-7.17 (2H, m), 6.85 (1H, d, J = 8.9 Hz), 4.02 (3H, s), 3.95-3.98 (2H, m), 2.36-2.50 (3H, m), 1.85-1.95 (4H, m).

Reference Example 12

General procedure for demethylation (exemplified by the synthesis of 5-((4-(4-(9H-fluoren-2-yl)phenyl)piperidin-1-yl)sulfonyl)-pyridin-2(1H)-one)

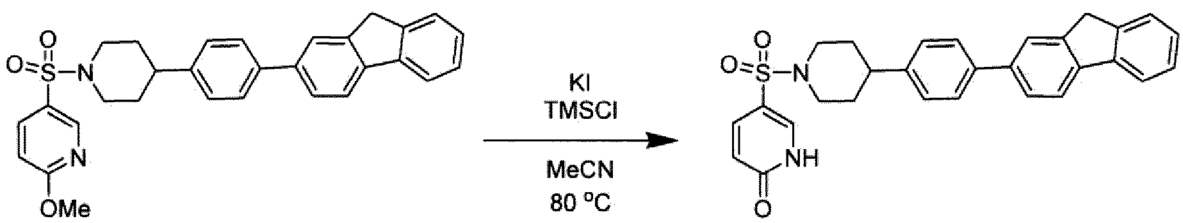


The methoxypyridine compound (5-((4-(4-(9H-fluoren-2-yl)phenyl)piperidin-1-yl)sulfonyl)-2-methoxypyridine obtained in Reference Example 9) (37.8 mg, 0.0762 mmol, 1.0 equiv), potassium iodide (63.2 mg, 0.381 mmol, 5.0 equiv) and chlorotrimethylsilane (50.3 μL, 0.381 mmol, 5.0 equiv) were added to a MeCN (48 mL), and the resulting mixture was stirred at 80°C for 3 hours. A 10% $Na_2S_2O_3$ aqueous solution was added, and the titled organic compound was extracted with EtOAc. The combined organic layer was washed with water and brine, dried over $Na_2SO_4$, filtered, and concentrated *in vacuo* to give the titled 5-((4-(4-(9H-fluoren-2-yl)phenyl)piperidin-1-yl)sulfonyl)-pyridin-2(1H)-one as a light brown oil (25.0 mg, 68% yield).
$^1$H NMR (400 MHz, $CDCl_3$) δ 7.94 (1H, m), 7.29-7.85 (9H, m), 7.16-7.18 (1H, m), 7.02 (1H, s), 6.83-6.86 (1H, m), 6.64-6.67 (1H, m), 3.96 (1H, m), 3.84 (2H, s), 2.51-2.57 (2H, m), 1.82-1.95 (6H, m).

Reference Example 13

Production of 5-((4-(4-(anthracen-9-yl)phenyl)piperidin-1-yl)sulfonyl)pyridin-2(1*H*)-one

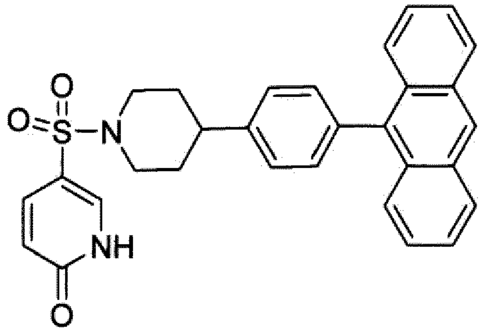

According to the general procedure for demethylation described in Reference Example 12 and using 5-((4-(4-(anthracen-9-yl)phenyl)piperidin-1-yl)sulfonyl)-2-methoxypyridine obtained in Reference Example 10 (18.3 mg, 0.36 mmol) as the methoxypyridine compound instead, the titled 5-((4-(4-(anthracen-9-yl)phenyl)piperidin-1-yl)sulfonyl)pyridin-2(1*H*)-one was obtained as a brown solid (15.6 mg, 0.031 mmol, 87.3% yield).

Reference Example 14

Production of 5-((4-(3',4',5'-trifluoro-[1,1'-biphenyl]-4-yl)piperidin-1-yl)sulfonyl)pyridin-2(1*H*)-one

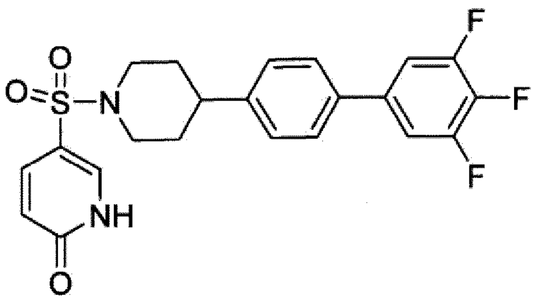

According to the general procedure for demethylation described in Reference Example 12 and using 2-methoxy-5-((4-(3',4',5'-trifluoro-[1,1'-biphenyl]-4-yl)piperidin-1-yl)sulfonyl)pyridine obtained in Reference Example 11 (248.8 mg, 0.538 mmol) as the methoxypyridine compound instead, the titled 5-((4-(3',4',5'-trifluoro-[1,1'-biphenyl]-4-yl)piperidin-1-yl)sulfonyl)pyridin-2(1*H*)-one was obtained as a brown solid (19.8 mg, 0.044 mmol, 92% yield).

Reference Example 15

Production of 2-bromo-1-(3'-(trifluoromethyl)-[1,1'-biphenyl]-4-yl)ethan-1-one

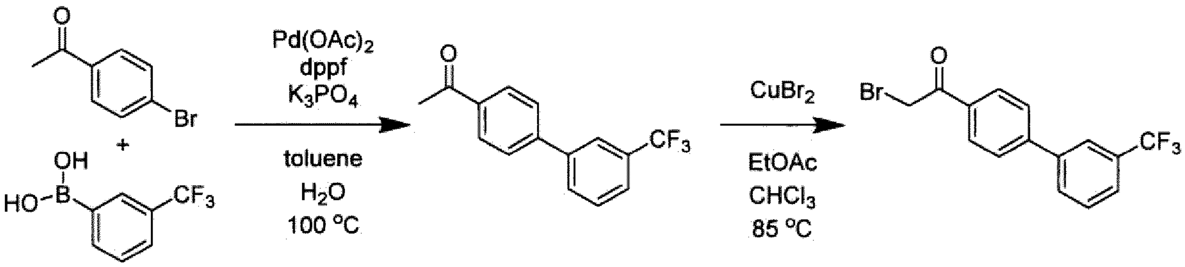


Step 1 (General procedure for Suzuki-Miyaura reaction 2):

A mixture of an aryl bromide compound (1-(4-bromophenyl)ethan-1-one, CAS: 99-90-1, purchased from Tokyo Chemical Industry Co., Ltd.) (1.3 g, 6.6 mmol, 1.0 equiv), palladium(II) acetate (5.3 mg, 0.023 mmol, 0.3 mol%), 1,1'-bis(diphenylphosphino)ferrocene (13 mg, 0.023 mmol, 0.3 mol%), potassium phosphate (3.9 g, 18.4 mmol, 2.8 equiv), and a boronic acid compound ((3-(trifluoromethyl)phenyl)boronic acid, CAS: 1423-26-3, purchased from Wako Pure Chemical Industries, Ltd.) (1.75 g, 9.2 mmol, 1.4 equiv) in a liquid mixture of toluene (30 mL) and degassed water (3 mL) was stirred at 100°C overnight. The reaction mixture was diluted with water and extracted with EtOAc. The combined organic layer was washed with water, dried over $Na_2SO_4$, filtered, and concentrated *in vacuo.* The residue was purified by column chromatography (EtOAc/hexane = 17%) to give 1-(3'-(trifluoromethyl)-[1,1'-biphenyl]-4-yl)ethan-1-one as a brown solid (1.62 g, 6.15 mmol, 93.1% yield).
$^1H$ NMR (400 MHz, $CDCl_3$) $\delta$ 8.03 (2H, dd, J = 8.0, 1.3 Hz), 7.84 (1H, s), 7.77 (1H, d, J = 7.6 Hz), 7.63 (3H, m), 7.56 (1H, t, J = 7.6 Hz), 2.64 (3H, s) .

Step 2:

A mixture of (3'-(trifluoromethyl)-[1,1'-biphenyl]-4-yl)ethan-1-one (1.62 g, 6.15 mmol, 1.0 equiv) and copper(II) bromide (2.7 g, 12.3 mmol, 2.0 equiv) in a liquid mixture of EtOAc (30 mL) and $CHCl_3$ (30 mL) was stirred at 85°C for 70 min. The mixture was concentrated *in vacuo,* and water was added. The organic compounds were extracted with EtOAc. The combined organic layer was washed with a saturated aqueous $Na_2S_2O_3$ solution and brine. The organic layer was dried over $Na_2SO_4$, filtered, and concentrated *in vacuo.* The residue was purified by column chromatography ($CH_2Cl_2$/hexane = 12-73%) to give the titled 2-bromo-1-(3'-(trifluoromethyl)-[1,1'-biphenyl]-4-yl)ethan-1-one as a white solid (3.24 mmol, 74.9% yield).
$^1H$ NMR (400 MHz, $CDCl_3$) $\delta$ 7.57 (2H, m), 7.35 (1H, s), 7.28 (1H, d, J = 7.6 Hz), 7.20 (2H, br d, J = 8.0 Hz), 7.15 (1H, d, J = 8.0 Hz), 7.09 (1H, t, J = 7.6 Hz), 3.98 (2H, s).

Production Example 1B

General procedure for alkylation reaction 1 of substituted aza-alicyclic compounds (pyridinone compounds) (compounds B) (exemplified by the synthesis of 5-((4-(4-(9H-fluoren-2-yl)phenyl)piperidin-1-yl)sulfonyl)-1-(2-oxo-2-(3'-(trifluoromethyl)-[1,1'-biphenyl]-4-yl)ethyl)pyridin-2(1H)-one (KS-46))

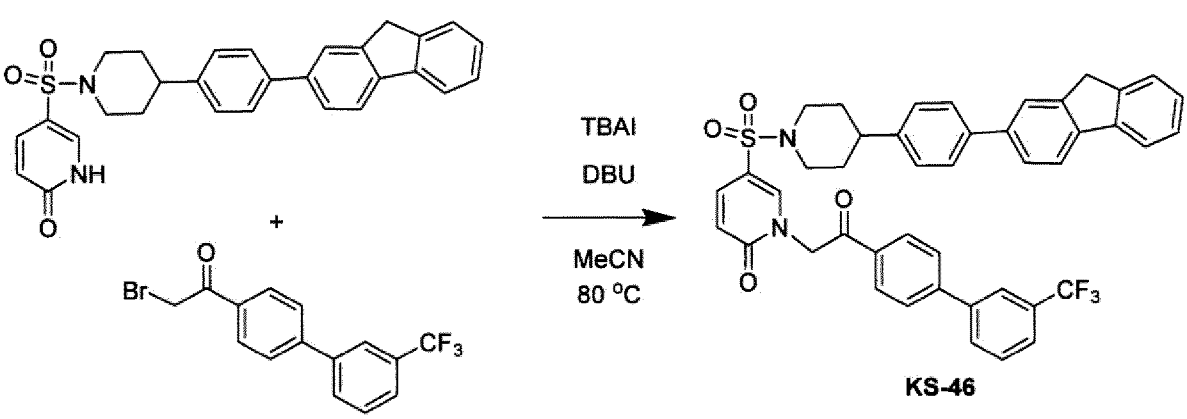


KS-46

5-((4-(4-(9H-Fluoren-2-yl)phenyl)piperidin-1-yl)sulfonyl)-pyridin-2(1H)-one produced in Reference Example 12 (25 mg, 0.0518 mmol, 1.0 equiv) and diazabicycloundecene (DBU) (11.5 $\mu$L, 0.077 mmol, 1.5 equiv) were added to MeCN (12 mL), and the mixture was stirred at 80°C for 1 hour. Then, a solution of an $\alpha$-bromoketone compound (2-bromo-1-(3'-(trifluoromethyl)-[1,1'-biphenyl]-4-yl)ethan-1-one produced in Reference Example 15) (26.4 mg, 0.077 mmol, 1.5 equiv) and tetrabutylammonium iodide (9.6 mg, 0.0259 mmol, 0.5 equiv) in MeCN (4 mL) was added, and the mixture was stirred at 80°C for 90 min. A saturated $Na_2S_2O_3$ aqueous solution was added thereto, and the titled organic compound was extracted with EtOAc. The combined organic layer was washed with water, dried over $Na_2SO_4$, filtered, and concentrated *in vacuo.* The residue was purified by column chromatography ($EtOAc/CH_2Cl_2$ = 0-50%) to give the titled 5-((4-(4-(9H-fluoren-2-yl)phenyl)piperidin-1-yl)sulfonyl)-1-(2-oxo-2-(3'-(trifluoromethyl)-[1,1'-biphenyl]-4-yl)ethyl)pyridin-2(1H)-one (KS-46) as a yellow solid (0.0119 mmol, 23% yield).
$^1H$ NMR (400 MHz, $CDCl_3$) $\delta$ 8.12-8.14 (m, 2H), 7.75-7.91(m, 7H), 7.56-7.70 (m, 7H), 7.36 (dd, J = 8.0 Hz, 1H), 7.27-7.30 (m, 3H), 7.18 (dd, J = 8.5 Hz, 1H), 6.68-6.70 (m, 1H), 5.49 (m, 2H), 5.29 (s, 2H), 3.95 (br, 3H), 2.64-2.67 (m, 3H), 1.87-2.01 (m, 4H) ; LRMS (ESI+) m/z 767 $[M + Na]^+$.

Production Example 2B

Production of 5-((4-(4-(anthracen-9-yl)phenyl)piperidin-1-yl)sulfonyl)-1-(2-oxo-2-(3'-(trifluoromethyl)-[1,1'-biphenyl]-4-yl)ethyl)pyridin-2(1H)-one (KS-47)

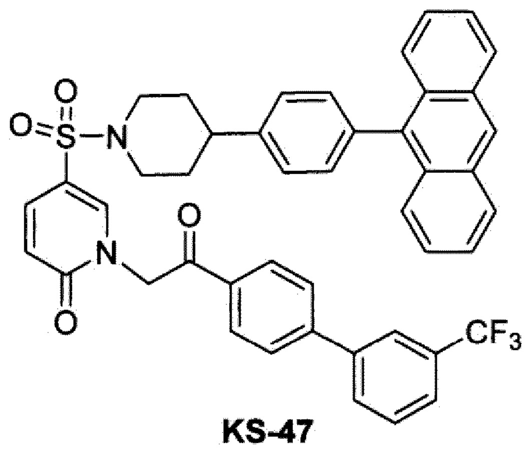

**KS-47**

According to the general procedure for alkylation reaction 1 of pyridinone compounds described in Production Example 1B and using 5-((4-(4-(anthracen-9-yl)phenyl)piperidin-1-yl)sulfonyl)pyridin-2(1*H*)-one produced in Reference Example 13 (15.6 mg, 0.031 mmol) as the pyridinone compound instead, the titled 5-((4-(4-(anthracen-9-yl)phenyl)piperidin-1-yl)sulfonyl)-1-(2-oxo-2-(3'-(trifluoromethyl)-[1,1'-biphenyl]-4-yl)ethyl)pyridin-2(1H)-one (KS-47) was obtained as a light brown solid (14.3 mg, 0.0189 mmol, 60% yield).
$^1H$ NMR (400 MHz, $CDCl_3$) $\delta$ 8.49 (1H, s), 8.13 (2H, d, J = 8.5 Hz), 8.03 (2H, d, J = 8.5 Hz), 7.95 (1H, d, J = 2.7 Hz), 7.88 (1H, s), 7.81 (1H, d, J = 7.6 Hz), 7.77 (2H, d, J = 8.5 Hz), 7.62-7.70 (5H, m), 7.32-7.47 (8H, m), 6.70 (1H, d, J = 9.4 Hz), 5.51 (2H, s), 3.91-4.02 (2H, m), 2.71-2.79 (3H, m), 2.14-2.20 (2H, m), 1.96-2.08 (2H, m); LRMS (ESI+) m/z757 $[M + H]^+$, 779 $[M + Na]^+$.

Production Example 3B

Production of 1-(2-oxo-2-(3'-(trifluoromethyl)-[1,1'-biphenyl]-4-yl)ethyl)-5-((4-(3',4',5'-trifluoro-[1,1'-biphenyl]-4-yl)piperidin-1-yl)sulfonyl)pyridin-2(1*H*)-one (KS-54)

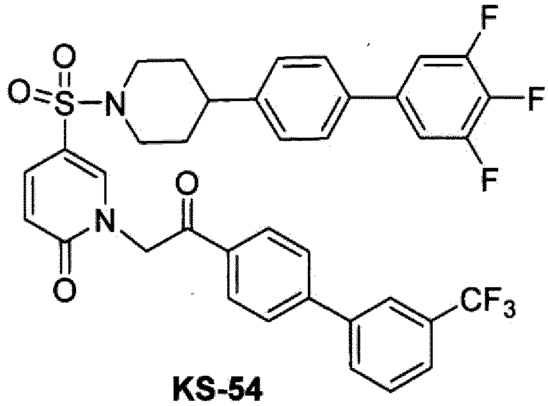

**KS-54**

According to the general procedure for alkylation reaction 1 of pyridinone compounds described in Production Example 1B and using 5-((4-(3',4',5'-trifluoro-[1,1'-biphenyl]-4-yl)piperidin-1-yl)sulfonyl)pyridin-2(1H)-one produced in Reference Example 14 (19.8 mg, 0.044 mmol) as the pyridinone compound instead, 31 mg of the titled 1-(2-oxo-2-(3'-(trifluoromethyl)-[1,1'-biphenyl]-4-yl)ethyl)-5-((4-(3',4',5'-trifluoro-[1,1'-biphenyl]-4-yl)piperidin-1-yl) sulfonyl)pyridin-2(1*H*)-one (KS-54) was obtained as a pale yellow oil (31 mg, 0.044 mmol, 100% yield).
$^1$H NMR (400 MHz, $CDCl_3$) δ 8.12 (2H, d, J = 8. 0 Hz), 7.88 (2H, m), 7.81 (1H, d, J = 7.4 Hz), 7.76 (2H, d, J = 8.0 Hz), 7.62-7.66 (2H, m), 7.44 (2H, d, J = 8.0 Hz), 7.27 (2H, d, J = 8.0 Hz), 7.15 (2H, t, J = 8.0 Hz), 6.68 (1H, d, J = 9.7 Hz), 5.49 (2H, s), 3.94 (2H, br d, J = 9.7 Hz), 2.65-2.70 (3H, m), 1.98 (2H, br d, J = 12.0 Hz), 1.88-1.90 (2H, m). LRMS (ESI+) m/z 710 $[M + H]^+$.

Reference Example 16

Production of 1-(2-(4-bromophenyl)-2-oxoethyl)-5-((4-(3',4',5'-trifluoro-[1,1'-biphenyl]-4-yl)piperidin-1-yl)sulfonyl)pyridin-2(1*H*)-one

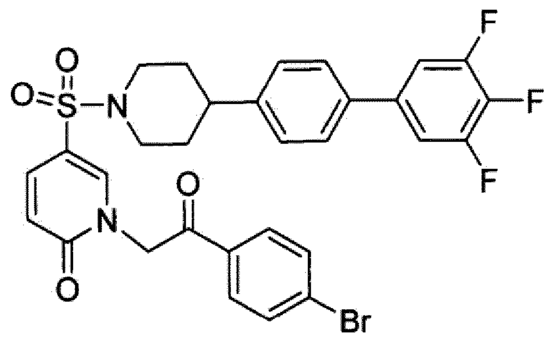

According to the general procedure for alkylation reaction 1 of pyridinone compounds described in Production Example 1B, and using 5-((4-(3',4',5'-trifluoro-[1,1'-biphenyl]-4-yl)piperidin-1-yl)sulfonyl)pyridin-2(1*H*)-one produced in Reference Example 14 (60 mg, 0.135 mmol) as the pyridinone compound and 2-bromo-1-(4-bromophenyl)ethan-1-one (37.5 mg, 0.135 mmol) as the α-bromoketone compound instead, the titled 1-(2-(4-bromophenyl)-2-oxoethyl)-5-((4-(3',4',5'-trifluoro-[1,1'-biphenyl]-4-yl)piperidin-1-yl)sulfonyl)pyridin-2(1*H*)-one was obtained as a brown solid (8.0 mg, 0.0124 mmol, 9.2% yield).
$^1$H NMR (400 MHz, $CDCl_3$) δ 7.87 (3H, dd, J = 8.5, 2.2 Hz), 7.68 (2H, d, J = 8.5 Hz), 7.63 (1H, dd, J = 9.8, 2.2 Hz), 7.44 (2H, d, J = 8.5 Hz), 7.29 (2H, s), 7.14 (2H, dd, J = 8.9, 8.5 Hz), 6.66 (1H, d, J = 9.8 Hz), 5.40 (2H, s), 3.92 (2H, br d, J = 11.7 Hz), 2.62 (3H, m), 1.86-1.97 (4H, m).

Production Example 4B

Production of 1-(2-oxo-2-(3',4',5'-trifluoro-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one (KS-59)

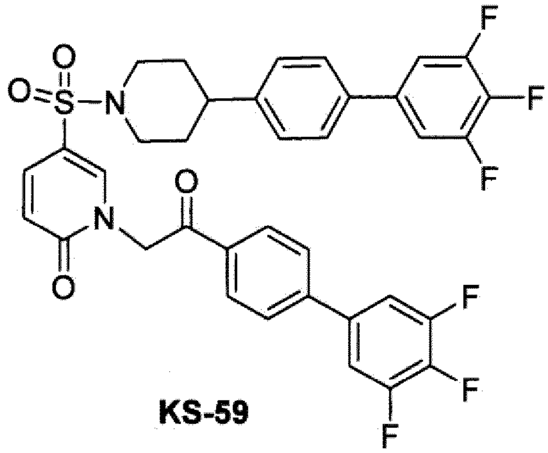

KS-59

A mixture of 1-(2-(4-bromophenyl)-2-oxoethyl)-5-((4-(3',4',5'-trifluoro-[1,1'-biphenyl]-4-yl)piperidin-1-yl)sulfonyl)pyridin-2(1*H*)-one produced in Reference Example 16 (8.0 mg, 0.0124 mmol, 1.0 equiv), palladium(II) acetate (0.13 mg, 0.0006 mmol, 5 mol%), Amphos (0.25 mg, 0.0009 mmol, 7.5 mol%), potassium carbonate (7 mg, 0.0496 mmol, 4.0 equiv), and 3,4,5-trifluorophenyl-boronic acid (4.4 mg, 0.0248 mmol, 2.0 equiv) in toluene (1 mL) and degassed water (0.1 mL) was stirred at 100°C overnight. The reaction mixture was diluted with water and extracted with EtOAc. The combined organic layer was washed with water, dried over $Na_2SO_4$, filtered, and concentrated *in vacuo*. The residue was purified by column chromatography (EtOAc/hexane = 0-12%) to give the titled 1-(2-oxo-2-(3',4',5'-trifluoro-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one (KS-59) as a yellow amorphous solid (3.7 mg, 0.0053 mmol, 42.8% yield).
$^1H$ NMR (400 MHz, $CDCl_3$) $\delta$ 8.09 (2H, d, J = 8.5 Hz), 7.89 (1H, d, J = 2.6 Hz), 7.65 (3H, m), 7.44 (1H, d, J = 8.0 Hz), 7.27 (6H, m), 7.14 (2H, dd, J = 8.9,8.5 Hz), 6.68 (1H, d, J = 9.4 Hz), 5.47 (2H, s), 3.94 (2H, br d, J = 11.2 Hz), 2.64-2.70 (3H, m), 1.88-2.01 (4H, m). LRMS (ESI+) m/z 719 $[M + Na]^+$.

Reference Example 17

General procedure for Miyaura-Ishiyama borylation (exemplified by the synthesis of 2-([1,1':4',1"-terphenyl]-4-yl)-4,4,5,5-tetramethyl-1,3,2-dioxaborolane)

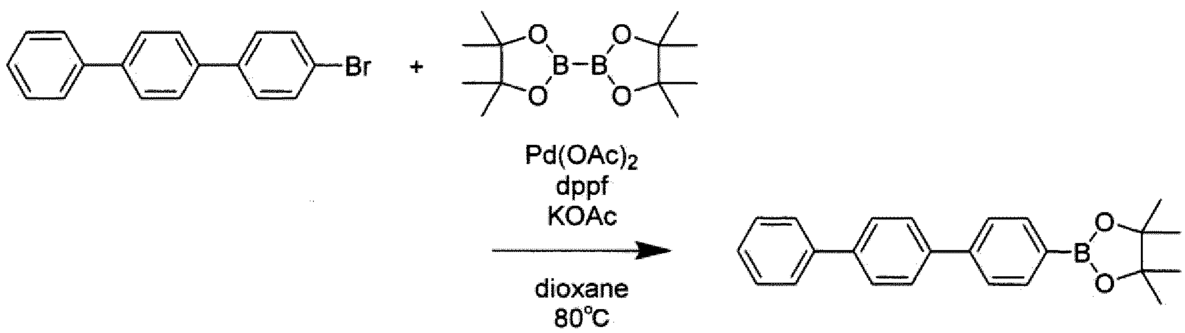


A mixture of an aryl bromide compound (200 mg, 0.646 mmol, 1.0 equiv), palladium(II) acetate (14.4 mg, 0.064 mmol, 10 mol%), 1,1'-bis(diphenylphosphino)ferrocene (35.5 mg, 0.064 mmol, 10 mol%), potassium acetate (126.8 mg, 1.29 mmol, 2.0 equiv), and bis(pinacolato)diboron (197 mg, 0.776 mmol, 1.2 equiv) in dioxane (12 mL) was stirred at

80°C for 3 hr. The reaction mixture was diluted with water and extracted with $CH_2Cl_2$. The combined organic layer was washed with brine. The organic layer was dried over $Na_2SO_4$, filtered, and concentrated *in vacuo.* The residue was purified by column chromatography (EtOAc/hexane = 0→18%) to give the titled 2-([1,1':4',1"-terphenyl]-4-yl)-4,4,5,5-tetramethyl-1,3,2-dioxaborolane as a brown solid (160.8 mg, 0.45 mmol, 69.8% yield).
$^1$H NMR (400 MHz, $CDCl_3$) δ 7.82 (2H, d, J = 8.5 Hz), 7.56-7.64 (8H, m), 7.37-7.41 (2H, m), 7.29-7.31 (1H, m), 1.29 (12H, s).

Reference Example 18

Production of *tert*-butyl 4-([1,1':4',1'':4",1'''-quaterphenyl]-4-yl)piperidine-1-carboxylate

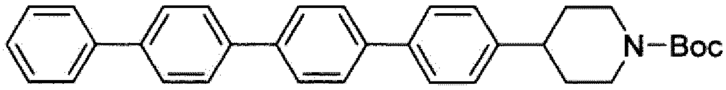

According to the general procedure for the Suzuki-Miyaura reaction 1 described in Reference Example 2 and using 2-([1,1':4',1"-terphenyl]-4-yl)-4,4,5,5-tetramethyl-1,3,2-dioxaborolane (160.8 mg, 0.45 mmol, 0.63 equiv) as the boronic acid compound instead, the titled *tert*-butyl 4-([1,1':4',1":4",1'''-quaterphenyl]-4-yl)piperidine-1-carboxylate was obtained as a brown solid (20.3 mg, 0.0415 mmol, 9.2% yield).
$^1$H NMR (400 MHz, $CDCl_3$) δ 7.26-7.72 (16H, m), 7.19-7.21 (1H, m), 4.26 (4H, m), 2.82 (2H, m), 2.63-2.70 (1H, m), 1.80-1.87 (2H, m), 1.61-1.67 (2H, m), 1.49 (9H, s).

Reference Example 19

Production of *tert*-butyl 4-(4-(pyren-1-yl)phenyl)piperidine-1-carboxylate

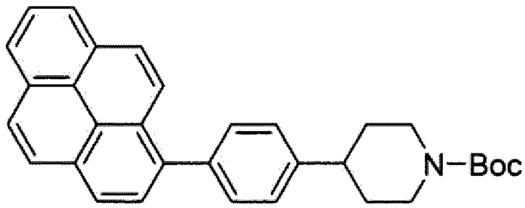

According to the general procedure for the Suzuki-Miyaura reaction 1 described in Reference Example 2 and using 1-pyreneboronic acid (177 mg, 0.72 mmol, 2.0 equiv) as the boronic acid compound instead, the titled tert-butyl 4-(4-(pyren-1-yl)phenyl)piperidine-1-carboxylate was obtained as a brown solid (123.7 mg, 0.267 mmol, 69.4% yield).
$^1$H NMR (400 MHz, $CDCl_3$) δ 8.18-8.21 (4H, m), 8.09 (2H, s), 7.96-8.03 (3H, m), 7.57 (2H, d, J = 8.0 Hz), 7.38 (2H, d, J = 7.6 Hz), 4.31 (2H, m), 2.75-2.88 (3H, m), 1.97 (2H, br d, J = 12.6 Hz), 1.68-1.80 (2H, m), 1.49 (9H, s).

Reference Example 20

Production of *tert*-butyl 4-([1,1'-biphenyl]-4-yl)piperidine-1-carboxylate

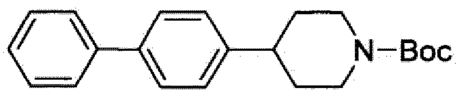

According to the general procedure for the Suzuki-Miyaura reaction 2 described in Reference Example 2 and using tert-butyl 4-(4-bromophenyl)piperidine-1-carboxylate (34 mg, 0.1 mmol, 1.0 equiv) as the aryl bromide compound and phenylboronic acid (84.1 mg, 0.68 mmol, 2.0 equiv) as the boronic acid compound instead, the titled tert-butyl 4-([1,1'-biphenyl]-4-yl)piperidine-1-carboxylate was obtained as a brown solid (108.3 mg, 0.32 mmol, 92.8% yield).
$^1$H NMR (400 MHz, $CDCl_3$) δ 7.50-7.55 (4H, m), 7.36-7.41 (2H, m), 7.30-7.31 (1H, m), 7.26 (1H, m), 7.23 (1H, m), 4.23

(2H, m), 2.79 (2H, m), 2.62-2.69 (1H, m), 1.81 (2H, br d, J = 12.6 Hz), 1.58-1.64 (2H, m), 1.46 (9H, s) .

Reference Example 21

Production of 4-([1,14',14",1'''-quaterphenyl]-4-yl)piperidine

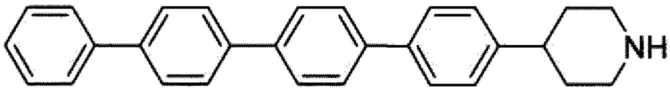

According to the general procedure for Boc removal described in Reference Example 5 and using *tert*-butyl 4-([1,1':4',1":4",1'''-quaterphenyl]-4-yl)piperidine-1-carboxylate (Reference Example 18) (20.3 mg, 0.04 mmol) instead, the titled 4-([1,1':4',1":4",1'''-quaterphenyl]-4-yl)piperidine was obtained as a brown solid (17.9 mg, 0.04 mmol, 100% yield). $^1$H NMR (400 MHz, $CDCl_3$) δ 7.22-7.72 (17H, m), 3.18-3.22 (2H, m), 2.75-2.79 (2H, m), 2.61-2.69 (1H, m), 2.00-2.10 (2H, m), 1.64-1.66 (2H, m).

Reference Example 22

Production of 4-(4-(pyren-1-yl)phenyl)piperidine

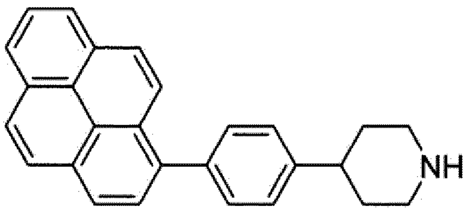

According to the general procedure for Boc removal described in Reference Example 5 and using *tert*-butyl 4-(4-(pyren-1-yl)phenyl)piperidine-1-carboxylate (Reference Example 19) (123.7 mg, 0.268 mmol) instead, the titled 4-(4-(pyren-1-yl)phenyl)piperidine was obtained as a brown oil (95.6 mg, 0.264 mmol, 98.7% yield).
$^1$H NMR (400 MHz, $CDCl_3$) δ 8.15-8.23 (4H, m), 8.09 (2H, s), 7.97-8.03 (3H, m), 7.57 (2H, d, J = 7.6 Hz), 7.40 (2H, d, J = 7.6 Hz), 3.23-3.36 (2H, m), 2.75-2.88 (4H, m), 1.98 (2H, br d, J = 12.1 Hz), 1.76-1.86 (2H, m).

Reference Example 23

Production of 4-([1,1'-biphenyl]-4-yl)piperidine

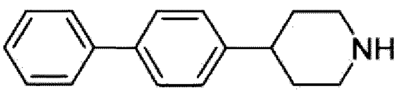

According to the general procedure for Boc removal described in Reference Example 5 and using *tert*-butyl 4-([1,1'-biphenyl]-4-yl)piperidine-1-carboxylate (Reference Example 20) (108.3 mg, 0.32 mmol) instead, the titled 4-([1,1'-biphenyl]-4-yl)piperidine was obtained as a brown oil (27 mg, 0.113 mmol, 35.5% yield).
$^1$H NMR (400 MHz, $CDCl_3$) δ 7.53-7.57 (4H, m), 7.41-7.44 (2H, m), 7.30-7.32 (3H, m), 2.79-2.86 (2H, m), 2.69-2.71 (1H, m), 1.90 (2H, br d, J = 12.6 Hz), 1.73-1.82 (2H, m) .

Reference Example 24

Production of 5-((4-([1,1':4',1":4",1'''-quaterphenyl]-4-yl)piperidin-1-yl)sulfonyl)-2-methoxypyridine

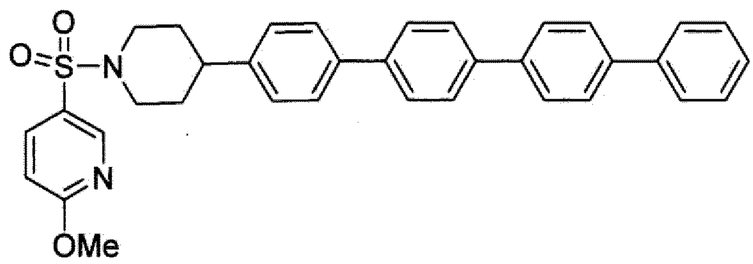

According to the general procedure for sulfonamide synthesis described in Reference Example 9 and using 4-([1,1':4',1':4",1'''-quaterphenyl]-4-yl)piperidine (Reference Example 21) (16.2 mg, 0.0415 mmol, 1.0 equiv) as the secondary amine compound instead, the titled 5-((4-([1,1':4',1":4",1'''-quaterphenyl]-4-yl)piperidin-1-yl)sulfonyl)-2-methoxypyridine was obtained as a brown solid (16.4 mg, 0.0292 mmol, 70.5% yield).
$^1$H NMR (400 MHz, $CDCl_3$) δ 8.55-8.62 (1H, m), 7.89-7.94 (1H, m),7.16-7.72 (17H, m), 6.85-6.87 (1H, m), 4.02 (3H, s), 3.94-3.99 (2H, m), 2.38-2.48 (3H, m), 1.83-1.96 (4H, m).

Reference Example 25

Production of 2-methoxy-5-((4-(4-(pyren-1-yl)phenyl)piperidin-1-yl)sulfonyl)pyridine

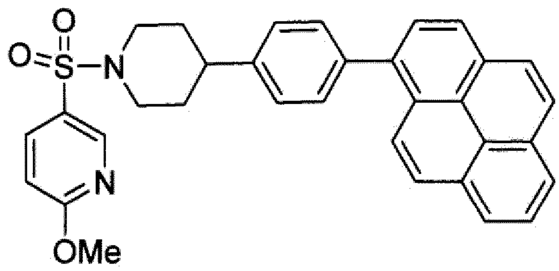

According to the general procedure for sulfonamide synthesis described in Reference Example 9 and using 4-(4-(pyren-1-yl)phenyl)piperidine (Reference Example 22) (95.6 mg, 0.264 mmol, 1.0 equiv) as the secondary amine compound instead, the titled 2-methoxy-5-((4-(4-(pyren-1-yl)phenyl)piperidin-1-yl)sulfonyl)pyridine was obtained as a brown solid (123.7 mg, 0.267 mmol, 69.4% yield).
$^1$H NMR (400 MHz, $CDCl_3$) δ 8.64 (1H, m), 8.15-8.21 (4H, m), 8.09 (2H, s), 8.01-8.02 (3H, m), 7.95-7.96 (2H, m), 7.56 (2H, d, J = 8.0 Hz), 7.32 (2H, d, J = 8.1 Hz), 6.87 (1H, d, J = 8.5 Hz), 4.04 (3H, s), 3.98 (2H, m), 2.54-2.58 (1H, m), 2.42-2.49 (2H, m), 1.98-2.03 (4H, m).

Reference Example 26

Production of 5-((4-([1,1'-biphenyl]-4-yl)piperidin-1-yl)sulfonyl)-2-methoxypyridine

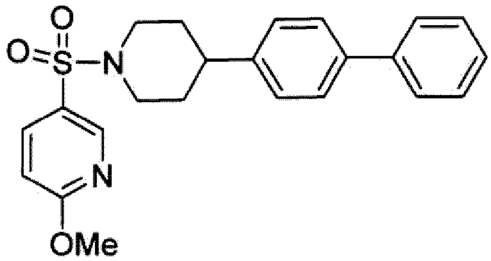

According to the general procedure for sulfonamide synthesis described in Reference Example 9 and using 4-([1,1'-biphenyl]-4-yl)piperidine (Reference Example 23) (29.9 mg, 0.126 mmol, 1.0 equiv) as the secondary amine compound instead, the titled 5-((4-([1,1'-biphenyl]-4-yl)piperidin-1-yl)sulfonyl)-2-methoxypyridine was obtained as a brown solid (18.4 mg, 0.045 mmol, 35.7% yield).
$^1$H NMR (400 MHz, $CDCl_3$) δ 8.73 (1H, m), 7.89-7.92 (1H, m), 7.51-7.54 (4H, m), 7.39-7.43 (2H, m), 7.32-7.34 (1H, m), 7.21-7.23 (2H, m), 6.85 (1H, d, J = 8.5 Hz), 4.01 (3H, s), 3.97 (2H, m), 2.38-2.52 (3H, m), 1.86-1.96 (4H, m).

Reference Example 27

Production of 2-methoxy-5-(pyrrolidin-1-yl-sulfonyl)pyridine

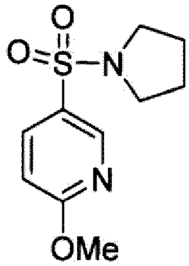

To a solution of 2-methoxypyridine-5-sulfonyl chloride (11.46 g, 55.2 mmol, 1.0 equiv) in $CH_2Cl_2$ (300 mL) was added pyrrolidine (12.5 mL, 150 mmol, 2.8 equiv). The mixture was stirred at room temperature overnight. The resulting mixture was extracted with EtOAc, and the combined organic layer was washed with water and brine. The organic layer was dried over $Na_2SO_4$, filtered, and concentrated *in vacuo*. The residue was purified by column chromatography (EtOAc = 100%) to give 2-methoxy-5-(pyrrolidin-1-yl-sulfonyl)pyridine as a pale yellow solid (13.2 g, 54.5 mmol, 98.7% yield).
$^1$H NMR (400 MHz, $CDCl_3$) δ 8.64 (d,J = 2.7 Hz, 1H), 7.93-7.97 (m, 2H), 6.84 (d, J = 8.5 Hz, 1H), 4.01 (s, 3H), 3.23-3.27 (m, 4H), 1.78-1.82 (m, 4H).

Reference Example 28

Production of 3-((6-methoxypyridin-3-yl)sulfonyl)thiazolidine

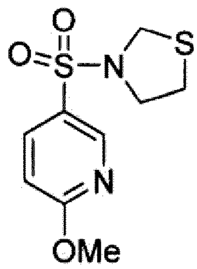

To a solution of 2-methoxypyridine-5-sulfonyl chloride (100 mg, 0.48 mmol, 1.0 equiv) in $CH_2Cl_2$ (5.0 mL) was

added thiazolidine (37.8 μL, 0.48 mmol, 1.0 equiv), N,N-dimethyl-4-aminopyridine (58.6 mg, 0.48 mmol, 1.0 equiv), and triethylamine (10 μL, 0.076 mmol, 1.0 equiv). The mixture was stirred at room temperature overnight. The resulting mixture was extracted with EtOAc, and the combined organic layer was washed with water and brine. The organic layer was dried over $Na_2SO_4$, filtered, and concentrated *in vacuo*. The residue was purified by column chromatography (MeOH/EtOAc = 30%) to give 3-((6-methoxypyridin-3-yl)sulfonyl)thiazolidine as a brown solid (151 mg, 0.504 mmol, 77.7% yield).
$^1$H NMR (500 MHz, $CDCl_3$) δ 8.64 (d, J = 1.5 Hz, 1H), 7.94-7.96 (m, 1H), 6.82 (d, J = 8.0 Hz, 1H), 4.44-4.50 (m, 2H), 4.01 (s, 3H), 3.62-3.65 (m, 2H), 2.77-2.81 (m, 2H).

Reference Example 29

Production of 5-((4-([1,1':4',1":4",1'''-quaterphenyl]-4-yl)piperidin-1-yl)sulfonyl)pyridin-2(1*H*)-one

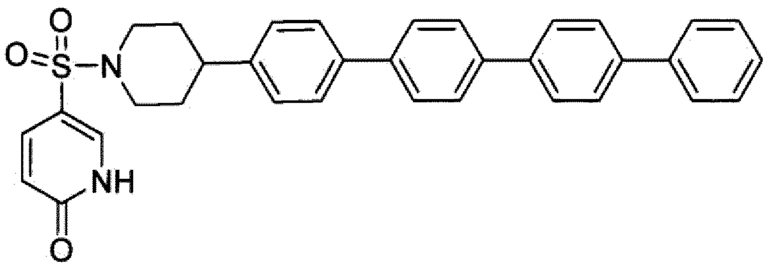

According to the general procedure for demethylation described in Reference Example 12 and using 5-((4-([1,1':4',1":4",1'''-quaterphenyl]-4-yl)piperidin-1-yl)sulfonyl)-2-methoxypyridine (Reference Example 24) (16.4 mg, 0.0292 mmol) as the methoxypyridine compound instead, the titled 5-((4-([1,1':4',1":4",1'''-quaterphenyl]-4-yl)piperidin-1-yl)sulfonyl)pyridin-2(1*H*)-one was obtained as a brown solid (10.7 mg, 0.0196 mmol, 67% yield).
$^1$H NMR (400 MHz, $CDCl_3$) δ 7.95 (1H, m), 7.68-7.70 (2H, m), 7.53-7.57 (3H, m), 7.43-7.44 (3H, m), 7.16-7.33 (12H, m), 6.65-6.67 (1H, m), 3.91 (2H, m), 2.52-2.60 (3H, m), 1.86-2.04 (4H, m).

Reference Example 30

Production of 5-((4-(4-(pyren-1-yl)phenyl)piperidin-1-yl)sulfonyl)pyridin-2(1*H*)-one

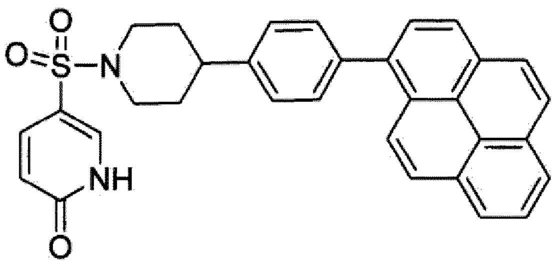

According to the general procedure for demethylation described in Reference Example 12 and using 2-methoxy-5-((4-(4-(pyren-1-yl)phenyl)piperidin-1-yl)sulfonyl)pyridine (Reference Example 25) (109.8 mg, 0.206 mmol) as the methoxypyridine compound instead, the titled 5-((4-(4-(pyren-1-yl)phenyl)piperidin-1-yl)sulfonyl)pyridin-2(1H)-one was obtained as a brown solid (23.9 mg, 0.049 mmol, 23.9% yield).
$^1$H NMR (400 MHz, $CDCl_3$) δ 8.62 (1H, m), 7.67-7.90 (10H, m), 7.50-7.54 (4H, m), 6.72 (1H, d, J = 8.9 Hz), 2.10-2.49 (2H, m), 1.86-1.92 (7H, m).

Reference Example 31

Production of 5-((4-([1,1'-biphenyl]-4-yl)piperidin-1-yl)sulfonyl)pyridin-2(1*H*)-one

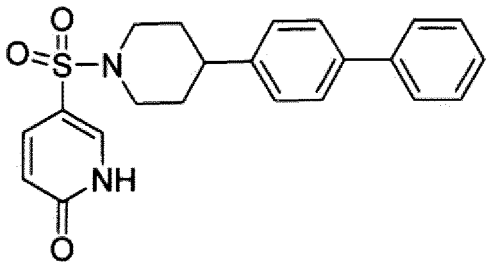

According to the general procedure for demethylation described in Reference Example 12 and using 5-((4-([1,1'-biphenyl]-4-yl)piperidin-1-yl)sulfonyl)-2-methoxypyridine (Reference Example 26) (18.4 mg, 0.045 mmol) as the methoxypyridine compound instead, the titled 5-((4-([1,1'-biphenyl]-4-yl)piperidin-1-yl)sulfonyl)pyridin-2(1*H*)-one was obtained as a brown solid (14.6 mg, 0.037 mmol, 82.2% yield).
$^1$H NMR (400 MHz, $CDCl_3$) δ 7.93 (1H, m), 7.67-7.70 (1H, m), 7.52-7.56 (4H, m), 7.40-7.42 (3H, m), 7.33-7.34 (2H, m), 6.64 (1H, d, J = 9.4 Hz), 3.90-3.93 (2H, m), 2.54-2.60 (3H, m), 1.86-2.01 (4H, m) .

Reference Example 32

Production of 5-(pyrrolidin-1-yl-sulfonyl)pyridin-2(1*H*)-one

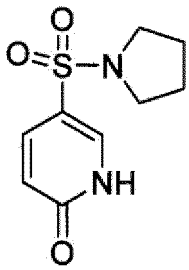

According to the general procedure for demethylation described in Reference Example 12 and using 2-methoxy-5-(pyrrolidin-1-yl-sulfonyl)pyridine (Reference Example 27) (4.2 g, 17.3 mmol) as the methoxypyridine compound instead, the titled 5-(pyrrolidin-1-yl-sulfonyl)pyridin-2(1*H*)-one was obtained as a white solid (3.9 g, 17.3 mmol, 100% yield).
$^1$H NMR (400 MHz, $CDCl_3$) δ 7.96 (d, J = 2.9 Hz, 1H), 7.73 (dd, J = 9.5, 2.9 Hz,1H), 6.63 (d, J = 9.5 Hz, 1H), 3.25-3.29 (m, 4H), 1.86-1.89 (m, 4H).

Reference Example 33

Production of 5-(thiazolidin-3-yl-sulfonyl)pyridin-2(1*H*)-one

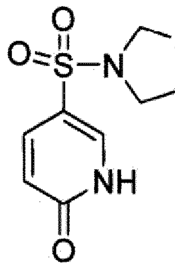

According to the general procedure for demethylation described in Reference Example 12 and using 3-((6-methoxypyridin-3-yl)sulfonyl)thiazolidine (Reference Example 28) (73.2 mg, 0.281 mmol) as the methoxypyridine compound instead, the titled 5-(thiazolidin-3-yl-sulfonyl)pyridin-2(1H)-one was obtained as a white solid (54.3 mg, 0.22 mmol, 78.4% yield).
$^1$H NMR (500 MHz, $CDCl_3$) δ 8.00 (d, J = 2.3 Hz, 1H), 7.71-7.73 (m, 1H), 6.60 (d, J = 9.7 Hz, 1H), 4.45 (m, 2H), 3.62-3.65 (m, 2H), 2.87-91 (m, 2H).

Production Example 5B

Production of 5-((4-([1,1':4',1":4",1'''-quaterphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one (KS-44)

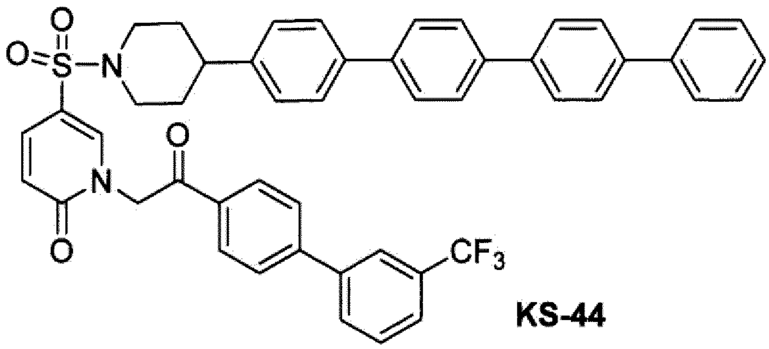

**KS-44**

According to the general procedure for alkylation reaction 1 of pyridinone compounds described in Production Example 1B and using 5-((4-([1,1':4',1":4",1'''-quaterphenyl]-4-yl)piperidin-1-yl)sulfonyl)pyridin-2(1*H*)-one (Reference Example 29) (10.7 mg, 0.0196 mmol) as the pyridinone compound instead, the titled 5-((4-([1,1':4',1":4",1'''-quaterphenyl]-yl)piperidin-1-yl)sulfonyl)-1-(2-oxo-2-(3'-(trifluoromethyl)-[1,1'-biphenyl]-4-yl)ethyl)pyridin-2(1H)-one (KS-44) was obtained as a brown oil (3.5 mg, 0.0432 mmol, 22% yield).
$^1$H NMR (400 MHz, $CDCl_3$) δ 8.12-8.14 (2H, m), 7.88-8.01 (2H, m), 7.81 (1H, m), 7.76 (3H, d, J = 8.5 Hz), 7.54-7.68 (12H, m), 7.41-7.43 (2H, m), 7.29-7.33 (3H, m), 7.21-7.26 (2H, m), 6.68-6.71 (1H, m), 5.49 (2H, s), 3.93 (2H, m), 2.64-2.67 (3H, m), 1.87-2.00 (4H, m). LRMS (ESI+) m/z 809 $[M + H]^+$.

Production Example 6B

Production of 1-(2-oxo-2-(3'-(trifluoromethyl)-[1,1'-biphenyl]-4-yl)ethyl)-5-((4-(4-(pyren-1-yl)phenyl)piperidin-1-yl)sulfonyl)pyridin-2(1*H*)-one (KS-45)

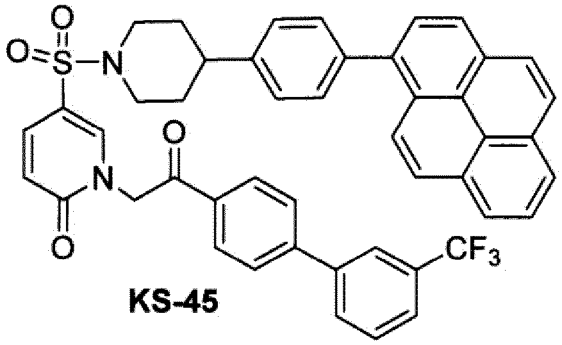

KS-45

According to the general procedure for alkylation reaction 1 of pyridinone compounds described in Production Example 1B and using 5-((4-(4-(pyren-1-yl)phenyl)piperidin-1-yl)sulfonyl)pyridin-2(1H)-one (Reference Example 30) (25.6 mg, 0.049 mmol) as the pyridinone compound instead, the titled 1-(2-oxo-2-(3'-(trifluoromethyl)-[1,1'-biphenyl]-4-yl)ethyl)-5-((4-(4-(pyren-1-yl)phenyl)piperidin-1-yl)sulfonyl)pyridin-2(1H)-one (KS-45) was obtained as a light brown oil (6.6 mg, 0.084 mmol, 17.2% yield).
$^1$H NMR (400 MHz, $CDCl_3$) $\delta$ 8.09-8.23 (7H, m), 7.88-8.05 (5H, m), 7.76-7.83 (3H, m), 7.59-7.72 (5H, m), 7.49-7.54 (1H, m), 7.38-7.40 (1H, m), 7.30-7.33 (1H, m), 6.67-6.73 (1H, m), 5.47-5.50 (2H, m), 3.91-4.04(2H, m), 2.58-2.73 (3H, m), 1.96-2.13 (4H, m). LRMS (ESI+) m/z 803 [M + Na]$^+$.

Production Example 7B

Production of 5-((4-([1,1'-biphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one (KS-43)

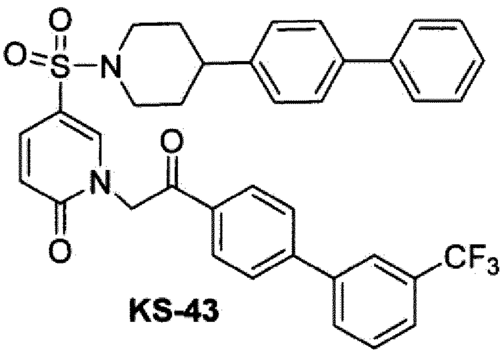

KS-43

5-((4-([1,1'-Biphenyl]-4-yl)piperidin-1-yl)sulfonyl)pyridin-2(1*H*)-one (Reference Example 31) (14.6 mg, 0.037 mmol, 1.2 equiv), potassium carbonate (9.1 mg, 0.066 mmol, 2.2 equiv), 2-bromo-1-(3'-(trifluoromethyl)-[1,1'-biphenyl]-4-yl)ethan-1-one (Reference Example 15) (10.3 mg, 0.03 mmol, 1.0 equiv), and tetrabutylammonium iodide (6.8 mg, 0.0185 mmol, 0.6 equiv) were added to MeCN (6 mL), and the mixture was stirred at 80°C for 2.5 hours. A saturated $Na_2S_2O_3$ aqueous solution was added to the mixture, and organic compounds were extracted with EtOAc. The combined organic layer was washed with water, dried over $Na_2SO_4$, filtered, and concentrated *in vacuo.* The residue was purified by column chromatography (EtOAc/$CH_2Cl_2$ = 0-50%) to give 5-((4-([1,1'-biphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one (KS-43) as a yellow solid (8.9 mg, 0.0136 mmol, 36.6% yield).
$^1$H NMR (400 MHz, $CDCl_3$) $\delta$ 8.12-8.14 (2H, m), 7.88-7.92 (2H, m), 7.76-7.81 (3H, m), 7.54-7.68 (7H, m), 7.41-7.43 (2H, m), 7.34-7.35 (1H, m), 7.28 (1H, m), 7.26 (1H, m), 6.68 (1H, d, J = 9.8 Hz), 5.49 (2H, s), 3.93-3.96 (2H, m), 2.61-2.70 (3H, m), 1.88-2.00 (4H, m); LRMS (ESI+) m/z 657 [M + H]$^+$.

Reference Example 34

Production of 1-(2-(4-bromophenyl)-2-oxoethyl)-5-(pyrrolidin-1-yl-sulfonyl)pyridin-2(1*H*)-one

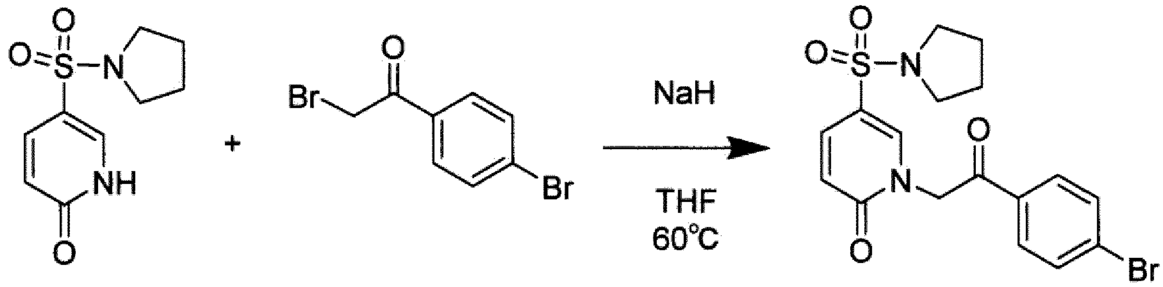


5-(Pyrrolidin-1-yl-sulfonyl)pyridin-2(1*H*)-one (Reference Example 32) (3.9 g, 17.3 mmol, 1.0 equiv) and sodium hydride (60% in mineral oil, 756 mg, 19.0 mmol, 1.1 equiv) were added to THF (110 mL), and the mixture was stirred at 60°C for 1 hour. Then, a solution of 2,4'-dibromoacetophenone (5.78 g, 20.8 mmol, 1.2 equiv) in THF (50 mL) was added, and the mixture was stirred at 60°C for 90 min. Organic compounds were extracted with $CH_2Cl_2$ from the reaction product. The combined organic layer was washed with water, dried over $Na_2SO_4$, filtered, and concentrated *in vacuo.* The residue was purified by column chromatography (EtOAc/$CH_2Cl_2$ = 0-58%) to give 1-(2-(4-bromophenyl)-2-oxoethyl)-5-(pyrrolidin-1-yl-sulfonyl)pyridin-2(1*H*)-one as a white solid (3.49 g, 8.21 mmol, 47.4% yield).
$^1$H NMR (500 MHz, Acetone): 8.27 (d, J = 2.6 Hz, 1H), 8.03-8.06 (m, 2H), 7.80-7.82 (m, 2H), 7.77 (dd, J = 9.5, 2.6 Hz, 1H), 6.55 (d, J = 9.5 Hz, 1H), 5.69 (s, 2H), 3.22-3.27 (m, 4H), 1.82-1.87 (m, 4H) .

Comparative Production Example 1

Production of 1-(2-oxo-2-(3'-(trifluoromethyl)-[1,1'-biphenyl]-4-yl)ethyl)-5-(thiazolidin-3-yl-sulfonyl)pyridin-2(1*H*)-one(KS-35)

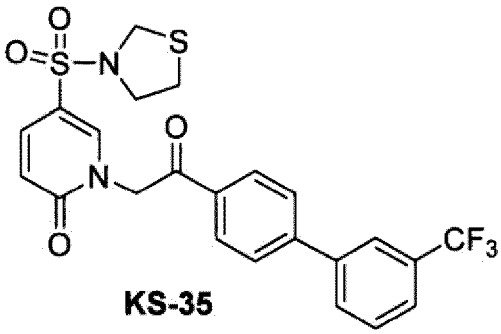

KS-35

According to the general procedure for alkylation reaction 1 of pyridinone compounds described in Production Example 1B and using 5-(thiazolidin-3-yl-sulfonyl)pyridin-2(1*H*)-one (Reference Example 33) (54.3 mg, 0.22 mmol) as the pyridinone compound instead, the titled 1-(2-oxo-2-(3'-(trifluoromethyl)-[1,1'-biphenyl]-4-yl)ethyl)-5-(thiazolidin-3-yl-sulfonyl)pyridin-2(1*H*)-one (KS-35) was obtained as a yellow solid (36.7 mg, 0.072 mmol, 65.5% yield).
$^1$H NMR (400 MHz, $CDCl_3$) $\delta$ 8.09 (2H, d, J = 8.5 Hz), 7.97-7.98 (1H, m), 7.86 (1H, s), 7.80 (1H, d, J = 7.6 Hz), 7.74 (2H, d, J = 8.5 Hz), 7.61-7.67 (3H, m), 6.61 (1H, d, J = 9.8 Hz), 5.47(2H, s), 4.49-4.50 (m, 2H), 3.65-3.67 (2H, m), 2.96-2.99 (2H, m).

Reference Example 35

Production of 1-(2-oxo-2-(4-(4,4,5,5-tetramethyl-1,3,2-dioxaborolan-2-yl)phenyl)ethyl)-5-(pyrrolidin-1-yl-sulfonyl)pyridin-2(1*H*)-one

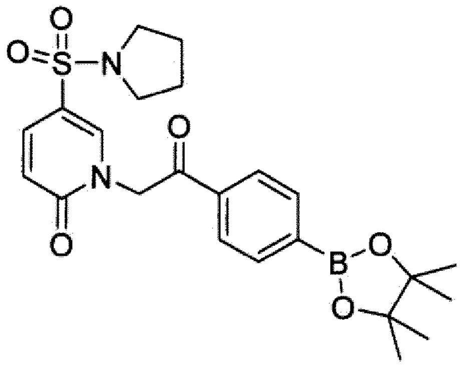

According to the general procedure for Miyaura-Ishiyama borylation and using 1-(2-(4-bromophenyl)-2-oxoethyl)-5-(pyrrolidin-1-yl-sulfonyl)pyridin-2(1*H*)-one produced in Reference Example 34 (2.0 g, 4.7 mmol) as the aryl bromide compound instead, the titled 1-(2-oxo-2-(4-(4,4,5,5-tetramethyl-1,3,2-dioxaborolan-2-yl)phenyl)ethyl)-5-(pyrrolidin-1-yl-sulfonyl)pyridin-2(1*H*)-one was obtained as a pale yellow solid (1.2 g, 2.55 mmol, 54.4% yield).
$^1H$ NMR (400 MHz, $CDCl_3$) $\delta$ 7.95 (4H, m), 7.88-7.89 (1H, m), 7.65-7.68 (1H, m), 6.63 (1H, d, J = 9.8 Hz), 5.45 (2H, s), 3.27 -3.31 (m, 4H), 1.90-1.91 (m, 4H), 1.36 (12H, s).

Reference Example 36

Production of (3-bromophenyl)(trifluoromethyl)sulfane

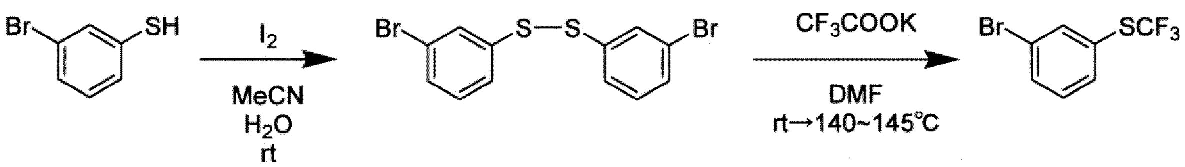


Step 1: To a solution of 3-bromobenzenethiol (1.89 g, 10 mmol, 1.0 equiv) in a liquid mixture of water and acetonitrile (water:acetonitrile = 1:5, 30 mL) was added iodine (1.27 g, 5 mmol, 0.5 equiv) at room temperature, and the reaction was immediately completed. A saturated $Na_2S_2O_3$ aqueous solution was added thereto, and organic compounds were extracted with $CH_2Cl_2$. The combined organic layer was dried over $Na_2SO_4$, filtered, and concentrated *in vacuo* to give 1,2-bis(3-bromophenyl)disulfane as a colorless solid (1.9 g, 100% yield) (Zeynizadeh, B.; J. Chem. Res. 2002, 11, 564).
$^1H$ NMR (400 MHz, $CDCl_3$) $\delta$ 7.62 (2H, s), 7.35-7.40 (4H, m), 7.14-7.19 (2H, m).

Step 2: To a solution of potassium trifluoroacetate (913 mg, 6 mmol, 1.2 equiv) in anhydrous DMF (12.5 mL) was added 1,2-bis(3-bromophenyl)disulfane obtained in step 1 (1.9 g, 5.0 mmol, 1.0 equiv) at room temperature. Then, the mixture was heated to 140 to 145°C overnight. This solution was poured into water and extracted with EtOAc/hexane (4/1). The combined organic layer was washed with water, dried over $Na_2SO_4$, filtered, and concentrated *in vacuo* to give the titled (3-bromophenyl)(trifluoromethyl)sulfane as a colorless solid (137.2 mg, 10.5% yield) (Roques, N. J. Fluor. Chem. 2001, 107, 311).
$^1H$ NMR (400 MHz, $CDCl_3$) $\delta$ 7.82 (1H, s), 7.61-7.62 (2H, m), 7.30-7.32 (1H, m).

Reference Example 37

Production of 4-bromo-*N*,*N*,2-trimethylaniline

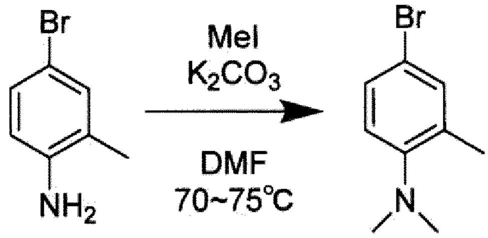


4-Bromo-2-methylaniline (930 mg, 5 mmol, 1.0 equiv) was added under stirring to a suspension of potassium carbonate (2.08 g, 15 mmol, 3.0 equiv) in DMF (7.5 mL). A solution of methyl iodide (0.95 mL, 15 mmol, 3.0 equiv) in DMF (7.5 mL) was gradually added, and the mixture was stirred for 2 hours at 70 to 75°C. Then, the reaction mixture was poured into water, and extracted with diethyl ether. The extract was washed with water, dried over $Na_2SO_4$, filtered, and concentrated *in vacuo* to give the titled 4-bromo-*N*,*N*,2-trimethylaniline as a pale yellow solid (338.7 mg, 1.58 mmol, 31.6% yield) (Kolchina, E.F.; Shekleina, N.V.; Shelkovnikov, V.V.; Russ. J. Org. Chem. 2011, 47, 855).
$^1$H NMR (400 MHz, $CDCl_3$) δ 7.21-7.26 (2H, m), 6.84-6.87 (1H, m), 2.64 (6H, s), 2.27 (3H, s).

Production Example 8B

General procedure for Suzuki-Miyaura reaction 3 (exemplified by the synthesis of 1-(2-oxo-2-(3'-((trifluoromethyl)thio)-[1,1'-biphenyl]-4-yl)ethyl)-5-(pyrrolidin-1-yl-sulfonyl)pyridin-2(1H)-one (KS-21))

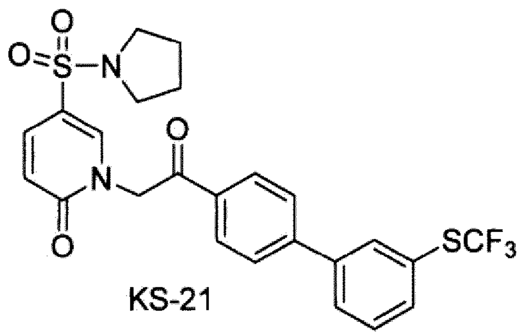

KS-21

A mixture of the aryl bromide compound (1,2-bis(3-bromophenyl)disulfane produced in Reference Example 36) (68.6 g, 0.267 mmol, 1.0 equiv), palladium(II) acetate (6.0 mg, 0.026 mmol, 10 mol%), 1,1'-bis(diphenylphosphino)ferrocene (14.8 mg, 0.026 mmol, 10 mol%), potassium phosphate (113.4 mg, 0.534 mmol, 2.0 equiv), and the dioxaborolane compound (1-(2-oxo-2-(4-(4,4,5,5-tetramethyl-1,3,2-dioxaborolan-2-yl)phenyl)ethyl)-5-(pyrrolidin-1-yl-sulfonyl)pyridin-2(1*H*)-one produced in Reference Example 35) (113.6 mg, 0.267 mmol, 1.0 equiv) in a liquid mixture of dioxane (3 mL) and degassed water (0.3 mL) was stirred at 100°C overnight. The mixture was diluted with water, and extracted with EtOAc. The combined organic layer was washed with water. The organic layer was dried over $Na_2SO_4$, filtered, and concentrated *in vacuo.* The residue was purified by column chromatography ($CH_2Cl_2$/EtOAc = 0-50%) to give 1-(2-oxo-2-(3'-((trifluoromethyl)thio)-[1,1'-biphenyl]-4-yl)ethyl)-5-(pyrrolidin-1-yl-sulfonyl)pyridin-2(1H)-one (KS-21) as a brown gummy material (79.8 mg, 0.152 mmol, 57.2% yield).
$^1$H NMR (400 MHz, $CDCl_3$) δ 8.09-8.10 (2H, m), 7.92-7.93 (2H, m), 7.73-7.75 (3H, m), 7.67-7.69 (2H, m), 7.53-7.57 (1H, m), 6.64 (1H, d, J = 9.4 Hz), 5.47 (2H, s), 3.29-3.32 (m, 4H), 1.90-1.91 (4H, m) .

Production Example 9B

Production of 1-(2-(4'-(dimethylamino)-3'-methyl-[1,1'-biphenyl]-4-yl)-2-oxoethyl)-5-(pyrrolidin-1-yl-sulfonyl)pyridin-2 (1*H*)-one (KS-16)

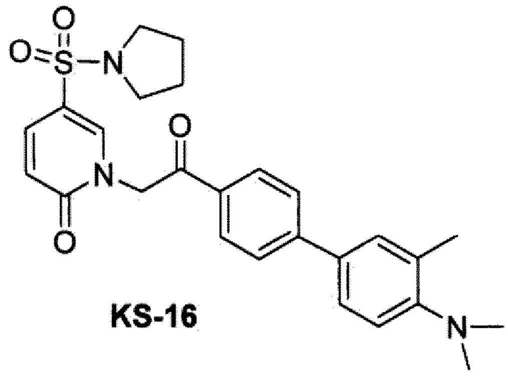

**KS-16**

According to the general procedure for Suzuki-Miyaura reaction 3 described in Production Example 8B and using 4-bromo-*N,N*,2-trimethylaniline (Reference Example 37) as the aryl bromide compound (45 mg) and 1-(2-oxo-2-(4-(4,4,5,5-tetramethyl-1,3,2-dioxaborolan-2-yl)phenyl)ethyl)-5-(pyrrolidin-1-yl-sulfonyl)pyridin-2(1*H*)-one (Reference Example 35) (55 mg) as the dioxaborolane compound instead, the titled 1-(2-(4'-(dimethylamino)-3'-methyl-[1,1'-biphenyl]-4-yl)-2-oxoethyl)-5-(pyrrolidin-1-yl-sulfonyl)pyridin-2(1*H*)-one (KS-16) was obtained as a yellow solid (17.5 mg, 0.0364 mmol, 34.4% yield).
$^1$H NMR (500 MHz, $CDCl_3$) δ 8.03-8.04 (2H, m), 7.91 (1H, m), 7.66-7.72 (3H, m), 7.42-7.46 (2H, m), 6.64 (1H, d, J = 9.7 Hz), 5.46 (2H, s), 3.30 (m, 4H), 1.90-1.91 (4H, m) .

Production Example 10B

Production of 1-(2-oxo-2-(4-(quinolin-7-yl)phenyl)ethyl)-5-(pyrrolidin-1-yl-sulfonyl)pyridin-2(1*H*)-one (KS-18)

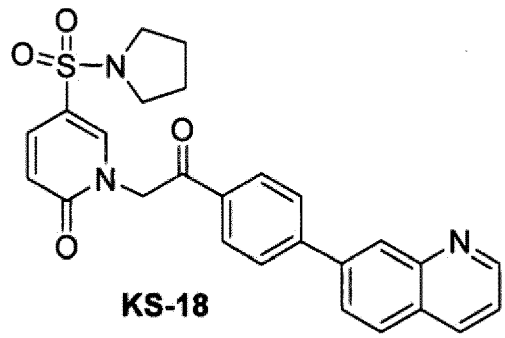

**KS-18**

According to the general procedure for Suzuki-Miyaura reaction 3 described in Production Example 8B and using 7-bromoquinoline (44 mg, 0.21 mmol) as the aryl bromide compound and 1-(2-oxo-2-(4-(4,4,5,5-tetramethyl-1,3,2-dioxaborolan-2-yl)phenyl)ethyl)-5-(pyrrolidin-1-yl-sulfonyl)pyridin-2(1*H*)-one (Reference Example 35) (50 mg, 0.105 mmol) as the dioxaborolane compound instead, the titled 1-(2-oxo-2-(4-(quinolin-7-yl)phenyl)ethyl)-·5-(pyrrolidin-1-yl-sulfonyl)pyridin-2(1*H*)-one (KS-18) was obtained as a light brown solid (35.7 mg, 0.0754 mmol, 71.1% yield).
$^1$H NMR (500 MHz, $CDCl_3$) δ 8.97 (1H, s), 8.38 (1H, s), 8.19 (1H, d, J = 8.0 Hz), 8.12 (2H, d, J = 8.0 Hz), 7.90-7.94 (4H, m), 7.83 (1H, d, J = 8.5 Hz), 7.66 (1H, d, J = 9.1 Hz), 7.45 (1H, m), 6.63 (1H, d, J = 9.1 Hz), 5.49 (2H, s), 3.30 (4H, m), 1.90 (4H, m).

Production Example 11B

Production of 1-(2-oxo-2-(4-(6-(trifluoromethyl)pyridin-2-yl)phenyl)ethyl)-5-(pyrrolidin-1-yl-sulfonyl)pyridin-2(1*H*)-one (KS-22)

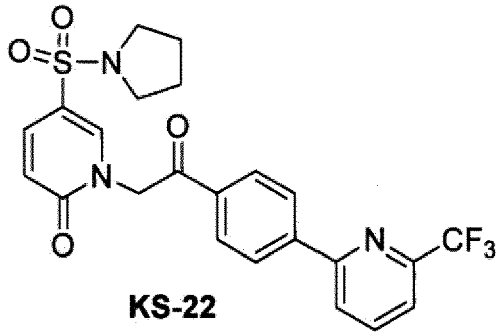

KS-22

According to the general procedure for Suzuki-Miyaura reaction 3 described in Production Example 9 and using 2-bromo-6-(trifluoromethyl)pyridine (71.9 mg, 0.31 mmol) as the aryl bromide compound instead, the titled 1-(2-oxo-2-(4-(6-(trifluoromethyl)pyridin-2-yl)phenyl)ethyl)-5-(pyrrolidin-1-yl-sulfonyl)pyridin-2(1*H*)-one (KS-22) was obtained as a brown solid (34.9 mg, 0.071 mmol, 70% yield).
$^1$H NMR (500 MHz, $CDCl_3$) $\delta$ 8.24-8.25 (2H, m), 8.12-8.13 (2H, m), 8.00-8.01 (2H, m), 7.91-7.92 (1H, m), 7.67-7.71 (2H, m), 6.65 (1H, d, J = 9.7 Hz), 5.48 (2H, s), 3.29-3.32 (4H, m), 1.90-1.92 (4H, m).

Production Example 12B

Production of 1-(2-oxo-2-(4-(quinolin-6-yl)phenyl)ethyl)-5-(pyrrolidin-1-yl-sulfonyl)pyridin-2(1*H*)-one (KS-23)

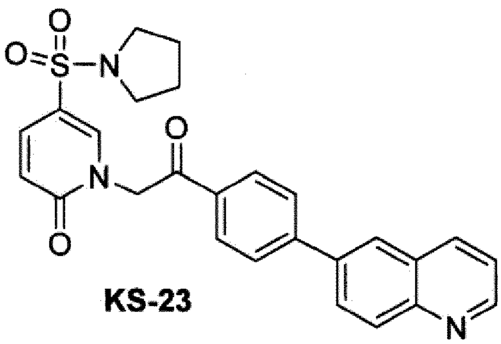

KS-23

According to the general procedure for Suzuki-Miyaura reaction 2 described in Production Example 8B and using 6-bromoquinoline (66.2 mg, 0.31 mmol) as the aryl bromide compound and 1-(2-oxo-2-(4-(4,4,5,5-tetramethyl-1,3,2-dioxaborolan-2-yl)phenyl)ethyl)-5-(pyrrolidin-1-yl-sulfonyl)pyridin-2(1*H*)-one (Reference Example 35) (50 mg, 0.105 mmol) as the dioxaborolane compound instead, the titled 1-(2-oxo-2-(4-(quinolin-6-yl)phenyl)ethyl)-5-(pyrrolidin-1-yl-sulfonyl)pyridin-2(1*H*)-one (KS-23) was obtained as a yellow solid (33.9 mg, 0.071 mmol, 67.5% yield).
$^1$H NMR (400 MHz, $CDCl_3$) $\delta$ 8.94-8.95 (1H, m), 8.20-8.22 (2H, m), 8.11-8.13 (2H, m), 8.07 (1H, m), 7.98-8.01 (1H, m), 7.94 (1H, m), 7.86-7.88 (2H, m), 7.65-7.68 (1H, m), 7.46-7.47 (1H, m), 6.63 (1H, d, J = 9.4 Hz), 5.49 (2H, s), 3.28-3.30 (4H, m), 1.90 (4H, m) .

Reference Example 38

Production of 4'-(2-(2-oxo-5-(pyrrolidin-1-yl-sulfonyl)pyridin-1(2*H*)-yl)acetyl)-[1,1'-biphenyl]-3-carbaldehyde

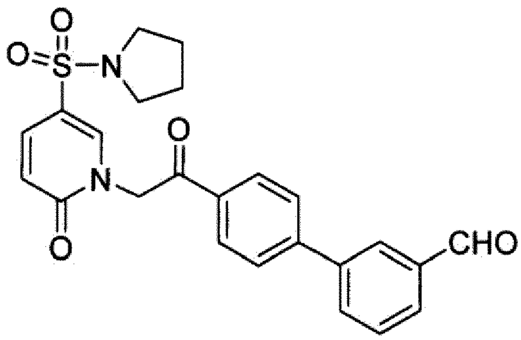

According to the general procedure for Suzuki-Miyaura reaction 2 described in Production Example 8B and using 3-bromobenzaldehyde (70 mg, 0.46 mmol) as the aryl bromide compound and 1-(2-oxo-2-(4-(4,4,5,5-tetramethyl-1,3,2-dioxaborolan-2-yl)phenyl)ethyl)-5-(pyrrolidin-l-yl-sulfonyl)pyridin-2(1*H*)-one (Reference Example 35) (100 mg, 0.211 mmol) as the dioxaborolane compound instead, the titled 4'-(2-(2-oxo-5-(pyrrolidin-1-yl-sulfonyl)pyridin-1(2*H*)-yl)acetyl)-[1,1'-biphenyl]-3-carbaldehyde was obtained as a white solid (19.7 mg, 0.0437 mmol, 18.6% yield). $^1$H NMR (400 MHz, $CDCl_3$) δ 10.12 (1H, s), 8.11-8.16 (3H, m), 7.91-7.93 (3H, m), 7.79-7.81 (2H, m), 7.67-7.70 (2H, m), 6.65 (1H, d, J = 9.8 Hz), 5.47 (2H, s), 3.30-3.31 (4H, m), 1.90-1.93 (4H, m).

Production Example 13B

Production of 1-(2-oxo-2-(3'-vinyl-[1,1'-biphenyl]-4-yl)ethyl)-5-(pyrrolidin-1-yl-sulfonyl)pyridin-2(1*H*)-one (KS-26)

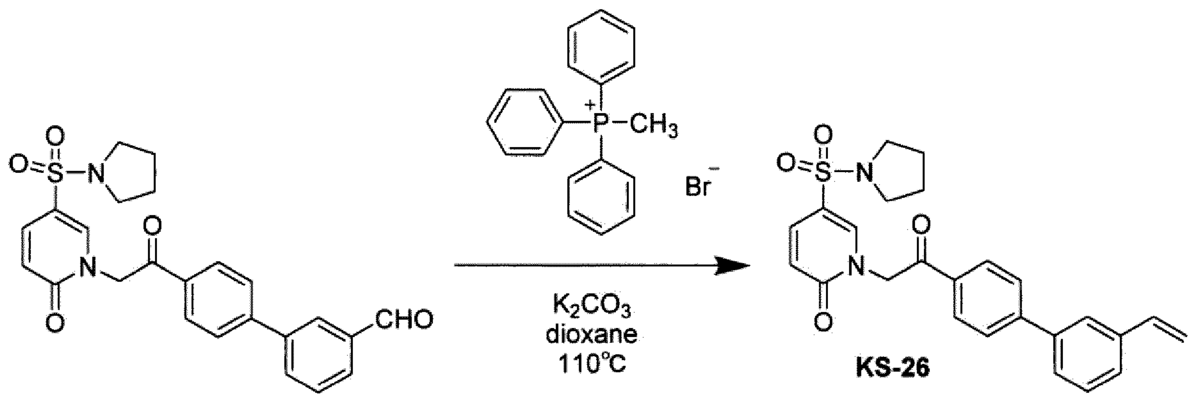


A mixture of 4'-(2-(2-oxo-5-(pyrrolidin-1-yl-sulfonyl)pyridin-1(2*H*)-yl)acetyl)-[1,1'-biphenyl]-3-carbaldehyde (50 mg, 0.11 mmol, 1.0 equiv), methyltriphenylphosphonium bromide (46.4 mg, 0.13 mmol, 1.2 equiv) and potassium carbonate (23.5 mg, 0.17 mmol, 1.5 equiv) in dioxane (2 mL) was stirred at 110°C overnight. The solvent was removed *in vacuo.* The residue was diluted EtOAc. The mixture was filtered through Celite, and concentrated *in vacuo.* The residue was purified by column chromatography ($CH_2Cl_2$/EtOAc = 0-100%) to give the titled 1-(2-oxo-2-(3'-vinyl-[1,1'-biphenyl]-4-yl)ethyl)-5-(pyrrolidin-1-yl-sulfonyl)pyridin-2(1*H*)-one (KS-26) as a yellow solid (6.8 mg, 0.0152 mmol, 13.8% yield). $^1$H NMR (500 MHz, $CDCl_3$) δ 8.08-8.09 (2H, m), 7.92 (1H, m), 7.75-7.77 (2H, m), 7.65-7.67 (3H, m), 7.43-7.52 (6H, m), 6.77 (1H, d, J = 10.9 Hz), 6.65 (1H, d, J = 9.7 Hz), 5.83 (1H, d, J = 17.8 Hz), 5.47 (2H, s), 5.33 (1H, d, J = 10.9 Hz), 3.30-3.31 (4H, m), 1.90-1.93 (4H, m).

Production Example 14B

Production method for 3-amino-1-(2-oxo-2-(3'-(trifluoromethyl)-[1,1'-biphenyl]-4-yl)ethyl)-5-(pyrrolidin-1-yl-sulfonyl)pyridin-2(1H)-one (RT-13)

(1) Production of 5-bromo-3-nitropyridin-2(1*H*)-one

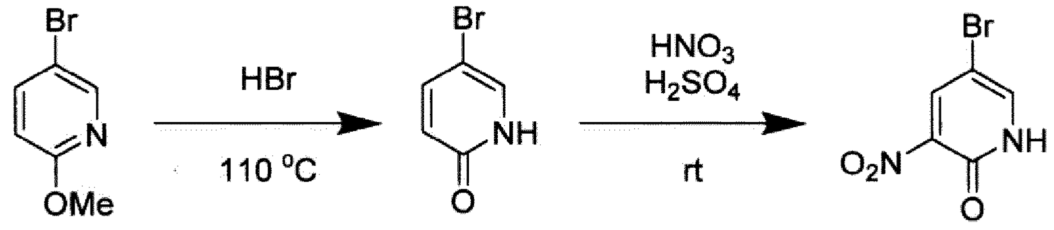


Step 1: 5-Bromo-2-methoxypyridine (95% purity, 507 μL, 4 mmol) and 48% HBr (8 mL) were heated together at 110°C for 4 hours. After the reaction mixture was cooled to room temperature, a saturated aqueous sodium bicarbonate solution was added and filtered. The filtrate was washed with water, 2-propanol, and diethyl ether. After separation of the organic layer, the aqueous layer was extracted with EtOAc, and the combined EtOAc layer was dried over $Na_2SO_4$ and filtered. Then, the solvent was evaporated to give 5-bromo-3-nitropyridin-2(1H)-one as a white solid (542.3 mg, 77% yield).
$^1$H NMR (400 MHz, $CDCl_3$) δ: 7.50 (2H, m), 6.51 (1H, m) .
Step 2: To a solution of 5-bromopyridin-2(1*H*)-one (1.75 g, 10.1 mmol) in sulfuric acid (10 mL) was added nitric acid (60-61%, 3.5 mL) at 0°C. The mixture was allowed to warm to room temperature, and stirred for 3 hours. The reaction mixture was poured into ice water, and the obtained precipitate was collected by filtration. The resulting product was washed with water and dried *in vacuo* to give 5-bromo-3-nitropyridin-2(1*H*)-one as a white solid (960 mg, 43% yield).
$^1$H NMR (500 MHz, $CDCl_3$) δ: 8.57 (s, 1H), 8.26 (s, 1H).

(2) Production of 5-bromo-3-nitro-1-(2-oxo-2-(3'-(trifluoromethyl)-[1,1'-biphenyl]-4-yl)ethyl)pyridin-2(1H)-one

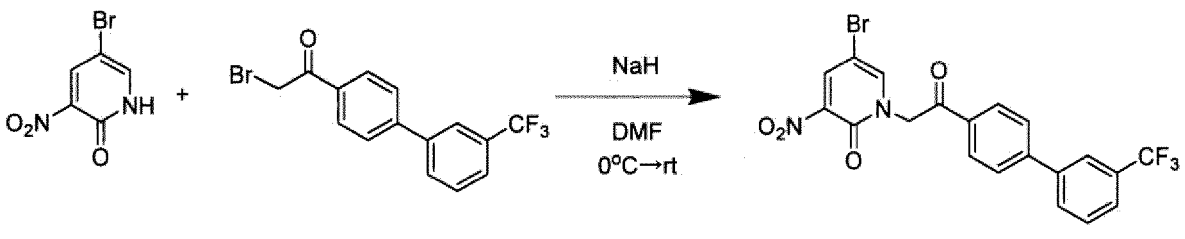


A flask containing 5-bromo-3-nitropyridin-2(1*H*)-one (601 mg, 2.7 mmol, 1.4 equiv) produced in (1) and sodium hydride (60% in mineral oil, 106 mg, 2.65 mmol, 1.4 equiv) was purged and filled with argon, and cooled to 0°C, to which DMF (4 mL) was added. The mixture was warmed to room temperature with stirring, 2-bromo-1-(3'-(trifluoromethyl)-[1,1'-biphenyl]-4-yl)ethan-1-one (640 mg, 1.9 mmol, 1.0 equiv) (Reference Example 15) was added and stirred for 30 min. After cooling to 0°C, a 1 M HCl aqueous solution was added, and insoluble materials were collected by filtration. The precipitate was taken up in EtOAc and concentrated *in vacuo*. The residue was purified by column chromatography (EtOAc/$CH_2Cl_2$ = 0-20%) to give the titled 5-bromo-3-nitro-1-(2-oxo-2-(3'-(trifluoromethyl)-[1,1'-biphenyl]-4-yl)ethyl)pyridin-2(1*H*)-one as a white solid (819 mg, 1.7 mmol, 90% yield).
$^1$H NMR (500 MHz, $CDCl_3$) δ 8.47 (1H, d, J = 2.2 Hz), 8.10 (2H, d, J = 8.0 Hz), 7.87 (1H, s), 7.80 (1H, d, J = 8.0 Hz), 7.76 (3H, m), 7.68 (1H, d, J = 8.0 Hz), 7.61 (1H, t, J = 8.0 Hz), 5.50 (2H, s).

(3) Production of 2-(5-bromo-2-oxo-1-(2-oxo-2-(3'-(trifluoromethyl)-[1,1'-biphenyl]-4-yl)ethyl)-1,2-dihydropyridin-3-yl)isoindoline-1,3-dione

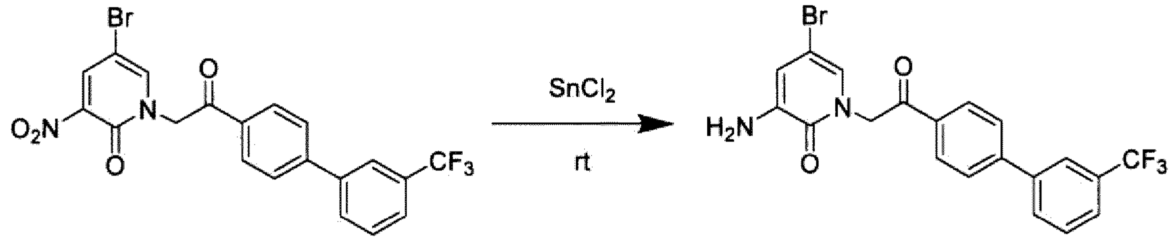


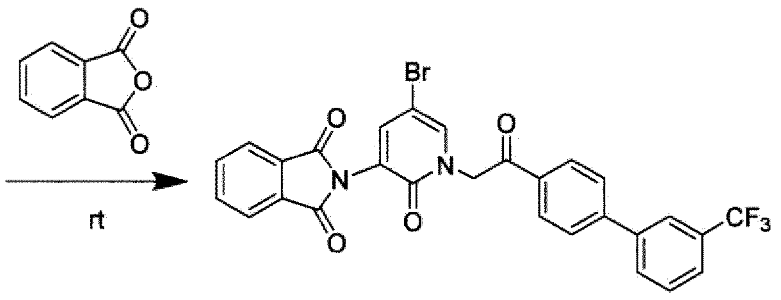


Step 1: To a solution of 5-bromo-3-nitro-1-(2-oxo-2-(3'-(trifluoromethyl)-[1,1'-biphenyl]-4-yl)ethyl)pyridin-2(1*H*)-one produced above in (2) (220 mg, 0.457 mmol, 1 equiv) in EtOAc (2 mL) was added $SnCl_2$ (250.5 mg, 1.3 mmol, 3 equiv) . The mixture was stirred at room temperature for 30 min. Thereafter, the solution was diluted with cold water and stirred for a few minutes, and then solid $K_2CO_3$ was added. The mixture was extracted with EtOAc/THF (3 times), washed with brine, dried over $Na_2SO_4$, filtered, and concentrated *in vacuo* to give crude amine. The crude amine was used for the next step without further purification.

$^1$H NMR (500 MHz, $CDCl_3$) $\delta$ 8.10 (2H, d, J = 8.0 Hz), 7.87 (1H, s), 7.80 (1H, d. J = 7.4 Hz), 7.73 (2H, d, J = 8.5 Hz), 7.67 (1H, d, J = 8.0 Hz), 7.59 (1H, m), 6.80 (1H, d, J = 2.2 Hz), 5.36 (2H, s) .

Step 2: A mixture of the crude amine obtained above (111 mg, 0.246 mmol, 1.0 equiv) and phthalic anhydride (427 mg, 0.27 mmol, 1.1 equiv) in toluene (2 mL) was stirred at 100°C overnight. The mixture was cooled to room temperature, and concentrated *in vacuo.* The residue was purified by column chromatography (EtOAc/hexane = 30-50%) to give 2-(5-bromo-2-oxo-1-(2-oxo-2-(3'-(trifluoromethyl)-[1,1'-biphenyl]-4-yl)ethyl)-1,2-dihydropyridin-3-yl)isoindoline-1,3-dione as a white solid (140 mg, 0.204 mmol, 82% yield).

$^1$H NMR (500 MHz, $CDCl_3$) $\delta$ 8.09 (2H, d, J = 8.0 Hz), 7.90-7.92 (2H, m), 7.84 (1H, s), 7.78 (1H, d, J = 8.0 Hz), 7.75-7.76 (2H, m), 7.71 (2H, d, J = 8.5 Hz), 7.66 (1H, d, J = 8.3 Hz), 7.58-7.63 (2H, m), 7.56 (1H, d, J = 2.2 Hz), 5.43 (2H, s).

<u>(4) Production of 2-(2-oxo-1-(2-oxo-2-(3'-(trifluoromethyl)-[1,1'-biphenyl]-4-yl)ethyl)-5-(pyrrolidin-1-yl-sulfonyl)-1,2-dihydropyridin-3-yl)isoindoline-1,3-dione</u>

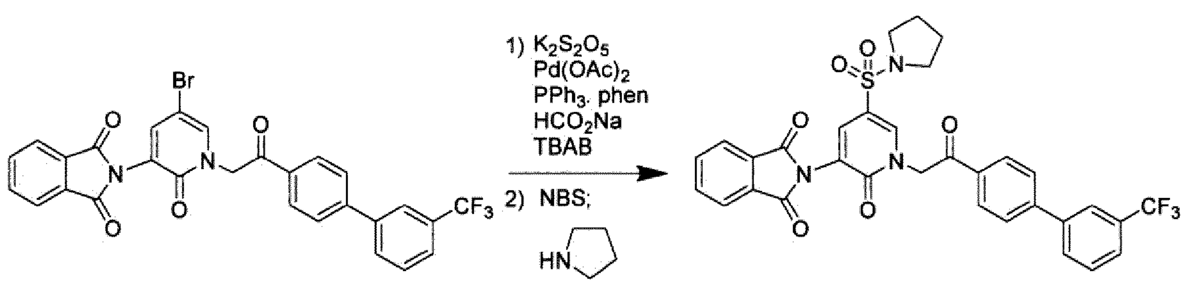


Sulfonamide was prepared by the following reported procedure (Shavnya, A.; Coffey, S.B.; Smith, A.C.; Mascitti, V.; Org. Lett. 2013, 15, 6226).

Step 1: A test tube was charged with 2-(5-bromo-2-oxo-1-(2-oxo-2-(3'-(trifluoromethyl)-[1,1'-biphenyl]-4-yl)ethyl)-1,2-dihydropyridin-3-yl)isoindoline-1,3-dione produced in (3) (122 mg, 0.209 mmol, 1.0 equiv), potassium metabisulfite (97.8 mg, 0.439 mmol, 2 equiv), tetrabutylammonium bromide (75.4 mg, 0.233 mmol, 1.1 equiv), sodium formate (31.3 mg, 0.459 mmol, 2.2 equiv), palladium acetate (2.3 mg, 0.01 mmol, 5 mol%), triphenylphosphine (8.2

mg, 0.031 mmol, 15 mol%), 1,10-phenanthroline (5.6 mg, 0.031 mmol, 15 mol%), and DMSO (0.5 mL). After the mixture was degassed by bubbling argon gas with stirring for 10 min, the mixture was stirred at 80°C for 4.5 h and then cooled to room temperature.

Step 2: A solution of pyrrolidine (2.0 equiv) in THF (0.5 mL) was added to the mixture obtained above in step 1, and the mixture was cooled to 0°C with an ice bath. While maintaining this temperature, N-bromosuccinimide (NBS) (74.3 mg, 418 mmol, 2.0 equiv) was added. The reaction mixture was warmed to room temperature and stirred overnight. The mixture was diluted with brine and water, and extracted with EtOAc (3 times). The obtained extract (organic layer) was dried over $Na_2SO_4$, filtered, and concentrated under reduced pressure. The crude residue was purified by column chromatography ($EtOAc/CH_2Cl_2$ = 0-10%) to give the titled 2-(2-oxo-1-(2-oxo-2-(3'-(trifluoromethyl)-[1,1'-biphenyl]-4-yl)ethyl)-5-(pyrrolidin-1-yl-sulfonyl)-1,2-dihydropyridin-3-yl)isoindoline-1,3-dione as a white solid (40 mg, 0.0629 mmol, 30% yield).

$^1H$ NMR (500 MHz, $CDCl_3$) δ 8.10 (2H, d, J = 8.0 Hz), 7.91 (2H, m), 7.83 (2H, s), 7.72-7.77 (4H, m), 7.52-7.69 (4H, m), 5.43 (2H, s), 3.31 (4H, m), 1.94 (4H, m).

(5) Production of 3-amino-1-(2-oxo-2-(3'-(trifluoromethyl)-[1,1'-biphenyl]-4-yl)ethyl)-5-(pyrrolidin-1-yl-sulfonyl)pyridin-2(1H)-one (RT-13)

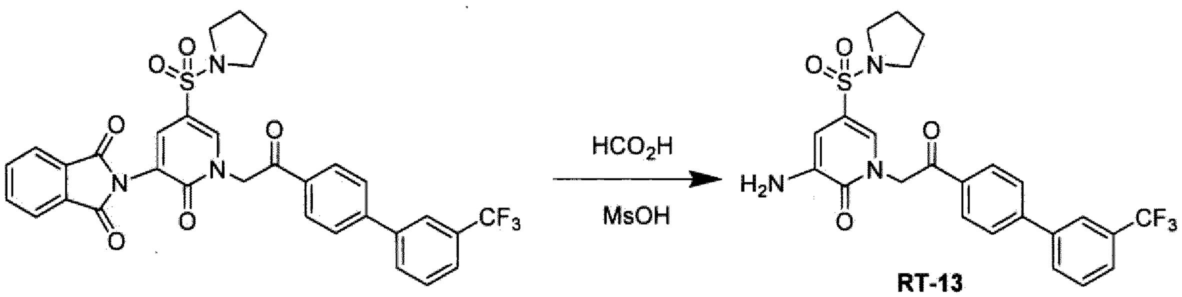


RT-13

A suspension of 2-(2-oxo-1-(2-oxo-2-(3'-(trifluoromethyl)-[1,1'-biphenyl]-4-yl)ethyl)-5-(pyrrolidin-1-yl-sulfonyl)-1,2-dihydropyridin-3-yl)isoindoline-1,3-dione (22.3 mg, 0.035 mmol) in formic acid (0.5 mL) and methanesulfonic acid (0.5 mL) was stirred at 50°C for 5 h. The reaction mixture was cooled to room temperature, and ice and a saturated aqueous $NaHCO_3$ solution were added to the mixture. The resulting mixture was extracted with EtOAc (2 times). The extract (organic layer) was dried over $Na_2SO_4$, filtered, and concentrated under reduced pressure. The crude residue was purified by column chromatography (EtOAc/hexane = 50-70%) to give the titled 3-amino-1-(2-oxo-2-(3'-(trifluoromethyl)-[1,1'-biphenyl]-4-yl)ethyl)-5-(pyrrolidin-1-yl-sulfonyl) pyridin-2(1H)-one (RT-13) as a white solid (13.7 mg, 0.027 mmol, 77% yield).

$^1H$ NMR (500 MHz, $CDCl_3$) δ 8.11 (2H, d, J = 1.7 Hz), 7.87 (1H, s), 7.81 (1H, d, J = 7.4 Hz), 7.76 (2H, d, J = 1.7 Hz), 7.68 (1H, d, J = 7.4 Hz), 7.60 (1H, t, J = 7.4 Hz), 7.37 (1H, d, J = 2.3 Hz) 6.79 (1H, d, J = 1.7 Hz), 5.47 (2H, s), 3.28 (4H, m), 1.87 (4H, m) .

Reference Example 39

Production of 1-(3-methyl-3'-(trifluoromethyl)-[1,1'-biphenyl]-4-yl)ethan-1-one

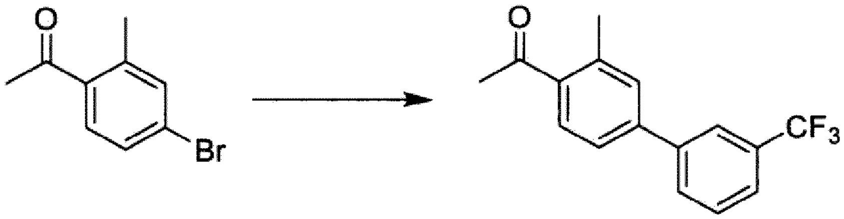

According to the general procedure for Suzuki-Miyaura reaction 2 described in Reference Example 15 and

using 1-(4-bromo-2-methylphenyl)ethan-1-one (144.1 µL, 0.94 mmol) and (3-(trifluoromethyl)phenyl)boronic acid (356.6 mg, 1.88 mmol) instead, the titled 1-(3-methyl-3'-(trifluoromethyl)-[1,1'-biphenyl]-4-yl)ethan-1-one was obtained as a white solid (232.1 mg, 0.834 mmol, 89% yield).
$^1H$ NMR (500 MHz, $CDCl_3$) δ 7.84 (br s, 1H), 7.81 (d, J = 8.0 Hz, 1H), 7.78 (d, J = 8.0 Hz, 1H), 7.64 (d, J = 8.1 Hz, 1H), 7.58 (dd, J = 8.1, 8.1 Hz, 1H), 7.49 (d, J = 8.0 Hz, 1H), 7.47 (br s, 1H), 2.63 (s, 3H), 2.625 (s, 3H).

Reference Example 40

General procedure for bromination by copper(II) bromide (exemplified by the synthesis of 2-bromo-1-(3-methyl-3'-(trifluoromethyl)-[1,1'-biphenyl]-4-yl)ethan-1-one)

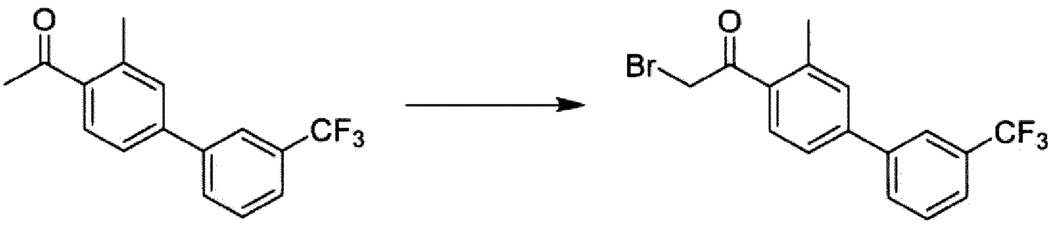

A mixture of copper(II) bromide (372.6 mg, 1.67 mmol, 2.0 equiv) in dry EtOAc (2.0 mL) was heated at a temperature higher than 80°C, to which a solution of the acetophenone compound (1-(3-methyl-3'-(trifluoromethyl)-[1,1'-biphenyl]-4-yl)ethan-1-one produced in Reference Example 39) (232.1 mg, 0.83 mmol, 1.0 equiv) in $CHCl_3$ (2.0 mL) was added and stirred overnight. The mixture was filtered and washed with EtOAc. The filtrate was washed with water and brine, dried over $Na_2SO_4$, filtered, and concentrated *in vacuo.* The residue was purified by column chromatography to give the titled 2-bromo-1-(3-methyl-3'-(trifluoromethyl)-[1,1'-biphenyl]-4-yl)ethan-1-one as a white solid (146.9 mg, 0.411 mmol, 49.0% yield).
$^1H$ NMR (500 MHz, $CDCl_3$) : δ 7.85 (br s, 1H), 7.80 (d, J = 8.6 Hz, 1H), 7.79 (d, J = 8.3 Hz, 1H), 7.66 (d, J = 8.3 Hz, 1H), 7.60 (dd, J = 8.3, 8.3 Hz, 1H), 7.52 (m, 3H), 4.46 (s, 2H), 2.63 (s, 3H) .

Production Example 15B

Production of 2-bromo-1-(3-methyl-3'-(trifluoromethyl)-[1,1'-biphenyl]-4-yl)ethan-1-one (AK-5)

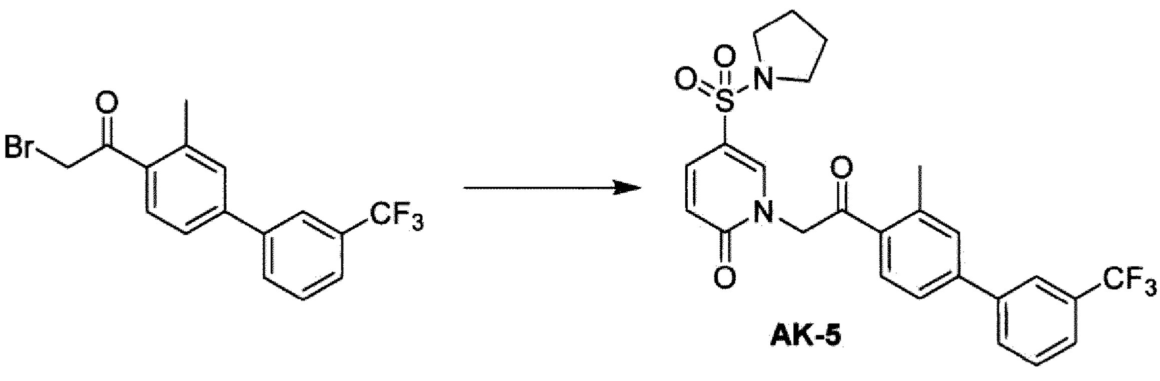

AK-5

5-(Pyrrolidin-1-yl-sulfonyl)pyridin-2(1*H*)-one (19.6 mg, 0.09 mmol, 1.0 equiv) and sodium hydride (50% in mineral oil, 6.2 mg, 0.13 mmol, 1.1 equiv) were added to dry THF (1.0 mL), and the mixture was stirred at 40°C for 10 min. Then, a solution of 2-bromo-1-(3-methyl-3'-(trifluoromethyl)-[1,1'-biphenyl]-4-yl)ethan-1-one (Reference Example 40) (36.7 mg, 0.1 mmol, 1.2 equiv) in THF (1.0 mL) was added to the reaction mixture, and the resulting mixture was stirred at 40°C for 1 hr. The reaction mixture was diluted with water, extracted with EtOAc, dried over $Na_2SO_4$, filtered, and concentrated *in vacuo.* The residue was purified by column chromatography to give the titled 2-bromo-1-(3-methyl-3'-(trifluoromethyl)-[1,1'-biphenyl]-4-yl)ethan-1-one (AK-5) as a colorless oil (7.6 mg, 0.015 mmol, 18.0% yield).

$^1H$ NMR (500 MHz, $CDCl_3$) δ 7.96 (d, J = 9.8 Hz, 1H), 7.95 (s, 1H), 7.86 (s, 1H), 7.80 (d, J = 8.1 Hz, 1H), 7.70-7.67 (m, 2H), 7.62-7.57 (m, 2H), 7.54 (s, 1H), 6.66 (d, J = 9.8 Hz, 1H), 5.34 (s, 2H), 3.32 (m, 4H), 2.62 (s, 3H), 1.90 (m, 4H); LRMS (ESI+) m/z527 [M + Na]$^+$.

Reference Example 41

Production of 1-(6-(3-(trifluoromethyl)phenyl)pyridin-3-yl)ethan-1-one

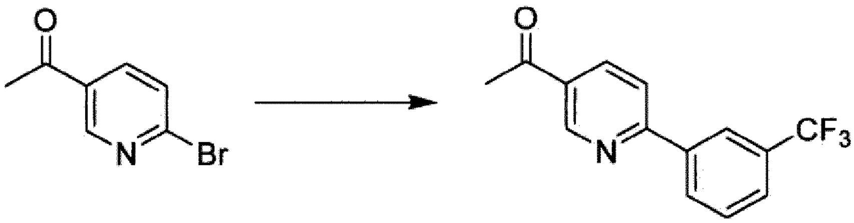

According to the general procedure for Suzuki-Miyaura reaction 2 described in Reference Example 15 and using 1-(6-bromopyridin-3-yl)ethan-1-one (250.0 mg, 1.2 mmol) as the aryl bromide compound and (3-(trifluoromethyl)phenyl)boronic acid (474.7 mg, 2.5 mmol) as the boronic acid compound instead, the titled 1-(6-(3-(trifluoromethyl)phenyl)pyridin-3-yl)ethan-1-one was obtained as a yellow solid (286.3 mg, 1.08 mmol, 87.0% yield).
$^1H$ NMR (400 MHz, $CDCl_3$) δ 9.26 (d, J = 2.3 Hz, 1H), 8.36 (d, J = 2.8 Hz, 1H), 8.34 (dd, J = 8.5, 2.3 Hz, 1H), 8.26 (br d, J = 8.4 Hz, 1H), 7.89 (d, J = 8.5 Hz, 1H), 7.73 (br d, J = 8.4 Hz, 1H), 7.64 (dd, J = 8.4, 8.4 Hz, 1H).

Reference Example 42

Production of 2-bromo-1-(6-(3-(trifluoromethyl)phenyl)pyridin-3-yl)ethan-1-one

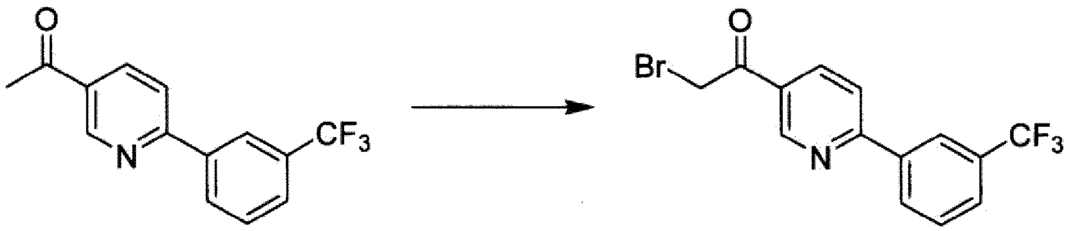

According to the general procedure for bromination by copper(II) bromide described in Reference Example 40 and using 1-(6-(3-(trifluoromethyl)phenyl)pyridin-3-yl)ethan-1-one produced in Reference Example 41 (482.2 mg, 2.2 mmol) as the acetophenone compound instead, the titled 2-bromo-1-(6-(3-(trifluoromethyl)phenyl)pyridin-3-yl)ethan-1-one was obtained as a brown solid (106.8 mg, 0.31 mmol, 29.0% yield).
$^1H$ NMR (400 MHz, $CDCl_3$) δ 9.29 (d, J = 2.3 Hz, 1H), 8.40-8.33 (m, 2H),8.27 (d, J = 6.4 Hz, 1H), 7.93 (d, J = 8.7 Hz, 1H), 7.75 (d, J = 7.8 Hz, 1H), 7.65 (dd, J = 7.8, 6.4 Hz, 1H), 4.47 (s, 2H).

Comparative Production Example 2

General procedure for alkylation reaction 2 of pyridinone compound (exemplified by the synthesis of 1-(2-oxo-2-(6-(3-(trifluoromethyl)phenyl)pyridin-3-yl)ethyl)-5-(pyrrolidin-1-yl-sulfonyl)pyridin-2(1H)-one (AK-6))

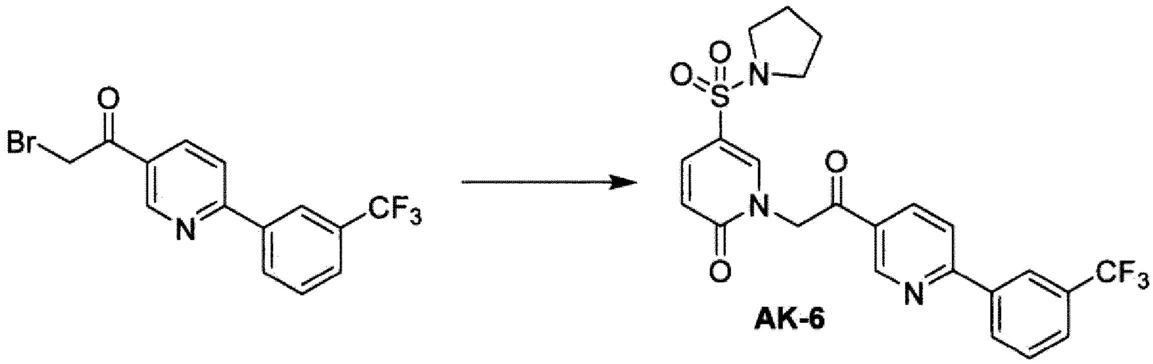


A suspension of the aryl bromide compound (2-bromo-1-(6-(3-(trifluoromethyl)phenyl)pyridin-3-yl)ethan-1-one produced in Reference Example 42) (106.8 mg, 0.31 mmol, 1.5 equiv), 5-(pyrrolidin-1-yl-sulfonyl)pyridin-2(1H)-one (Reference Example 32) (47.2 mg, 0.21 mmol, 1.0 equiv), and potassium carbonate (62.9 mg, 0.46 mmol, 2.2 equiv) in dry MeCN (13.8 mL) was stirred at room temperature overnight. The reaction mixture was diluted with water, extracted with $CH_2Cl_2$, and washed with brine. The organic layer was dried over $Na_2SO_4$, filtered, and concentrated *in vacuo.* The residue was purified by column chromatography to give the titled 1-(2-oxo-2-(6-(3-(trifluoromethyl)phenyl)pyridin-3-yl)ethyl)-5-(pyrrolidin-1-yl-sulfonyl)pyridin-2(1*H*)-one (AK-6) as an orange solid (56.1 mg, 0.114 mmol, 55.0% yield).
$^1$H NMR (500 MHz, $CDCl_3$) δ 9.33 (br s, 1H), 8.39 (m, 2H), 8.27 (br d, J = 6.4 Hz, 1H), 7.97 (d, J = 8.6 Hz, 1H), 7.94 (d, J = 2.9 Hz, 1H), 7.77 (d, J = 8.5 Hz, 1H), 7.72-7.65 (m, 2H), 6.67 (d, J = 9.2 Hz, 1H), 5.45 (s, 2H), 3.33 (m, 4H), 1.93 (m, 4H).

Reference Example 43

Production of 1-(4-bromo-2-methoxyphenyl)ethan-1-one

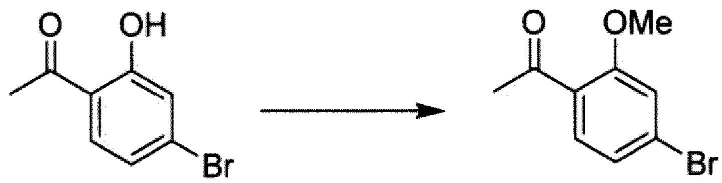

To a solution of 1-(4-bromo-2-hydroxyphenyl)ethan-1-one (200 mg, 0.9 mmol, 1.0 equiv) in dry DMF (9 mL) were added potassium carbonate (192.8 mg, 1.4 mmol, 1.5 equiv) and methyl iodide (115.9 μL, 1.9 mmol, 2.0 equiv), and the mixture was stirred for 3.5 h at room temperature. The mixture was poured into water, and extracted with EtOAc. The extract (organic layer) was washed with brine, dried over $Na_2SO_4$, filtered, and concentrated *in vacuo.* The residue was purified by column chromatography to give the titled 1-(4-bromo-2-methoxyphenyl)ethan-1-one as a white solid (238.4 mg, 1.0 mmol, 100% yield).
$^1$H NMR (400 MHz, $CDCl_3$) 57.63 (d, J = 7.4 Hz, 1H), 7.16 (d, J = 1.8 Hz, 1H), 7.13 (dd, J = 7.4, 1.8 Hz, 1H), 3.92 (s, 3H), 2.59 (s, 3H).

Reference Example 44

Production of 2-bromo-1-(4-bromo-2-methoxyphenyl)ethan-1-one

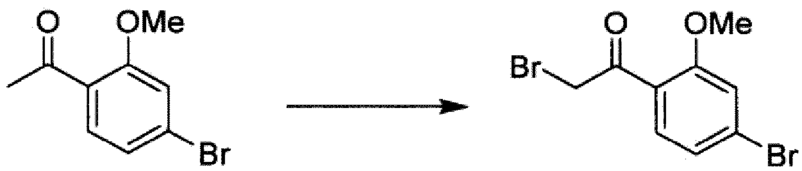

According to the general procedure for bromination by copper(II) bromide described in Reference Example 40 and using 1-(4-bromo-2-methoxyphenyl)ethan-1-one (Reference Example 43) (311 mg, 1.4 mmol) instead, the titled 2-bromo-1-(4-bromo-2-methoxyphenyl)ethan-1-one was obtained as a pale yellow solid (387.2 mg, 1.26 mmol, 93.0% yield).
$^1$H NMR (400 MHz, $CDCl_3$) δ7.71 (d, J = 8.1 Hz, 1H), 7.20 (dd, J = 8.1, 1.5 Hz, 1H), 7.15 (d, J = 1.5 Hz, 1H), 4.55 (s, 2H), 3.96 (s, 3H).

Reference Example 45

Production of 1-(2-(4-bromo-2-methoxyphenyl)-2-oxoethyl)-5-(pyrrolidin-1-yl-sulfonyl)pyridin-2(1*H*)-one

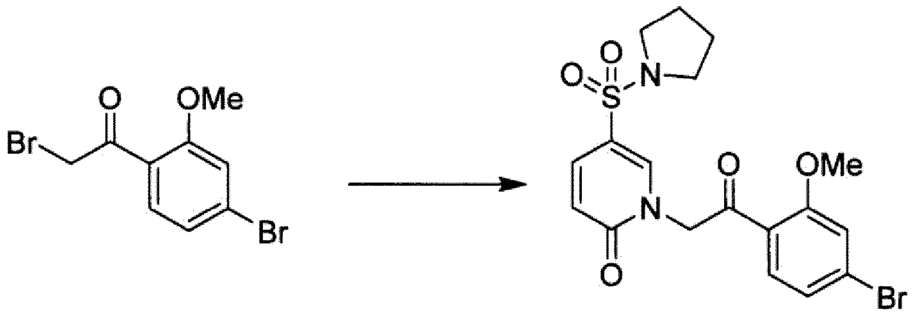

According to the general procedure for alkylation reaction 2 of pyridinone compound described in Production Example 15B and using 2-bromo-1-(4-bromo-2-methoxyphenyl)ethan-1-one (Reference Example 44) (387.2 mg, 1.26 mmol) as the aryl bromide compound instead, the titled 1-(2-(4-bromo-2-methoxyphenyl)-2-oxoethyl)-5-(pyrrolidin-1-yl-sulfonyl)pyridin-2(1*H*)-one was obtained as a pale yellow solid (175 mg, 0.38 mmol, 46.0% yield). $^1$H NMR (500 MHz, $CDCl_3$) δ7.87 (d, J = 2.3 Hz, 1H), 7.78 (d, J = 8.0 Hz, 1H), 7.65 (dd, J = 9.2, 2.3 Hz, 1H), 7.21 (m, 2H), 6.62 (d, J = 9.7 Hz, 1H), 5.31 (s, 2H), 4.01 (s, 3H), 3.29 (m, 4H), 1.90 (m, 4H).

Production Example 16B

Production of 1-(2-(3-methoxy-3'-(trifluoromethyl)-[1,1'-biphenyl]-4-yl)-2-oxoethyl)-5-(pyrrolidin-1-yl-sulfonyl)pyridin-2(1H)-one (AK-14)

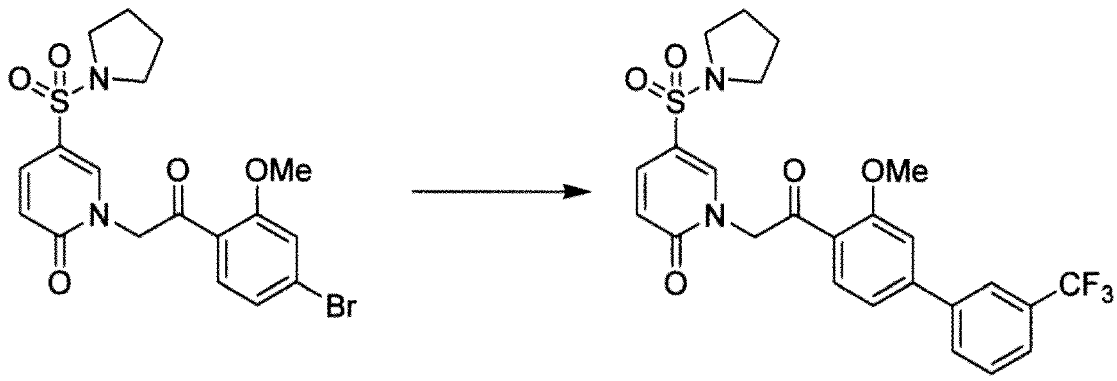

According to the general procedure for Suzuki-Miyaura reaction 2 described in Reference Example 15 and using 1-(2-(4-bromo-2-methoxyphenyl)-2-oxoethyl)-5-(pyrrolidin-1-yl-sulfonyl)pyridin-2(1*H*)-one (Reference Example 45) (175 mg, 0.38 mmol) as the aryl bromide compound and (3-(trifluoromethyl)phenyl)boronic acid (146 mg, 0.77 mmol) as the boronic acid compound instead, the titled 1-(2-(3-methoxy-3'-(trifluoromethyl)-[1,1'-biphenyl]-4-yl)-2-oxoethyl)-5-(pyrrolidin-1-yl-sulfonyl)pyridin-2(1*H*)-one (AK-14) was obtained as a white amorphous solid (165 mg, 0.509 mmol, 82.0% yield).

$^1$H NMR (500 MHz, $CDCl_3$) δ8.01 (d, J = 8.0 Hz, 1H), 7.91 (d, J = 2.6 Hz, 1H), 7.85 (br s, 1H), 7.79 (d, J = 7.9 Hz, 1H), 7.69 (d, J = 7.9 Hz, 1H), 7.66 (dd, J = 9.5, 2.6 Hz, 1H), 7.62 (dd, J = 7.9, 7.9 Hz, 1H), 7.28 (dd, J = 8.0, 1.8 Hz, 1H), 7.20 (d, J = 1.8 Hz, 1H), 7.63 (d, J = 9.5 Hz, 1H), 5.40 (s, 2H), 4.10 (s, 3H), 3.30 (m, 4H), 1.91 (m, 4H).

Production Example 17B

Production of 1-(2-(3-hydroxy-3'-(trifluoromethyl)-[1,1'-biphenyl]-4-yl)-2-oxoethyl)-5-(pyrrolidin-1-yl-sulfonyl)pyridin-2(1H)-one (AK-15)

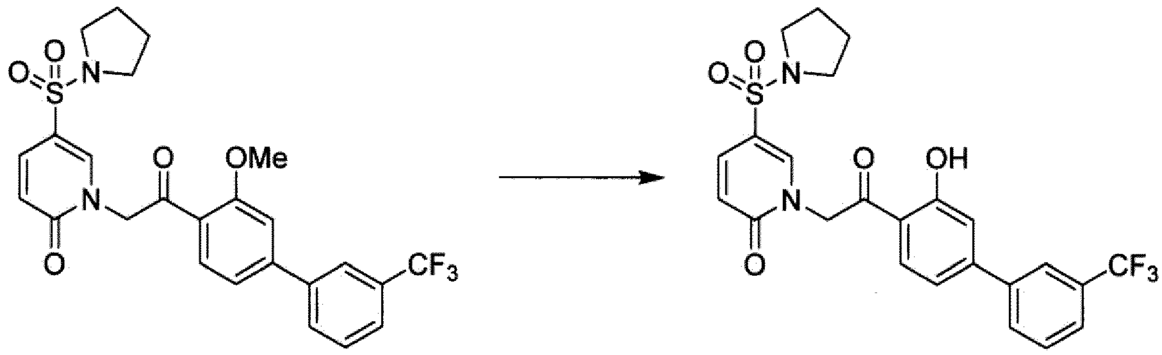

A mixture of 1-(2-(3-methoxy-3'-(trifluoromethyl)-[1,1'-biphenyl]-4-yl)-2-oxoethyl)-5-(pyrrolidin-1-yl-sulfonyl)pyridin-2(1*H*)-one (AK-14) (55.0 mg, 0.1 mmol, 1.0 equiv) in dry $CH_2Cl_2$ (1.0 mL) was cooled to -78°C, to which boron tribromide (11 μL, 1.1 mmol, 1.1 equiv) was added, followed by stirring for 2 h. Boron tribromide (20 μL, 2 mmol, 2.0 equiv) was further added to the mixture, stirred overnight, and allowed to warm to room temperature. After reaction, MeOH was added to the mixture, and stirred for 30 min at room temperature. The solvent was removed *in vacuo.* The resulting product was diluted with EtOAc, washed with water, dried over $Na_2SO_4$, filtered, and concentrated *in vacuo.* The residue was purified by column chromatography to give the titled 1-(2-(3-hydroxy-3'-(trifluoromethyl)-[1,1'-biphenyl]-4-yl)-2-oxoethyl)-5-(pyrrolidin-1-yl-sulfonyl)pyridin-2(1H)-one (AK-15) as a white amorphous solid (52.2 mg, 0.103 mmol, 97.0% yield).
$^1$H NMR (500 MHz, $CDCl_3$) δ11.53 (s, 1H), 7.93 (d, J = 2.3 Hz, 1H), 7.89 (d, J = 8.6 Hz, 1H), 7.88 (br s, 1H), 7.82 (d, J = 8.0 Hz, 1H), 7.70 (m, 2H), 7.62 (dd, J = 8.0, 8.0 Hz, 1H), 7.28 (m, 1H), 7.24 (d, J = 1.7 Hz, 1H), 6.68 (d, J = 9.8 Hz, 1H), 5.49 (s, 2H), 3.30 (m, 4H), 1.89 (m, 4H).

Production Example 18B

Production of 1-(2-hydroxy-2-(3-hydroxy-3'-(trifluoromethyl)-[1,1'-biphenyl]-4-yl)ethyl)-5-(pyrrolidin-1-yl-sulfonyl)pyridin-2(1H)-one (AK-16)

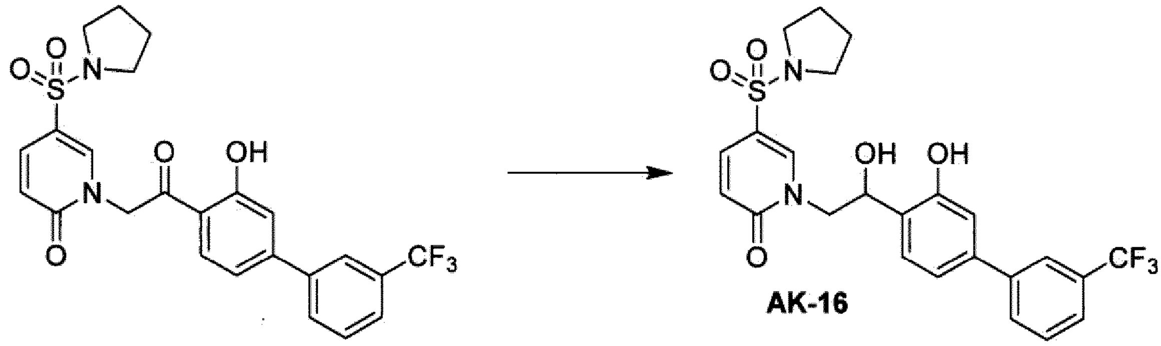

AK-16

A mixture of 1-(2-(3-hydroxy-3'-(trifluoromethyl)-[1,1'-biphenyl]-4-yl)-2-oxoethyl)-5-(pyrrolidin-1-yl-sulfonyl)pyridin-2(1*H*)-one (AK-15) (26.1 mg, 0.05 mmol, 1.0 equiv) in dry MeOH (0.5 mL) was cooled to 0°C, to which sodium borohydride (3.9 mg, 0.1 mmol, 2.0 equiv) was added, followed by stirring for 1.5 h. After reaction, the solvent was removed *in vacuo.* The resulting product was diluted with water, extracted with $CH_2Cl_2$, washed with brine, dried over $Na_2SO_4$, filtered, and concentrated *in vacuo.* The residue was purified by column chromatography to give the titled 1-(2-hydroxy-2-(3-hydroxy-3'-(trifluoromethyl)-[1,1'-biphenyl]-4-yl)ethyl)-5-(pyrrolidin-1-yl-sulfonyl)pyridin-2(1*H*)-one (AK-16) as a white solid (10.8 mg, 0.021 mmol, 41.0% yield).
$^1$H NMR (500 MHz, acetone-$d_6$) δ8.15 (d, J = 2.9 Hz, 1H), 7.91 (m, 2H), 7.73-7.69 (m, 3H), 7.51 (d, J = 8.0 Hz, 1H), 7.52 (dd, J = 8.1, 1.8 Hz, 1H), 6.54 (d, J = 9.2 H1z, 1H), 5.39 (dd, J = 8.0, 3.4 Hz, 1H), 4.54 (dd, J = 13.5, 3.5 Hz, 1H), 7.23 (dd, J = 13.5, 8.0 Hz, 1H), 3.16 (m, 4H), 1.75 (m, 4H).

Production Example 19B

Production of 1-((2,2-dimethyl-7-(3-(trifluoromethyl)phenyl)-4H-benzo[d][1,3]dioxin-4-yl)methyl)-5-(pyrrolidin-1-yl-sulfonyl)pyridin-2(1H)-one (AK-17)

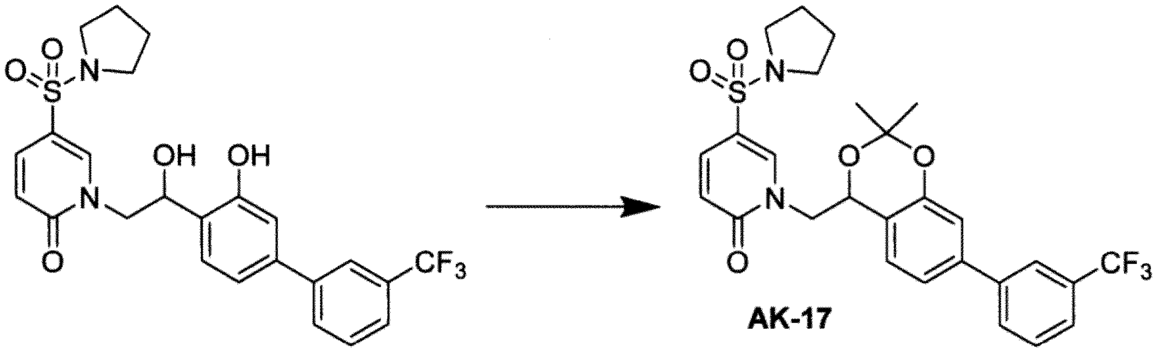

To a solution of 1-(2-hydroxy-2-(3-hydroxy-3'-(trifluoromethyl)-[1,1'-biphenyl]-4-yl)ethyl)-5-(pyrrolidin-1-yl-sulfonyl)pyridin-2(1H)-one (10.8 mg, 0.02 mmol, 1.0 equiv) in dry acetone (1 mL) were added 2,2-dimethoxypropane (52 μL, 0.2 mmol, 20 equiv) and pyridinium p-toluenesulfonate (1 mg, 0.04 mmol, 0.2 equiv), and the mixture was stirred for 12 hours at room temperature. The mixture was poured into saturated aqueous $NaHCO_3$ solution, and extracted with EtOAc. The extract was washed with brine, dried over $Na_2SO_4$, filtered, and concentrated under reduced pressure. The residue was purified by column chromatography to give 1-((2,2-dimethyl-7-(3-(trifluoromethyl)phenyl)-4H-benzo[d][1,3]dioxin-4-yl)methyl)-5-(pyrrolidin-1-yl-sulfonyl)pyridin-2(1H)-one (AK-17) as a white solid (7.4 mg, 0.013 mmol, 70.0% yield). $^1$H NMR (400 MHz, $CDCl_3$) δ8.18 (d, J = 2.2 Hz, 1H), 7.80 (s, 1H), 7.74 (d, J = 7.6 Hz, 1H), 7.63-7.60 (m, 2H), 7.57-7.52 (m, 1H), 7.38 (d, J = 8.0 Hz, 1H), 7.22 (dd, J =8.0,1.8 Hz, 1H), 7.07 (d, J = 1.3 Hz, 1H), 6.62 (d, J = 9.8 Hz, 1H), 5.30-27 (m, 1H), 4.94 (dd, J =13.5,1.8 Hz, 1H), 3.93 (dd, J =13.5,8.5 Hz, 1H), 3.24 (m, 4H), 1.84 (m, 4H), 1.59 (s, 3H), 1.45 (s, 3H).

Production Example 20B

Production of 1-(2-(3-isobutoxy-3'-(trifluoromethyl)-[1,1'-biphenyl]-4-yl)-2-oxoethyl)-5-(pyrrolidin-1-yl-sulfonyl)pyridin-2(1H)-one (AK-22)

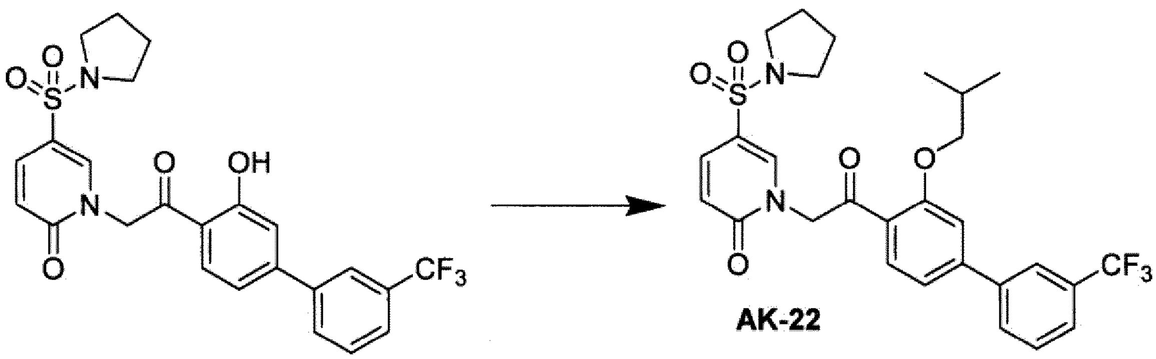
AK-22

To a mixture of 1-(2-(3-hydroxy-3'-(trifluoromethyl)-[1,1'-biphenyl]-4-yl)-2-oxoethyl)-5-(pyrrolidin-1-yl-sulfonyl)pyridin-2(1H)-one (37.8 mg, 0.07 mmol, 1.0 equivalent) in dry DMF (0.75 mL) were added potassium carbonate (41.3 mg, 0.3 mmol, 4.0 equiv) and 1-iodo-2-methylpropane (34.4 μL, 0.3 mmol, 4.0 equiv), and the mixture was stirred for 24 hours at room temperature. After the reaction, the mixture was diluted with water, extracted with EtOAc, washed with brine, dried over $Na_2SO_4$, filtered, and concentrated under reduced pressure. The residue was purified by column chromatography to give 1-(2-(3-isobutoxy-3'-(trifluoromethyl)-[1,1'-biphenyl]-4-yl)-2-oxoethyl)-5-(pyrrolidin-1-yl-sulfonyl)pyridin-2(1H)-one (AK-22) as a colorless oil (16.4 mg, 0.029 mmol, 44.0% yield).
$^1H$ NMR (500 MHz, $CDCl_3$) δ8.01 (d, J = 8.5 Hz, 1H), 7.91 (d, J = 2.2 Hz, 1H), 7.83 (s, 1H), 7.79 (d, J = 7.4 Hz, 1H), 7.69-7.65 (m, 2H), 7.62-7.59 (m, 1H), 7.26 (m, 1H), 7.17 (m, 1H), 6.64 (d, J = 9.7 Hz, 1H), 5.42 (s, 2H), 4.03 (d, J =6.8 Hz, 2H), 3.31 (m, 4H), 2.33-2.25 (m, 1H), 1.91 (m, 4H), 1.16 (d, J = 6.8 Hz, 6H).

Production Example 21B

Production of 1-(1-(3-(hexyloxy)-3'-(trifluoromethyl)-[1,1'-biphenyl]-4-yl)-1-oxooctan-2-yl)-5-(pyrrolidin-1-yl-sulfonyl)pyridin-2(1H)-one (AK-23)

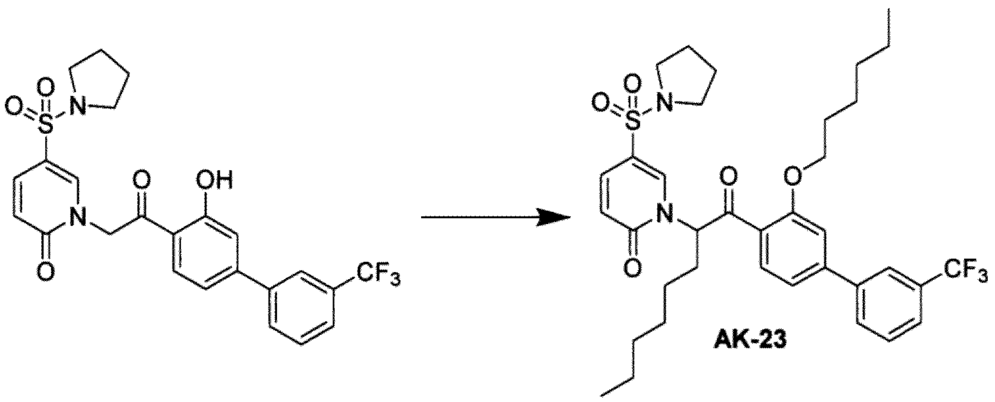
AK-23

To a solution of 1-(2-(3-hydroxy-3'-(trifluoromethyl)-[1,1'-biphenyl]-4-yl)-2-oxoethyl)-5-(pyrrolidin-1-yl-sulfonyl)pyridin-2-(1H)-one (37.8 mg, 0.07 mmol, 1.0 equiv) and potassium carbonate (41.3 mg, 0 .30 mmol, 4.0 equiv) in dry DMF (0.75 mL) was added 1-iodohexane (44.1 μL, 0.3 mmol, 4.0 equiv), and the mixture was stirred for 24 hours at room temperature. The mixture was poured into water, and extracted with EtOAc. The extract was washed with brine, dried over $Na_2SO_4$, filtered, and concentrated under reduced pressure. The residue was purified by column chromatography to give 1-(1-(3-(hexyloxy)-3'-(trifluoromethyl)-[1,1'-biphenyl]-4-yl)-1-oxooctan-2-yl)-5-(pyrrolidin-1-yl-sulfonyl)pyridin-2(1H)-one (AK-23) as a colorless oil (12.5 mg, 0.018 mmol, 26.4% yield).
$^1H$ NMR (500 MHz, $CDCl_3$) δ8.01 (d, J = 2.8 Hz, 1H), 7.85-7.81 (m, 2H), 7.77 (d, J = 8.0 Hz, 1H), 7.68 (d, J = 8.0 Hz, 1H), 7.61-7.60 (m, 2H), 7.23 (d, J = 6.8 Hz, 1H), 7.13 (s, 1H), 6.58 (d, J = 4.0 Hz, 1H), 4.25-4.18 (m, 2H), 3.31 (m, 4H), 2.24 (m, 1H), 2.01-2.00 (m, 2H), 1.88 (m, 4H), 1.39-1.22 (m, 18H), 0.93 (t, J = 6.8 Hz, 3H), 0.86 (d, J = 6.8 Hz, 3H).

Production Example 22B

Production of 1-(2-oxo-2-(3-phenethoxy-3'-(trifluoromethyl)-[1,1'-biphenyl]-4-yl)ethyl)-5-(pyrrolidin-1-yl-sulfonyl)pyridin-2(1H)-one (AK-24)

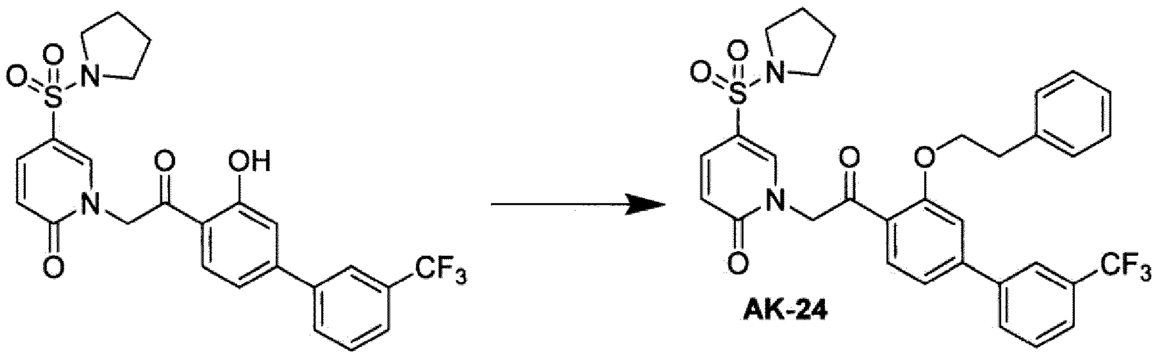

AK-24

A solution of 1-(2-(3-hydroxy-3'-(trifluoromethyl)-[1,1'-biphenyl]-4-yl)-2-oxoethyl)-5-(pyrrolidin-1-yl-sulfonyl)pyridin-2-(1H)-one (37.8 mg, 0.07 mmol, 1.0 equiv) and potassium carbonate (41.3 mg, 0.30 mmol, 4.0 equiv) in dry DMF (0.75 mL) were added (2-iodoethyl)benzene (42.5 μL, 0.3 mmol, 4.0 equiv), and the mixture was stirred for 24 hours at room temperature. The mixture was poured into water, and extracted with EtOAc. The extract was washed with brine, dried over $Na_2SO_4$, filtered, and concentrated under reduced pressure. The residue was purified by column chromatography to give 1-(2-oxo-2-(3-phenethoxy-3'-(trifluoromethyl)-[1,1'-biphenyl]-4-yl)ethyl)-5-(pyrrolidin-1-yl-sulfonyl)pyridin-2(1H)-one (AK-24) as a white amorphous solid (12.8 mg, 0.020 mmol, 34.0% yield).
$^1$H NMR (500 MHz, $CDCl_3$) δ7.97 (d, J = 8.0 Hz, 1H), 7.82 (br s, 1H), 7.78 (d, J = 8.0 Hz, 1H), 7.69-7.58 (m, 5H), 7.33-7.30 (m, 3H), 7.27 (m, 1H), 7.25 (m, 1H), 7.19 (m, 1H), 6.60 (d, J = 2.2 Hz, 1H), 4.92 (s, 2H), 4.58 (m, 2H), 3.29 (m, 6H), 1.90 (m, 4H).

Production Example 22B

Production of 4-(2-(2-oxo-5-(pyrrolidin-1-yl-sulfonyl)pyridin-1(2H)-yl)acetyl)-3'-(trifluoromethyl)-[1,1'-biphenyl]-3-ylacetate (AK-25)

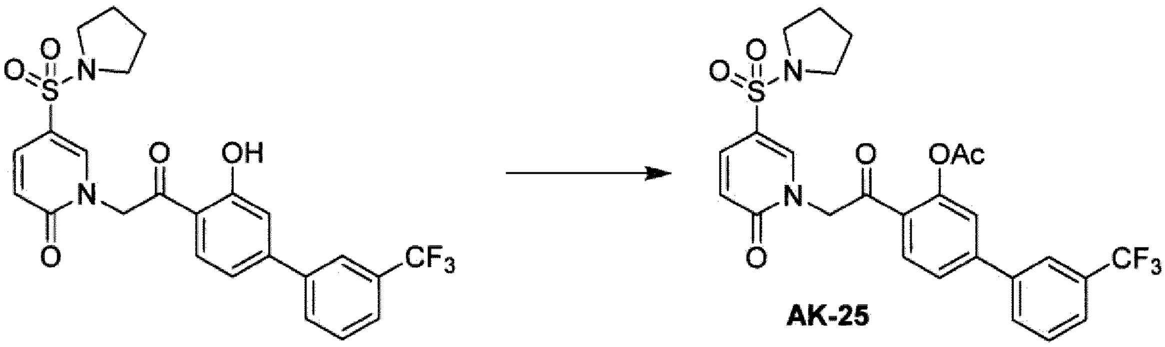

AK-25

To a solution of 1-(2-(3-hydroxy-3'-(trifluoromethyl)-[1,1'-biphenyl]-4-yl)-2-oxoethyl)-5-(pyrrolidin-1-yl-sulfonyl)pyridin-2(1H)-one (AK-15) (37.8 mg, 0.07 mmol, 1.0 equiv) and potassium carbonate (41.3 mg, 0.30 mmol, 4.0 equiv) in dry DMF (0.75 mL) was added acetyl chloride (21.2 μL, 0.3 mmol, 4.0 equiv), and the mixture was stirred for 24 h at room temperature. The mixture was poured into water, and extracted with EtOAc. The extract (organic layer) was washed with brine, dried over $Na_2SO_4$, filtered, and concentrated *in vacuo.* The residue was purified by column chromatography to give the titled 4-(2-(2-oxo-5-(pyrrolidin-1-yl-sulfonyl)pyridin-1(2H)-yl)acetyl)-3'-(trifluoromethyl)-[1,1'-biphenyl]-3-yl acetate (AK-25) as a white amorphous solid (16.2 mg, 0.029 mmol, 39.0% yield).

$^1H$ NMR (500 MHz, $CDCl_3$) δ8.29 (d, J = 8.6 Hz, 1H), 7.92 (br s, 1H), 7.87 (d, J = 2.3 Hz, 1H), 7.86 (d, J = 11.0 Hz, 1H), 7.76 (d, J = 1.2 Hz, 1H), 7.73-7.69 (m, 4H), 7.66 (dd, J = 6.0, 6.0 Hz, 1H), 6.71 (d, J = 9.7 Hz, 1H), 5.30 (s, 2H), 3.35 (m, 4H), 2.42 (s, 3H), 1.75 (m, 4H).

Reference Example 46

Production of 2-bromo-1-((3*S*,10*R*,13*S*,17S)-10,13-dimethyl-3-((trimethylsilyl)oxy)-2,3,4,7,8,9,10,11,12,13,14,15,16,17-tetradecahydro-1*H*-cyclopenta[a]phenanthren-17-yl)ethan-1-one

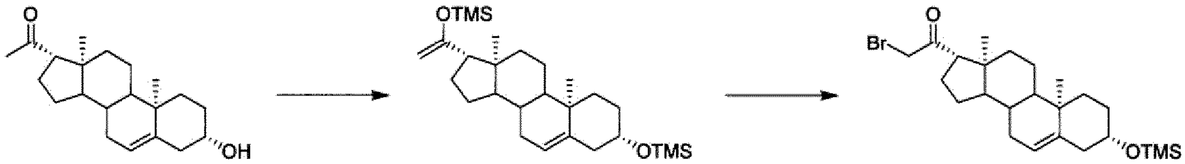

Step 1: Dry THF (4.0 mL) and diisopropylamine (221 μL, 1.6 mmol, 2.5 equiv) in a flask was cooled to -3°C, to which n-butyllithium (2.6 M solution in hexane, 618 μL, 1.6 mmol, 2.5 equiv) was added, followed by stirring for 40 min. The solution was cooled to -78°C, to which 1-((3S,10R,13S,17S)-3-hydroxy-10,13-dimethyl-2,3,4,7,8,9,10,11,12,13,14,15,16,17-tetradecahydro-1*H*-cyclopenta[a]phenanthren-17-yl)ethan-1-one (200 mg, 0.6 mmol, 1.0 equiv) in dry THF (2.3 mL) was added, and the mixture was stirred for 40 min. Then, trimethylsilyl chloride (201 μL, 1.6 mmol, 2.5 equiv) was added to the solution, and the mixture was stirred overnight and allowed to warm to room temperature. After reaction, the reaction mixture was filtered through a small amount of silica gel, and carried on to the next step without further purification.

Step 2: To a solution of the crude mixture obtained above in step 1 (291 mg, 0.63 mmol, 1.0 equiv) in dry DMF (6.3 mL) was added NBS (123.6 mg, 0.69 mmol, 1.1 equiv), and the mixture was stirred for 90 min at room temperature. The solvent was removed *in vacuo*. The resulting product was diluted with water, and extracted with $CH_2Cl_2$ and EtOAc. The extract (organic layer) was dried over $Na_2SO_4$, filtered, and concentrated *in vacuo*. The residue was purified by column chromatography to give the titled 2-bromo-1-((3*S*,10*R*,13*S*,17*S*)-10,13-dimethyl-3-((trimethylsilyl)oxy)-2,3,4,7,8,9,10,11,12,13,14,15,16,17-tetradecahydro-1*H*-cyclopenta[a]phenanthren-17-yl)ethan-1-one as a white solid (121.6 mg, 0.026 mmol, 41.0% yield).

$^1H$ NMR (500 MHz, $CDCl_3$) δ5.32 (m, 1H), 3.93 (d, J = 13.2 Hz, 1H), 3.90 (d, J = 13.2 Hz, 1H), 3.48 (m, 1H), 2.83 (dd, J = 8.6, 8.6 Hz, 1H), 2.32-2.15 (m, 3H), 2.04-1.94 (m, 2H), 1.85-1.69 (m, 4H), 1.65-1.42 (m, 6H), 1.28 (m, 1H), 1.18 (m, 1H), 1.08 (m, 1H), 1.04-0.95 (m, 1H), 0.99 (s, 3H), 0.65 (s, 3H), 0.12 (s, 8H).

Production Example 24B

Production of 1-(2-((3S,10R,13S,17S)-3-hydroxy-10,13-dimethyl-2,3,4,7,8,9,10,11,12,13,14,15,16,17-tetradecahydro-1H-cyclopenta[a]phenanthren-17-yl)-2-oxoethyl)-5-(pyrrolidin-1-yl-sulfonyl)pyridin-2(1H)-one (AK-20)

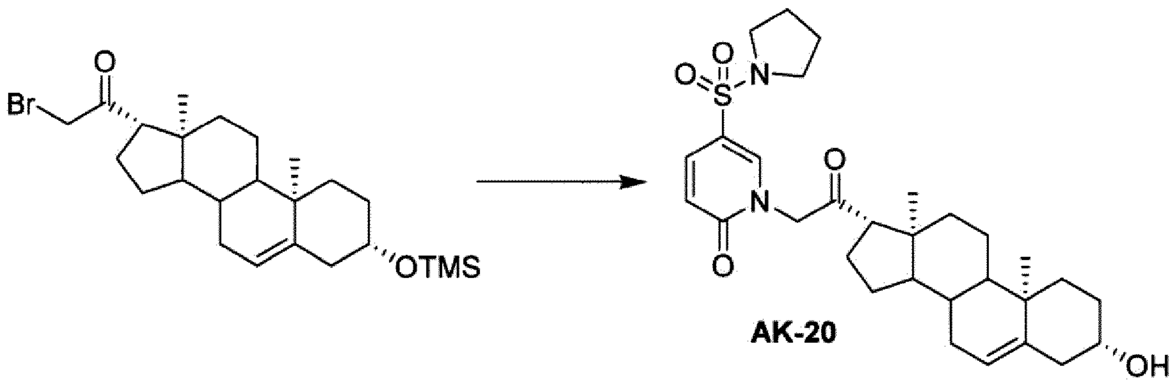

AK-20

A suspension of 2-bromo-1-((3*S*,10*R*,13*S*,17*S*)-10,13-dimethyl-3-((trimethylsilyl)oxy)-2,3,4,7,8,9,10,11,12,13,14,15,16,17-tetradecahydro-1*H*-cyclopenta[a]phenanthren-17-yl)ethan-1-one (Reference Example 46) (121.6 mg, 0.26 mmol, 1.5 equiv), 5-(pyrrolidin-1-yl-sulfonyl)pyridin-2(1H)-one (Reference Example 32) (39.6 mg, 0.17 mmol, 1.0 equiv), and potassium carbonate (62.9 mg, 0.46 mmol, 2.2 equiv) in dry MeCN (12 mL) was stirred at room temperature overnight. To the mixture, dry $CH_2Cl_2$ (2.5 mL) was added, and the mixture was stirred for 24 hr. The solvent was removed *in vacuo.* The resulting compounds were diluted with water, extracted with $CH_2Cl_2$, and washed with brine. The obtained organic layer was dried over $Na_2SO_4$, filtered, and concentrated *in vacuo.* The residue was purified by column chromatography to give the titled 1-(2-((3*S*,10*R*,13*S*,17*S*)-3-hydroxy-10,13-dimethyl-2,3,4,7,8,9,10,11,12,13,14,15,16,17-tetradecahydro-1H-cyclopenta[a]phenanthren-17-yl)-2-oxoethyl)-5-(pyrrolidin-1-yl-sulfonyl)pyridin-2(1H)-one (AK-20) as a white solid (58.8 mg, 0.095 mmol, 62.0% yield).
$^1$H NMR (500 MHz, $CDCl_3$) δ7.77 (d, J = 2.6 Hz, 1H), 7.62 (dd, J = 9.5, 2.6 Hz, 1H), 6.59 (d, J = 9.5 Hz, 1H), 5.35 (m, 1H), 4.96 (d, J = 17.2 Hz, 1H), 4.50 (d, J = 17.2 Hz, 1H), 3.53 (m, 1H), 3.27 (m, 4H), 2.66 (dd, J = 8.6, 8.6 Hz, 1H), 2.33-2.17 (m, 4H), 1.99 (m, 1H), 1.90-1.82 (m, 6H), 1.81-1.71 (m, 2H), 1.63-1.44 (m, 7H), 1.34-1.18 (m, 1H), 1.11 (ddd, J = 13.2, 13.2, 3.4 Hz, 1H), 1.01 (s, 3H), 0.70 (s, 3H).

Reference Example 47

Production of methyl 4-(pyrrolidin-1-yl-sulfonyl)-1H-pyrrole-2-carboxylate

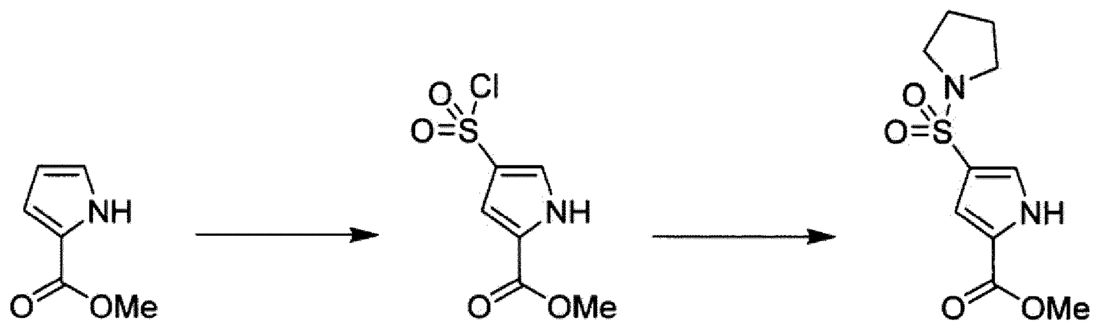

Methyl 1*H*-pyrrole-2-carboxylate (1.29 g, 0.01 mol) was added to chlorosulfuric acid (5.6 mL, 0.08 mol) at 0°C, and the reaction mixture was stirred at room temperature for 4 h. After cooling down to 0°C, the reaction was quenched by adding ice and water. The mixture was filtered to give methyl 4-(chlorosulfonyl)-1*H*-pyrrole-2-carboxylate as an off-white solid, which was used for the next step without further purification. To a stirred solution of the filtrate above (methyl 4-(chlorosulfonyl)-1*H*-pyrrole-2-carboxylate) in dry $CH_2Cl_2$ (20 mL) and the dry THF (10 mL) was added pyrrolidine (25 mL) at room temperature. The mixture was stirred for 24 h, and then concentrated. The residue was purified by column chromatography on silica gel to give the titled methyl 4-(pyrrolidin-1-yl-sulfonyl)-1*H*-pyrrole-2-carboxylate (1.77 g, 67% yield, in 2 steps) as a white solid.
$^1$H NMR (500 MHz, $CDCl_3$) δ9.65 (br s, 1H), 7.42 (dd, J = 2.9, 1.8 Hz, 1H), 7.14 (dd, J = 1.8, 1.8 Hz, 1H), 3.88 (s, 3H), 3.23 (m, 4H), 1.78 (m, 4H).

Reference Example 48

Production of 4-(pyrrolidin-1-yl-sulfonyl)-1H-pyrrole-2-carboxylic acid

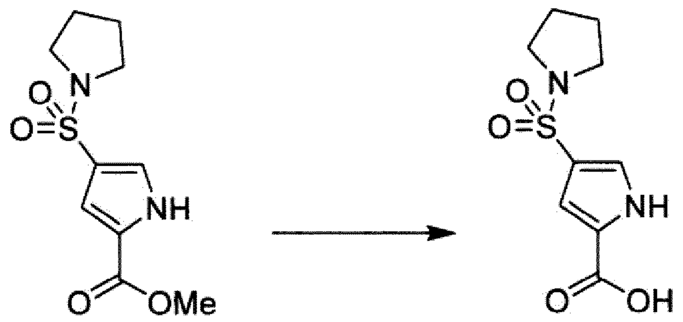

A solution of methyl 4-(pyrrolidin-1-yl-sulfonyl)-1H-pyrrole-2-carboxylate (Reference Example 47) (561 mg, 2.17 mmol) in MeOH (10 mL) and 2M KOH aqueous solution (10 mL) was stirred at reflux for 3 h. The mixture was washed with $CH_2Cl_2$ and $CHCl_3$, then treated with a 2 M HCl aqueous solution, and extracted with EtOAc. The combined extract was concentrated to give the titled 4-(pyrrolidin-1-yl-sulfonyl)-1*H*-pyrrole-2-carboxylic acid (349 mg, 66% yield) as a white solid, which was used for the next step without further purification.
$^1H$ NMR (400 MHz, $CDCl_3$) δ9.48 (br s, 1H), 7.47 (br s, 1H), 7.24 (br s, 1H), 3.25 (m, 4H), 1.80 (m, 4H).

Reference Example 49

Production of 4-(pyrrolidin-1-yl-sulfonyl)-1H-pyrrole-2-carboxamide

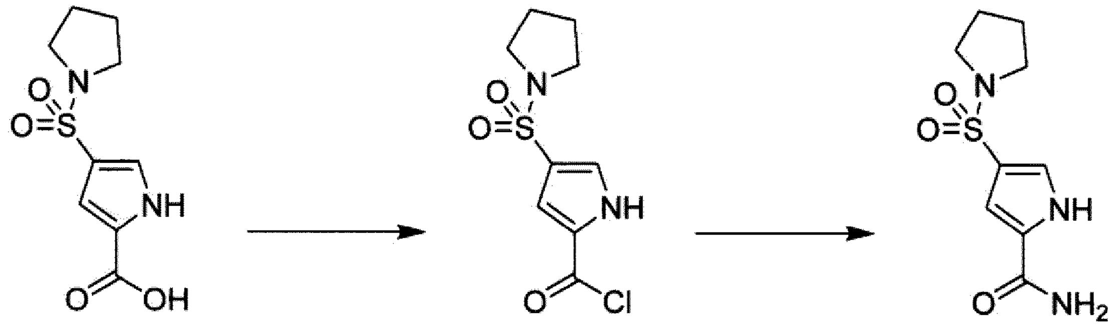

A solution of 4-(pyrrolidin-1-yl-sulfonyl)-1*H*-pyrrole-2-carboxylic acid (Reference Example 48) (561 mg, 2.17 mmol) in $SOCl_2$ (10 mL) was stirred at 60°C for 2 h. After cooling down to room temperature, the reaction mixture was concentrated to give 4-(pyrrolidin-1-yl-sulfonyl)-1*H*-pyrrole-2-carbonyl chloride (347 mg, 92% yield) as a white solid, which was used for the next step without further purification. To a stirred solution of 4-(pyrrolidin-1-yl-sulfonyl)-1*H*-pyrrole-2-carbonyl chloride (50.2 mg, 0.19 mol) in THF (0.4 mL) were added a 28% $NH_3$ aqueous solution (0.15 mL) at 0°C. The reaction mixture was stirred at room temperature for 24 h, and then concentrated. The residue was purified by column chromatography on silica gel to give the titled 4-(pyrrolidin-1-yl-sulfonyl)-1*H*-pyrrole-2-carboxamide (28.9 mg, 62% yield) as a white solid.
$^1H$ NMR (400 MHz, $CDCl_3$) δ7.42 (m, 1H), 6.88 (m, 1H), 3.23 (m, 4H), 1.79 (m, 4H).

Reference Example 50

Production of N,N-dimethyl-4-(pyrrolidin-1-yl-sulfonyl)-1H-pyrrole-2-carboxamide

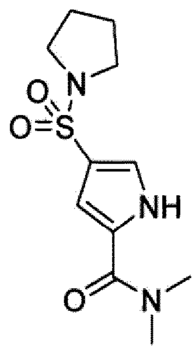

A solution of 4-(pyrrolidin-1-yl-sulfonyl)-1H-pyrrole-2-carboxylic acid (Reference Example 48) (10.9 mg, 0.05 mol) in $SOCl_2$ (0.25 mL) was treated as in Reference Example 49 to give 4-(pyrrolidin-1-yl-sulfonyl)-1*H*-pyrrole-2-carbonyl chloride as a white solid. A solution of 4-(pyrrolidin-1-ylsulfonyl)-1*H*-pyrrole-2-carbonyl chloride and a 50% $Me_2NH_3$ aqueous solution (0.02 mL) in dry THF (0.25 mL) was treated as in Reference Example 49 to give the titled *N,N*-dimethyl-4-(pyrrolidin-1-yl-sulfonyl)-1H-pyrrole-2-carboxamide (9.9 mg, 82% yield) as a white solid.
$^1$H NMR (500 MHz, Acetone-$d_6$) δ11.34 (br s, 1H), 7.45 (m 1H), 6.87 (m, 1H), 3.16 (m, 4H), 2.84 (s, 6H), 1.72 (m, 4H).

Reference Example 51

Production of 2-(4-(Pyrrolidin-1-yl-sulfonyl)-1H-pyrrol-2-yl)oxazole

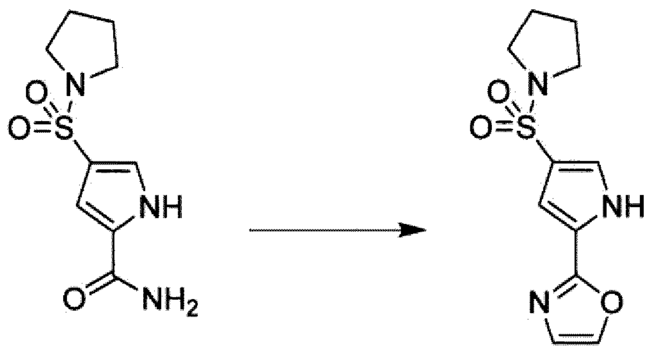

To a stirred solution of 4-(pyrrolidin-1-yl-sulfonyl)-1*H*-pyrrole-2-carboxamide (Reference Example 49) (28.9 mg, 0.12 mol) in N-methylpyrrolidone (0.6 mL) were added 2-bromo-1,1-dimethoxyethane (0.09 mL, 0.60 mol) and TsOH • $H_2O$ (1.4 mg, cat.) at room temperature. The reaction mixture was stirred at 110°C for 19 h. After cooling down to room temperature, the reaction mixture was diluted with a saturated aqueous $NaHCO_3$ solution, and extracted with EtOAc. The combined extract (organic layer) was washed with water, dried over $Na_2SO_4$, and concentrated. The residue was purified by column chromatography on silica gel to give the titled 2-(4-(pyrrolidin-1-yl-sulfonyl)-1*H*-pyrrol-2-yl)oxazole (12.0 mg, 38% yield) as a yellow solid.
$^1$H NMR (500 MHz, $CDCl_3$) δ10.71 (br s, 1H), 7.68 (s, 1H), 7.42 (dd, J = 2.9, 2.3 Hz, 1H), 7.20 (s, 1H), 7.14 (dd, J = 2.3, 2.3 Hz, 1H), 3.26 (m, 4H), 1.79 (m, 4H).

Reference Example 52

Production of N-methoxy-N-methyl-4-(pyrrolidin-1-yl-sulfonyl)-1H-pyrrole-2-carboxamide

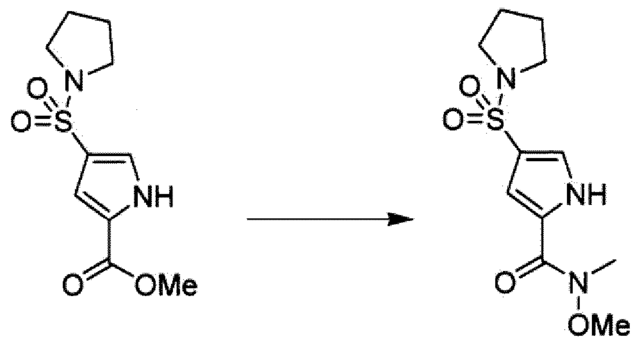

(a) To a stirred solution of bis-(trimethylsilyl)-amine (4.4 mL, 21.0 mmol) in dry THF (8.0 mL) was added nBuLi (2.6 M solution in hexane, 7.6 mL, 20.1 mmol) at -78°C. The reaction mixture was stirred at 0°C for 30 min.
(b) To a stirred solution of 4-(pyrrolidin-1-yl-sulfonyl)-1H-pyrrole-2-carboxylate (646.0 mg, 2.5 mmol) in dry THF (8.0 mL) was added *N,O*-dimethylhydroxylamine hydrochloride (317.6 mg, 3.3 mmol) at -78°C.
(c) The solution from step (a) was added to the solution from step (b). The mixture was stirred at -78°C for 40 min, diluted with a saturated aqueous $NaHCO_3$ solution, and extracted with EtOAc. The combined extract (organic layer) was washed with brine, dried over $Na_2SO_4$, and concentrated. The residue was purified by column chromatography on silica gel to give the titled *N*-methoxy-*N*-methyl-4-(pyrrolidin-1-yl-sulfonyl)-1*H*-pyrrole-2-carboxamide (675.2 mg, 92% yield) as a white solid. $^1H$ NMR (500 MHz, $CDCl_3$) δ7.42 (m, 1H), 7.13 (m, 1H), 3.80 (s, 1H), 3.37 (s, 1H), 3.23 (m, 4H), 1.78 (m, 4H).

Reference Example 53

Production of 1-(4-(pyrrolidin-1-yl-sulfonyl)-1H-pyrrol-2-yl)propan-1-one

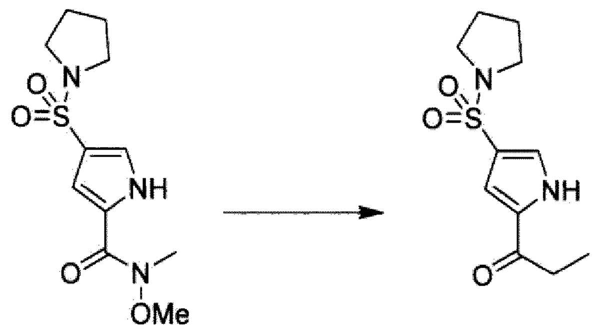

To a solution of N-methoxy-N-methyl-4-(pyrrolidin-1-yl-sulfonyl)-1*H*-pyrrole-2-carboxamide (Reference Example 52) (167.8 mg, 0.58 mmol) in dry THF (4.0 mL) was added ethylmagnesium bromide (3.0 M solution in $Et_2O$, 1.74 mL, 5.22 mmol) at room temperature. The reaction mixture was stirred for 7.5 h, diluted with a saturated aqueous $NaHCO_3$ solution, and extracted with EtOAc. The combined extract (organic layer) was washed with brine, dried over $Na_2SO_4$, and concentrated. The residue was purified by column chromatography on silica gel to give the titled 1-(4-(pyrrolidin-1-yl-sulfonyl)-1*H*-pyrrol-2-yl)propan-1-one (129.9 mg, 88% yield) as a white solid.
$^1H$ NMR (500 MHz, $CDCl_3$) δ9.61 (br s, 1H), 7.45 (m, 1H), 7.12 (m, 1H), 3.24 (m, 4H), 2.83 (q, J = 7.0 Hz, 2H), 1.80 (m, 4H), 1.22 (t, J = 7.0 Hz 3H).

Reference Example 54

Production of 1-(4-(pyrrolidin-1-yl-sulfonyl)-1H-pyrrol-2-yl)ethan-1-one

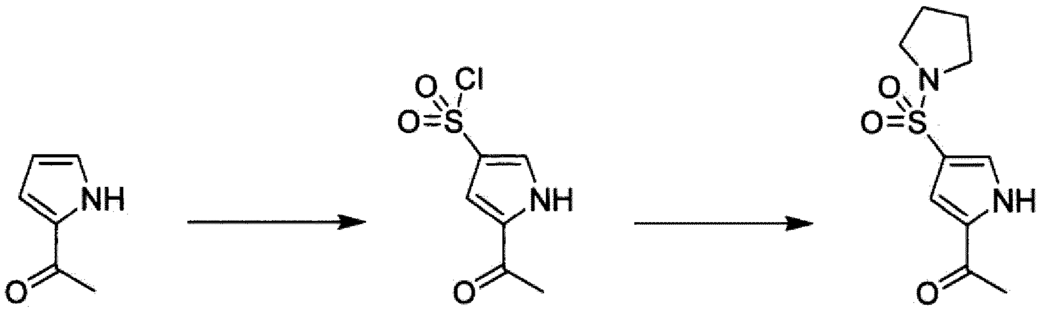

To a stirred chlorosulfuric acid (2.0 mL, 30 mmol) was added 1-(1*H*-pyrrol-2-yl)ethan-1-one (545.7 mg, 5.0 mmol) at 0°C. The reaction mixture was stirred at room temperature for 1.5 h. After cooling down to 0°C, the reaction was quenched by adding ice and water. The reaction mixture was filtered to give 5-acetyl-1*H*-pyrrole-3-sulfonyl chloride as a pale pink powder, which was used for the next step without further purification. To a solution of 5-acetyl-1*H*-pyrrole-3-sulfonyl chloride in $CH_2Cl_2$ (8 mL) was added pyrrolidine (0.8 mL, 9.7 mmol) at room temperature. The mixture was stirred at room temperature for 14 h, and then the mixture was concentrated. The residue was purified by column chromatography on silica gel to give the titled 1-(4-(pyrrolidin-1-yl-sulfonyl)-1*H*-pyrrol-2-yl)ethan-1-one (398.5 mg, 32% yield in 2 steps) as a white powder.
$^1$H NMR (500 MHz, $CDCl_3$) δ7.46 (d, J = 1.43 Hz, 1H), 7.12 (br s, 1H), 3.25 (m, 4H), 2.47 (s, 1H), 1.81 (m, 4H).

Production Example 1A

Production of methyl 1-(2-oxo-2-(3'-(trifluoromethyl)-[1,1'-biphenyl]-4-yl)ethyl)-4-(pyrrolidin-1-yl-sulfonyl)-1H-pyrrole2-carboxylate (RT-22)

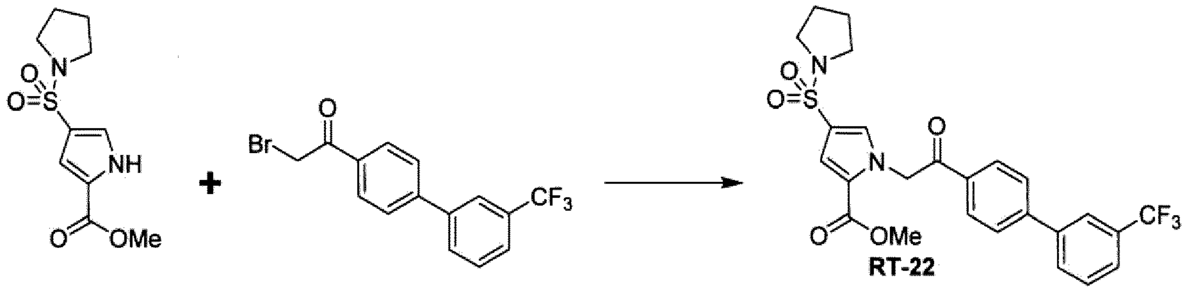
RT-22

A solution of methyl 4-(pyrrolidin-1-yl-sulfonyl)-1H-pyrrole-2-carboxylate (Reference Example 47) (12.9 mg, 0.050 mmol), 2-bromo-1-(3'-(trifluoromethyl)-[1,1'-biphenyl]-4-yl)ethan-1-one (Reference Example 15) (18.2 mg, 0.055 mmol), and potassium carbonate (21.8 mg, 0.158 mmol) in dry MeCN (0.25 mL) was stirred at room temperature for 7.5 h. Then, the reaction mixture was diluted with water and extracted with EtOAc. The combined extract (organic layer) was dried over $Na_2SO_4$ and concentrated. The residue was purified by column chromatography on silica gel to give the titled methyl 1-(2-oxo-2-(3'-(trifluoromethyl)-[1,1'-biphenyl]-4-yl)ethyl)-4-(pyrrolidin-1-yl-sulfonyl)-1*H*-pyrrole-2-carboxylate (RT-22) as a pale yellow amorphous (19.3 mg, 74% yield).
$^1$H NMR (500 MHz, $CDCl_3$) δ 8.10 (d, J = 8.3 Hz, 2H), 7.87 (s, 1H), 7.81 (d, J = 8.0 Hz, 1H), 7.77 (d, J = 8.3 Hz, 2H), 7.68 (d, J = 8.0 Hz, 1H) 7.26 (dd, J = 8.0, 8.0 Hz 1H), 7.32 (br s, 1H), 7.28 (d, J = 1.7 Hz, 1H), 5.83 (s, 2H), 3.77 (s, 3H), 3.28 (m, 4H), 1.83 (m, 4H).

Production Example 2A

Production of N,N-dimethyl-1-(2-oxo-2-(3'-(trifluoromethyl)-[1,1'-biphenyl]-4-yl)ethyl)-4-(pyrrolidin-1-yl-sulfonyl)-1H-pyrrole-2-carboxamide (RT-23)

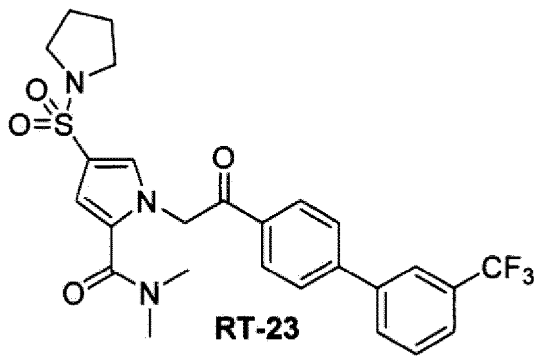

RT-23

A solution of *N,N*-dimethyl-4-(pyrrolidin-1-yl-sulfonyl)-1*H*-pyrrole-2-carboxamide (Reference Example 50) (9.9 mg, 0.037 mmol), 2-bromo-1-(3'-(trifluoromethyl)-[1,1'-biphenyl]-4-yl)ethan-1-one (Reference Example 15) (14.2 mg, 0.042 mmol), and potassium carbonate (13.6 mg, 0.098 mmol) in dry MeCN (0.20 mL) was treated as in Production Example 1A to give the titled N,N-dimethyl-1-(2-oxo-2-(3'-(trifluoromethyl)-[1,1'-biphenyl]-4-yl)ethyl)-4-(pyrrolidin-1-yl-sulfonyl)-1*H*-pyrrole-2-carboxamide (RT-23) as a white solid (15.0 mg, 77% yield).

Production Example 3A

Production of 2-(2-(oxazol-2-yl)-4-(pyrrolidin-1-yl-sulfonyl)-1H-pyrrol-1-yl)-1-(3'-(trifluoromethyl)-[1,1'-biphenyl]-4-yl)ethan-1-one (RT-58)

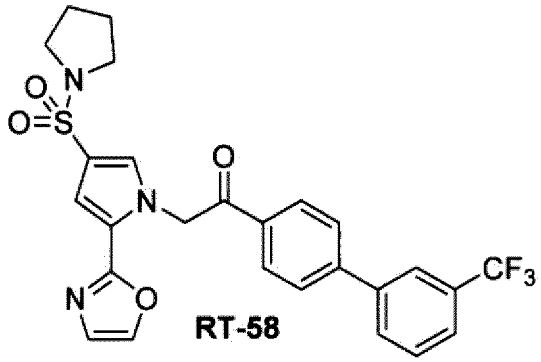

RT-58

A solution of 2-(4-(pyrrolidin-1-yl-sulfonyl)-1H-pyrrol-2-yl)oxazole (Reference Example 51) (12.0 mg, 0.045 mmol), 2-bromo-1-(3'-(trifluoromethyl)-[1,1'-biphenyl]-4-yl)ethan-1-one (Reference Example 15) (29.6 mg, 0.086 mmol), and potassium carbonate (21.6 mg, 0.156 mmol) in dry MeCN (0.40 mL) was treated as in Production Example 1A to give the titled 2-(2-(oxazol-2-yl)-4-(pyrrolidin-1-yl-sulfonyl)-1*H*-pyrrol-1-yl)-1-(3'-(trifluoromethyl)-[1,1'-biphenyl]-4-yl)ethan-1-one (RT-58) as a yellow oil (9.9 mg, 42% yield).
$^1$H NMR (500 MHz, $CDCl_3$) $\delta$ 8.13 (d, J = 8.3 Hz, 2H), 7.89 (s, 1H), 7.83 (d, J = 7.7 Hz, 1H), 7.77 (d, J = 8.3 Hz, 2H), 7.69 (d, J = 7.7 Hz, 1H), 7.63 (dd, J = 7.7, 7.7 Hz, 1H), 7.57 (s, 1H), 7.30 (d, J = 1.4 Hz, 1H), 7.16 (d, 1.4 Hz, 1H), 7.00 (s, 1H), 6.05 (s, 2H), 3.30 (m, 4H), 1.83 (m, 4H).

Production Example 4A

Production of 2-(2-acetyl-4-(pyrrolidin-1-yl-sulfonyl)-1H-pyrrol-1-yl)-1-(3'-(trifluoromethyl)-[1,1'-biphenyl]-4-yl)ethane-1-one (HS-16)

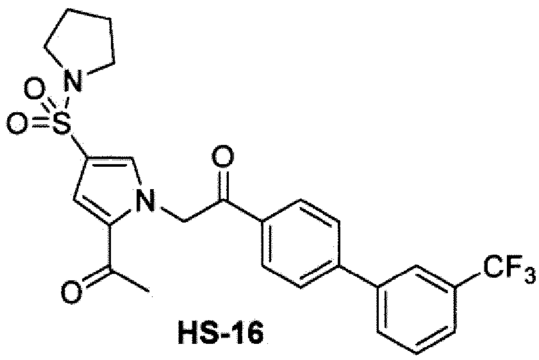

HS-16

A solution of 1-(4-(pyrrolidin-1-yl-sulfonyl)-1*H*-pyrrol-2-yl)ethan-1-one (Reference Example 54) (24.4 mg, 0.10 mmol), 2-bromo-1-(3'-(trifluoromethyl)-[1,1'-biphenyl]-4-yl)ethan-1-one (Reference Example 15) (40.4 mg, 0.12 mmol), and potassium carbonate (21.1 mg, 0.15 mmol) in dry MeCN (0.50 mL) was treated as in Production Example 1A to give the titled 2-(2-acetyl-4-(pyrrolidin-1-ylsulfonyl)-1*H*-pyrrol-1-yl)-1-(3'-(trifluoromethyl)-[1,1'-biphenyl]-4-yl)ethane-1-one (HS-16) as a yellow oil (47.9 mg, 95% yield).
$^1$H NMR (500 MHz, $CDCl_3$) $\delta$ 8.10 (d, J = 8.3 Hz, 2H), 7.87 (s, 1H), 7.81 (d, J = 7.9 Hz, 1H), 7.76 (d, J = 8.3 Hz, 2H), 7.69 (d, J = 8.3 Hz, 1H), 7.62 (dd, J = 7.9, 7.9 Hz, 1H), 7.33 (d, J = 1.6 Hz, 1H), 7.29 (d, J = 1.6 Hz, 1H), 5.80 (s, 2H), 3.29 (m, 4H), 2.45 (s, 3H), 1.86 (m, 4H).

Production Example 5A

Production of 1-(1-(2-oxo-2-(3'-(trifluoromethyl)-[1,1'-biphenyl]-4-yl)ethyl)-4-(pyrrolidin-1-yl-sulfonyl)-1H-pyrrol-2-yl)propan-1-one (HS-17)

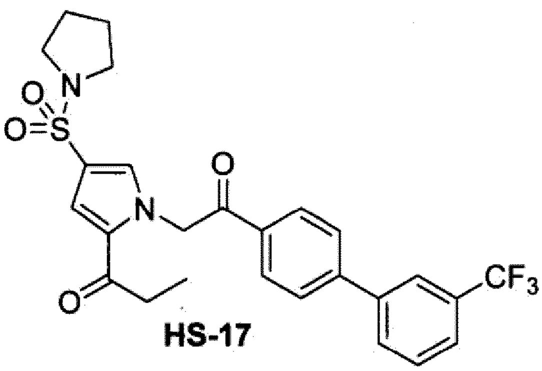

HS-17

A solution of 1-(4-(pyrrolidin-1-yl-sulfonyl)-1H-pyrrol-2-yl)propan-1-one (Production Example 4A) (26.1 mg, 0.10 mmol), 2-bromo-1-(3'-(trifluoromethyl)-[1,1'-biphenyl]-4-yl)ethan-1-one (Reference Example 15) (37.8 mg, 0.11 mmol), and potassium carbonate (22.3 mg, 0.16 mmol) in dry MeCN (1.0 mL) was treated as in Production Example 1A to give the titled 1-(1-(2-oxo-2-(3'-(trifluoromethyl)-[1,1'-biphenyl]-4-yl)ethyl)-4-(pyrrolidin-1-yl-sulfonyl)-1*H*-pyrrol-2-yl)propan-1-one (HS-17) as a yellow oil (50.9 mg, 50.9% yield).
$^1$H NMR (500 MHz, $CDCl_3$) $\delta$ 8.10 (d, J = 8.2 Hz, 2H), 7.87 (s, 1H), 7.81 (d, J = 7.9 Hz, 1H), 7.76 (d, J = 8.2 Hz, 2H), 7.69 (d, J = 7.9 Hz, 1H), 7.62 (dd, J = 7.9 Hz, 1H), 7.32 (d, J = 1.5 Hz, 1H), 7.30 (d, J = 1.5 Hz, 1H), 5.83 (s, 2H), 3.28 (m, 4H), 2.84 (q, J = 7.4 Hz, 2H), 1.85 (m, 4H), 1.12 (t, J = 7.4 Hz, 3H).

Production Example 6A

Production of methyl 1-(2-hydroxy-2-(3'-(trifluoromethyl)-[1,1'-biphenyl]-4-yl)ethyl)-4-(pyrrolidin-1-yl-sulfonyl)-1H-pyrrole-2-carboxylate (HS-6)

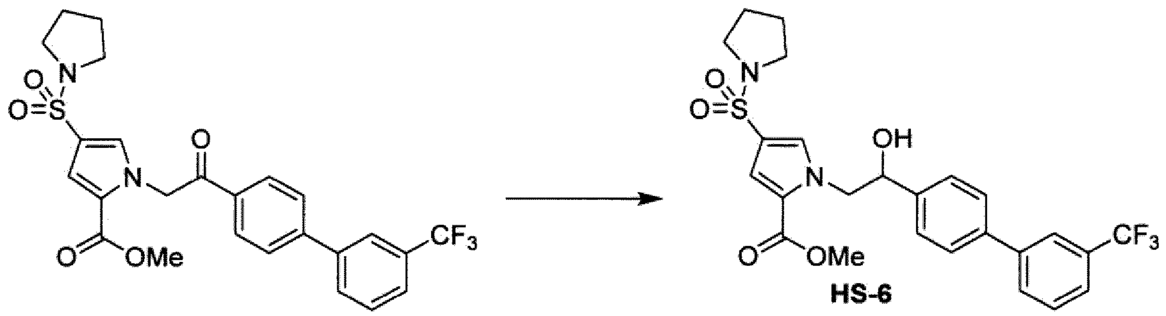


To a solution of methyl 1-(2-oxo-2-(3'-(trifluoromethyl)-[1,1'-biphenyl]-4-yl)ethyl)-4-(pyrrolidin-1-yl-sulfonyl)-1*H*-pyrrole-2-carboxylate (RT-22) (Production Example 1A) (129.6 mg, 0.25 mmol) in dry MeOH (2.5 mL) was added sodium borohydride (14.8 mg, 0.39 mmol) at 0°C. The reaction mixture was stirred at room temperature for 3 h, diluted with a saturated aqueous $NH_4Cl$ solution, and extracted with EtOAc. The combined extract (organice layer) was washed with brine, dried over $Na_2SO_4$, and concentrated. The residue was purified by column chromatography on silica gel to give the titled methyl 1-(2-hydroxy-2-(3'-(trifluoromethyl)-[1,1'-biphenyl]-4-yl)ethyl)-4-(pyrrolidin-1-yl-sulfonyl)-1*H*-pyrrole-2-carboxylate (HS-6) as a pale yellow solid (92.5 mg, 72% yield).
$^1$H NMR (500 MHz, $CDCl_3$) δ 7.81 (s, 1H), 7.75 (d, J = 7.7 Hz, 1H), 7.62-7.55 (m, 4H), 7.49 (d, J = 7.7 Hz, 2H), 7.25 (br s, 1H), 7.22 (br s, 1H), 5.14 (br d, J = 3.9 Hz, 1H), 4.85 (dd, J = 14.0, 3.9 Hz, 1H), 4.32 (dd, J = 14.0, 7.7 Hz, 1H), 3.87 (s, 3H), 3.16 (m, 4H), 1.75 (m, 4H).

Production Example 7A

Production of 2-(2-(hydroxymethyl)-4-(pyrrolidin-1-yl-sulfonyl)-1H-pyrrol-1-yl)-1-(3'-(trifluoromethyl)-[1,1'-biphenyl]-4-yl)ethan-1-ol (HS-14)

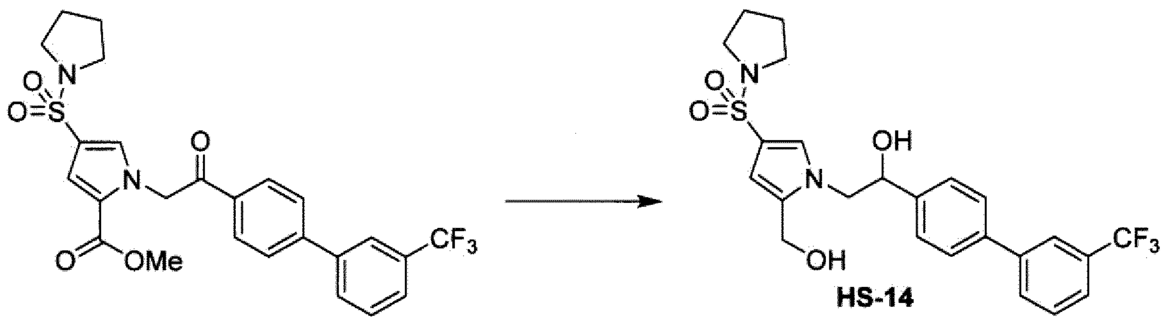


To a solution of methyl 1-(2-oxo-2-(3'-(trifluoromethyl)-[1,1'-biphenyl]-4-yl)ethyl)-4-(pyrrolidin-1-yl-sulfonyl)-1*H*-pyrrole-2-carboxylate (RT-22) (Production Example 1A) (120.0 mg, 0.23 mmol) in dry THF (2.0 mL) was added lithium aluminium hydride (8.5 mg, 0.22 mmol) at 4°C. The reaction mixture was stirred at same temperature for 1.5 h, and then lithium aluminium hydride (4.5 mg, 0.22 mmol) was added to the reaction mixture and mixed. In two hours, lithium aluminium hydride (3.3 mg, 0.09 mmol) was added again. The reaction mixture was stirred at 0°C for 0.5 h, diluted with a saturated aqueous $NH_4Cl$ solution, and extracted with EtOAc. The combined extract (organic layer) was washed with brine, dried over $Na_2SO_4$, and concentrated. The residue was purified by column chromatography on silica gel to give the titled 2-(2-(hydroxymethyl)-4-(pyrrolidin-1-yl-sulfonyl)-1H-pyrrol-1-yl)-1-(3'-(trifluoromethyl)-[1,1'-biphenyl]-4-yl)ethan-1-ol (HS-14) as a pale yellow solid (104.9 mg, 91% yield).
$^1$H NMR (400 MHz, $CDCl_3$) δ 7.80 (s, 2H), 7.75 (d, J = 7.6 Hz, 1H), 7.63-7.52 (m, 4H), 7.45 (d, J = 8.2 Hz, 2H), 7.15 (d, J = 1.7 Hz, 1H), 6.38 (d, J = 1.7 Hz, 1H), 5.08 (m, 1H), 4.58 (d, J = 13.8 Hz, 1H), 4.53 (d, J = 13.8 Hz, 1H), 4.25 (dd, J = 15.1, 3.4 Hz, 1H), 4.13 (dd, J = 15.1, 10.1 Hz, 1H), 3.16 (m, 4H), 1.74 (m, 4H) .

Production Example 8A

Production of 2-(pyrrolidin-1-yl-sulfonyl)-6-(3'-(trifluoromethyl)-[1,1'-biphenyl]-4-yl)indolizin-8-ylacetate (HS-20)

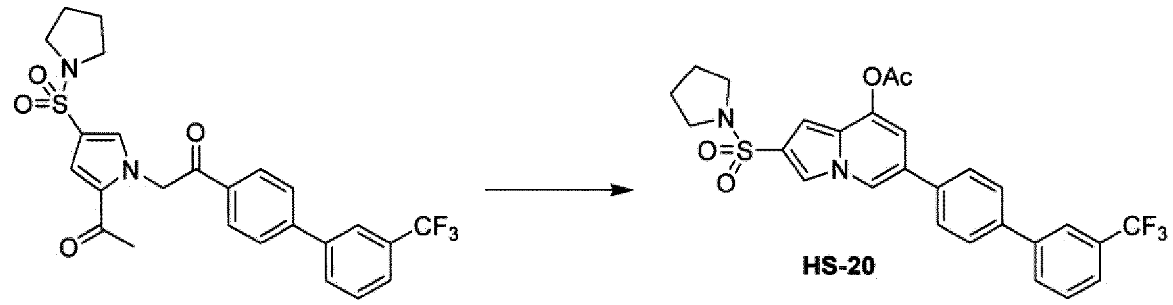

To a solution of 2-(2-acetyl-4-(pyrrolidin-1-yl-sulfonyl)-1*H*-pyrrol-1-yl)-1-(3'-(trifluoromethyl)-[1,1'-biphenyl]-4-yl)ethan-1-one (HS-16) (Production Example 4A) (17.9 mg, 0.035 mmol) in dry THF (0.7 mL) was added sodium hydride (4.8 mg, 0.11 mmol) at 0°C. The reaction mixture was stirred at room temperature for 2.5 h, and then acetic anhydride (6.6 μL, 0.07 mmol) was added to the reaction mixture. The reaction mixture was stirred at room temperature for 3 h, diluted with water, and extracted with EtOAc. The combined extract (organic layer) was washed with brine, dried over $Na_2SO_4$, and concentrated. The residue was purified by column chromatography on silica gel to give the titled 2-(pyrrolidin-1-yl-sulfonyl)-6-(3'-(trifluoromethyl)-[1,1'-biphenyl]-4-yl)indolizin-8-yl acetate (HS-20) as a white solid (57% yield).
$^1$H NMR (400 MHz, $CDCl_3$) δ 8.07 (br s, 1H), 7.87 (br s, 1H), 7.82-7.80 (m, 2H), 7.70 (d, J = 8.5 Hz, 2H), 7.62 (d, J = 8.5 Hz, 2H), 7.60 (m, 2H), 7.03 (d, J = 1.1 Hz, 2H), 6.71 (br s, 1H), 3.30 (m, 4H), 2.44 (s 3H), 1.78 (m, 4H).

Production Example 9A

Production of 3-(2-oxo-2-(3'-(trifluoromethyl)-[1,1'-biphenyl]-4-yl)ethyl)-5-((phenylsulfonyl)methyl)imidazolidine-2,4-dione (RT-35)

(1) Production of (5R,5'R)-5,5'-(disulfanediylbis(methylene))bis(imidazolidine-2,4-dione)

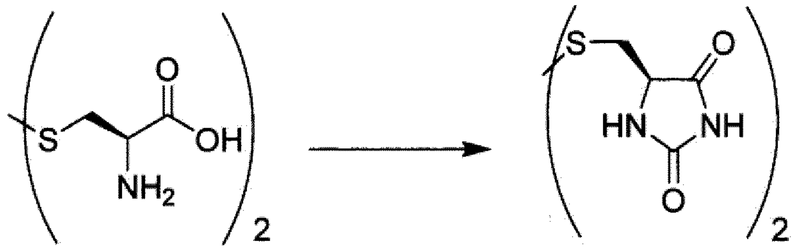

To a stirred solution of L-(-)-cystine (2.00 g, 8.32 mmol) in water (10 mL) was added sodium cyanate (1.21 g, 18.6 mmol) at room temperature. The reaction mixture was stirred at 100°C for 1 h. After cooling down to room temperature, a concentrated aqueous hydrochloric acid solution (7 mL) was added to the reaction mixture, and the mixture was stirred at 100°C for 1 h. The reaction mixture was cooled down to 0°C to give the titled (5*R*,5'*R*)-5,5'-(disulfanediylbis(methylene))bis(imidazolidine-2,4-dione) (1.77 g, 73% yield) as a white solid.
$^1$H NMR (500 MHz, DMSO-$d_6$) δ 10.78 (s, 2H), 8.06 (s, 2H), 4.35 (m, 1H), 3.15 (dd, J = 14.0, 4.6 Hz, 2H), 3.02 (dd, J = 14.0, 4.6 Hz, 2H) .

(2) Production of 5-methylene-3-(2-oxo-2-(3'-(trifluoromethyl)-[1,1'-biphenyl]-4-yl)ethyl)imidazolidine-2,4-dione

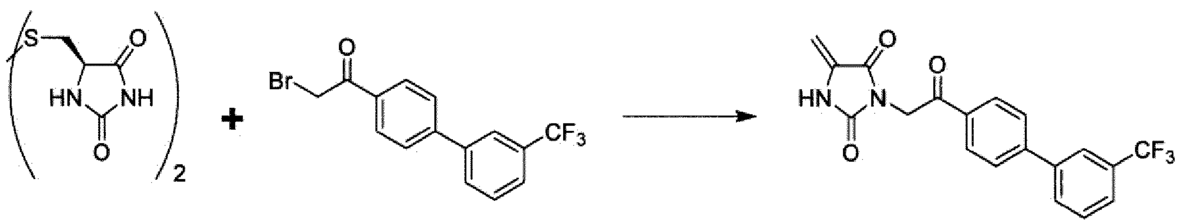

A solution of (5*R*,5'*R*)-5,5'-(disulfanediylbis(methylene))bis(imidazolidine-2,4-dione) (28.2 mg, 0.097 mmol), 2-bromo-1-(3'-(trifluoromethyl)-[1,1'-biphenyl]-4-yl)ethan-1-one (Reference Example 15) (71.2 mg, 0.207 mmol), and potassium carbonate (40.8 mg, 0.295 mmol) in dry DMF (0.5 mL) was stirred at room temperature for 9 h. Then, the reaction mixture was diluted with water, and extracted with EtOAc. The combined extract (organic layer) was dried over $Na_2SO_4$, and concentrated. The residue was purified by column chromatography on silica gel to give the titled 5-methylene-3-(2-oxo-2-(3'-(trifluoromethyl)-[1,1'-biphenyl]-4-yl)ethyl)imidazolidine-2,4-dione as a colorless oil (27.8 mg, 38% yield).
$^1$H NMR (500 MHz, $CDCl_3$) δ 8.08 (d, J = 8.3 Hz, 2H), 7.87 (s, 1H), 7.83 (br s, 1H), 7.81 (d, J = 8.0 Hz, 1H), 7.74 (d, J = 8.3 Hz, 2H), 7.68 (d, J = 8.0 Hz, 1H), 7.61 (dd, J = 8.0, 8.0 Hz, 1H), 5.52 (d, J = 1.7 Hz, 1H), 5.05 (s, 2H), 5.02 (d, J = 1.7 Hz, 1H).

(3) Production of 3-(2-oxo-2-(3'-(trifluoromethyl)-[1,1'-biphenyl]-4-yl)ethyl)-5-((phenylsulfonyl)methyl)imidazolidine-2,4-dione (RT-35)

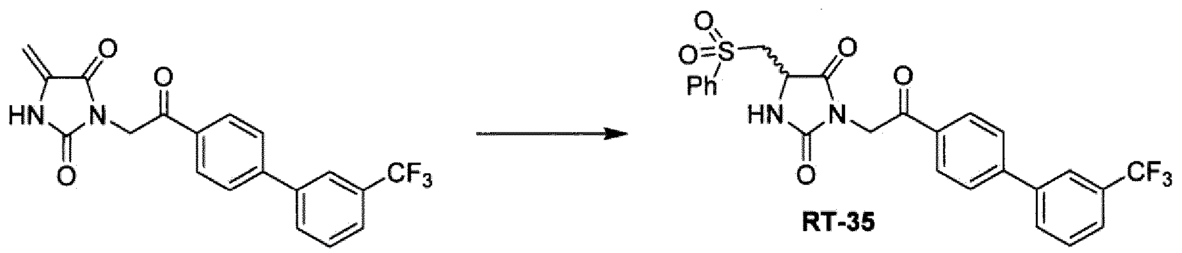

RT-35

To a stirred solution of 5-methylene-3-(2-oxo-2-(3'-(trifluoromethyl)-[1,1'-biphenyl]-4-yl)ethyl)imidazolidine-2,4-dione (27.8 mg, 0.074 mmol) in dry DMF (0.3 mL) was added sodium benzenesulfinate dihydrate (61.0 mg, 0.37 mmol) at 60°C. The reaction mixture was stirred at the same temperature for 7.5 h. After cooling down to room temperature, the reaction was quenched by adding acetic acid (0.05 mL), and the reaction mixture was stirred at room temperature for 1.5 h and filtered to give a residue as a pale yellow solid. The residue was purified by column chromatography on silica gel to give the titled 3-(2-oxo-2-(3'-(trifluoromethyl)-[1,1'-biphenyl]-4-yl)ethyl)-5-((phenylsulfonyl)methyl)imidazolidine-2,4-dione (RT-35) as a white solid (6.6 mg, 17% yield).
$^1$H NMR (500 MHz, $CDCl_3$) δ 8.04 (d, J = 8.6 Hz, 2H), 7.98 (d, J = 7.4 Hz, 2H), 7.86 (s,1H), 7.81-7.72 (m, 4H), 7.69-7.59 (m, 4H), 6.36 (s, 1H), 4.98 (s, 2H), 4.66 (d, J = 11.2 Hz, 1H), 3.76 (dd, J = 13.7, 1.2 Hz, 1H), 3.42 (dd, J = 13.7, 11.2 Hz, 1H); ESI-MS m/z 515.0 $[M-H]^-$.

The compounds produced according to the methods described above (including 33 different types of compounds of the present invention) were subjected as test compounds to various pharmacological tests according to the following methods.

Pharmacological Test 1: *in vitro* GEF Assay

DOCK1 and DOCK2, i.e., DOCK-A subfamily members, both activate Rac by catalyzing the GTP-GDP exchange reaction for Rac via their DHR-2 domains. Therefore, in this Pharmacological Test, the inhibitory effect of the compounds of the present invention on Rac activation by DOCK1 or DOCK2 was assessed in an *in vitro* GEF assay by using polypeptide fragments corresponding to the DHR-2 domain of DOCK1 or DOCK2 from mice and humans. The *in vitro* GEF assay was performed using a labeled GTP (Bodipy-FL-GTP, Invitrogen), which has a property of increasing the fluorescence intensity when bound to Rac.

The polypeptide fragments corresponding to the DHR-2 domain of DOCK1 or DOCK2 were prepared by expression in *E. coli* Arctic express (DE3) cells as N-terminal Histidine-SUMO tag-fused recombinant proteins, followed by purification by affinity column chromatography on nickel-NTA columns.

Rac was prepared by expression in *E. coli* BL21 (DE3) cells as an N-terminal GST tag-fused recombinant protein, followed by purification by affinity column chromatography on glutathione-Sepharose columns.

Each of the polypeptide fragments corresponding to the DHR-2 domain of DOCK1 or DOCK2, and a predetermined concentration of each test compound (the compounds of the present invention: 33 types of compounds; and comparative compound: TBOPP) dissolved in DMSO were incubated in a reaction solution A (pH 7.0), which contained 20 mM of MES-NaOH, 150 mM of NaCl, 10 mM of $MgCl_2$, and 20 $\mu$M of GDP for 20 minutes at room temperature while being shielded from light to thus prepare GEF pretreated products. The final concentration of DMSO was adjusted to 1 mass% in all of the test samples. For a control test, a GEF pretreated product (control) was prepared by incubation in the same manner using DMSO with no test compounds, instead of the DMSO used above in which each test compound was dissolved.

Separately, Rac was added to the reaction solution A (pH 7.0) to a concentration of 15 $\mu$M, and was allowed to stand on ice for 30 min to form a GDP-Rac complex. Bodipy-FL-GTP was added to a concentration of 3.6 $\mu$M to 100 $\mu$L of the reaction solution A that contained the thus-prepared GDP-Rac complex, and equilibrated at 30°C for 2 minutes. After equilibration, 50 $\mu$L of the GEF pretreated product prepared as described above was added and reacted at 30°C.

Changes in fluorescence intensity of Bodipy-FL-GTP in the reaction solutions were monitored using an EnSpire spectrofluorometer (Perkin Elmer) (excitation wavelength: 488 nm, fluorescence wavelength: 514 nm). The measured values were corrected so that the fluorescence intensity at the beginning of the reaction (0 sec) was 0. Then, an approximate curve (hyperbola) was obtained by plotting the calculated correction values on the y-axis and the time (t) on the x-axis using GraphPad Prism5 (GraphPad software), and the slope at t = 0 to 10 seconds was defined as the initial velocity of the guanine nucleotide exchange reaction. The $IC_{50}$ value was calculated taking the initial reaction velocity of the control, to which only a solvent (DMSO) was added, as 100%.

Based on the obtained results, columns A and B of Fig. 1, Fig. 2, Fig. 3-1, and Fig. 3-2 show the results evaluating the inhibitory effect on Rac activation by DOCK1 (DHR-2 domain) ($IC_{50}$ value), and DOCK1-selective inhibitory effect (DOCK1/DOCK2 selectivity) of each test compound. The inhibitory effect on Rac activation by DOCK1 (DHR-2 domain) ($IC_{50}$ value) was determined from the reaction inhibition curve plotted using values of Rac activation by the DOCK1 DHR-2 domain in the presence of various concentrations of the test compounds, taking the values of the control as 100%. The DOCK1-selective inhibitory effect was determined by comparing the inhibitory effect of each test compound on Rac activation by DOCK1 (DHR-2 domain) with the inhibitory effect on Rac activation by DOCK2 (DHR-2 domain), and can be determined by dividing the $IC_{50}$ value representing the inhibitory effect on Rac activation by DOCK2 by the $IC_{50}$ value representing the inhibitory effect on Rac activation by DOCK1. The higher value indicates a higher DOCK1-selective inhibitory effect. Additionally, a comparative control experiment was performed in the same manner as above by using TBOPP, which is known as a DOCK1-selective inhibitor, instead of the compounds of the present invention. As a result, the inhibitory effect ($IC_{50}$ value) on Rac activation by DOCK1 (DHR-2 domain) under the experimental conditions was 8.4 $\mu$M, and the DOCK1-selective inhibitory effect (DOCK1/DOCK2 selectivity) was 2.6.

As shown in Fig. 1, Fig. 2, Fig. 3-1, and Fig. 3-2, the compounds of the present invention were all confirmed to have a high inhibitory effect against DOCK1, in particular a high DOCK1-selective inhibitory effect. Further, as shown in Fig. 1, all of the compounds encompassed by Compounds A except for HS-14 had a higher DOCK1-inhibiting activity than TBOPP. In particular, RT-22, HS-16, RT-23, RT-58, HS-6, HS-14, and HS-20 were confirmed to show higher selective inhibitory activity against DOCK1 than against DOCK2. Compared to TBOPP, HS-14 showed slightly lower DOCK1-inhibiting activity, but showed higher selective inhibitory activity against DOCK1. Further, as shown in Fig. 2, all of the compounds encompassed by Compounds Ba showed higher DOCK1-inhibiting activity than TBOPP. In particular, KS-47, KS-54, KS-43, KS-59, and KS-46 showed higher selective inhibitory activity against DOCK1 than against DOCK2. Furthermore, as shown in Figs. 3-1 and 3-2, all of the compounds encompassed by Compounds Bb except for AK-16 and RT-13 showed higher DOCK1-inhibiting activity than TBOPP; and of these compounds, AK-17, AK-5, AK-15, AK-23, and AK-24 showed higher selective inhibitory activity against DOCK1 than against DOCK2. Compared to TBOPP, AK-16 showed slightly lower DOCK1-inhibiting activity, but showed higher selective inhibitory activity against DOCK1.

As representative examples of the test compounds, Figs. 4 (A) to 4(E) show the results of evaluating the DOCK1-selective inhibitory effect of RT-22 (Production Example 1A) and KS-59 (Production Example 4B). Fig. 4(A) is a graph showing reaction inhibition curves plotting the values of Rac activation by the mouse DOCK1 DHR-2 domain (-●-) and DOCK2 DHR-2 domain **(-▲-)** in the presence of various concentrations of RT-22, taking the values obtained with the addition of DMSO (control) as 100%. The numerical values in the graph are the $IC_{50}$ values for mouse DOCK1 and DOCK2 in order from the top, respectively. Fig. 4(B) is a graph showing reaction inhibition curves plotting the values of Rac activation by the mouse DOCK1 DHR-2 domain (-●-) and DOCK2 DHR-2 domain **(-▲-)** in the presence of various concentrations of KS-59, taking the values obtained with the addition of DMSO (control) as 100%. The numerical values in the graph are the $IC_{50}$ values for mouse DOCK1 and DOCK2 in order from the top, respectively. Fig. 4(C) is a graph showing the reaction inhibition curves plotting the values obtained from an experiment performed in the same manner as above using the human DOCK1 DHR-2 domain (-●-) and DOCK2 DHR-2 domain **(-▲-)** in the presence of various

concentrations of RT-22. The numerical values in the graph are the $IC_{50}$ values for DOCK1 and DOCK2 in order from the top, respectively. Fig. 4(D) is a graph showing the reaction inhibition curves plotting the values obtained from an experiment performed in the same manner using the human DOCK1 DHR-2 domain (-●-) and DOCK2 DHR-2 domain **(-▲-)** in the presence of various concentrations of KS-59. The numerical values in the graph are the $IC_{50}$ values for DOCK1 and DOCK2 in order from the top, respectively. Fig. 4(E) is a graph showing reaction inhibition curves plotting the values of Rac activation by the mouse Trio DH-PH domain (-●-) and Tiam1 DH-PH domain **(-▲-)** in the presence of various concentrations of RT-22, taking the values obtained with the addition of DMSO (control) as 100%. The numerical values in the graph are the $IC_{50}$ values for the mouse Trio DH-PH domain and Tiam1 DH-PH domain in order from the top, respectively.

These results confirmed that the compounds of the present invention show a high inhibitory effect on Rac activation by DOCK1, while showing a significantly lower inhibitory effect on Rac activation by DOCK2, indicating that the compounds of the present invention exhibit a selective inhibitory effect on Rac activation by DOCK1. The results also confirmed that the compounds of the present invention do not affect other GEF family members, in addition to DOCK2.

Pharmacological Test 2: Cell Invasion Inhibition Assay

In this Pharmacological Test, the inhibitory effect of each test compound on cell invasion response was evaluated using a mouse lung carcinoma cell line (3LL cells), a mouse pancreatic cancer cell line (PANC02 cells), and a human breast cancer cell line (MDA-MB-157 cells). Of these, the mouse lung carcinoma cell line (3LL) is a cancer cell line with mutant Ras (K-Ras (G12C)), and the human breast cancer cell line (MDA-MB-157) is a cancer cell line with mutant Rac (Rac1 (P29S)). The experiments were performed in BD BioCoat Matrigel invasion chambers (BD Biosciences).

Specifically, an upper chamber with a Matrigel-coated membrane insert (8 μm pores) at the bottom was equilibrated with DMEM (serum-free medium) for 120 minutes. Thereafter, 300 μL of a suspension of a cancer cell line in DMEM (serum-free) was placed in the upper chamber while 500 μL of DMEM (with 10 mass% FCS) was placed in the lower chamber. Each test compound dissolved in DMSO at a predetermined concentration was added to both the culture media in the upper and lower chambers. As a control, DMSO alone was added to both the culture media in the upper and lower chambers. In all of the experiments, the final DMSO concentration in the culture media was adjusted to 0.2 mass%.

The resulting products were cultured under 5% $CO_2$ at 37°C for 22 hours (3LL and PANC02 cells) or 48 hours (MDA-MB-157 cells). Then, the upper chamber was removed, and the culture medium was removed. Thereafter, non-invading cells remaining in the upper chamber were removed using a cotton swab, and the cells remaining outside the bottom of the upper chamber (cells that had invaded into the Matrigel, and passed through the membrane insert) were stained with Diff-Quick (Sysmex). After staining, the membrane inserts with Matrigel were cut out using a scalpel to prepare slides, and the number of invading cells was counted under an optical microscope.

The results were evaluated based on percent cell invasion (%: Invasion) and percent inhibition (%: Inhibition). The percent cell invasion was obtained by converting the number of invading cells in the presence of each test compound into a percentage, taking the number of invading cells in the control test, in which DMSO was added alone, as 100%; and the percent inhibition was obtained by deducting the percent cell invasion from 100.

The results are shown in column C in Fig. 1, Fig. 2, Fig. 3-1, and Fig. 3-2. Column C in Fig. 1, Fig. 2, Fig. 3-1, and Fig. 3-2 shows the percent cell invasion inhibition (%: Inhibition) of each test compound (33 types) against the mouse lung carcinoma cell line (3LL cells). A comparative control experiment was also performed in the same manner using TBOPP, which is known as a DOCK1-selective inhibitor, instead of the compounds of the present invention, which resulted in the percent cell invasion inhibition of 95%. As representative examples of the test compounds, Figs. 5-1 (A) (B) and 5-2 (C) (D) show the results of cancer cell-invasion inhibitory effect of RT-22 (Production Example 1A), KS-59 (Production Example 4B), HS-6 (Production Example 6A), HS-20 (Production Example 8A), and RT-13 (Production Example 14B), in comparison with the invasion inhibitory effect of TBOPP. Fig. 5-1 (A) is a graph showing the effect of RT-22 and KS-59 on the mouse lung carcinoma cell line (3LL cells). Fig. 5-1(B) is a graph showing the effect of HS-6, HS-20 and RT-13 on the mouse lung carcinoma cell line (3LL cells). Fig. 5-2(C) is a graph showing the effect of RT-22 on the mouse pancreatic cancer cell line (PANC02 cells). Fig. 5-2(D) is a graph showing the effect of RT-22 on the human breast cancer cell line (MDA-MB-157 cells). In all of the graphs, the vertical axis represents the percent cell invasion (%), taking the number of invading cells in the control test as 100%.

As shown in Fig. 1, Fig. 2, Fig. 3-1, and Fig. 3-2, the compounds of the present invention were all confirmed to have high percent cell invasion inhibition against cancer cells. Further, as shown in Fig. 1, Fig. 2, Fig. 3-1, and Fig. 3-2, it was confirmed that RT-22, HS-16, HS-17, RT-58, and HS-6 from among the compounds encompassed by Compound A; the compounds encompassed by Compound Ba except for KS-43; and KS-21, RT-13, KS-16, KS-22, KS-18, and KS-23 from among the compounds encompassed by Compound Bb, in particular, have higher percent cell invasion inhibition than TBOPP.

These results confirmed that the compounds of the present invention show high activity in terms of inhibiting

the invasive potential of cancer cells. In particular, RT-22, which is encompassed by Compound A, and KS-59, which is encompassed by Compound B, were confirmed to show DOCK1-inhibiting activity, DOCK1-selective inhibitory activity, and a cancer cell invasion inhibitory effect that are all higher than those of TBOPP.

Pharmacological Test 3: Inhibition of Macropinocytosis in Cancer Cells

Macropinocytosis is a phenomenon in which cells take up various substances together with extracellular fluid while extending cellular membranes. Remodeling of actin cytoskeleton through Rac activity is known to be important. It has recently been revealed that oncogenic Ras stimulates uptake of extracellular high-molecular-weight proteins by means of macropinocytosis to use them as a source of glutamine supply, which plays an essential function for the survival and growth of cancer cells in a nutrient-poor environment; macropinocytosis has thus received significant attention as a novel target of cancer treatment (NPL 9 and 10).

Therefore, this Pharmacological Test evaluated the inhibitory effect of the compounds of the present invention on macropinocytosis in a mouse lung carcinoma cell line (3LL cells) and a mouse pancreatic cancer cell line (PANC02 cells) as a target. Specifically, 150 μL of mouse lung carcinoma cell line (3LL) or mouse pancreatic cancer cell line (PANC02) ($4x10^4$ cells) was seeded on the glass portion of fibronectin-coated glass-bottom culture dishes. After 16-hour culture at 37°C, the medium was changed to DMEM (serum free), and cultured for another 24 hours to allow the cells to be serum-starved. Subsequently, the medium was changed to 2 mL of serum-free medium containing DMSO alone (control) or DMSO in which each test compound was dissolved, followed by culture for 1 hour for pretreatment. The final concentration of DMSO was adjusted to 0.2 mass%.

DMSO alone (control) or DMSO in which a predetermined concentration of each test compound was dissolved was added in the same manner as above to TMR-dextran (final concentration: 500 μg/mL)-containing DMEM (with 10 mass% FBS), and the thus-obtained medium (180 μL) was added to the cancer cells pretreated as above, followed by incubation at 37°C for 1 hour. The cancer cells were then fixed by 60-minute incubation at room temperature in a 4% paraformaldehyde solution. After washing with PBS 3 times, the nuclei were stained with DAPI (1/3000 dilution) by 5-minute incubation at room temperature, followed by washing with PBS 4 times. Thereafter, observation was performed with a confocal laser point scanning microscope (Zeiss LSM510 META).The TMR-dextran taken up by the cancer cells was observed as spots in cytoplasm. The macropinocytosis activity was measured based on the number of TMR-dextran spots per cell.

The results were shown based on the macropinocytosis activity (the number of TMR-dextran uptake per cell) in terms of each test compound, relative to the macropinocytosis activity in the control test (DMSO was added alone) taken as 1. As representative examples of the test compounds, Figs. 6A to 6D show the results of the measurement of the macropinocytosis activity in the mouse lung carcinoma cell line (3LL cells) (Fig. 6(A) and 6(B)) and the mouse pancreatic cancer cell line (PANC02 cells) (Fig. 6(C) and 6(D)) in terms of RT-22 (Production Example 1A) .

These results confirm that RT-22 and other compounds of the present invention have an inhibitory effect on macropinocytosis activity in cancer cells.

Pharmacological Test 4: Evaluation on T Lymphocyte Migration

Lymphocyte migration plays key roles in immune responses. T cell stimulation by chemokines, such as CCL21, induces actin cytoskeletal remodeling through Rac activation. This drives the cells to migrate towards the source of chemokines. To activate Rac in lymphocytes, the function of DOCK2 is indispensable, and migration of DOCK2-deficient T cells is thus significantly impaired (NPL 4). In contrast, DOCK1 is not expressed in T cells, and lymphocyte migration does not depend on the function of DOCK1. Accordingly, in order to verify the DOCK1 selectivity of the compounds of the present invention at the cellular level, it is useful to confirm the effect of the compounds of the present invention on the lymphocyte migration.

In view of the above, the effect of RT-22 (Production Example 1A), which serves as a representative of the compounds of the present invention, on the lymphocyte migration was studied. Specifically, mouse spleen cells ($1\times10^7$ cells/mL) were first precultured at 37°C for 1 hour in 0.5 mass% BSA-containing RPMI-1640 (Transwell medium), to which DMSO alone or DMSO in which a predetermined concentration of each test compound was dissolved was added. Subsequently, 500 μL of Transwell medium containing 300 ng/mL of CCL21 and DMSO in which a predetermined concentration of each test compound was dissolved was added to a 24-well plate. Then, Transwell inserts (Corning, pore size: 5 μm) were placed in the wells, into which the precultured mouse spleen cells were loaded at $1x10^6$ cells/100 μL.

After 2-hour incubation at 37°C, the cells that migrated to the lower chamber were collected and stained with PE-labeled anti-Thy1.2 antibody (53-2-1, BD Pharmingen) and APC-labeled anti-B220 antibody (RA-6B2, eBioscience). The percentage (%) of the migrated cells was calculated by dividing the number of $Thy1.2^+$ cells (T cells) in the lower chamber by the number of $Thy1.2^+$ cells (T cells) placed into the Transwell inserts.

Fig. 7 shows the results of RT-22. Fig. 7 clearly indicates that RT-22 had no effect on the migration of CCL21-stimulated T cells. In other words, RT-22 and the other compounds of the present invention were confirmed at the cellular level to selectively inhibit Rac activation by DOCK1. This suggests that the compounds of the present invention are useful as an active ingredient of anticancer agents, with fewer side effects.

Pharmacological Test 5: Effect on Viability of Lymphocytes

As stated above, it is useful to confirm the effect of the compounds of the present invention on the viability of lymphocytes in order to verify the DOCK1 selectivity of the compounds of the present invention at the cellular level. Therefore, the effect of RT-22 (Production Example 1A), which serves as a representative of the compounds of the present invention, on the viability of lymphocytes was studied.

Specifically, $1 \times 10^6$ mouse spleen cells were suspended in 100 μL of RPMI-1640 medium containing 0.5% BSA, and DMSO in which a predetermined concentration of each test compound was dissolved or DMSO alone was added thereto to a final DMSO concentration of 0.2%. After 1-hour culture at 37°C, 2 μL of a propidium iodide staining solution (BD Pharmingen) was added and incubated on ice for 30 minutes, followed by flow cytometry analysis. The percentage (%) of the viable cells under each condition was calculated, considering negative cells to be viable cells.

Fig. 8 shows the results of RT-22. Fig. 8 clearly indicates that RT-22 had no effect also on the viability of T cells. This also suggests that RT-22 and the other compounds of the present invention are useful as an active ingredient of anticancer agents with fewer side effects.

Pharmacological Test 6: Inhibition of Growth of Mouse Lung Carcinoma Cells

The mouse lung carcinoma cell line (3LL) was suspended in 200 μL of PBS(-), and transplanted ($5 \times 10^5$ cells per mouse) into the dorsal subcutaneous region of C57BL/6 mice (6 weeks old). To a 6:1:1 mixture of PBS/Cremophor EL/ethanol, RT-22 as a representative of the compounds of the present invention was added to a concentration of 0.25 mg/300 μL, and the resulting mixture was loaded into an SMP-300 micro infusion pump (iPRECIO), which was subcutaneously implanted on the previous day of the transplantation, to perform continuous intravenous administration (n = 5 mice). The control group was only administered with an equivalent volume of a solvent (a 6:1:1 mixture of PBS/Cremophor EL/ethanol) (n = 6 mice). The transverse and longitudinal diameters of the tumor mass were measured over time for a period of 4 weeks from the start of administration, and the tumor volume (V) was calculated according to the formula: $V = \pi / 6 x \sqrt{((\text{transverse diameter x longitudinal diameter})^3)}$.

Fig. 9 is a graph showing the results regarding the inhibitory effect of RT-22 (Production Example 1A) as a representative of the compounds of the present invention on tumor growth in a model to which the mouse lung carcinoma cell line (3LL) was subcutaneously transplanted. As can be seen from Fig. 9, the administration of RT-22 was confirmed to clearly reduce the tumor tissue size, compared with the control group; and on day 26 to day 29, the tumor size was reduced to 64 to 69% of that of the control group. A similar experiment was performed with the use of TBOPP at a concentration of 0.25 mg/300 μL, which is known as a DOCK1-selective inhibitor with an inhibitory effect on the growth of mouse lung carcinoma cells. On day 26 to day 29, the tumor size was reduced by only 21-24% compared to the control group, indicating that RT-22, which is a compound of the present invention, is more effective in inhibiting tumor growth than TBOPP.

## Claims

1. A compound represented by the following formula (A):

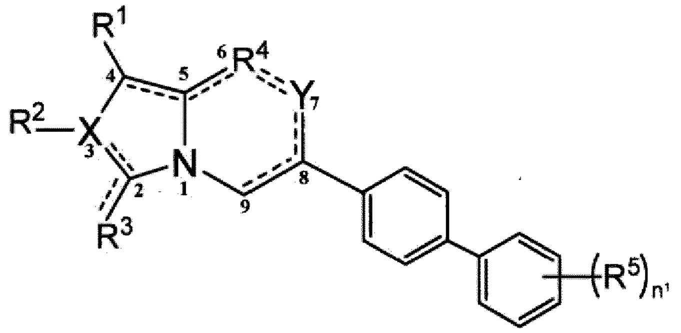

(A)

wherein

X represents a carbon atom or a nitrogen atom;
Y represents an oxygen atom, a hydroxy group, or a hydrocarbon group;
$R^1$ and $R^2$ are different, and each represents a hydrogen atom or a group represented by the following formula (A-1):

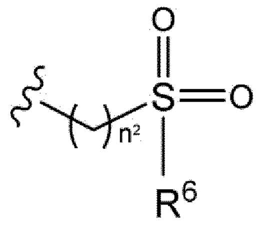

(A－1)

wherein $R^6$ represents a pyrrolidino group or a phenyl group, and $n^2$ is 0 or 1;
$R^3$ represents -CO-$R^7$, wherein $R^7$ is an alkoxy group, an alkyl group, or an alkylamino group, a 1,3-oxazole group, an alkylhydroxy group, a hydrogen atom, or an oxygen atom;
$R^4$ represents a hydrogen atom, an oxygen atom, or a hydrocarbon group in which one or more hydrogen atoms may be replaced by one or more substituents;
$R^5$ represents a halogen atom, a halogenated alkyl group, or a halogenated alkylthio group; and
$n^1$ is an integer of 0 to 5; and

in the skeleton of the compound of formula (A),

each single solid line represents a single bond;
each double line, which consists of a solid line and a dotted line, represents a single bond or a double bond; and two dotted lines represent no bond or a double bond; or
a salt thereof.

**2.** The compound according to claim 1,
wherein in formula (A),

X is a carbon atom;
Y is an oxygen atom or a hydroxy group;
$R^1$ is a group represented by formula (A-1), wherein $R^6$ is a pyrrolidino group and $n^2$ is 0;
$R^2$ is a hydrogen atom;
$R^3$ is -CO-$R^7$, wherein $R^7$ is an alkoxy group, an alkyl group, or an alkylamino group, a 1,3-oxazole group, or an alkylhydroxy group;
$R^4$ is a hydrogen atom;
$R^5$ is a halogen atom, a halogenated alkyl group, or a halogenated thio group; and
$n^1$ is an integer of 1 to 3; and

in the skeleton of the compound,

the double line between 2-position and 3-position and the double line between 4-position and 5-position, which each consist of a solid line and a dotted line, represent a double bond;
the double line between 7-position and 8-position, which consists of a solid line and a dotted line, represents a single bond when Y is a hydroxy group, and the double line represents a double bond when Y is an oxygen atom;
the double line between 5-position and 6-position and the double line between 8-position and 9-position, which each consist of a solid line and a dotted line, represent a single bond;
the two dotted lines between 6-position and 7-position represent no bond; and
$R^3$ is bound to 2-position by a single bond.

**3.** The compound according to claim 1,
wherein in formula (A),

X is a carbon atom;
Y is an oxygen atom;
$R^1$ is a group represented by formula (A-1), wherein $R^6$ is a pyrrolidino group and $n^2$ is 0;
$R^2$ is a hydrogen atom;
$R^3$ is -CO-$R^7$, wherein $R^7$ is an alkoxy group, an alkyl group, or an alkylamino group, or a 1,3-oxazole group;
$R^4$ is a hydrogen atom;
$R^5$ is a halogenated alkyl group; and
$n^1$ is 1; and

in the skeleton of the compound,

the double line between 2-position and 3-position, the double line between 4-position and 5-position, and the double line between 7-position and 8-position, which each consist of a solid line and a dotted line, represent a double bond;
the double line between 5-position and 6-position and the double line between 8-position and 9-position, which each consist of a solid line and a dotted line, represent a single bond;
the two dotted lines between 6-position and 7-position represent no bond; and
$R^3$ is bound to 2-position by a single bond.

**4.** A compound selected from the group consisting of the following compounds:

(A1) methyl 1-(2-oxo-2-(3'-(trifluoromethyl)-[1,1'-biphenyl]-4-yl)ethyl)-4-(pyrrolidin-1-yl-sulfonyl)-1H-pyrrole-2-carboxylate;
(A2) 2-(2-acetyl-4-(pyrrolidin-1-yl-sulfonyl)-1H-pyrrol-1-yl)-1-(3'-(trifluoromethyl)-[1,1'-biphenyl]-4-yl)ethane;
(A3) 1-(1-(2-oxo-2-(3'-(trifluoromethyl)-[1,1'-biphenyl]-4-yl)ethyl)-4-(pyrrolidin-1-yl-sulfonyl)-1H-pyrrol-2-yl)propan-1-one;
(A4) N,N-dimethyl-1-(2-oxo-2-(3'-(trifluoromethyl)-[1,1'-biphenyl]-4-yl)ethyl)-4-(pyrrolidin-1-yl-sulfonyl)-1H-pyrrole-2-carboxamide;
(A5) 2-(2-(oxazol-2-yl)-4-(pyrrolidin-1-yl-sulfonyl)-1H-pyrrol-1-yl)-1-(3'-(trifluoromethyl)-[1,1'-biphenyl]-4-yl)ethan-1-one;
(A6) methyl 1-(2-hydroxy-2-(3'-(trifluoromethyl)-[1,1'-biphenyl]-4-yl)ethyl)-4-(pyrrolidin-1-yl-sulfonyl)-1H-pyrrole-2-carboxylate;
(A7) 2-(2-(hydroxymethyl)-4-(pyrrolidin-1-yl-sulfonyl)-1H-pyrrol-1-yl)-1-(3'-(trifluoromethyl)-[1,1'-biphenyl]-4-yl)ethan-1-ol;
(A8) 2-(pyrrolidin-1-yl-sulfonyl)-6-(3'-(trifluoromethyl)-[1,1'-biphenyl]-4-yl)indolizin-8-yl acetate; and
(A9) 3-(2-oxo-2-(3'-(trifluoromethyl)-[1,1'-biphenyl]-4-yl)ethyl)-5-((phenylsulfonyl)methyl)imidazolidine-2,4-dione; or

a salt thereof.

**5.** A compound represented by the following formula (B):

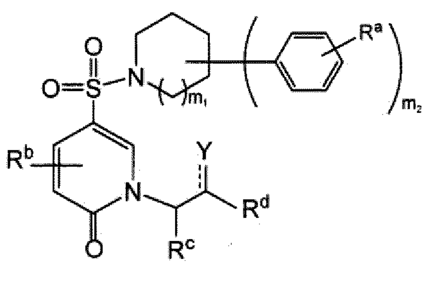
(B)

wherein

$R^a$ is one of the groups represented by the following formulas (B-1) to (B-4):

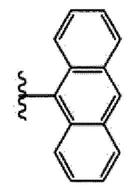

(B-1) ,

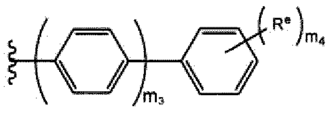

(B-2)

wherein $R^e$ is the same or different, and each represents a halogen atom, a trihaloalkyl group, or a trihaloalkylthio group; $m_3$ is an integer of 0 to 2; and $m_4$ is an integer of 0 to 5,

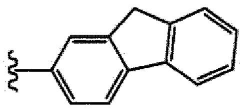

(B-3) , and

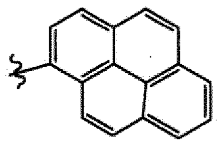

(B-4) ;

$R^b$ is a hydrogen atom or an amino group;
$R^c$ is a hydrogen atom or an alkyl group;
$R^d$ is
a group represented by the following formula (B-5):

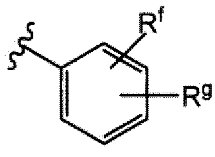

(B-5)

wherein $R^f$ is a hydrogen atom, a hydroxy group, an alkyl group, an acyloxy group, or an alkoxy group, wherein the alkoxy group may be linked to Y to form a ring; and $R^g$ is a group represented by the following formula (B-6), or a quinolyl group:

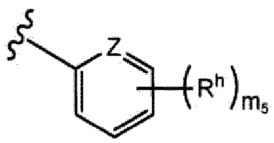

(B-6)

wherein Z is a carbon atom or a nitrogen atom; $R^h$ is the same or different, and each represents a halogen atom, a halogenated alkyl group, a halogenated alkylthio group, an alkyl group, an alkenyl group, or an alkylamino group; and $m_5$ is an integer of 1 to 3, or
a group represented by formula (B-7):

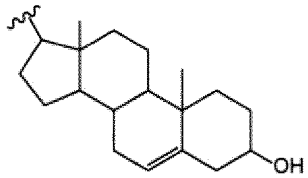

(B-7) ;

Y is an oxygen atom, a hydroxy group, or an alkoxy group, wherein when $R^d$ is a group represented by formula (B-5), the alkoxy group may be linked to an alkoxy group represented by $R^f$ to form a ring;
$m_1$ is 0 or 1;
$m_2$ is 0 or 1; and
the double line, which consists of a dotted line and a solid line, represents a double bond when Y is an oxygen atom, and the double line represents a single bond when Y is a hydroxy group or an alkoxy group; or
a salt thereof.

**6.** The compound according to Claim 5,
wherein in formula (B),

$m_1$ and $m_2$ are both 1;
Y is an oxygen atom;
$R^a$ is one of the groups represented by the following formulas (B-1) to (B-4):

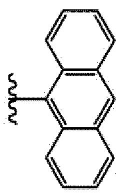

(B-1) ,

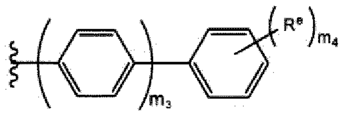

(B-2)

wherein $R^e$ is the same or different, and each represents a halogen atom, a halogenated alkyl group, or a halogenated alkylthio group; $m_3$ is an integer of 0 to 2; and $m_4$ is an integer of 0 to 5;

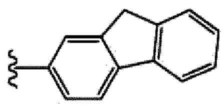

(B-3) , and

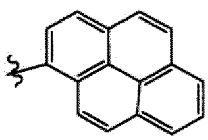

(B-4) ;

$R^b$ and $R^c$ are both a hydrogen atom; and
$R^d$ is a group represented by the following formula (B-5) :

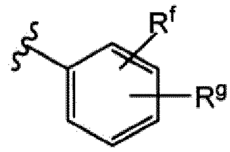

(B − 5)

wherein $R^f$ is a hydrogen atom, and $R^g$ is a group represented by the following formula (B-6):

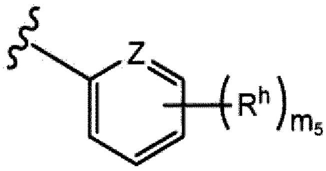

(B − 6)

wherein Z is a carbon atom; $R^h$ is the same or different, and each represents a halogen atom, a halogenated alkyl group, or a halogenated alkylthio group; and $m_5$ is an integer of 1 to 3.

**7.** The compound according to claim 5,
wherein in formula (B),

$m_1$ and $m_2$ are both 0;
$R^b$ is a hydrogen atom or an amino group;
$R^c$ is a hydrogen atom or an alkyl group;
$R^d$ is
a group represented by the following formula (B-5):

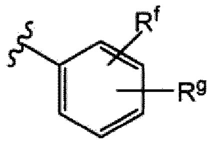

(B − 5)

wherein $R^f$ is the same or different, and each represents a hydrogen atom, a hydroxy group, an alkyl group, an acyloxy group, or an alkoxy group, wherein the alkoxy group may be linked to Y to form a ring; $R^g$ is a group represented by the following formula (B-6), or a quinolyl group:

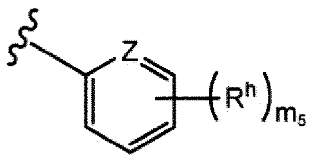

(B − 6)

wherein Z is a carbon atom or a nitrogen atom; $R^h$ is the same or different, and each represents a halogen atom, a halogenated alkyl group, a halogenated alkylthio group, an alkyl group, an alkylene group, or an alkylamino group; and $m_5$ is an integer of 1 to 3; or
a group represented by the following formula (B-7):

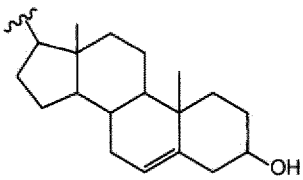

(B − 7)

;

Y is an oxygen atom, a hydroxy group, or an alkoxy group, wherein when $R^d$ is a group represented by formula (B-5), the alkoxy group may be linked to an alkoxy group represented by $R^f$ to form a ring.

**8.** A compound selected from the group consisting of the following compounds:

(B1) 5-((4-(4-(anthracen-9-yl)phenyl)piperidin-1-yl)sulfonyl)-1-(2-oxo-2-(3'-(trifluoromethyl)-[1,1'-biphenyl]-4-yl)ethyl)pyridin-2(1H)-one,
(B2) 1-(2-oxo-2-(3'-(trifluoromethyl)-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one,
(B3) 5-((4-([1,1':4',1":4",1"'-quaterphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one,
(B4) 5-((4-([1,1'-biphenyl]-4-yl)piperidin-1-yl)sulfonyl)-1-(2-oxo-2-(3'-(trifluoromethyl)-[1,1'-biphenyl]-4-yl)ethyl)pyridin-2 (1H)-one,
(B5) 1-(2-oxo-2-(3',4',5'-trifluoro-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one,
(B6) 5-((4-(4-(9H-fluoren-2-yl)phenyl)piperidin-1-yl)sulfonyl)-1-(2-oxo-2-(3'-(trifluoromethyl)-[1,1'-biphenyl]-4-yl)ethyl)pyridin-2(1H)-one, and
(B7) 1-(2-oxo-2-(3'-(trifluoromethyl)-[1,1'-biphenyl]-4-yl)ethyl)-5-((4-(4-(pyren-1-yl)phenyl)piperidin-1-yl)sulfonyl)pyridin-2(1H)-one; or

a salt thereof.

**9.** A compound selected from the group consisting of the following compounds:

(B8) 1-(2-hydroxy-2-(3-hydroxy-3'-(trifluoromethyl)-[1,1'-biphenyl]-4-yl)ethyl)-5-(pyrrolidin-1-yl-sulfonyl)pyridin-2(1H)-one,
(B9) 1-((2,2-dimethyl-7-(3-(trifluoromethyl)phenyl)-4H-benzo[d][1,3]dioxin-4-yl)methyl)-5-(pyrrolidin-1-yl-sulfonyl)pyridin-2(1H)-one,
(B10) 1-(2-oxo-2-(3'-((trifluoromethyl)thio)-[1,1'-biphenyl]-4-yl)ethyl)-5-(pyrrolidin-1-yl-sulfonyl)pyridin-2(1H)-one,
(B11) 3-amino-1-(2-oxo-2-(3-(trifluoromethyl)-[1,1'-biphenyl]-4-yl)ethyl)-5-(pyrrolidin-1-yl-sulfonyl)pyridin-2(1H)-one,
(B12) 2-bromo-1-(3-methyl-3'-(trifluoromethyl)-[1,1'-biphenyl]-4-yl)ethan-1-one,
(B13) 1-(2-(3-hydroxy-3'-(trifluoromethyl)-[1,1'-biphenyl]-4-yl)-2-oxoethyl)-5-(pyrrolidin-1-yl-sulfonyl)pyridin-2(1H)-one,
(B14) 1-(2-(3-methoxy-3'-(trifluoromethyl)-[1,1'-biphenyl]-4-yl)-2-oxoethyl)-5-(pyrrolidin-1-yl-sulfonyl)pyridin-2(1H)-one,
(B15) 4-(2-(2-oxo-5-(pyrrolidin-1-yl-sulfonyl)pyridin-1(2H)-yl)acetyl)-3'-(trifluoromethyl)-[1,1'-biphenyl]-3-yl-acetate, (B16) 1-(2-(3-isobutoxy-3'-(trifluoromethyl)-[1,1'-biphenyl]-4-yl)-2-oxoethyl)-5-(pyrrolidin-1-yl-sulfonyl)pyridin-2(1H)-one,
(B17) 1-(1-(3-(hexyloxy)-3'-(trifluoromethyl)-[1,1'-biphenyl]-4-yl)-1-oxooctan-2-yl)-5-(pyrrolidin-1-yl-sulfonyl)pyridin-2(1H)-one,
(B18) 1-(2-oxo-2-(3-phenethoxy-3'-(trifluoromethyl)-[1,1'-biphenyl]-4-yl)ethyl)-5-(pyrrolidin-1-yl-sulfonyl)pyridin-2(1H)-one,
(B19) 1-(2-oxo-2-(3'-vinyl-[1,1'-biphenyl]-4-yl)ethyl)-5-(pyrrolidin-1-yl-sulfonyl)pyridin-2(1H)-one,
(B20) 1-(2-(4'-(dimethylamino)-3'-methyl-[1,1'-biphenyl]-4-yl)-2-oxoethyl)-5-(pyrrolidin-1-yl-sulfonyl)pyridin-2(1H)-one,
(B21) 1-(2-oxo-2-(4-(6-(trifluoromethyl)pyridin-2-yl)phenyl)ethyl)-5-(pyrrolidin-1-yl-sulfonyl)pyridin-2(1H)-one,
(B22) 1-(2-oxo-2-(4-(quinolin-7-yl)phenyl)ethyl)-5-(pyrrolidin-1-yl-sulfonyl)pyridin-2(1H)-one,
(B23) 1-(2-oxo-2-(4-(quinolin-6-yl)phenyl)ethyl)-5-(pyrrolidin-1-yl-sulfonyl)pyridin-2(1H)-one, and
(B24) 1-(2-((3S,10R,13S,17S)-3-hydroxy-10,13-dimethyl-2,3,4,7,8,9,10,11,12,13,14,15,16,17-tetradecahydro-1H-cyclopenta[a]phenanthren-17-yl)-2-oxoethyl)-5-(pyrrolidin-1-yl-sulfonyl)pyridin-2(1H)-one;

or a salt thereof.

**10.** A DOCK1 selective inhibitor comprising as an active ingredient at least one compound selected from the group consisting of the compounds of any one of claims 1 to 9 and salts thereof.

**11.** A pharmaceutical composition comprising at least one member selected from the group consisting of the compounds of any one of claims 1 to 9 and salts thereof, and a pharmaceutically acceptable carrier or an additive.

**12.** The pharmaceutical composition according to claim 11, which is for use in the treatment and/or prevention of a cancer.

**13.** The pharmaceutical composition according to claim 12, wherein the cancer is an invasive or metastatic cancer.

Fig. 1

| | | | A | B | C |
|---|---|---|---|---|---|
| | Pyrrole-type compound A | Chemical formula | GEF assay ($IC_{50}$) against DOCK1 | Selectivity (DOCK1/DOCK2) | Cell invasion assay against 3LL cells |
| RT-22 | Methyl 1-(2-oxo-2-(3'-(trifluoromethyl)-[1,1'-biphenyl]-4-yl)ethyl)-4-(pyrrolidin-1-yl-sulfonyl)-1H-pyrrole-2-carboxylate | 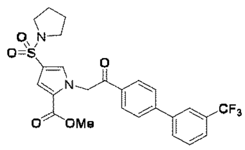 | 3.6 | 2.9 | 98 |
| HS-16 | 2-(2-Acetyl-4-(pyrrolidin-1-ylsulfonyl)-1H-pyrrol-1-yl)-1-(3'-(trifluoromethyl)-[1,1'-biphenyl]-4-yl)ethane | 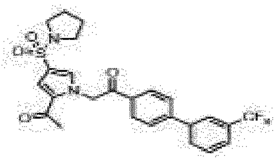 | 4.8 | 2.9 | 96 |
| HS-17 | 1-(1-(2-Oxo-2-(3'-(trifluoromethyl)-[1,1'-biphenyl]-4-yl)ethyl)-4-(pyrrolidin-1-ylsulfonyl)-1H-pyrrol-2-yl)propan-1-one | 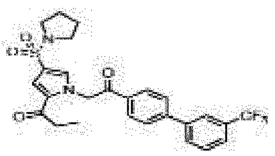 | 5.9 | 2.2 | 96 |
| RT-23 | N,N-Dimethyl-1-(2-oxo-2-(3'-(trifluoromethyl)-[1,1'-biphenyl]-4-yl)ethyl)-4-(pyrrolidin-1-ylsulfonyl)-1H-pyrrole-2-carboxamide | 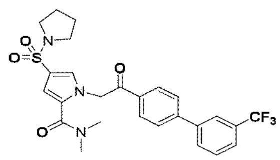 | 7.2 | 2.6 | 85 |
| RT-58 | 2-(2-(Oxazol-2-yl)-4-(pyrrolidin-1-ylsulfonyl)-1H-pyrrol-1-yl)-1-(3'-(trifluoromethyl)-[1,1'-biphenyl]-4-yl)ethan-1-one | 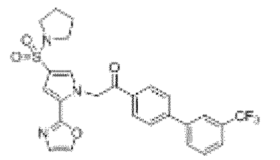 | 2.5 | 3 | 97 |
| HS-6 | Methyl 1-(2-hydroxy-2-(3'-(trifluoromethyl)-[1,1'-biphenyl]-4-yl)ethyl)-4-(pyrrolidin-1-yl-sulfonyl)-1H-pyrrole-2-carboxylate | 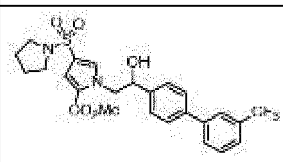 | 8.2 | 2.9 | 99 |
| HS-14 | 2-(2-(Hydroxymethyl)-4-(pyrrolidin-1-ylsulfonyl)-1H-pyrrol-1-yl)-1-(3'-(trifluoromethyl)-[1,1'-biphenyl]-4-yl)ethan-1-ol | 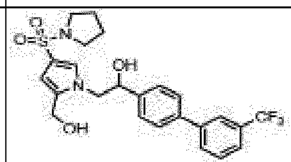 | 10 | 3.5 | 93 |
| HS-20 | 2-(Pyrrolidin-1-ylsulfonyl)-6-(3'-(trifluoromethyl)-[1,1'-biphenyl]-4-yl)indolizin-8-yl acetate | 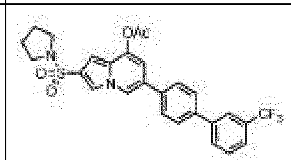 | 2.3 | 3.7 | 91 |
| RT-35 | 3-(2-Oxo-2-(3'-(trifluoromethyl)-[1,1'-biphenyl]-4-yl)ethyl)-5-((phenylsulfonyl)methyl)imidazolidine-2,4-dione | 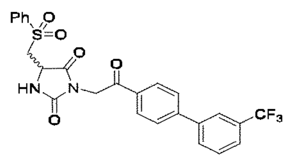 | 2.7 | 2.4 | 88 |

Fig. 2

| | | | A | B | C |
|---|---|---|---|---|---|
| | Substituted aza-alicyclic compound Ba | Chemical formula | GEF assay ($IC_{50}$) against DOCK1 | Selectivity (DOCK1/DOCK2) | Cell invasion assay against 3LL cells |
| KS-47 | 5-((4-(4-(Anthracen-9-yl)phenyl)piperidin-1-yl)sulfonyl)-1-(2-oxo-2-(3'-(trifluoromethyl)-[1,1'-biphenyl]-4-yl)ethyl)pyridin-2(1H)-one | 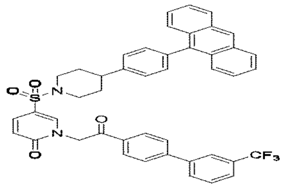 | 1.3 | 3 | 96 |
| KS-54 | 1-(2-Oxo-2-(3'-(trifluoromethyl)-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one | 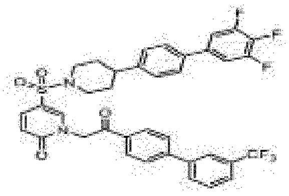 | 2.8 | 2.7 | 96 |
| KS-44 | 5-((4-([1,1':4',1'':4'',1'''-Quaterphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one | 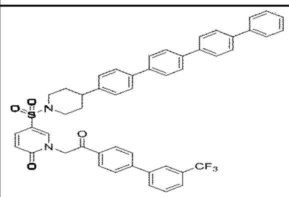 | 1.4 | 2.5 | 99 |
| KS-43 | 5-((4-([1,1'-Biphenyl]-4-yl)piperidin-1-yl)sulfonyl)-1-(2-oxo-2-(3'-(trifluoromethyl)-[1,1'-biphenyl]-4-yl)ethyl)pyridin-2(1H)-one | 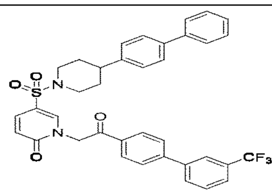 | 1.9 | 2.9 | 88 |
| KS-59 | 1-(2-Oxo-2-(3',4',5'-trifluoro-[1,1'-biphenyl]-4-yl)ethyl)-5-((4-(3',4',5'-trifluoro-[1,1'-biphenyl]-4-yl)piperidin-1-yl)sulfonyl)pyridin-2(1H)-one | 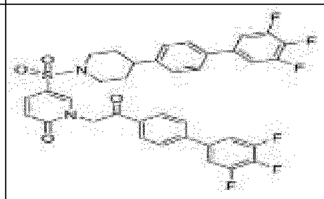 | 1.7 | 3.3 | 96 |
| KS-46 | 5-((4-(4-(9H-Fluoren-2-yl)phenyl)piperidin-1-yl)sulfonyl)-1-(2-oxo-2-(3'-(trifluoromethyl)-[1,1'-biphenyl]-4-yl)ethyl)pyridin-2(1H)-one | 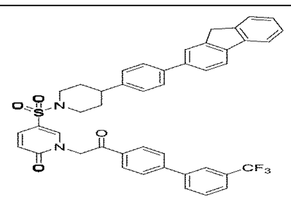 | 1.7 | 2.6 | 98 |
| KS-45 | 1-(2Oxo-2-(3'-(trifluoromethyl)-[1,1'-biphenyl]-4-yl)ethyl)-5-((4-(4-(pyren-1-yl)phenyl)piperidin-1-yl)sulfonyl)pyridin-2(1H)-one | 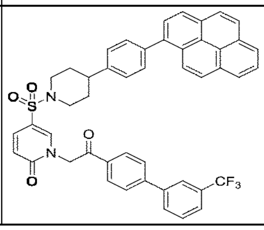 | 1.4 | 2.3 | 97 |

Fig. 3-1

| | Substituted aza-alicyclic compound Bb | Chemical formula | A | B | C |
|---|---|---|---|---|---|
| | | | GEF assay ($IC_{50}$) against DOCK1 | Selectivity (DOCK1/DOCK2) | Cell invasion assay against 3LL cells |
| AK-16 | 1-(2-Hydroxy-2-(3-hydroxy-3'-(trifluoromethyl)-[1,1'-biphenyl]-4-yl)ethyl)-5-(pyrrolidin-1-ylsulfonyl)pyridin-2(1H)-one | 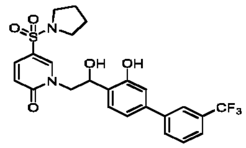 | 16.5 | 2.9 | 90 |
| AK-17 | 1-((2,2-Dimethyl-7-(3-(trifluoromethyl)phenyl)-4H-benzo[d][1,3]dioxin-4-yl)methyl)-5-(pyrrolidin-1-ylsulfonyl)pyridin-2(1H)-one | 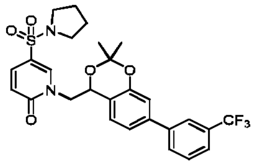 | 5.5 | 2.8 | — |
| KS-21 | 1-(2-Oxo-2-(3'-((trifluoromethyl)thio)-[1,1'-biphenyl]-4-yl)ethyl)-5-(pyrrolidin-1-ylsulfonyl)pyridin-2(1H)-one | 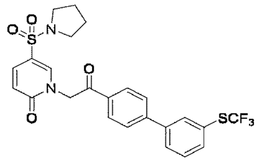 | 5 | 1.9 | 98 |
| RT-13 | 3-Amino-1-(2-oxo-2-(3'-(trifluoromethyl)-[1,1'-biphenyl]-4-yl)ethyl)-5-(pyrrolidin-1-ylsulfonyl)pyridin-2(1H)-one | 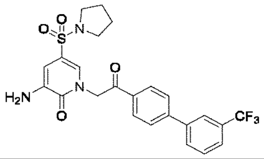 | 8.7 | 2 | 100 |
| AK-5 | 2-Bromo-1-(3-methyl-3'-(trifluoromethyl)-[1,1'-biphenyl]-4-yl)ethan-1-one | 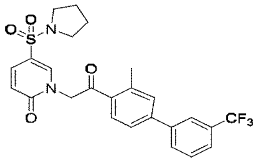 | 4.5 | 2.8 | 88 |
| AK-15 | 1-(2-(3-Hydroxy-3'-(trifluoromethyl)-[1,1'-biphenyl]-4-yl)-2-oxoethyl)-5-(pyrrolidin-1-ylsulfonyl)pyridin-2(1H)-one | 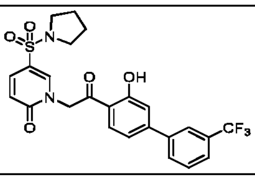 | 5.8 | 2.7 | — |
| AK-14 | 1-(2-(3-Methoxy-3'-(trifluoromethyl)-[1,1'-biphenyl]-4-yl)-2-oxoethyl)-5-(pyrrolidin-1-ylsulfonyl)pyridin-2(1H)-one | 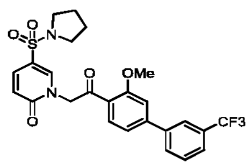 | 5.6 | 2.4 | — |
| AK-25 | 4-(2-(2-Oxo-5-(pyrrolidin-1-ylsulfonyl)pyridin-1(2H)-yl)acetyl)-3'-(trifluoromethyl)-[1,1'-biphenyl]-3-yl acetate | 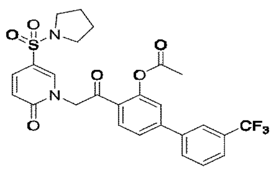 | 6.3 | 2 | 79 |
| AK-22 | 1-(2-(3-Isobutoxy-3'-(trifluoromethyl)-[1,1'-biphenyl]-4-yl)-2-oxoethyl)-5-(pyrrolidin-1-ylsulfonyl)pyridin-2(1H)-one | 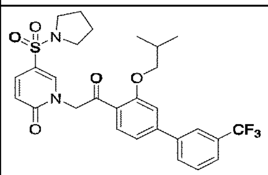 | 4.5 | 2.3 | — |

Fig. 3-2

| | Substituted aza-alicyclic compound Bb | Chemical formula | A<br>GEF assay ($IC_{50}$) against DOCK1 | B<br>Selectivity (DOCK1/DOCK2) | C<br>Cell invasion assay against 3LL cells |
|---|---|---|---|---|---|
| AK-23 | 1-(1-(3-(Hexyloxy)-3'-(trifluoromethyl)-[1,1'-biphenyl]-4-yl)-1-oxooctan-2-yl)-5-(pyrrolidin-1-ylsulfonyl)pyridin-2(1H)-one | 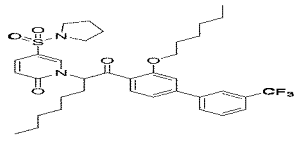 | 4.4 | 3.5 | — |
| AK-24 | 1-(2-Oxo-2-(3-phenethoxy-3'-(trifluoromethyl)-[1,1'-biphenyl]-4-yl)ethyl)-5-(pyrrolidin-1-ylsulfonyl)pyridin-2(1H)-one | 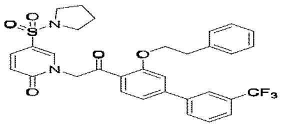 | 3.7 | 2.7 | — |
| KS-26 | 1-(2-Oxo-2-(3'-vinyl-[1,1'-biphenyl]-4-yl)ethyl)-5-(pyrrolidin-1-yl-sulfonyl)pyridin-2(1H)-one | 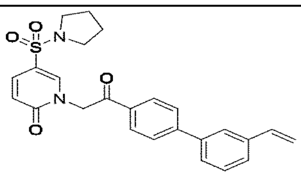 | 6 | 2 | 86 |
| KS-16 | 1-(2-(4'-(Dimethylamino)-3'-methyl-[1,1'-biphenyl]-4-yl)-2-oxoethyl)-5-(pyrrolidin-1-ylsulfonyl)pyridin-2(1H)-one | 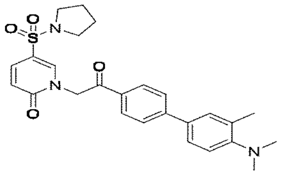 | 7.1 | 1.7 | 96 |
| KS-22 | 1-(2-Oxo-2-(4-(6-(trifluoromethyl)pyridin-2-yl)phenyl)ethyl)-5-(pyrrolidin-1-ylsulfonyl)pyridin-2(1H)-one | 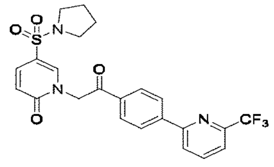 | 8.4 | 1.6 | 97 |
| KS-18 | 1-(2-Oxo-2-(4-(quinolin-7-yl)phenyl)ethyl)-5-(pyrrolidin-1-ylsulfonyl)pyridin-2(1H)-one | 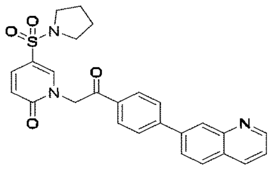 | 8.1 | 2 | 97 |
| KS-23 | 1-(2-Oxo-2-(4-(quinolin-6-yl)phenyl)ethyl)-5-(pyrrolidin-1-ylsulfonyl)pyridin-2(1H)-one | 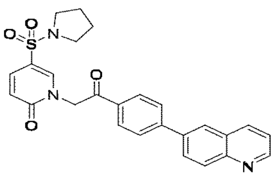 | 8 | 1.7 | 95 |
| AK-20 | 1-(2-((3S,10R,13S,17S)-3-Hydroxy-10,13-dimethyl-2,3,4,7,8,9,10,11,12,13,14,15,16,17-tetradecahydro-1H-cyclopenta[a]phenanthren-17-yl)-2-oxoethyl)-5-(pyrrolidin-1-ylsulfonyl)pyridin-2(1H)-one | 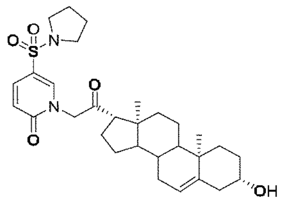 | 6.4 | 1.9 | 92 |

Fig. 4

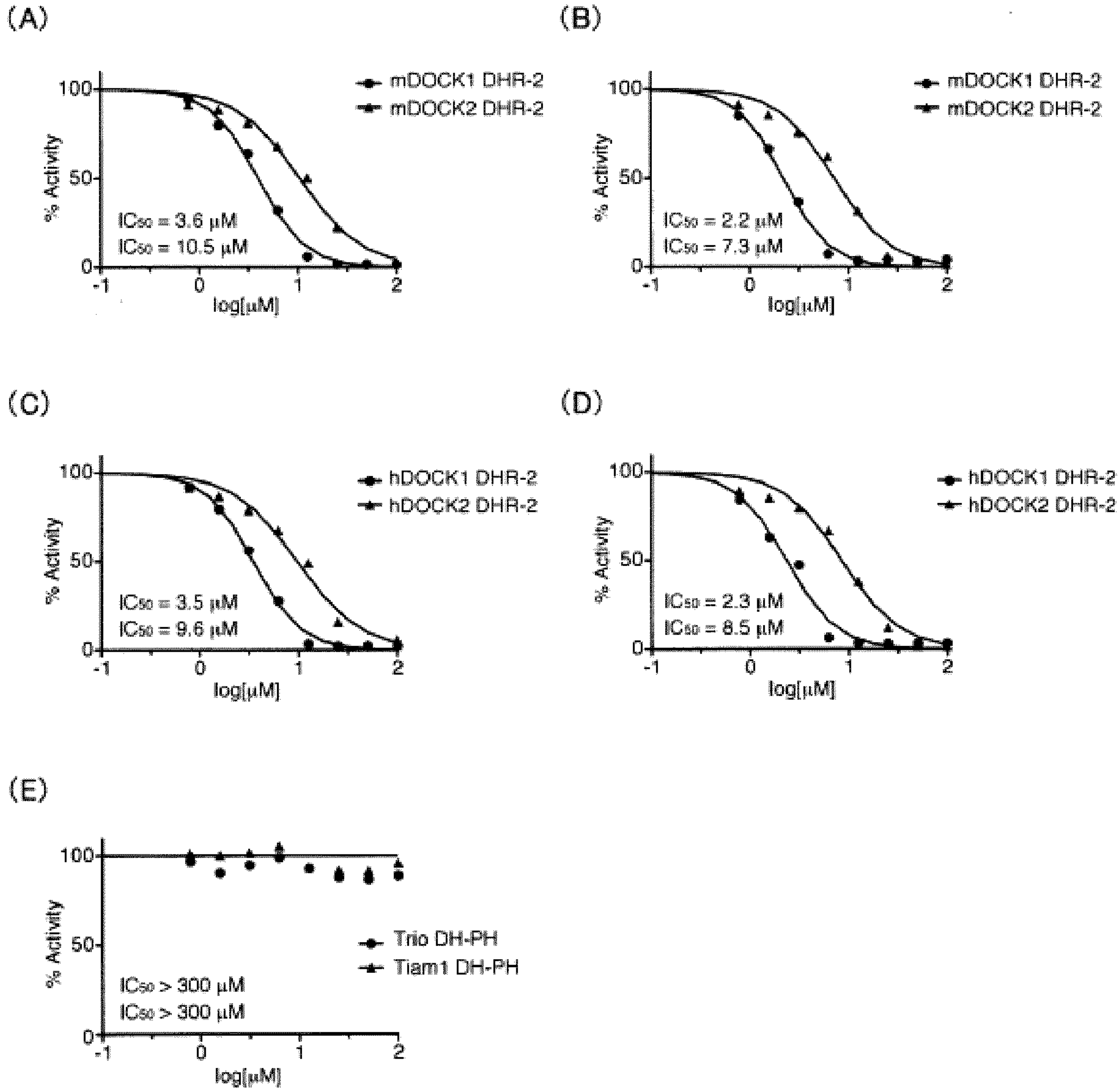
(A)
mDOCK1 DHR-2
mDOCK2 DHR-2
% Activity
log[μM]
IC50 = 3.6 μM
IC50 = 10.5 μM
(B)
mDOCK1 DHR-2
mDOCK2 DHR-2
% Activity
log[μM]
IC50 = 2.2 μM
IC50 = 7.3 μM
(C)
hDOCK1 DHR-2
hDOCK2 DHR-2
% Activity
log[μM]
IC50 = 3.5 μM
IC50 = 9.6 μM
(D)
hDOCK1 DHR-2
hDOCK2 DHR-2
% Activity
log[μM]
IC50 = 2.3 μM
IC50 = 8.5 μM
(E)
Trio DH-PH
Tiam1 DH-PH
% Activity
log[μM]
IC50 > 300 μM
IC50 > 300 μM

Fig. 5-1

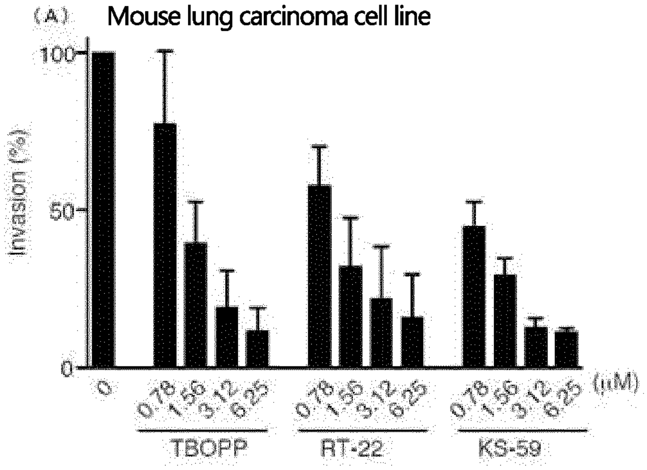
(A) Mouse lung carcinoma cell line
Invasion (%)
100
50
0
0
0.78
1.56
3.12
6.25
(μM)
TBOPP
RT-22
KS-59

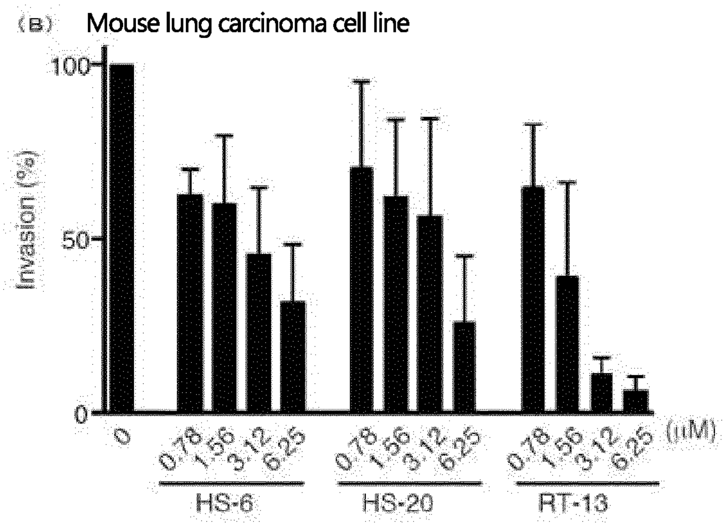
(B) Mouse lung carcinoma cell line
Invasion (%)
100
50
0
0
0.78
1.56
3.12
6.25
(μM)
HS-6
HS-20
RT-13

Fig. 5-2

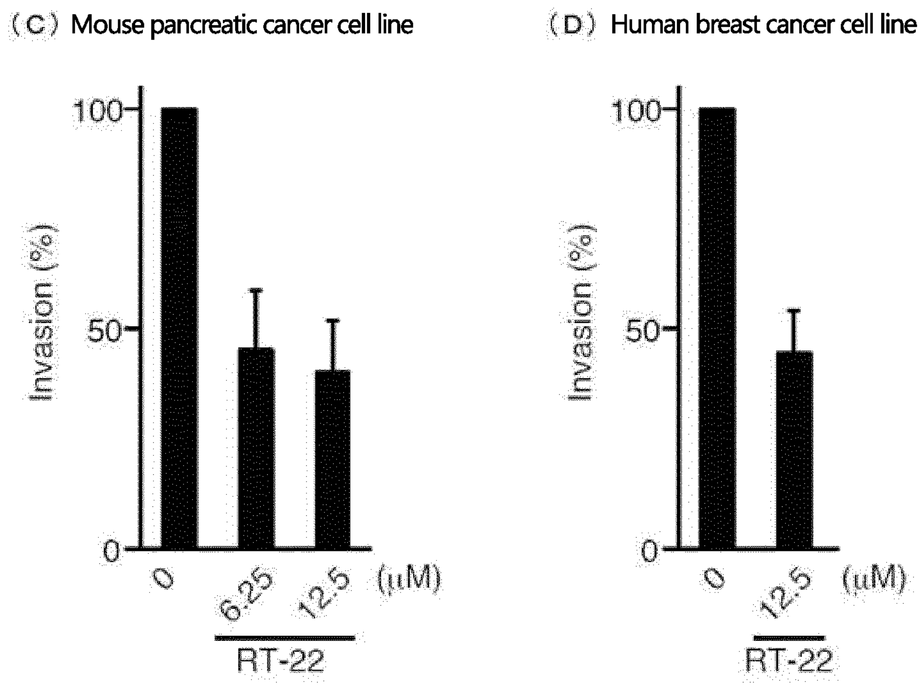
(C) Mouse pancreatic cancer cell line
Invasion (%)
100
50
0
0
6.25
12.5
(μM)
RT-22
(D) Human breast cancer cell line
Invasion (%)
100
50
0
0
12.5
(μM)
RT-22

Fig. 6

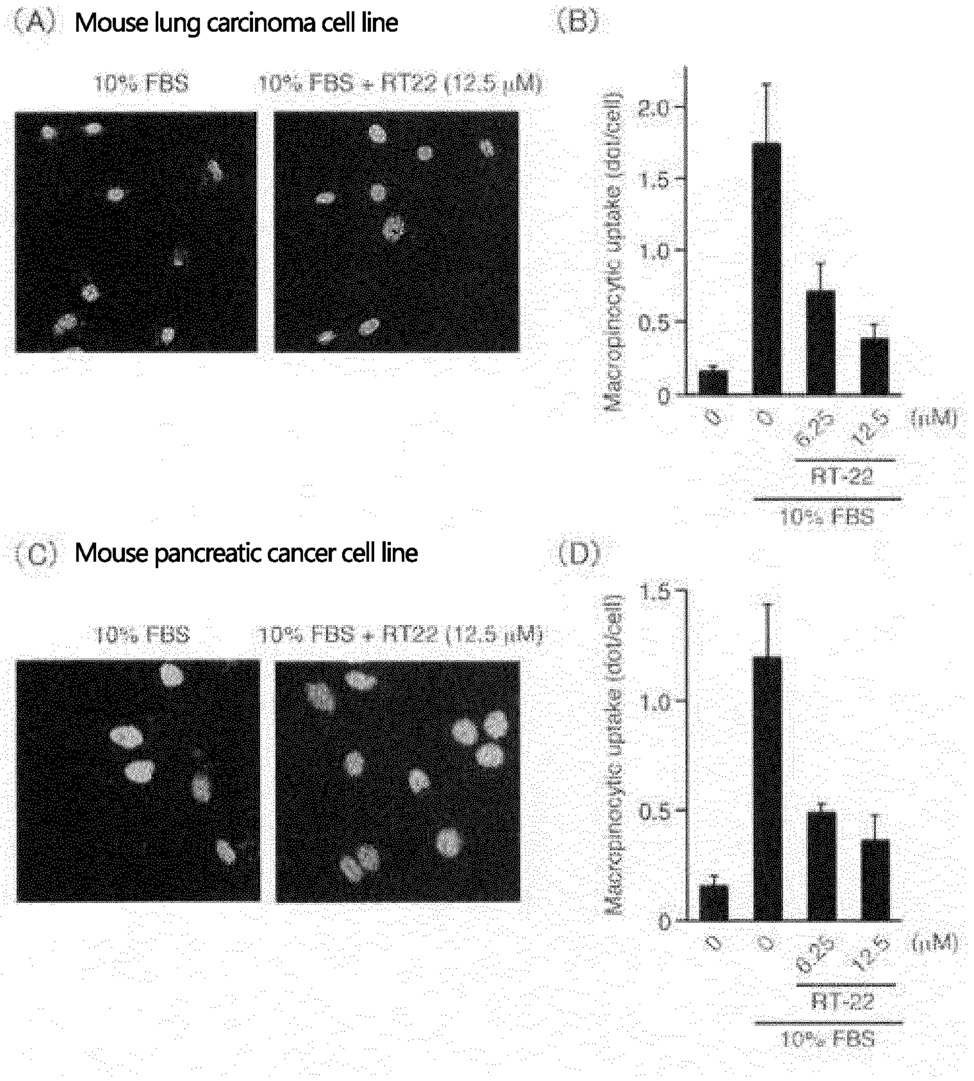
(A) Mouse lung carcinoma cell line
10% FBS
10% FBS + RT22 (12.5 μM)
(B)
Macropinocytic uptake (dot/cell)
2.0
1.5
1.0
0.5
0
0
0
6.25
12.5
(μM)
RT-22
10% FBS
(C) Mouse pancreatic cancer cell line
10% FBS
10% FBS + RT22 (12.5 μM)
(D)
Macropinocytic uptake (dot/cell)
1.5
1.0
0.5
0
0
0
6.25
12.5
(μM)
RT-22
10% FBS

Fig. 7

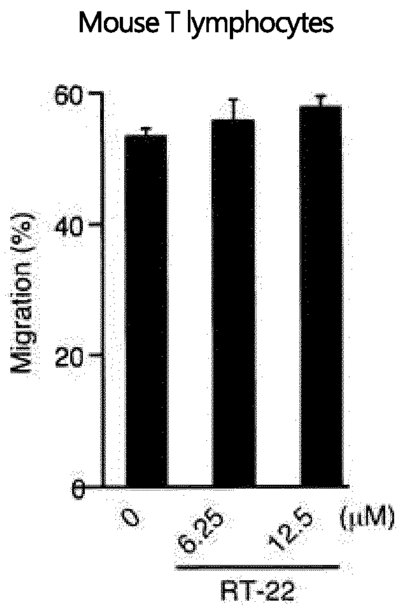
Mouse T lymphocytes
Migration (%)
60
40
20
0
0
6.25
12.5
(μM)
RT-22

Fig. 8

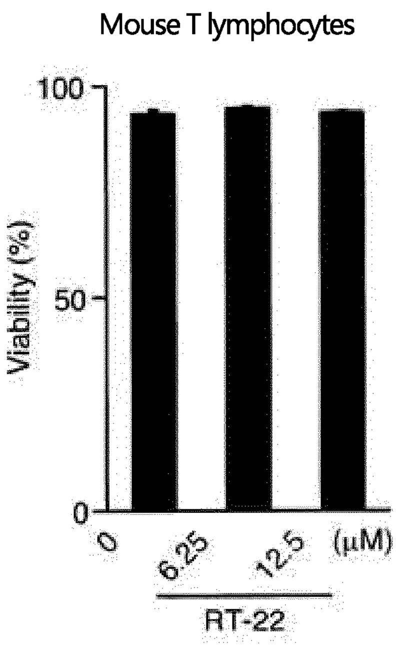
Mouse T lymphocytes
Viability (%)
100
50
0
0
6.25
12.5
(μM)
RT-22

Fig. 9

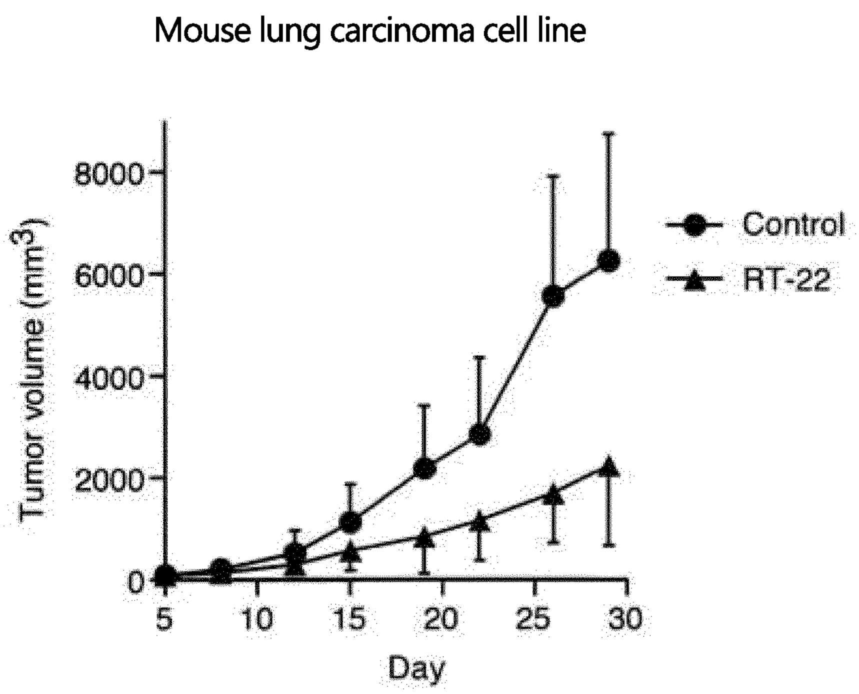
Mouse lung carcinoma cell line
8000
6000
4000
2000
0
Tumor volume (mm3)
5
10
15
20
25
30
Day
Control
RT-22

## INTERNATIONAL SEARCH REPORT

International application No.
PCT/JP2019/046862

A. CLASSIFICATION OF SUBJECT MATTER
Int. Cl. A61K31/40(2006.01)i; A61K31/401(2006.01)i; A61K31/4174(2006.01)i; A61K31/422(2006.01)i; A61K31/437(2006.01)i; A61K31/4439(2006.01)i; A61K31/444(2006.01)i; A61P35/00(2006.01)i; A61P35/04(2006.01)i; A61P43/00(2006.01)i; C07D207/36(2006.01)i; C07D233/78(2006.01)i; C07D401/10(2006.01)i; C07D401/12(2006.01)i; C07D405/06(2006.01)i; C07D413/04(2006.01)i; C07D471/04(2006.01)i; C07D213/71(2006.01)i; C07D213/73(2006.01)i
FI: C07D207/36 CSP; A61P43/00 111; A61P35/00; A61P35/04; A61K31/401; A61K31/422; A61K31/40; A61K31/437; A61K31/4174; A61K31/444; A61K31/4439; C07D413/04; C07D233/78; C07D471/04 104A; C07D213/71; C07D405/06; C07D401/12; C07D213/73; C07D401/10
According to International Patent Classification (IPC) or to both national classification and IPC

B. FIELDS SEARCHED

Minimum documentation searched (classification system followed by classification symbols)
Int. Cl. A61K31/40; A61K31/401; A61K31/4174; A61K31/422; A61K31/437; A61K31/4439; A61K31/444; A61P35/00; A61P35/04; A61P43/00; C07D207/36; C07D233/78; C07D401/10; C07D401/12; C07D405/06; C07D413/04; C07D471/04; C07D213/71; C07D213/73

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII): CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | US 2011/0319459 A1 (QUEENS UNIVERSITY AT KINGSTON) 29 December 2011, QC-166, QC-276, QC-304 | 1, 10-13 |
| X | SALERNO, L. et al., Potholing of the hydrophobic heme oxygenase-1 western region for the search of potent and selective imidazole-based inhibitors, European Journal of Medicinal Chemistry, 2018, vol. 148, pp. 54-62, table 2.3, compounds 33, 34 | 1, 10-13 |
| X | WO 2018/216822 A1 (TAISHO PHARMACEUTICAL CO., LTD.) 29 November 2018, compounds 33, 34 | 1, 11 |
| X | JP 51-143667 A (ROHM AND HAAS COMPANY) 10 December 1976, example 34 | 1 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30.01.2020 | 10.02.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/046862

C (Continuation). DOCUMENTS CONSIDERED TO BE RELEVANT

| Category* | Citation of document, with indication, where appropriate, of the relevant passages | Relevant to claim No. |
|---|---|---|
| X | CHEN, Y. et al., Synthesis of novel triazole and imidazole derivatives containing biphenyl, Huaxue Yanjiu Yu Yingyong, 2011, vol. 23, no. 3, pp. 302-304, compounds 4, 6 | 1 |
| X | NARDI, D. et al., α-Aminomethyl ketones, α-aminoethanols, and their quaternary salts, with possible pharmacological activity. Derivatives of biphenyl and diphenyl ether, Bollettino Chimico Farmaceutico, 1963, vol. 102, no. 11, pp. 765-776, Sostanza VI, XXIII | 1 |
| X<br>Y<br>A | WO 2016/136985 A1 (KYUSHU UNIVERSITY) 01 September 2016, claims, preparation examples, fig. 2-7 | 5, 7, 9-13<br>9<br>1-4, 6, 8 |
| X<br>Y<br>A | TAJIRI, H. et al., Targeting Ras-Driven Cancer Cell Survival and Invasion through Selective Inhibition of DOCKI, Cell Reports, 2017, vol. 19, no. 5, pp. 969-980, summary, fig. 4, page 975 | 5, 7, 9-13<br>9<br>1-4, 6, 8 |

Form PCT/ISA/210 (continuation of second sheet) (January 2015)

**INTERNATIONAL SEARCH REPORT**

International application No.
PCT/JP2019/046862

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[see extra sheet]

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**
Information on patent family members

International application No.
PCT/JP2019/046862

| Patent Documents referred to in the Report | Publication Date | Patent Family | Publication Date |
|---|---|---|---|
| US 2011/0319459 A1 | 29.12.2011 | WO 2008/151437 A1<br>table 1<br>EP 2173740 A1 | |
| WO 2018/216822 A1 | 29.11.2018 | (Family: none) | |
| JP 51-143667 A | 10.12.1976 | US 4073921 A<br>example 34 | |
| WO 2016/136985 A1 | 01.09.2016 | US 2018/0028515 A1<br>claims, example, figure<br>EP 3263133 A1 | |

Form PCT/ISA/210 (patent family annex) (January 2015)

**INTERNATIONAL SEARCH REPORT**

International application No.
PCT/JP2019/046862

(Continuation of Box No. III)

This International Searching Authority has found that this international application has two or more inventions, as shall be explained below.

The inventions in claims 1-13 are composed of the following two inventions that pertain to compounds of chemical formulae.

Invention 1: Invention as in claims 1-4 and invention as in claims 10-13 where claims 1-4 are cited, i.e., an invention pertaining to the compound of formula (A)

Invention 2: Invention as in claims 5-9 and invention as in claims 10-13 where claims 5-9 are cited, i.e., an invention pertaining to the compound of formula (B)

A compound of formula (A) and compound of formula (B) differ significantly from one another in chemical structure as regards i) to iv):

i) formula (A) is a 5-membered ring-containing compound and formula (B) is a 6-membered ring-containing compound;

ii) groups directly connected to the ring described in i), i.e., groups corresponding to R1 and R2 in formula (A), may be hydrogen atoms or the like in formula (A) and are not limited to cyclic amine-sulfonate of formula (B);

iii) a ring group linked via a chain to the ring described in i), i.e., the group corresponding to Rd in formula (B) may be a sterol ring, a phenyl ring substituted with pyridyl or quinolyl, or the like in formula (B) and is not limited to p-biphenylene of formula (A); and

iv) in formula (B), Y is limited to an oxygen-containing group, but in formula (A), Y7 may be a hydrocarbon group or a hydrocarbon group that is part of a ring, and thus has a wider range than Y of formula (B).

Invention 1 and Invention 2 therefore undeniably differ in the special chemical features thereof.

As such, this application is classified into two inventions composed of the invention 1 and invention 2 above.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1016136985 A

### Non-patent literature cited in the description


- *Genes Dev,* 2014, vol. 28, 533-547
- *Proc. Natl. Acad. Sci. USA,* 2013, vol. 110, 7434-7439
- *Cell Reports,* 2017, vol. 19, 969-980
- *Nature,* 2001, vol. 412, 826-831
- *Cell Res,* 2013, vol. 319, 2343-2349
- *Biochem. Biophys. Res. Commun.,* 2018, vol. 497, 298-304
- *Cancer Research,* 2012, vol. 72, 2457-2467
- *Nature Reviews Drug Discovery,* 2014, vol. 13, 828-851
- *Nature,* 2013, vol. 497, 633-638
- *Cell Res,* 2013, vol. 23, 982-983
- **ALLWOOD, D.M. ; BLAKEMORE, D.C. ; BROWN, A.D. ; LEY, S.V.** *J. Org. Chem.,* 2014, (79), 328
- **ALLWOOD, D.M. ; BLAKEMORE, D.C. ; BROWN, A.D. ; LEY, S.V.** *J. Org. Chem.,* 2014, vol. 79, 328
- *CHEMICAL ABSTRACTS,* 143418-49-9
- **HOGAN, P.J. ; COX, B.G.** *Org. Proc. Res. Dev.,* 2009, vol. 13, 875
- *CHEMICAL ABSTRACTS,* 99-90-1
- *CHEMICAL ABSTRACTS,* 1423-26-3
- **ZEYNIZADEH, B.** *J. Chem. Res.,* 2002, vol. 11, 564
- **ROQUES, N.** *J. Fluor. Chem.,* 2001, vol. 107, 311
- **KOLCHINA, E.F. ; SHEKLEINA, N.V. ; SHELKOVNIKOV, V.V.** *Russ. J. Org. Chem.,* 2011, vol. 47, 855
- **SHAVNYA, A. ; COFFEY, S.B. ; SMITH, A.C. ; MASCITTI, V.** *Org. Lett.,* 2013, vol. 15, 6226